# EUROPEAN PATENT SPECIFICATION

(11) **EP 2 685 976 B1**
(45) Date of publication and mention of the grant of the patent: **27.12.2017**
(21) Application number: 12710797.7
(22) Date of filing: 26.02.2012
(51) Int. Cl.: A61K 31/4375, A61K 31/4745, A61K 31/551, C12Q 1/68, A61P 37/06, A61P 25/06

(54) **QUINOLONE ANALOGS FOR TREATING AUTOIMMUNE DISEASES**
CHINOLONANALOGA ZUR BEHANDLUNG VON AUTOIMMUNKRANKHEITEN
ANALOGUES DE QUINOLONE POUR LE TRAITEMENT DE MALADIES AUTO-IMMUNES

(30) Priority: 17.03.2011 US 201161457396 P
(43) Date of publication of application: 22.01.2014
(73) Proprietor: Tel HaShomer Medical Research Infrastructure and Services Ltd., 52 621 Ramat Gan (IL)
(72) Inventor: ACHIRON, Anat, 69016 Tel -Aviv (IL); GUREVICH, Michael, 76276 Rechovot (IL)
(74) Representative: V.O.
(86) International application number: PCT/IL2012/050061
(87) International publication number: WO 2012/123938

(56) References cited:
- WO-A1-2008/131134
- WO-A1-2009/046383
- WO-A1-2010/113096
- WO-A2-2007/022474
- DRYGIN DENIS ET AL: "Targeting RNA polymerase I with an oral small molecule CX-5461 inhibits ribosomal RNA synthesis and solid tumor growth.", CANCER RESEARCH 15 FEB 2011 LNKD- PUBMED:21159662, vol. 71, no. 4, 15 February 2011 (2011-02-15), pages 1418-1430, XP002678820, ISSN: 1538-7445

### SEQUENCE LISTING

<110> Tel HaShomer Medical Research Infrastructure and Services Ltd.
   Achiron, Anat
   Gurevich, Michael
<120> METHODS OF TREATING AUTOIMMUNE DISEASES
<130> 53050
<150> US 61/457,396
   <151> 2011-03-17
<160> 126
<170> PatentIn version 3.5
<210> 1
   <211> 296
   <212> DNA
   <213> Homo sapiens
<400> 1
   gatgaccaga tcatcaactg gctgctagaa ttccgttctt ctgtcatgta cttgacaaaa       60
   gactttgagc aacttatcag tattatattg agattgcctt ggttgaatag aagtcaaaca      120
   gtagtggaag agtatttggc ttttcttggt aatcttgtat cagcatagac tgttttcctc      180
   agaccgtgtc tcagcatgat tgcttcccat tttgtgcctc cccgagtgat cattaaggaa      240
   ggcgatgtag atgtttcaga ttctgatgat gaagatgata atcttcctgc aaattt          296
<210> 2
   <211> 476
   <212> DNA
   <213> Homo sapiens
<220>
   <221> misc_feature
   <222> (58)..(58)
   <223> n is a, c, g, or t
<220>
   <221> misc_feature
   <222> (68)..(68)
   <223> n is a, c, g, or t
<400> 2
   tttccttcaa cttgtggatg caggcaaggt ggatgatgcc agagctctcc tacagagnat      60
   gtggtgcnaa ttgctgaaca aaccccgatt ttgttgttgt tcctccttag gaattctagg     120
   aaacaaggaa aggcatcaac tgtgaaatct gtgttagaat tgattcctga attaaatgaa     180
   aaggaagaag catacaattc cctcatgaaa agctatgtct cagagaaaga tgtcacatct     240
   gctaaagcac tgtatgaaca tttgactgca aagaatacaa aattggatga tctgtttcta     300
   aagcgttacg catctttgct gaagtatgct ggagagcctg tccctttcat tgaaccccct     360
   gaaagctttg aattttatgc acagcagcta agaaaattga gggaaaactc ttcttgaaat     420
   aaccaggcga tactttgttt tgtatatatt tgtgattctg tgtctacatg ttattt         476
<210> 3
   <211> 533
   <212> DNA
   <213> Homo sapiens
<400> 3
   aggcttagca ggatgggcgc agtggctcac gccactaatc ccaacatttt aggaggccta      60
   ggcaggagca atcacttgtg cctgggagtt ctagactagc ctgggcgaga cttcatctct     120
   acaaaaaaag caacaacgac aaaaaaaatt agccaagcat agtggcacac ccctgtagtc     180
   ccagctactt gggaggctga ggtgggagga ttgcttgaac ccaagaggtc gaggctgcag     240
   tgagccaaga ttgtgccact gcactccagc ctgggtgaca gagcaggacc ctgtctctat     300
   tttataaatt aaaaaaggct gggtgtggtg gctcacaccc ataatcccaa cactttggct     360
   cagcagattg cttgaaccca ggaattcaag tccaatctgg gcaacatggg gaaaccccag     420
   ctctacaaaa aaaattagcc tggtgtggtg gcacatgcct gtagttccag ctactcagga     480
   ggctgaggtg ggagaatctc ctgagcctgg aaggtccagg cagtgagcca aga            533
<210> 4
   <211> 411
   <212> DNA
   <213> Homo sapiens
<400> 4
   ccaggcatat gacatggatc cccctgggga accaggctga tctctttcca gagggcacta 60
   tccgaccagt gcatgatgat atcctcatcg ctcagctgcg gcctggccaa gaaattgacc 120
   tgctcatgca ctgtgtcaag ggcattggca aagatcatgc caagttttca ccagtggcaa 180
   cagccagtta caggctcctg ccagacatca ccctgcttga gcccgtggaa ggggaggcag 240
   ctgaggagtt gagcaggtgc ttctcacctg gtgttattga ggtgcaggaa gtccaaggta 300
   aaaaggtggc cagagttgcc aacccccggc tggatacctt cagcagagaa atcttccgga 360
   atgagaagct aaagaaggtt gtgaggcttg cccgggttcg agatcattat a 411
<210> 5
   <211> 291
   <212> DNA
   <213> Homo sapiens
<400> 5
   gttgagtcaa cgggggtgtt gccaccagat gtgctggtga gtgaagccat caaagtactg 60
   atggggaagt gccggcgctt cttggatgaa ctagatgcgg ttcagatgga ctgagcttgg 120
   atgcttctga ggcaagctga agctttgggt tctgactgac ccaccctaca ggactgctga 180
   acagagagcc cagtgtgact agggatcctg agttttctgg gacaattcca gctttaatca 240
   atacattttg ttaaatgtgc cataaaatga gactttttac gcctttataa g 291
<210> 6
   <211> 441
   <212> DNA
   <213> Homo sapiens
<400> 6
   gcacgaggaa gaccataccc taggaaattc tctacgttac atgatcatga agaacccgga 60
   agtggaattt tgtggttaca ctacgaccca tccttcagag agcaaaatta atttacgcat 120
   tcagactcga ggtacccttc cagctgttga gccatttcag agaggcctga atgagctcat 180
   gaatgtctgc caacatgtgc ttgacaagtt tgaggccagc ataaaggact ataaggatca 240
   aaaagcaagc agaaatgaat ccacattcta gtcctttatg cagtatacaa ggagaactgt 300
   cctgtaggat attctcttcc tgatggtgca gaacccagaa ttagaagttt gtggttacag 360
   catactctgt ccttcagaaa ggcgtgattc tagctgttga ccccttgcag ctgttggaat 420
   ctctgcaaga acctctgtat t 441
<210> 7
   <211> 393
   <212> DNA
   <213> Homo sapiens
<400> 7
   gctatcagcc cggatgacag tgacgaggag aactgagggc acgtggggtg cggcagcggg      60
   ctagggccca gggcagcttg cccgtgctgc cgtgcagttc ttgcctccct cacggggcgt     120
   cacccccagc ccagctccgt tgtacataaa taccttgtga cagagctccc ggtgaacttc     180
   tggatcccgt ttctgatgca gattcttgtc ttgttctcca cttgtgctgt tagaactcac     240
   tggcccagtg gtgttctacc tcctacccca cccaccccct gcctgtcccc aaattgaaga      300
   tccttccttg cctgtggctt gatgcggggc gggtaaaggg tattttaact taggggtagt     360
   tcctgctgtg agtggttaca gctgatcctc ggg                                  393
<210> 8
   <211> 238
   <212> DNA
   <213> Homo sapiens
<400> 8
   gttctggcct agagctgtat gcggaatgga agcacgttaa tgaggactct caggagaaga      60
   agatcctgct gagtccagag cgagtgcatg agatcttcaa acgcatctca gatgaggagt     120
   gttttgtgct gggcatcgag ccccgctatg cacggccaga gtcgatgatt gtcacagtgc     180
   tgcccgtgcc cccgctgtcc gtgcggcctg ctgttgtgat gcagggctct gcccgtaa       238
<210> 9
   <211> 545
   <212> DNA
   <213> Homo sapiens
<400> 9
   tttattggcc ccacttatta ccagcgtttg aagcatatgg tggatgataa gattcactct      60
   cgtgctaggg gacctattca gatcctcaat agacagccca tggagggtag atctcgtgat     120
   ggtggcctgc gttttggaga aatggaacga gattgtcaga ttgcccatgg agcagcccag     180
   tttttaaggg aaagattgtt tgaggcatca gatccatatc aggttcatgt ttgcaatctt     240
   tgtggaataa tggcgattgc caacaccagg acccatacat atgaatgcag gggctgccgc     300
   aataaaaccc agatttcttt ggtgcgaatg ccttacgcat gcaaactatt gtttcaggaa     360
   cttatgtcta tgagtattgc accgcgaatg atgagtgttt agctatttta caggagtcaa     420
   caagataatt aaatatcttg gtgtcttgtt tctattgtgt ggctttataa aaatgacaaa     480
   tatgtactgt gttgtgataa aaagtatttt atttgtttaa tgatatgcat gcttttcttc     540
   tgtaa                                                                 545
<210> 10
   <211> 439
   <212> DNA
   <213> Homo sapiens
<400> 10
   ttaactgcag cttgcctgct tcagcaaaaa cggagattca ggccagcagc tggatatggg      60
   ggtctctctt cagactcttc tcgtttctga gaatctagtc tactgttggt tgagcttctt     120
   ggcaggacat cagtaccaac tagaagtggg tcatagatag attaccaggg atgcagtggt     180
   gtttaggcag gataggtctt tactggccct gactgctgtt aataattggc agcagtgctc     240
   cccagatccc agaaggtccc tgctggagtg tttccagtgc acctgtaggg aaccaactag     300
   acttctctcc tggttagtcc agctctttac tctaaaccct ttctgtccaa aatgagtcat     360
   tttcagttgt accttagatg tctggtgttg aggatcaagt gccatagcct ttattcaggg     420
   ggcctataaa cccttccag                                                  439
<210> 11
   <211> 350
   <212> DNA
   <213> Homo sapiens
<220>
   <221> misc_feature
   <222> (55)..(55)
   <223> n is a, c, g, or t
<220>
   <221> misc_feature
   <222> (68)..(68)
   <223> n is a, c, g, or t
<220>
   <221> misc_feature
   <222> (74)..(74)
   <223> n is a, c, g, or t
<220>
   <221> misc_feature
   <222> (76)..(76)
   <223> n is a, c, g, or t
<220>
   <221> misc_feature
   <222> (138)..(138)
   <223> n is a, c, g, or t
<220>
   <221> misc_feature
   <222> (174)..(175)
   <223> n is a, c, g, or t
<220>
   <221> misc_feature
   <222> (180)..(180)
   <223> n is a, c, g, or t
<220>
   <221> misc_feature
   <222> (185)..(187)
   <223> n is a, c, g, or t
<220>
   <221> misc_feature
   <222> (189)..(190)
   <223> n is a, c, g, or t
<220>
   <221> misc_feature
   <222> (193)..(193)
   <223> n is a, c, g, or t
<400> 11
   caattcccag ggtgtcccta atttgagaag taaccttttg gaatagcatt agacnctggc       60
   tgtccccntc cccncncaaa taaacatgat atttcattct ctgtccagca gtcatgaacc      120
   ccttcacctc caatgacntg atcatttagt ttggtggggg tgggggtggg ggtnngggtn      180
   ggggnnnann canccgcagg agcaagggcc cctcccacat acacaggagg agtatttcat      240
   ttctccttaa tgaaggctct ggccctaacc cctcagcact gtctccagat aggaacatgc      300
   acaaagcagt taattaggca gcctggagaa aaccagagat ccagtacaga                 350
<210> 12
   <211> 554
   <212> DNA
   <213> Homo sapiens
<220>
   <221> misc_feature
   <222> (270)..(270)
   <223> n is a, c, g, or t
<220>
   <221> misc_feature
   <222> (361)..(369)
   <223> n is a, c, g, or t
<220>
   <221> misc_feature
   <222> (376)..(376)
   <223> n is a, c, g, or t
<220>
   <221> misc_feature
   <222> (507)..(507)
   <223> n is a, c, g, or t
<400> 12
   cctgctagcg cttcaaattc ccaggtgtcc ctaatttgag aagtaacctt ttggaatagc      60
   aggagcaagg gcccctccca catacacagg aggagtattt catttctcct taatgaaggc     120
   tctggcccta acccctcagc actgtctcca gataggaaca tgcacaaagc agttaattag     180
   gcagcctgga gaaaaccaga gatccagtac agaaaggaaa ggatatttat tgattaacag     240
   aagttgtctt tttaaaagtg tttatttttn gcaataaaga gcacaacata atctccttca     300
   tgcgtcatcg tgccacttag ctgagccagc cagctctgcc tccctcccca aagacctttg     360
   nnnnnnnnng ctttanagga tgaatctgtt gaggaggtct gtttccgagc tggcctgggc     420
   tgccccatac ccctcaccag catagctgac ggcttcggcg atgttctggg aaccagtgat     480
   gtggtgccag taggatcgcc gcggcanact gcgtcgtggg tgtgcaggga tacgagtaat     540
   gttccgtgcc cttg                                                       554
<210> 13
   <211> 338
   <212> DNA
   <213> Homo sapiens
<400> 13
   ggtcgtgcca aagaggtcca caaccaggtg tgcactgttc actgcagccc atttgctgta      60
   tgaactgtgg ttgttgtgtg cccaatgaca aggctactaa gaaattcatc atttgaaacg     120
   tagaggccgc agcagtcagc gatgtttctg aaatgagcat ccttgacgcc tgtgtacttc     180
   ccaggctgga tgtgaagcta cattaccatg tgagttgtgc cattcacagc acagtggtga     240
   ggaattgagc tcatgaagca ggcaaggacc gaacacctcc accccaacgt agacctgcag     300
   gtgctgcccc atgacctcca ccaaagccca tataagga                             338
<210> 14
   <211> 349
   <212> DNA
   <213> Homo sapiens
<220>
   <221> misc_feature
   <222> (73)..(74)
   <223> n is a, c, g, or t
<400> 14
   ttttttactt cataggccag gcttgaagtt ctgaacactt tgaagtctcc aattatgaga        60
   gatccagtct aannctctgg cctgctaatt agcaataagt gctttatttg gaaggaggga       120
   gtcatccact cttgagccac tgcagtgaag tcacttgatc tcagtctggg ggaaaacact       180
   tcaatagcta aacattctag ctttgatttt tctgaaggga atacacttgt tttcaatttt       240
   ggggtttttc tttggggcac ttgcttgact ctgtatgaac ttgtgatcca aggaaaaagg       300
   agaaagaaca gtgttggctt ttaaaatcag gatggtttta tgtttgcta                   349
<210> 15
   <211> 416
   <212> DNA
   <213> Homo sapiens
<220>
   <221> misc_feature
   <222> (45)..(45)
   <223> n is a, c, g, or t
<220>
   <221> misc_feature
   <222> (179)..(179)
   <223> n is a, c, g, or t
<220>
   <221> misc_feature
   <222> (202)..(203)
   <223> n is a, c, g, or t
<220>
   <221> misc_feature
   <222> (286)..(288)
   <223> n is a, c, g, or t
<220>
   <221> misc_feature
   <222> (334)..(334)
   <223> n is a, c, g, or t
<400> 15
   accgaccaga tgtttgtgtt ctttccagag gagcccaagg tgggnatcaa gaccatcaag        60
   gtgtactgcc agcgcatgca ggaggagaac atcacacggg ctctcatcgt ggtgcagcag       120
   ggcatgacac cctccgccaa gcagtccctg gtcgacatgg cccccaagta catcctggna       180
   gcagtttctg cagcaggagc tnntcatcaa catcacggag cacgagctag tccctgagca       240
   cgtcgtcatg accaaggagg aggtgacaga gctgctggcc cgatannnta agctccgaga       300
   gaaccagctg cccaggatcc aggcggggga cccntgtggc gcgctacttt gggataaagc       360
   gtgggcaggt ggtgaagatc atccggccca gtgagacggc tggcaggtac atcacc           416
<210> 16
   <211> 339
   <212> DNA
   <213> Homo sapiens
<400> 16
   gccccattca cctgtggatt tgtgcgagat gcagcctcag aaggaacaag gcccccagag      60
   ggaggtcacc tgggggcaac tggtgccggg tcttcaccca gaccacgctg ggtcccctct     120
   gttgggggtt tggggtccgg gtctcccacc agccactgct tcctcctggg ccctcaattc     180
   cacccctcgt cttccctccc tcggggccct gatgcgtggc ggcccccacc cggcctcggc     240
   tctttactcc attcacagcc atgcacgcgc tcaagtacac cagggtgcga gatgccagct     300
   ctggagttct cggttgttgt aggaggttgg gtgttttca                            339
<210> 17
   <211> 485
   <212> DNA
   <213> Homo sapiens
<400> 17
   agataagcta ggagccgcgc gagtcgtagt gtcgctgttt gcgggtctcc gcgcgggacc      60
   ggggcgcagc ggggtcgctg aggcgagggt gtcatgtcag acaacgagga caattttgat     120
   ggcgacgact ttgatgatgt ggaggaggat gaagggctag atgacttgga gaatgccgaa     180
   gaggaaggcc aggagaatgt cgagatcctc ccctctgggg agcgaccgca ggccaaccag     240
   aagcgaatca ccacaccata catgaccaag tacgagcgag cccgcgtgct gggcacccga     300
   gcgctccaga ttgcgatgtg tgcccctgtg atggtggagc tggaggggga gacagatcct     360
   ctgctcattg ccatgaagga actcaaggcc cgaaagatcc ccatcatcat tcgccgttac     420
   ctgccagatg ggagctatga agactggggg gtggacgagc tcatcatcac cgactgagct     480
   ggagt                                                                 485
<210> 18
   <211> 543
   <212> DNA
   <213> Homo sapiens
<400> 18
   gtatggcttt gtaattgctg tcaccaccat tgacaatatt ggtgctggtg tgatccagcc      60
   aggccgaggc tttgtccttt atccagttaa gtacaaggcc attgttttcc ggccatttaa     120
   aggggaggtc gtggatgctg ttgtcactca ggtcaacaag gttggactct tcacagaaat 180
   tgggcccatg tcttgcttca tctctcgaca ttccatccct tcagagatgg agtttgatcc 240
   taactccaac ccaccatgtt acaagacaat ggatgaggat attgtgattc agcaggacga 300
   tgagatccgc ttaaagattg tggggacccg tgtggacaag aatgacattt ttgctattgg 360
   ctccctgatg gacgattact tggggcttgt aagctgagcc tggtggcctc ctacccttgg 420
   tcctactcta ggaagtgtga ttgtcacact tatcatgttg tccagaggtc cagtctggct 480
   gctgttgtgg aggcaaggaa ggcaactcat cccagaaggc atctggtgct tcttgtagct 540
   taa 543
<210> 19
   <211> 377
   <212> DNA
   <213> Homo sapiens
<400> 19
   ccttccaggg ctgaccagtt tgagtatgta atgtatggaa aagtgtacag gattgaggga 60
   gatgaaactt ctactgaagc agcaacacgc ctctctgcgt acgtgtccta tgggggcctg 120
   ctcatgaggc tgcaggggga tgccaacaac ctgcatggat tcgaggtgga ctccagagtt 180
   tatctcctga tgaagaagct agccttctga acctcgcctg aagccagcct ctctgccaag 240
   tcactcaggt catgggcatt gttcaagcct gagtggcagc cgctcttgct cacctgttga 300
   ggaagggctg gctcactgtc caccgtggcg gcatctttaa ctggcctcca ctcaatggga 360
   aactgactcg cctgtga 377
<210> 20
   <211> 305
   <212> DNA
   <213> Homo sapiens
<400> 20
   tggagcccga cgggacttac gagccgggct tcgtgggtat tcgcttctgc caggaatgta 60
   acaacatgct gtaccccaag gaagacaagg agaaccgcat tctgctctac gcgtgccgga 120
   actgtgatta ccagcaggag gccgacaaca gctgcatcta tgtcaacaag atcacgcacg 180
   aagtggacga actgacccag attatcgccg acgtgtccca ggaccccacg ttgccgcgga 240
   ccgaggacca cccgtgccaa aagtgcggcc acaaggaggc tgtgttcttc cagtcacaca 300
   gtgcg 305
<210> 21
   <211> 515
   <212> DNA
   <213> Homo sapiens
<220>
   <221> misc_feature
   <222> (173)..(173)
   <223> n is a, c, g, or t
<220>
   <221> misc_feature
   <222> (175)..(175)
   <223> n is a, c, g, or t
<220>
   <221> misc_feature
   <222> (177)..(178)
   <223> n is a, c, g, or t
<220>
   <221> misc_feature
   <222> (180)..(180)
   <223> n is a, c, g, or t
<220>
   <221> misc_feature
   <222> (182)..(182)
   <223> n is a, c, g, or t
<220>
   <221> misc_feature
   <222> (298)..(299)
   <223> n is a, c, g, or t
<220>
   <221> misc_feature
   <222> (301)..(301)
   <223> n is a, c, g, or t
<400> 21
   gttcttgctc ttcgagggcg agaagaagat caccattaac aaggacacca aggtacccaa       60
   tgcctgttta ttcaccatca acaaagaaga ccacacactg ggaaacatca ttaaatcaca      120
   actcctaaaa gacccgcaag tgctatttgc tggctacaaa gtcccccacc ccntngnngn      180
   anaagatcat catccgagtg cagaccacgc cggactacag cccccaggaa gcctttacca      240
   acgccatcac cgacctcatc agtgagctgt ccctgctgga ggagcgcttt cgggtggnnc      300
   nataaaagac aagcaggaag gaattgagta ggggccagag ggggctctgc tcggcctgtg      360
   agccccgttc ctacctgtgc ctgaccctcc gctccaggta ccacaccgag gagagcggcc      420
   ggtcccagcc atggcccgcc ttgtggccac ccctcaccct gacaccgacg tgttggccac      480
   ccctcaccct gacaccgacg tgtcctgtac ataga                                 515
<210> 22
   <211> 354
   <212> DNA
   <213> Homo sapiens
<220>
   <221> misc_feature
   <222> (252)..(253)
   <223> n is a, c, g, or t
<400> 22
   gttcttgctc ttcgagggcg agaagatcac cattaacaag gacaccaagg tacccaatgc        60
   ctgtttattc accatcaaca aagaagacca cacactggga aacatcatta aatcacgtgc       120
   ctgcttcccc ttcgccttct gccgtgattg tcagtttcct gaggcctccc cagccacgct       180
   tcctgtacag cctgcagaac tctgccccag agcacatcag ctatgtgccc cagctctcaa       240
   acgacacctt gnncggggag gctcaccctg tccaccttca cgctggagca gcctctaggc       300
   cagttcagca gccacaacat ctctgacttg gataccatct ggctggtggt ggcc             354
<210> 23
   <211> 299
   <212> DNA
   <213> Homo sapiens
<400> 23
   cagccggtcc cctggataaa gaggtgaggg ggtgtgaagt ctgggagccc ccctcccagg        60
   gccaggctac agggggacag cagggctata ccaggagtgc cacagagctt gcatttgggt        120
   gtgatctaat ccccagtcct gccctgacct cctgggtgct cttagccaag ccctcttccc       180
   tgtgtgtgct agaaagtgag ggtcactccc ttccacacaa gggagtgaat tgagggtggg       240
   gatctgagct ctctgagcag caaagctgga cacaggccaa attgccaatc ctgagtccc        299
<210> 24
   <211> 537
   <212> DNA
   <213> Homo sapiens
<220>
   <221> misc_feature
   <222> (83)..(83)
   <223> n is a, c, g, or t
<220>
   <221> misc_feature
   <222> (92)..(92)
   <223> n is a, c, g, or t
<220>
   <221> misc_feature
   <222> (141)..(141)
   <223> n is a, c, g, or t
<220>
   <221> misc_feature
   <222> (154)..(154)
   <223> n is a, c, g, or t
<220>
   <221> misc_feature
   <222> (173)..(173)
   <223> n is a, c, g, or t
<220>
   <221> misc_feature
   <222> (177)..(177)
   <223> n is a, c, g, or t
<220>
   <221> misc_feature
   <222> (191)..(191)
   <223> n is a, c, g, or t
<400> 24
   ccagtgaact cctgaaacct caaacagtac caaaccttta tacactgttt tttccatata      60
   tatataccta tgataaagta tanatgtata anattaagca tagcaagaga ttaataataa     120
   tgtaatagaa caatgataac natactataa taanaagtta tgtgaatgtg gtntggntct     180
   ctcttgcttt ncaaaatatc ttcttgtaca gtactcacct attttagaat gtggttgact     240
   acaggtaacc aaaaccacag aaagggaaac tttggatgag gggggcacta ctgtacttag     300
   gaatacaact atatacatat gattttattt ttaagaccat attatatttg ggtatctact     360
   aatattttgt ataaagcaat tttttgttcc attacgtgac tttttgtttt attgtatatg     420
   taatttaaca cacaataaag ggtaaagttg cttccccaaa ccacactttt aatcaaaacc     480
   tagaatcatc tgcagtcctt gttaaaaatg caggtttcta gaaccctctg aagttct        537
<210> 25
   <211> 253
   <212> DNA
   <213> Homo sapiens
<400> 25
   ttccgtgctg tgtaggggct aacaatggac acccagaagg acgttcaacc tccaaagcag 60
   caaccaatga tatatatctg tggagagtgt cacacagaaa atgaaataaa atctagggat 120
   ccaatcagat gcagagaatg tggatacaga ataatgtaca agaaaaggac taaaagattg 180
   gtcgtttttg atgctcgatg aatgctggga attcagagga atgtcttcac ttatacttgg 240
   atttgctctc ttc 253
<210> 26
   <211> 231
   <212> DNA
   <213> Homo sapiens
<220>
   <221> misc_feature
   <222> (63)..(63)
   <223> n is a, c, g, or t
<400> 26
   ctttcacact gtggcagccc agtgaagcag actgggccat gaactctcct agccctgggg 60
   ccnagcctgt tccacaggca cccctgcagg aggcgctgcc aggagagcct tccatctcgg 120
   ggctctttga ggttccctcc ttctgggtgt tcttcaggct gagcagagag gctcctgtac 180
   cctctctctc ggaatctgaa gagccagatt taggccgggc aaaggggctc a 231
<210> 27
   <211> 349
   <212> DNA
   <213> Homo sapiens
<400> 27
   ctgcttcact tgtggcaaga tcgtcggcaa caagtgggag gcttacctgg ggctgctgca 60
   ggccgagtac accgaggggg atgcgctgga tgccctgggc ctgaagcgct actgctgccg 120
   ccggatgctg ctggcccacg tggacctgat cgagaagctg ctcaattatg cacccctgga 180
   gaagtgacca cgctggaacc cacccacccg ctgtgctgac catgggccct gagcgtcctg 240
   ccccgaattc acgaggctga ggcatccggg agctggcgta atgcctggcc gcagtgtgtg 300
   tgtatcccat accccactct ggaaggaacc atccagtaaa ggtctttca 349
<210> 28
   <211> 503
   <212> DNA
   <213> Homo sapiens
<400> 28
   aagaattccc ttgcgtattc tctctctaaa acaaccaaaa aaaaatggag aggcttttta 60
   tatactctaa gactggctaa acaaccttga tcattgagcc tcgagccatg ggagagatgc 120
   tgaccatgtg gactgcaagg ctgcttgatt cacagatgga tgtgacctaa aggataaata 180
   agctattact tatgtgctga tctcttgaca ttcactcatt agaagacctt actccttcaa 240
   gcaaatgttt ggggtcaaat ttaccatatc ttctggctaa ccatattcaa gattcttctg 300
   aaacttggag gatgtaaaga atccatttga tttggtcagc ctggcttttg tcgtggtggc 360
   tggctcggat aaattttccc aacaattaaa tcttgccttt acacacccaa actttgtaat 420
   tttagtcttg gtgaaatata atgaatttgt tcctaccttg tcaagcaaga atgtcgtctt 480
   ctcctatgga ctcaattgct att 503
<210> 29
   <211> 276
   <212> DNA
   <213> Homo sapiens
<400> 29
   ccccatccag gaccttctat ttatatactg tgaacatcct gtcagctgcc cgaatgttgt 60
   tcgacaggtg ctacaagagc atagccaact tgatagaaag gaggcaattt gaaaccaaag 120
   agaataagcg tctactagaa aaatctcaga gggtagaagc catcattgca tctatgcagg 180
   ctactggtgc agaggaagca cagttacaag aaatagagga gatgatcaca gctcctgaac 240
   gtcagcagct agagaccctg aaacgtaatg tcaaca 276
<210> 30
   <211> 517
   <212> DNA
   <213> Homo sapiens
<400> 30
   gagtgagatt accacttcct ctagtgctcc cttcacccag ccattgtctt ccaatgagat 60
   cttcagatcc ctacctgttg gctataacat ctctaagcaa gttcttgatc agtatctcac 120
   tctgctggca gatgatccac tagagtttgt tggaaagtct ggcgacagtg gtggaggaat 180
   gtatgtcatc aacctccata aggcattagc atccctagcc acagccactc tgaagtccgt 240
   cgtacaggag agatttgggt ctcgctgtgc tagaatattc cgtctagttt tgcagaagaa 300
   acacatagag cagaagcaag tggaagactt tgcaatgatt cctgcaaagg aggcaaagga 360
   tatgctatat aagatgctct cagaaaattt catgtcactc caggttggat gccagtgaga 420
   tccaggtgga cgaaaccatc ttcctgctgg agtcttacat tgagtgcacc atgaagagac 480
   agtgatccag aagaagcatc ttcctcagaa gatctgg 517
<210> 31
   <211> 530
   <212> DNA
   <213> Homo sapiens
<400> 31
   cctgctccag acattttgtt cttgaatctg tgagacccag aagggcccac tgagcccact 60
   cactccacct ttggcaacca ttgttccagg tcccccaggg cttcctccca cagcagctgt 120
   gaatggcaca gtgaccttcc tgcagcgtgg agatggcaca tccttgctgc tggggacttg 180
   gccctgctat ttatttttgt atttatgtct taatctcttc cactgatgca tcctccaagg 240
   gtagatgggg agggtctgtg tgaaggggcc ggcttctctt ggtgcctgct gggttgcagg 300
   ggcaggaagc gtgtggactg cagcttctgc tggtgctccc cccgtcctcc tggaggcagt 360
   ataggagaga gagcaaggat tgagtctgag acttaagcac tcggtcccag cttgccagtt 420
   cctggttctg tgtccttgga aaactaccta acctttctga gcctcctata ctatccgaca 480
   caaatgggga tgatacctac ctccagggtt ggcgtgagga ttcatgggct 530
<210> 32
   <211> 382
   <212> DNA
   <213> Homo sapiens
<400> 32
   ggtacgccga aacatgatcc agtctcggtt gactcaagag tgtggagaag atctcagtaa 60
   acaggaggtg gataaagtac taaaggactg ctgtgtaagc tatggtggca tgtggtacct      120
   taaagggaca gtacagtctt gacaatagta gcaaactact aacccagcaa atctaagccc      180
   aaggaagaag ggcggaacca gaagtagggc ctcgacttgc ttcagacgac acagagcaag      240
   aggaactgac catctcatga cctgtggcat tgcacggtgc agtggacaga agggattatc      300
   ctcagccagt cgcagggtca gcttaagtta gttagatcac tcccagaaga gaccagctgg      360
   gaccttcttt gcagtacaat tt                                               382
<210> 33
   <211> 216
   <212> DNA
   <213> Homo sapiens
<220>
   <221> misc_feature
   <222> (55)..(56)
   <223> n is a, c, g, or t
<220>
   <221> misc_feature
   <222> (59)..(59)
   <223> n is a, c, g, or t
<400> 33
   agagtggaac atgccttttc cgcacaatat taattccttt ttgtatcaga aaggnnctnt       60
   taggagttat gctaccatac ttacttcaaa cccaatgact actgtcaagg tcatattttc      120
   agtacataaa tactatcatt ttcattctaa agaatatttt cactgttcct tctttcttaa      180
   agtcttatgt ttcactcttt aactcaaatg tattct                                216
<210> 34
   <211> 391
   <212> DNA
   <213> Homo sapiens
<220>
   <221> misc_feature
   <222> (295)..(295)
   <223> n is a, c, g, or t
<400> 34
   gataaccact tgaaccaggc aaatacactg aagataaagg ttatttcttt ttttagcttt 60
   gaatttgtca tgaccatttt agtcttgcag atagcagggc agccccttgg ggcaaggatt 120
   tactctggga ggtaccgtta agagccttct ttcccccttt gaaagatcct tttacaatgt 180
   taaagtatac tagttgcaag aacaagcagg atttgcaggt tgctttacca gcatgagtct 240
   catttttctg gcttaaaatc tgggactgtg aaattattcc ataggaaagt gaatnttatt 300
   ttgcagaatt agcctcttac ataaaagtat ttgttgaagt gtctttaaaa ttgctatcat 360
   gagcaaaact ggttgctgta atgcttgttt t 391
<210> 35
   <211> 442
   <212> DNA
   <213> Homo sapiens
<400> 35
   ggataccaga ttggagaaga cttccaagag agatgatgcc aagaaataaa tgtaaaaaag 60
   caggcccaaa acccaaaaag gcaaaagacg caggcaaagg cacaccactc actaatactg 120
   aagatgtgtt gaaaaaaatg gtggaattgg aaaaaagagg tgatggtgaa aaatcagatg 180
   aggaaaatga agagaaagaa ggaagcaaag agaaaagtaa agaaggtgat gatgacgatg 240
   acgatgatgc cgcagaacag gaggaatatg atgaagaaga gcaagaagag gaaaatgact 300
   acattaattc atactttgaa gatggagatg attttggcgc agacgtgatg acaacatgga 360
   tgaggcaacc tattaggcat gaaatttttc aaaaaatatt tttatgatgc agcttctgaa 420
   catttggaca gacttgattt gt 442
<210> 36
   <211> 299
   <212> DNA
   <213> Homo sapiens
<400> 36
   ctgcgggaac gggctgatcg tggaggaggg acaacgctgc caccgcttcg cctgcaacac 60
   gtgcccctac gtgcacaaca tcacccgcaa ggtaacaaat cggaagtacc caaaactgaa 120
   agaagtggat gatgtgcttg gtggagcagc tgcctgggag aatgttgact ctactgcaga 180
   gtcgtgtccc aaatgcgaac atcctcgtgc ttacttcatg cagcttcaga cccgctctgc 240
   agatgagccg atgaccacct tctacaagtg ctgcaatgct cagtgtggac accgctgga 299
<210> 37
   <211> 441
   <212> DNA
   <213> Homo sapiens
<400> 37
   tatcgcctgg actccaaggt caagcaaata ggaggtggaa ttcagagcat cacctacacc 60
   cacaacggag acatcagccg aaagcccaac acacgtaagc agaagaacgg cttcccgccc 120
   aacttcatcc actcgctgga ctcctcccac atgatgctca ccgccctgca ctgctacagg 180
   aagggcctga ccttcgtctc tgtgcacgac tgttactgga ctcacgcagc tgatgtctcc 240
   gtcatgaacc aggtgtgccg ggagcagttt gtccgcttgc acagcgagcc catcctgcag 300
   gacctgtcca gattcctggt caagcggttc tgctctgagc cccagaagat cttggaggcc 360
   agccagctga aggagacact gcaggcggtg cccaagccag gggccttcga cctggagcag 420
   gtgaagcgtt ccacctactt c 441
<210> 38
   <211> 406
   <212> DNA
   <213> Homo sapiens
<220>
   <221> misc_feature
   <222> (73)..(73)
   <223> n is a, c, g, or t
<400> 38
   gctgaccttc gaggcggtgg atgtacccat gctttgcccc ccgctgccct ggacatcgcc 60
   gcactctggt gcnttcctgc tcagccccac caagctgatg cgcacggtgg aaggcgccac 120
   gcagcaccag gagctgctgg aaacctgccc gcccaccgcg ctgcatggcg cactggacgc 180
   cctcacccaa ctgggcaact gcgcctggcg cgtcaacggg cgcgtgctgg acctggtgct 240
   gcagctcttc caggccaagg gctgccccca gctaggcgtg ccggccccgc cctccgaggc 300
   gccccagccg cccgaggccc acctgccgca cagcgccgcg cccgcccgca aggccgagct 360
   gcgccgtgag ctggcgcact gccagaaggt ggcccgggag atgcac 406
<210> 39
   <211> 449
   <212> DNA
   <213> Homo sapiens
<400> 39
   ctctgaatgt gatcgacgcc cagcaaggac aagctttaaa atgtctgcgg tctgcccttt        60
   tgaagcagga ctggctcact ctgtcattgg gagctgtcag ctgcgactgc aggttctcta       120
   ggaggcattc cagaatagag tagcacactg tgtctgcagt tctcgatgac cgaaagttat       180
   caaaaatatt taaaatattt aaattgtgaa cctattgata aagaatattt ataaaaactg       240
   atctgtaggc ctgtactaat ctctacgcat tagcaatatt gactgtaaac ccacattaag       300
   gaaaccacta cgggtctggc agtgcgtgtc ccgtggggtg tgcattttaa aactcgattc       360
   atagacacag gtaccatgtt ccatttccgt catggtgaag caaatgaatt ggcctggcta       420
   ccactgtggt cgcgtgctac aggtttgac                                         449
<210> 40
   <211> 201998
   <212> DNA
   <213> Homo sapiens
<400> 40
<210> 41
   <211> 3762
   <212> DNA
   <213> Homo sapiens
<400> 41
   acagaggctg tggctggaag gagctgggca tccggcctga ggcgcagcgg tcgcgttagt 60
   tcggcccaat ggcggcaccg ctgcttcaca cgcgtttgcc gggagatgcg gccgcttcgt 120
   cctctgcagt taagaagctg ggcgcgtcga ggactgggat ttcaaatatg cgtgcattag 180
   agaatgactt tttcaattct cccccaagaa aaactgttcg gtttggtgga actgtgacag 240
   aagtcttgct gaagtacaaa aagggtgaaa caaatgactt tgagttgttg aagaaccagc 300
   tgttagatcc agacataaag gatgaccaga tcatcaactg gctgctagaa ttccgttctt 360
   ctatcatgta cttgacaaaa gactttgagc aacttatcag tattatatta agattgcctt 420
   ggttgaatag aagtcaaaca gtagtggaag agtatttggc ttttcttggt aatcttgtat 480
   cagcacagac tgttttcctc agaccgtgtc tcagcatgat tgcttcccat tttgtgcctc 540
   cccgagtgat cattaaggaa ggcgatgtag atgtttcaga ttctgatgat gaagatgata 600
   atcttcctgc aaattttgac acatgtcaca gagccttgca aataatagca agatatgtac 660
   catcgacacc gtggtttctc atgccaatac tggtggaaaa atttccattt gttcgaaaat 720
   cagagagaac actggaatgt tacgttcata acttactaag gattagtgta tattttccaa 780
   ccttgaggca tgaaattctg gagcttatta ttgaaaaact actcaagttg gatgtgaatg 840
   catcccggca gggtattgaa gatgctgaag aaacagcaac tcaaacttgt ggtgggacag 900
   attccacgga aggattgttt aatatggatg aagatgaaga aactgaacat gaaacaaagg 960
   ctggtcctga acggctcgac cagatggtgc atcctgtagc cgagcgcctg gacatcctga 1020
   tgtctttggt tttgtcctac atgaaggatg tctgctatgt agatggtaag gttgataacg 1080
   gcaaaacaaa ggatctatat cgcgacctga taaacatctt tgacaaactc ctgttgccca 1140
   cccatgcctc ctgccatgta cagtttttca tgttttacct ctgtagtttc aaattgggat 1200
   tcgcagaggc atttttggaa catctctgga aaaaattgca ggacccaagt aatcctgcca 1260
   tcatcaggca ggctgctgga aattatattg gaagcttttt ggcaagagct aaatttattc 1320
   ctcttattac tgtaaaatca tgcctagatc ttttggttaa ctggctgcac atatacctta 1380
   ataaccagga ttcgggaaca aaggcattct gcgatgttgc tctccatgga ccattttact 1440
   cagcctgcca agctgtgttc tacacctttg tttttagaca caagcagctt ttgagcggaa 1500
   acctgaaaga aggtttgcag tatcttcaga gtctgaattt tgagcggata gtgatgagcc 1560
   agctaaatcc cctgaagatt tgcctgccct cagtggttaa cttttttgct gcaatcacaa 1620
   ataagtacca gctcgtcttc tgctacacca tcattgagag gaacaatcgc cagatgctgc 1680
   cagtcattag gagtaccgct ggaggagact cagtgcagat ctgcacaaac ccgctggaca 1740
   ccttcttccc ctttgatccc tgtgtgctga agaggtcaaa gaaattcatt gatcctattt 1800
   atcaggtgtg ggaagacatg agtgctgaag agctacagga gttcaagaaa cccatgaaaa 1860
   aggacatagt ggaagatgaa gatgatgact ttctgaaagg cgaagtgccc cagaatgata 1920
   ccgtgattgg gatcacacca agctcctttg acacgcattt ccgaagtcct tcaagtagtg 1980
   tgggctcccc acccgtgttg tacatgcaac ccagtcccct ctgacggcag aaatttgtga 2040
   ctgagatgtg acatttggga ttccccatca cttgtcatgc cctcagcacc cagcttgtgc 2100
   cattgggcat tgatggcatt gaactagagc gagtgcctgc ctcggctgtg gcacttccag 2160
   gttcgactga atcaagcatc tgaagactgg gtttttttgt tgttgttgtt ccccttacag 2220
   acaaaatgaa gactatcatg tgcaatcttt tacagtgggg ttgatgatac atttggaagg 2280
   atttgcttgt ttaatatgta cattttttgt gttaacagct ttttgacaca attactgggt 2340
   aatttctaat ataggcagca gactgtttta cgggttgctg ttttaacatg ggtttttgtc 2400
   agatccatgg tcttaggact tgactgatga gctttcagtg aagaatcctc taagataaaa 2460
   cttctattta aagactttaa ctagaaagtg tttattttgg ctacattgtt caccttctgc 2520
   tgtattggta tttgtctgtt gggatttcaa gggagtgtag agaagacaga aggaaagctg 2580
   agagctggcc cgacatggtc tgggacacag agttggagct ggcactgaag atctccaggg 2640
   acttcagaga ccaataaaag cccataggga agagagagag gatataggga aacagaatca 2700
   gatgtgtaat atacttggca cagcgaaaaa atggatttaa aagacaaaaa tggaggtcca 2760
   ggtagatgta attcacacag actgaaagtg agttcgggct tgtgtaaaac acatgagatt 2820
   ggatttgacc ccttggctct caagtgtccc cttagatcta gaactgctcc ttggtggcca 2880
   ttagatcgag tcagttttga tctgcatcac ttagttattg ggaatttctt tgttggaaac 2940
   aggaaaattt ttttagatta tttggtgtac ggttttgctc acaacaatag gtggaagttg 3000
   ctagtgcagt cttggtctga tggctgtgtg catcgcacat tcggcttggt gaaatccttc 3060
   tctaaagcct ctttttgtat ttttataact aaacagagga agtcttcaga agacctcgct 3120
   ttaaaacaaa tttgtgcaaa cactgctaga gtcattttga agctcaagca ttttcacttt 3180
   gtttcttaca tgtgtacttt tttgtttact tgtgaaaatg gccatcttta agcatattta 3240
   ttttctgcca ctttatttaa aggcaagcaa tattttcttg atcataaata ttttgtaatg 3300
   aaatacttcc tcttttccag ggctttgtat gcacttgtat aattacattg atggcaatgt 3360
   agagtttgaa tttcagtctg taaatacttt tttggaaaat agaaattttt attgctttaa 3420
   agttttggat atgggtggtt ttcttttccg ggtttggtgg aaagtaattt gagaacttta 3480
   aggttgtctt tttaactgct ggcaaaatgt tgatttttta atattagata aaacgagtaa 3540
   acgaaattcc ccagaaatta gtagtaagtg gggtctttgt gggttgggaa gtagttttaa 3600
   tgtagaaaga catttacata taagtctgtt taatttcaaa ggagtttgtg aaaaaaaatc 3660
   catggtgaaa atgaaacaat gacatggtta atctggaact tacgttctta taccaataaa 3720
   aggtacctca atacatgttc tttcaaaaaa aaaaaaaaaa aa 3762
<210> 42
   <211> 719
   <212> DNA
   <213> Homo sapiens
<220>
   <221> misc_feature
   <222> (520)..(520)
   <223> n is a, c, g, or t
<220>
   <221> misc_feature
   <222> (645)..(645)
   <223> n is a, c, g, or t
<220>
   <221> misc_feature
   <222> (682)..(682)
   <223> n is a, c, g, or t
<400> 42
   acataaagaa aattttcatt tatttttccc tcagatatac ttcaaaataa catgtagaca      60
   cagaatcaca aatatataca aaacaaagta tcgcctggtt atttcaagaa gagttttccc     120
   tcaattttct tagctgctgt gcataaaatt caaagctttc agggggttca atgaaaggga    180
   caggctctcc agcatacttc agcaaagatg cgtaacgctt tagaaacaga tcatccaatt     240
   ttgtattctt tgcagtcaaa tgttcataca gtgctttagc agatgtgaca tctttctctg     300
   agacatagct tttcatgagg gaattgtatg cttcttcctt ttcatttaat tcaggaatca     360
   attctaacac agatttcaca gttgatgcct ttccttgttt cctagaattc ctaaggagga     420
   acaacaacaa aatcggggtt tgttcagcaa ttgcaccaca tctctgtagg agagctctgg     480
   catcatccac cttgcctgca tccacaagtt gaaggaaaan atcagtgaca ggtttataaa     540
   ttgcaaactg attggccaat ctctccgcca tgatggctta tctttcaaac tgctggtcca     600
   actgctcctc tattactttc tggataagat gccaaggccg agtantcggg tcaatgactt     660
   tatcctctga agtaagctat tntcatggtt tctattgcgg catctatgta atcattctt      719
<210> 43
   <211> 5095
   <212> DNA
   <213> Homo sapiens
<400> 43
   acatgctcct cctgtccttc tggcggagcg tgcttcccgc tgcggggacg ttcgagcaat      60
   ggcagccctg ctgagatccg cgcgttggtt gctgcgtgcc ggggcggccc cgcgcctccc     120
   gctctccctg cgcctcctcc ctggcggccc gggccggctg catgccgcct cctatctgcc     180
   cgccgctcgc gccgggcccg tggccggagg actactgagc ccagccaggc tgtatgccat     240
   tgctgccaaa gaaaaagata ttcaagagga gtccactttt tcttctagga agatttccaa     300
   tcagtttgat tgggctctaa tgagactaga tctttctgtt cgaagaactg gccgcattcc     360
   aaagaagctt ctacaaaaag tttttaatga tacctgccgc tcaggtggcc taggtggtag     420
   tcatgccttg cttctactac gtagttgtgg ttctctcttg cctgaactaa agcttgaaga 480
   gagaacagaa tttgctcata ggatatggga cacacttcag aaattaggtg ctgtgtatga 540
   tgtgagtcac tataatgctt tacttaaagt ctatcttcaa aatgaatata aattctcacc 600
   aactgatttc ctggcaaaaa tggaggaagc aaacattcaa ccaaatcgag tgacatacca 660
   gagattgatt gcttcttatt gtaatgtagg agatattgaa ggtgccagca agattcttgg 720
   atttatgaaa actaaggatc tcccagttac agaggcagta ttcagtgccc ttgtgacagg 780
   gcatgccaga gctggtgata tggagaatgc agaaaacatt ctcacagtga tgagagatgc 840
   cggaattgag cctggtccag acacatacct cgcattattg aatgcatatg ctgagaaggg 900
   cgacattgac catgttaagc agactctgga gaaggtggag aagtccgagc ttcaccttat 960
   ggaccgtgat ttactgcaaa ttatttttag cttcagtaaa gctgggtatc ctcagtatgt 1020
   ctcagaaatt ttggaaaaag ttacatgtga aagaagatat attccagatg caatgaacct 1080
   cattttactt ttagtcactg aaaaattgga agatgtagcg ttgcaaattt tactagcatg 1140
   ccccgtatca aaggaagatg gcccaagtgt ctttggcagt ttctttttac aacactgtgt 1200
   gactatgaat acgcctgtgg agaagctaac agactactgt aagaagttaa aggaagtcca 1260
   gatgcactcc tttcctctgc agttcaccct ccattgtgct ttactcgcca ataaaactga 1320
   tttggcaaaa gccttaatga aggctgtgaa ggaggaaggt tttcctatca gacctcacta 1380
   tttctggcca ttgctagttg gacgtcggaa ggaaaaaaat gttcaaggta taattgaaat 1440
   cctcaaagga atgcaagaat tgggagtaca tcctgatcag gaaacatata cagattatgt 1500
   gattccatgc tttgatagtg taaactcagc acgagccatt ttgcaggaaa atggatgtct 1560
   gtctgatagt gatatgtttt ctcaagctgg attgagaagt gaagcagcaa atgggaactt 1620
   agactttgta ttatcatttt tgaaatcaaa tacattgccc atctcgctgc agtctataag 1680
   aagtagccta ctgctaggct tcaggaggtc tatgaatata aatctttgga gcgagataac 1740
   agaattgttg tacaaggatg gacgttattg ccaggagcct cgaggaccga cggaagctgt 1800
   tggctatttt ctttataact tgattgacag catgagtgac tcagaagtac aggccaagga 1860
   ggagcatttg agacaatact tccatcagct ggagaagatg aatgtaaaaa ttcctgaaaa 1920
   tatctacaga ggcattcgta atctcctgga aagctaccat gttcctgaat tgattaagga 1980
   tgctcacttg ttggttgaga gtaagaattt agactttcaa aaaactgtgc aacttacatc 2040
   atctgaattg gagtccacac ttgaaacact aaaagctgaa aatcaaccta taagagatgt 2100
   cctaaagcaa ctcatattag tgctttgttc agaagagaat atgcaaaaag cccttgaatt 2160
   gaaagcaaaa tatgaatccg acatggttac tggtggctat gcagctttaa taaatttatg 2220
   ctgtcgacat gataaagtag aagatgcctt gaacttgaaa gaagaatttg accgcttaga 2280
   ttcatctgct gtccttgaca ccggcaagta tgtaggcctt gtaagagtat tggcaaagca 2340
   tggcaagctc caagatgcta ttaacattct gaaggagatg aaagagaagg atgttcttat 2400
   caaagataca acagccttgt cctttttcca catgctaaat ggcgcagctt taagaggtga 2460
   aattgaaaca gtaaaacagt tgcatgaagc catcgtgact ctagggttag cagaaccatc 2520
   caccaacata agtttcccat tggtcactgt acacttggaa aagggcgacc tatctactgc 2580
   tcttgaggtc gccattgact gctatgaaaa gtataaagta ttaccaagga ttcatgatgt 2640
   cttgtgtaaa ctggtagaga aaggcgagac tgatctaatt cagaaagcaa tggactttgt 2700
   gagccaagaa caaggtgaaa tggtgatgct ctatgatctc ttctttgcct tcctacaaac 2760
   aggaaattac aaagaggcca agaagatcat tgagactcca gggattagag ctcgatctgc 2820
   aaggcttcag tggttttgtg acagatgtgt tgcaaataat caggttgaaa ctctggaaaa 2880
   attagtggag ctgacacaga agctatttga atgtgataga gaccagatgt actacaatct 2940
   gctaaaactg tataaaataa acggtgactg gcaaagagct gatgcagtct ggaataaaat 3000
   ccaagaagaa aatgttattc ctcgtgaaaa gacattaaga ttattagcag aaatccttag 3060
   agagggtaac caggaagttc cgtttgacgt acctgagttg tggtatgaag atgaaaaaca 3120
   ttccctgaat tcttcgtcag cctcaaccac agaacctgat ttccagaaag atatattgat 3180
   tgcctgccga ttgaaccaaa aaaaaggggc atatgatatt ttcctgaatg caaaagagca 3240
   aaacattgtg tttaatgctg aaacctacag caatctcatt aaattactga tgtcagaaga 3300
   ttattttaca caagcaatgg aagtgaaagc attcgcggag acccacatca agggcttcac 3360
   actgaacgat gctgccaaca gccgcctcat cataacgcaa gttaggcggg attatttgaa 3420
   agaggctgtg acaacactga aaacagtatt ggatcagcag cagacccctt ctaggttagc 3480
   agtgacccgt gtcatccagg cattggccat gaagggtgat gttgaaaaca tagaagtagt 3540
   tcagaagatg ttaaatggac tcgaagactc cattggactt tcaaaaatgg ttttcatcaa 3600
   taacattgct ttggctcaaa taaagaataa taacatagat gccgcaatag aaaacattga 3660
   aaatatgctt acttcagaga ataaagtcat tgaaccccaa tacttcggct tggcatactt 3720
   attcagaaaa gtaatagagg agcagttgga accagcagtt gaaaagataa gcatcatggc 3780
   ggagagattg gccaatcagt ttgcaattta taaacctgtc actgattttt tccttcaact 3840
   tgtggatgca ggcaaggtgg atgatgccag agctctccta cagagatgtg gtgcaattgc 3900
   tgaacaaacc ccgattttgt tgttgttcct ccttaggaat tctaggaaac aaggaaaggc 3960
   atcaactgtg aaatctgtgt tagaattgat tcctgaatta aatgaaaagg aagaagcata 4020
   caattccctc atgaaaagct atgtctcaga gaaagatgtc acatctgcta aagcactgta 4080
   tgaacatttg actgcaaaga atacaaaatt ggatgatctg tttctaaagc gttacgcatc 4140
   tttgctgaag tatgctggag agcctgtccc tttcattgaa ccccctgaaa gctttgaatt 4200
   ttatgcacag cagctaagaa aattgaggga aaactcttct tgaaataacc aggcgatact     4260
   ttgttttgta tatatttgtg attctgtgtc tacatgttat tttgaagtat atctgaggga     4320
   aaaataaatg aaaattttct ttatgtactt atgtatgtgt gatgcatgtt caaagtctta     4380
   ttgaccataa ctctgtgcac ttggttattg gacatttttg gagttttttt ctctgggaaa     4440
   aatcgatagt gttttcttca atgctgctgc tgtgtgaagc catacttttt caggattctt     4500
   cccctaattg gctctttggt ttccctgctc tgtttcattt atttcattaa aatgttattc     4560
   ctttatttaa gattcactta ttagtctgct gtttctctga aaaattttag agctaggtat     4620
   agtgaccgtg aactttctaa cgcataatat tctgtgatac agccattccg tacatgtgtg     4680
   aagtcctgca taactttcga actttgttaa atgttggcac taggagtcat cagatctagg     4740
   cttcatcatt ttccagtgag aagcagagac ccaaagggcc tgttacttgt gcttggtcag     4800
   gggactgtct gtcatgcctg gaggctcttc ggcacacttc cccatctttc ccttctgcca     4860
   ctgtggcttc aagcacctct gttcatagag cgtctctgaa attgagtctc ggtcatgact     4920
   tatcccgaag tagagcaatg tgtttcctct cattgtagtt tcaggacttt gtcagtacaa     4980
   gctctgccct aggcttgtta ctttatactc atatcctgaa aagatgtgat ttcatctatg     5040
   aaggggtaaa atattggttt gtatttaatt gtttgaaata aaagtgatcc ctata           5095
<210> 44
   <211> 5427
   <212> DNA
   <213> Homo sapiens
<400> 44
   agatgcgacg aggttagcgg ggcccgacca ctccttggct tcccaggggt gagcctcgcg      60
   agttaggagt tgggtagaga gtcagcccgg ggcccggcat ccgctttttc gttgaagcaa     120
   cgactttggc cggatgactc cccagggtcg gcatcagcgt gggactggga acaggtgaag     180
   ggaaacagaa gagagcctga gaagaccgct ctcctccgat cctaattgac tgagccaatg     240
   agagccaaag aggtcacatg ctcaactggg cggggagcgg gtttccacct gcggcatctt     300
   tcgcgagcgg ggagatgagt ggggcggaat atgggagaaa gggagggccc gccacgctct     360
   ggctggacac ggccttaatc ggcccgttca ctcgacgttt ttggttctac gttgaccccg     420
   agaaaccgaa accgcagggc ctagggcggg tggaacgaga gggaactaca tttcccagca     480
   ggctgcggaa acgggactgc ggccactact tccggcgtgt accgagagac tggcgtccgg     540
   cgtgtaccga gagactggcg tccggtgtgc aggtggccac atggatcctg gcagccggtg     600
   gcggaacctg cccagcgggc ctagcctaaa gcacttgact gacccctctt atggaatccc     660
   gcgggaacag caaaaggcag cgttgcagga gctgacgcgg gcgcacgtgg agtccttcaa     720
   ctacgctgtg cacgagggtc tcggcctcgc ggtgcaggct atacctccct ttgaatttgc     780
   tttcaaagat gagcgtatct cttttactat tctggatgct gttatcagtc cacctacagt 840
   tccaaaaggg accatctgca aagaggccaa tgtttatcca gcagaatgcc ggggccgaag 900
   gagtacctac cgtgggaagt tgacagctga tatcaactgg gcagtgaatg gaatctcaaa 960
   aggaatcatt aagcagtttc ttggctatgt tcccatcatg gtgaaatcca agctttgcaa 1020
   cttacgtaac cttcccccac aagccctcat tgagcaccat gaggaggcag aggaaatggg 1080
   gggctatttt ataatcaatg gcattgaaaa agtcatccga atgttgatta tgcctcggag 1140
   aaattttccc attgcaatga taagaccaaa atggaaaacc agagggcctg gttatactca 1200
   gtatggagtt tcaatgcact gtgtgaggga agaacattcc gctgtcaata tgaacctcca 1260
   ctacttggaa aatggcactg ttatgttgaa ctttatttac cgaaaagaac tgttctttct 1320
   tcctttggga tttgcactta aggcacttgt cagcttttct gattatcaga tctttcagga 1380
   gctcatcaaa ggaaaagagg atgattcttt ccttaggaac tctgtttctc agatgttaag 1440
   gattgtaatg gaagagggtt gttcgacaca aaaacaggtc cttaactacc taggtgaatg 1500
   cttcagagta aaactcaatg ttcctgactg gtacccaaat gagcaagctg cggagttcct 1560
   gtttaaccag tgcatctgta tccacttgaa atccaatact gaaaagtttt atatgctttg 1620
   tctcatgacg cgaaagctct ttgctttagc caaaggagag tgcatggagg acaatcctga 1680
   tagtttggtg aaccaggaag tcctcacacc gggtcagctc ttccttatgt tcctgaagga 1740
   aaaactggaa ggttggttag tgtctattaa aatagctttt gataagaagg ctcagaagac 1800
   cagtgtttcc atgaacactg acaatttgat gaggattttt acaatgggca tagaccttac 1860
   aaaaccattt gaataccttt ttgctactgg gaatctgcgt tctaaaacag gtcttggcct 1920
   cctacaagat tctggacttt gtgttgtggc tgacaagctg aacttcatac gctacctctc 1980
   ccatttccgc tgcgtgcaca gaggggctga ttttgccaag atgaggacca ccacagtacg 2040
   caggctgctg ccagagtcct ggggcttcct ttgtcccgtg cataccccag acggggagcc 2100
   ctgtggcctg atgaaccacc taactgccgt atgtgaggtt gtcacacagt ttgtgtatac 2160
   ggcatctatt ccagctttac tgtgcaactt gggggtcact cccattgatg gagctcccca 2220
   ccgatcatac agtgagtgct accctgtcct gctggacggt gtcatggttg gctgggtgga 2280
   taaggatctt gctccaggca tcgcagattc tcttcgtcat tttaaggtgt tgagagagaa 2340
   aagaattcct ccctggatgg aagtggtcct tatacccatg acaggaaaac caagtctgta 2400
   cccaggattg ttccttttta ccactccttg tagactggta cggcctgtgc agaacttagc 2460
   attgggcaaa gaagagctaa ttggaactat ggaacagatc ttcatgaatg tcgctatctt 2520
   tgaggatgaa gtttttgctg gagttaccac acaccaggaa ctctttccac acagcctgct 2580
   gagtgtgatt gccaacttca tccctttctc tgatcacaac cagagtccac ggaacatgta 2640
   ccaatgccag atgggtaagc aaactatggg ctttccactt ctcacttatc aagaccgatc 2700
   ggataacaaa ctgtatcgtc ttcagactcc tcagagtccc ttggtgagac cctccatgta 2760
   tgattattat gacatggata actatccaat tgggaccaat gccatcgttg ctgtgatttc 2820
   ttacactggc tatgatatgg aagatgccat gattgtgaat aaggcctctt gggaacgagg 2880
   ctttgcccat ggaagtgtct acaagtctga gttcatagac ctctctgaaa aaattaaaca 2940
   aggagatagt agcctggtgt ttggcatcaa acctggtgac ccacgcgttc tgcagaagtt 3000
   agatgacgat ggattgccgt ttataggagc aaaactgcag tacggagatc cgtattacag 3060
   ctacctcaac ctcaacaccg gggaaagttt tgtgatgtac tataagagta aagaaaattg 3120
   tgttgtggat aacatcaaag tgtgcagtaa tgacactggg agtggaaaat tcaagtgtgt 3180
   ttgcatcact atgagagtgc ctcggaaccc aactatcgga gataaatttg ccagtcgcca 3240
   tgggcagaag ggcattttaa gcagattgtg gccggctgag gacatgcctt ttactgagag 3300
   tgggatggtc ccagacattc tgttcaatcc ccatggtttt ccatcccgca tgaccattgg 3360
   gatgttaatt gagagtatgg ccgggaagtc tgcagctttg catggtctct gccatgatgc 3420
   tacacccttc atcttctcag aggagaactc ggccttagaa tactttggtg agatgttaaa 3480
   ggctgctggc tacaatttct atggcaccga gaggttatat agtggcatca gtgggctaga 3540
   actggaagca gacatcttca taggagtggt ttattatcag cgcttacgcc atatggtctc 3600
   agacaaattt caagtaagga caactggagc ccgagacaga gtcaccaacc agcctattgg 3660
   gggaagaaat gtccagggtg gaatccgttt tggggagatg gaacgggatg cgcttttagc 3720
   tcatggtaca tcttttctcc ttcatgaccg cctcttcaac tgctcagatc ggtcggtagc 3780
   ccatgtgtgt gtgaagtgtg gcagtttact ctctccactg ttggagaagc cacccccttc 3840
   ttggtctgcc atgcgcaaca gaaaatacaa ctgtactctg tgtagtcgca gtgacactat 3900
   cgatactgtt tctgtgcctt atgtttttcg gtattttgta gctgaactgg cagctatgaa 3960
   catcaaagtg aaactggatg ttgtttaact tgatgttgac cttttggatt aagaggacta 4020
   tcagattaaa gcaaaatgta attttaattc aatgaagata tcattaccag gttactcttg 4080
   agatttttca acggtgttag aactctcaac caagacctga aaaccaagta tgcaaggttt 4140
   ctgaatctct ctggtagatt aactattgac aatgattttc tgttatcttt gttcaaaaag 4200
   ttcatgtctt ctcaaaatat gaaatattga taaatggaag agcatacggt gacaagtctc 4260
   ctttccaacc ccaggttccc tacaccctgc tctcagcagg cagtgagtgt cacacacctg 4320
   ttaatccatc ttgagcagga cagtactata caaatagaat gcaagctgta atgtaatttt 4380
   atattttctt atagccacgt tgaagtaaaa acaaacaggt acagtgtttt ttaccagctt 4440
   tatagaagta cagttgttac atatttaatg aatacaattt gatgggtctg actatatgca 4500
   cacacctttg ataccatcac cacaatcagg gtaataaaca tacctgtcat ctccacaagt 4560
   ttcctcctgc ccctttgttt tttgcttttt ggttgctgtt gagtttttgt tttgtcttct 4620
   gtggtaagaa cacttaactc aagacctacc ctcttaacaa atctttaagt gcacgatata 4680
   gtattgttaa ttccaggcac catgttgtac aacagatctt tagaccttac ttgtcttgca 4740
   taactgaagc tttatacctg ttgaacaact ctccatttcc ctggccccta gcaaccaccc 4800
   ttctaccctg tttctatgag tttgactatt acagatatct catatagtgg gatcatgcaa 4860
   tatttgtcct gtgactggct tatttcactt agcatagtga aataagattc atccattttg 4920
   gaagccaggc atggtgctgt gcatctatag tccctgctat ttgagaggct gaggtgggag 4980
   gatcatttga gtgcaggagt tcaaggacag cctgggtaat ataggaagac cctgtcttga 5040
   agaccctgac ctcaagtgat ccacccacct cggcctccga aagtgctagg attacaggtg 5100
   tgagccactg tgcctggcct ccggtgagta ttttatattt agtctacact tccatacttg 5160
   gctttttctt gcttttatat tgatctgctt tcatagcagt gtgtagagtg ccacttatgt 5220
   tttctttctt gtgtacagta ttttattgta tggatttacc atcccctgtg tatttaagtt 5280
   gttccattct ttggccatta taactttttt ctgcaaatat tctggtgact tatctttggc 5340
   cattataaac tgttgataat agatcatctt gtatatactt ctgcaattat aagatgtttt 5400
   ttgatgatgt aaaaaaaaaa aaaaaaa 5427
<210> 45
   <211> 1138
   <212> DNA
   <213> Homo sapiens
<400> 45
   agggctacta ggacgcacgc gcgagataga acctctagtc tcgtggagag attgaagatg 60
   gcggcttctc aggcggtgga ggaaatgcgg agccgcgtgg ttctggggga gtttggggtt 120
   cgcaatgtcc atactactga ctttcccggt aactattccg gttatgatga tgcctgggac 180
   caggaccgct tcgagaagaa tttccgtgtg gatgtagtac acatggatga aaactcactg 240
   gagtttgaca tggtgggaat tgacgcagcc attgccaatg cttttcgacg aattctgcta 300
   gctgaggtgc caactatggc tgtggagaag gtcctggtgt acaataatac atccattgtt 360
   caggatgaga ttcttgctca ccgtctgggg ctcattccca ttcatgctga tccccgtctt 420
   tttgagtatc ggaaccaagg agatgaagaa ggcacagaga tagatactct acagtttcgt 480
   ctccaggtca gatgcactcg gaacccccat gctgctaaag attcctctga ccccaacgaa 540
   ctgtacgtga accacaaagt gtataccagg catatgacat ggatccccct ggggaaccag 600
   gctgatctct ttccagaggg cactatccga ccagtgcatg atgatatcct catcgctcag 660
   ctgcggcctg gccaagaaat tgacctgctc atgcactgtg tcaagggcat tggcaaagat 720
   catgccaagt tttcaccagt ggcaacagcc agttacaggc tcctgccaga catcaccctg 780
   cttgagcccg tggaagggga ggcagctgag gagttgagca ggtgcttctc acctggtgtt 840
   attgaggtgc aggaagtcca aggtaaaaag gtggccagag ttgccaaccc ccggctggat 900
   accttcagca gagaaatctt ccggaatgag aagctaaaga aggttgtgag gcttgcccgg 960
   gttcgagatc attatatctg taagaaagat ttgctggctg cggtggctca cacctgtaat 1020
   cccagcactt tgggaggcca aggcgagtgg atcacggggt caagagagcg agaccatcct 1080
   ggctaacatg gtgaaacccc gtctctataa aaaaaaaaaa aaaaaaaaaa aaaaaaaa 1138
<210> 46
   <211> 1277
   <212> DNA
   <213> Homo sapiens
<400> 46
   gagagagaga agatggcggc ttctcaggcg gtggaggaaa tgcggagccg cgtggttctg      60
   ggggagtttg gggttcgcaa tgtccatact actgactttc ccggtaacta ttccggttat     120
   gatgatgcct gggaccagga ccgcttcgag aagaatttcc gtgtggatgt agtacacatg     180
   gatgaaaact cactggagtt tgacatggtg ggaattgacg cagccattgc caatgctttt     240
   cgacgaattc tgctagctga ggtgccaact atggctgtgg agaaggtcct ggtgtacaat     300
   aatacatcca ttgttcagga tgagattctt gctcaccgtc tggggctcat tcccattcat     360
   gctgatcccc gtctttttga gtatcggaac caaggagatg aagaaggcac agagatagat     420
   actctacagt ttcgtctcca ggtcagatgc actcggaacc cccatgctgc taaagattcc     480
   tctgacccca acgaactgta cgtgaaccac aaagtgtata ccaggcatat gacatggatc     540
   cccctgggga accaggctga tctctttcca gagggcacta tccgaccagt gcatgatgat     600
   atcctcatcg ctcagctgcg gcctggccaa gaaattgacc tgctcatgca ctgtgtcaag     660
   ggcattggca aagatcatgc caagttttca ccagtggcaa cagccagtta caggctcctg     720
   ccagacatca ccctgcttga gcccgtggaa ggggaggcag ctgaggagtt gagcaggtgc     780
   ttctcacctg gtgttattga ggtgcaggaa gtccaaggta aaaaggtggc cagagttgcc     840
   aacccccggc tggatacctt cagcagagaa atcttccgga atgagaagct aaagaaggtt     900
   gtgaggcttg cccgggttcg agatcattat atcttctctg ttgagtcaac gggggtgttg     960
   ccaccagatg tgctggtgag tgaagccatc aaagtactga tggggaagtg ccggcgcttc    1020
   ttggatgaac tagatgcggt tcagatggac tgagcttgga tgcttctgag gcaagctgaa    1080
   gctttgggtt ctgactgacc caccctacag gactgctgaa cagagagccc agtgtgacta    1140
   gggatcctga gttttctggg acaattccag ctttaatcaa tacattttgt taaatgtgcc    1200
   ataaaatgag actttttacg cctttataag gccttagatg taaataaact cacccaaaca    1260
   aaaaaaaaaa aaaaaaa                                                   1277
<210> 47
   <211> 725
   <212> DNA
   <213> Homo sapiens
<400> 47
   ccttccttcc tgcttcgcct ccgcgcctcg cgctatggga cagagccccc gatccgccag 60
   caccacctga ggatccagaa accgccccag cgatggaaga ggatcaggag ctggagagaa 120
   aaatatctgg attgaagacc tcaatggctg aaggcgagag gaagacagcc ctggaaatgg 180
   tccaggcagc tggaacagat agacactgtg tgacatttgt attgcacgag gaagaccata 240
   ccctaggaaa ttctctacgt tacatgatca tgaagaaccc ggaagtggaa ttttgtggtt 300
   acactacgac ccatccttca gagagcaaaa ttaatttacg cattcagact cgaggtaccc 360
   ttccagctgt tgagccattt cagagaggcc tgaatgagct catgaatgtc tgccaacatg 420
   tgcttgacaa gtttgaggcc agcataaagg actataagga tcaaaaagca agcagaaatg 480
   aatccacatt ctagtccttt atgcagtata caaggagaac tgtcctgtag gatattctct 540
   tcctgatggt gcagaaccca gaattagaag tttgtggtta cagcatactc tgtccttcag 600
   aaaggcgtga ttctagctgt tgaccccttg cagctgttgg aatctctgca agaacctctg 660
   tattcttcta ataaattccc tcttttattt aaaaaaaaaa aaaaaaaaaa aaaaaaaaaa 720
   aaaaa 725
<210> 48
   <211> 6584
   <212> DNA
   <213> Homo sapiens
<400> 48
   tttttttttc ctttttggga gctgaaaaat ttccggtaag ggaaagaagg gctcctttcg 60
   ctccttattt ccccgcctcc ttccctcccc caccttcccc tcctccggct ttttcctccc 120
   aactcgggga ggtccttccc ggtggccgcc ctgacgaggt ctgagcacct aggcggaggc 180
   ggcgcaggct ttttgtagtg aggtttgcgc ctgcgcagcg cgcctgcctc cgccatgcac 240
   gggggtggcc ccccctcggg ggacagcgca tgcccgctgc gcaccatcaa gagagtccag 300
   ttcggagtcc tgagtccgga tgaactgaag cgaatgtctg tgacggaggg tggcatcaaa 360
   tacccagaga cgactgaggg aggccgcccc aagcttgggg ggctgatgga cccgaggcag 420
   ggggtgattg agcggactgg ccgctgccaa acatgtgcag gaaacatgac agagtgtcct 480
   ggccactttg gccacattga actggccaag cctgtgtttc acgtgggctt cctggtgaag 540
   acaatgaaag ttttgcgctg tgtctgcttc ttctgctcca aactgcttgt ggactctaac 600
   aacccaaaga tcaaggatat cctggctaag tccaagggac agcccaagaa gcggctcaca 660
   catgtctacg acctttgcaa gggcaaaaac atatgcgagg gtggggagga gatggacaac 720
   aagttcggtg tggaacaacc tgagggtgac gaggatctga ccaaagaaaa gggccatggt 780
   ggctgtgggc ggtaccagcc caggatccgg cgttctggcc tagagttgta tgcggaatgg 840
   aagcacgtta atgaggactc tcaggagaag aagatcctgc tgagtccaga gcgagtgcat 900
   gagatcttca aacgcatctc agatgaggag tgttttgtgc tgggcatgga gccccgctat 960
   gcacggccag agtggatgat tgtcacagtg ctgcctgtgc cccgctctcc cgtgcggcct 1020
   gctgttgtga tgcagggctc tgcccgtaac caggatgacc tgactcacaa actggctgac 1080
   atcgtgaaga tcaacaatca gctgcggcgc aatgagcaga acggcgcagc ggcccatgtc 1140
   attgcagagg atgtgaagct cctccagttc catgtggcca ccatggtgga caatgagctg 1200
   cctggcttgc cccgtgccat gcagaagtct gggcgtcccc tcaagtccct gaagcagcgg 1260
   ttgaagggca aggaaggccg ggtgcgaggg aacctgatgg gcaaaagagt ggacttctcg 1320
   gcccgtactg tcatcacccc cgaccccaac ctctccattg accaggttgg cgtgccccgc 1380
   tccattgctg ccaacatgac ctttgcggag attgtcaccc ccttcaacat tgacagactt 1440
   caagaactag tgcgcagggg gaacagccag tacccaggcg ccaagtacat catccgagac 1500
   aatggtgatc gcattgactt gcgtttccac cccaagccca gtgaccttca cctgcagacc 1560
   ggctataagg tggaacggca catgtgtgat ggggacattg ttatcttcaa ccggcagcca 1620
   actctgcaca aaatgtccat gatggggcat cgggtccgca ttctcccatg gtctaccttt 1680
   cgcttgaatc ttagtgtgac aactccgtac aatgcagact ttgacgggga tgagatgaac 1740
   ttgcacctgc cacagtctct ggagacgcga gcagagatcc aggagctggc catggttcct 1800
   cgcatgattg tcacccccca gagcaatcgg cctgtcatgg gtattgtgca ggacacactc 1860
   acagcagtgc gcaaattcac caagagagac gtcttcctgg agcggggtga agtgatgaac 1920
   ctcctgatgt tcctgtcgac gtgggatggg aaggtcccac agccggccat cctaaagccc 1980
   cggcccctgt ggacaggcaa gcaaatcttc tccctcatca tacctggtca catcaattgt 2040
   atccgtaccc acagcaccca tcccgatgat gaagacagtg gcccttacaa gcacatctct 2100
   cctggggaca ccaaggtggt ggtggagaat ggggagctga tcatgggcat cctgtgtaag 2160
   aagtctctgg gcacgtcagc tggctccctg gtccacatct cctacctaga gatgggtcat 2220
   gacatcactc gcctcttcta ctccaacatt cagactgtca ttaacaactg gctcctcatc 2280
   gagggtcata ctattggcat tggggactcc attgctgatt ctaagactta ccaggacatt 2340
   cagaacacta ttaagaaggc caagcaggac gtaatagagg tcatcgagaa ggcacacaac 2400
   aatgagctgg agcccacccc agggaacact ctgcggcaga cgtttgagaa tcaggtgaac 2460
   cgcattctta acgatgcccg agacaagact ggctcctctg ctcagaaatc cctgtctgaa 2520
   tacaacaact tcaagtctat ggtcgtgtcc ggagctaaag gttccaagat taacatctcc 2580
   caggtcattg ctgtcgttgg acagcagaac gtcgagggca agcggattcc atttggcttc 2640
   aagcaccgga ctctgcctca cttcatcaag gatgactacg ggcctgagag ccgtggcttt 2700
   gtggagaact cctacctagc cggcctcaca cccactgagt tctttttcca cgccatgggg 2760
   ggtcgtgagg ggctcattga cacggctgtc aagactgctg agactggata catccagcgg 2820
   cggctgatca agtccatgga gtcagtgatg gtgaagtacg acgcgactgt gcggaactcc 2880
   atcaaccagg tggtgcagct gcgctacggc gaagacggcc tggcaggcga gagcgttgag 2940
   ttccagaacc tggctacgct taagccttcc aacaaggctt ttgagaagaa gttccgcttt 3000
   gattatacca atgagagggc cctgcggcgc actctgcagg aggacctggt gaaggacgtg 3060
   ctgagcaacg cacacatcca gaacgagttg gagcgggaat ttgagcggat gcgggaggat 3120
   cgggaggtgc tcagggtcat cttcccaact ggagacagca aggtcgtcct cccctgtaac 3180
   ctgctgcgga tgatctggaa tgctcagaaa atcttccaca tcaacccacg ccttccctcc 3240
   gacctgcacc ccatcaaagt ggtggaggga gtcaaggaat tgagcaagaa gctggtgatt 3300
   gtgaatgggg atgacccact aagtcgacag gcccaggaaa atgccacgct gctcttcaac 3360
   atccacctgc ggtccacgtt gtgttcccgc cgcatggcag aggagtttcg gctcagtggg 3420
   gaggccttcg actggctgct tggggagatt gagtccaagt tcaaccaagc cattgcgcat 3480
   cccggggaaa tggtgggggc tctggctgcg cagtcccttg gagaacctgc cacccagatg 3540
   accttgaata ccttccacta tgctggtgtg tctgccaaga atgtgacgct gggtgtgccc 3600
   cgacttaagg agctcatcaa catttccaag aagccaaaga ctccttcgct tactgtcttc 3660
   ctgttgggcc agtccgctcg agatgctgag agagccaagg atattctgtg ccgtctggag 3720
   catacaacgt tgaggaaggt gactgccaac acagccatct actatgaccc caacccccag 3780
   agcacggtgg tggcagagga tcaggaatgg gtgaatgtct actatgaaat gcctgacttt 3840
   gatgtggccc gaatctcccc ctggctgttg cgggtggagc tggatcggaa gcacatgact 3900
   gaccggaagc tcaccatgga gcagattgct gaaaagatca atgctggttt tggtgacgac 3960
   ttgaactgca tctttaatga tgacaatgca gagaagctgg tgctccgtat tcgcatcatg 4020
   aacagcgatg agaacaagat gcaagaggag gaagaggtgg tggacaagat ggatgatgat 4080
   gtcttcctgc gctgcatcga gtccaacatg ctgacagata tgaccctgca gggcatcgag 4140
   cagatcagca aggtgtacat gcacttgcca cagacagaca acaagaagaa gatcatcatc 4200
   acggaggatg gggaattcaa ggccctgcag gagtggatcc tggagacgga cggcgtgagc 4260
   ttgatgcggg tgctgagtga gaaggacgtg gaccccgtac gcaccacgtc caatgacatt 4320
   gtggagatct tcacggtgct gggcattgaa gccgtgcgga aggccctgga gcgggagctg 4380
   taccacgtca tctcctttga tggctcctat gtcaattacc gacacttggc tctcttgtgt 4440
   gataccatga cctgtcgtgg ccacttgatg gccatcaccc gacacggagt caaccgccag 4500
   gacacaggac cactcatgaa gtgttccttt gaggaaacgg tggacgtgct tatggaagca 4560
   gccgcacacg gtgagagtga ccccatgaag ggggtctctg agaatatcat gctgggccag 4620
   ctggctccgg ccggcactgg ctgctttgac ctcctgcttg atgcagagaa gtgcaagtat 4680
   ggcatggaga tccccaccaa tatccccggc ctgggggctg ctggacccac cggcatgttc 4740
   tttggttcag cacccagtcc catgggtgga atctctcctg ccatgacacc ttggaaccag 4800
   ggtgcaaccc ctgcctatgg cgcctggtcc cccagtgttg ggagtggaat gaccccaggg 4860
   gcagccggct tctctcccag tgctgcgtca gatgccagcg gcttcagccc aggttactcc 4920
   cctgcctggt ctcccacacc gggctccccg gggtccccag gtccctcaag cccctacatc 4980
   ccttcaccag gtggtgccat gtctcccagc tactcgccaa cgtcacctgc ctacgagccc 5040
   cgctctcctg ggggctacac accccagagt ccctcttatt cccccacttc accctcctac 5100
   tcccctacct ctccatccta ttctccaacc agtcccaact atagtcccac atcacccagc 5160
   tattcgccaa cgtcacccag ctactcaccg acctctccca gctactcacc cacctctccc 5220
   agctactcgc ccacctctcc cagctactcg cccacctctc ccagctactc acccacttcc 5280
   cctagctact cgcccacttc ccctagctac tcgccaacgt ctcccagcta ctcaccgaca 5340
   tctcccagct actcgccaac ttcacccagc tattctccca cttctcccag ctactcacct 5400
   acctctccaa gctattcacc cacctccccc agctactcac ccacttcccc aagttactca 5460
   cccaccagcc cgaactattc tccaaccagt cccaattaca ccccaacatc acccagctac 5520
   agcccgacat cacccagcta ttcacctact agtcccaact acacacctac cagccctaac 5580
   tacagcccaa cctctccaag ctactctcca acatcaccca gctattcccc gacctcacca 5640
   agttactccc cttccagccc acgatacaca ccacagtctc caacctatac cccaagctca 5700
   cccagctaca gccccagctc gcccagctac agcccaacct cacccaagta caccccaacc 5760
   agtccttctt acagtcccag ctccccagag tataccccaa cctctcccaa gtactcacct 5820
   accagtccca aatattcacc cacctctccc aagtactcgc ctaccagtcc cacctattca 5880
   cccaccaccc caaaatactc cccaacatct cctacttatt ccccaacctc tccagtctac 5940
   accccaacct ctcccaagta ctcacctact agccccactt actcgcccac ttcccccaag 6000
   tactcgccca ccagccccac ctactcgccc acctccccca aaggctcaac ctactctccc 6060
   acttcccctg gttactcgcc caccagcccc acctacagtc tcacaagccc ggctatcagc 6120
   ccggatgaca gtgacgagga gaactgaggg cacgtggggt gcggcagcgg gctagggccc 6180
   agggcagctt gcccgtgctg ctgtgcagtt cttgcctccc tcacggggcg tcacccccag 6240
   cccagctccg ttgtacataa atgccttgtg gcagagctcc cggtgaactt ctggatcccg 6300
   tttctgatgc agactcttgt cttgttctcc acttgtgctg ttagaactca ctggcccagt 6360
   ggtgttctca ctcctacccc acccaccccc tgcctgtccc caaattgaag atccttcctt 6420
   gcctgtggct tgatgcgggg cgggtaaagg gtattttaac ttaggggtag ttcctgctgt 6480
   gagtggttac agctgatcct cgggaagaac aaagctaaag ctgccttttg tctgttattt 6540
   tatttttttg aagtttaaat aaagtttact aattttgacc aaaa 6584
<210> 49
   <211> 342
   <212> DNA
   <213> Homo sapiens
<400> 49
   atccggcgtt ctggcctaga gctgtatgcg gaatggaagc acgttaatga ggactctcag 60
   gagaagaaga tcctgctgag tccagagcga gtgcatgaga tcttcaaacg catctcagat 120
   gaggagtgtt ttgtgctggg catcgagccc cgctatgcac ggccagagtc gatgattgtc 180
   acagtgctgc ccgtgccccc gctgtccgtg cggcctgctg ttgtgatgca gggctctgcc 240
   cgtaaccagg tcagtggctc cagggctctg cctcttagct ggagggcgag gtgtctaaag 300
   ggaggaagac ctgtaggtcg ggatggtgat gacctggttt ga 342
<210> 50
   <211> 3748
   <212> DNA
   <213> Homo sapiens
<400> 50
   cttttgattt caagagttag gagctcgaga accgtttggc aatatgtacg acgcggatga      60
   ggatatgcaa tatgatgagg atgatgatga aatcaccccg gatttgtggc aagaagcatg     120
   ctggattgta atcagttcct attttgacga gaaaggcttg gttagacaac agctggattc     180
   ttttgatgag tttattcaga tgtctgttca aagaattgtg gaagacgctc ctcctataga     240
   cctacaggct gaagctcagc atgctagtgg agaagttgaa gaaccgccac gatatttgct     300
   gaagtttgaa caaatttatc tttccaagcc tacccattgg gaaagagatg gtgctccttc     360
   accaatgatg cccaatgaag ctagattaag gaatctcacg tattctgctc cgctttatgt     420
   tgatataaca aaaacagtca ttaaagaagg tgaagaacaa cttcagactc agcatcagaa     480
   aacttttata ggaaaaattc caattatgtt gcggtcaact tactgccttt tgaatggctt     540
   gacagatcgt gatctttgtg agttaaatga atgccctttg gatcctggtg gctatttcat     600
   tattaatgga tcagaaaagg ttctgattgc ccaagagaaa atggcaacaa acacagttta     660
   tgtgtttgcc aaaaaggatt ctaaatatgc ctacacagga gagtgtagat catgtcttga     720
   gaattcttcc cgacccacca gtactatatg ggttagcatg ctggcaagag gaggacaggg     780
   tgccaagaag agtgctattg gtcagcgcat tgtggcaact ctaccatata tcaagcaaga     840
   agttcccatc attattgtgt tcagagcatt aggttttgtg tccgacagag atattttaga 900
   acatattatt tatgattttg aagatccaga gatgatggaa atggttaaac cttctctcga 960
   tgaagctttt gtcatccaag aacagaatgt tgcactaaat ttcattggtt cacgaggagc 1020
   aaagcctggt gttactaaag agaaaagaat taaatatgca aaggaagttt tacaaaaaga 1080
   aatgctccct catgttggtg tcagtgattt ttgtgagacc aaaaaagcct atttcttggg 1140
   atacatggtt cataggttac ttctggcagc tttgggtaga agagaactag atgacagaga 1200
   tcactatgga aacaagagat tggatcttgc tgggccgctg cttgcattct tatttagagg 1260
   tatgtttaag aatttgctta aagaagtgcg gatctatgca cagaaattta ttgatcgagg 1320
   aaaggatttt aacttggagt tggcaattaa aacacggatc atatctgatg gcctaaaata 1380
   ctctttagct actggaaact ggggtgatca aaagaaagct catcaagcca gagctggagt 1440
   atctcaggtg ttaaaccgcc tgacttttgc gtctactctt tctcacctgc gtcgtttaaa 1500
   ttctcctatt ggtagagacg gcaagctagc aaaaccaaga cagttgcata atacgttgtg 1560
   gggaatggtg tgtcctgccg agaccccaga gggccatgct gtaggacttg tgaagaattt 1620
   agccttgatg gcgtatattt cagttggatc tcaaccatct ccaattctgg aatttttaga 1680
   agaatggagt atggaaaatt tagaagaaat ttctcctgca gctattgctg atgcaaccaa 1740
   gatttttgtt aatggctgct gggttggaat acataaagat cccgaacaac ttatgaacac 1800
   cctaaggaaa ttgagacgtc agatggacat cattgtgtct gaagtttcta tgatcagaga 1860
   tattcgagag agggagattc ggatctatac ggatgcaggc cgtatttgta gaccacttct 1920
   gattgtggaa aaacaaaagc tacttttgaa gaagaggcat attgaccaat tgaaagagag 1980
   agaatataac aactatagtt ggcaggatct tgtggccagt ggggtagtgg agtatattga 2040
   taccctggaa gaagaaacag tgatgcttgc aatgactcca gatgatttac aggagaaaga 2100
   agtagcttat tgttccacat atacacactg tgagattcat ccctcaatga tccttggtgt 2160
   ctgtgcatct attattccct ttcctgatca taaccagtcc cctagaaaca cataccagtc 2220
   tgctatgggt aagcaggcta tgggagttta catcaccaac ttccatgttc gcatggacac 2280
   attggcccat gttctctatt atcctcaaaa gccacttgtg actacacggt ctatggaata 2340
   tctacgattt agagagctgc cagcaggcat caactcaatt gtggccattg catcatacac 2400
   tggatataat caggaagact ctgttatcat gaatcgttca gctgtagacc gcggcttctt 2460
   caggtctgtt ttctatcgct catacaaaga acaggagtct aaaaaaggat ttgatcaaga 2520
   agaagttttt gagaagccta cacgtgaaac atgccagggc atgaggcatg ccatttacga 2580
   caagctggat gatgatggtt tgatagctcc aggggttcgt gtatcaggag atgatgttat 2640
   tataggcaaa acagtcacct tgcctgaaaa tgaagatgaa ttggagagca ccaatagacg 2700
   ctataccaag agagactgta gcacttttct cagaactagt gagacgggca ttgtggatca 2760
   ggttatggta actctcaatc aggaaggata taaattttgt aaaataaggg tacgctctgt 2820
   taggattcca cagattggag acaaatttgc tagtcgacat ggtcaaaagg gtacttgtgg 2880
   tattcagtat agacaagagg atatgccttt cacctgtgaa ggtatcaccc ctgatatcat 2940
   catcaatccc catgccatcc cctctcgtat gactattggt cacttaattg aatgccttca 3000
   agggaaggta tcggctaaca agggtgaaat tggtgatgcc actccattta atgatgctgt 3060
   taacgtgcag aagatttcta atcttttatc tgattatggc tatcatctca gaggaaatga 3120
   ggtcctgtac aatgggttca ctggtcgaaa aatcacatca caaatattta ttggccccac 3180
   ttattaccag cgtttgaagc atatggtgga tgataagatt cactctcgtg ctaggggacc 3240
   tattcagatc ctcaatagac agcccatgga gggtagatct cgtgatggtg gcctgcgttt 3300
   tggagaaatg gaacgagatt gtcagattgc ccatggagca gcccagtttt taagggaaag 3360
   attgtttgag gcatcagatc catatcaggt tcatgtttgc aatctttgtg gaataatggc 3420
   gattgccaac accaggaccc atacatatga atgcaggggc tgccgcaata aaacccagat 3480
   ttctttggtg cgaatgcctt acgcatgcaa actattgttt caggaactta tgtctatgag 3540
   tattgcaccg cgaatgatga gtgtttagct attttacagg agtcaacaag ataattaaat 3600
   atcttggtgt cttgtttcta ttgtgtggct ttataaaaat gacaaatatg tactgtgttg 3660
   tgataaaaag tattttattt gtttaatgat atgcatgctt ttcttctgta aatatataat 3720
   aaatttttgt agatagtctt gatgtgtg 3748
<210> 51
   <211> 1422
   <212> DNA
   <213> Homo sapiens
<400> 51
   ggtggcccct gggcgagatg ccgtacgcca accagcctac cgtgcggatc acggagctca 60
   ctgacgagaa tgtcaagttc atcatcgaga acaccgacct ggcggtggcc aattcgattc 120
   ggagggtctt catcgctgag gttcccataa tagccattga ctgggttcag attgatgcca 180
   attcctcagt ccttcatgat gaattcattg ctcacaggct tggattaatt cccctcatta 240
   gtgatgacat tgtggacaag ctgcagtact ctcgggactg cacatgtgag gagttctgcc 300
   ccgagtgctc ggtggagttc accctcgatg tgcggtgcaa tgaagaccag acgcgacatg 360
   tcacgtctcg agacctcatc tccaacagcc cccgggtcat tccggtgaca tcccggaacc 420
   gagataatga ccccaatgac tacgtggagc aggatgacat cctcatcgtc aagttgagaa 480
   agggccagga gctgagactt cgagcctatg ccaaaaaggg ctttggcaag gagcatgcca 540
   agtggaaccc tactgcaggg gtggcttttg aatacgatcc agacaatgcc ctgaggcaca 600
   cagtgtaccc caagcccgag gaatggccaa agagtgagta ctcggagctg gatgaggatg 660
   agtcgcaggc tccctatgac cccaacggca agccagaaag gttttactac aatgtggagt 720
   cctgtggctc tctgcgtcct gaaaccattg tcctgtcagc cctctcagga ttgaagaaga 780
   aactgagtga tttacaaact caattaagcc acgagatcca gagtgatgtg ctaaccataa 840
   attaactgca gcttgcctgc ttcagcaaaa acggagattc aggccagcag ctggatatgg 900
   gggtctctct tcagactctt ctcgtttctg agaatctagt ctactgttgg ttgagcttct 960
   tggcaggaca tcagtaccaa ctagaagtgg gtcatagata gattaccagg gatgcagtgg 1020
   tgtttaggca ggataggtct ttactggccc tgactgctgt taataattgg cagcagtgct 1080
   ccccagatcc cagaaggtcc ctgctggagt gtttccagtg cacctgtagg gaaccaacta 1140
   gacttctctc ctggttagtc cagctcttta ctctaaaccc tttctgtcca aaatgagtca 1200
   ttttcagttg taccttagat gtctggtgtt gaggatcaag tgccatagcc tttattcagg 1260
   gggcctataa acccttccag ttcttgcccc agggctggcc tgctagcgct tcaaattccc 1320
   aggtgtccct aatttgagaa gtaacctttt ggaatagcat tagaccctgg ctgtcccctc 1380
   cccaccaaat aaacatgata tttcaaaaaa aaaaaaaaaa aa 1422
<210> 52
   <211> 778
   <212> DNA
   <213> Homo sapiens
<400> 52
   tatgttgtgc tctttattgc caaaaataaa cacttttaaa aagacaactt ctgttaatca       60
   ataaatatcc tttcctttct gtactggatc tctggttttc tccaggctgc ctaattaact      120
   gctttgtgca tgttcctatc tggagacagt gctgaggggt tagggccaga gccttcatta      180
   aggagaaatg aaatactcct cctgtgtatg tgggaggggc ccttgctcct gcggctgctt      240
   cacccccacc cccaccccca cccccaccaa actaaatgat cacggcattg gaggtgaagg      300
   ggctcatgac tgctggacag acaatgaaat atcatgttta tttggtggcg agggcacagc      360
   cactgtctaa tgctattcca aaaggttact gatcacacta cggacacctg cgaatgctaa      420
   gcgctagcat gccacccctg ggtcaagaac tgcaagggct tataggcccc tgaataaagg      480
   ctatggcact tgatcctgaa caccagacat ctaaggtaca actgacaatg actcattccg      540
   gacagaaatg gcttagacta aagagctgga ctaaccagcg aaagaagtct agttgatccc      600
   ttcaggtgca ctggaaacac ttcagccggg acctttctgg atctggggac cactgctgcc      660
   caacataaac tgcagaccag gccatttaag acctatgctt tgctaacaca actggtgccc      720
   tgatatccat ttatgaccca ctttttattg ccactgtgac cttgccagaa agctaacc        778
<210> 53
   <211> 1616
   <212> DNA
   <213> Homo sapiens
<400> 53
   accgcggagc agacgcggag gctggtggcc ctgggcgaga tgccgtacgc caaccagcct      60
   accgtgcgga tcacggagct cactgacgag aatgtcaagt tcatcatcga gaacaccgac     120
   ctggcggtgg ccaattcgat tcggagggtc ttcatcgctg aggttcccat aatagccatt     180
   gactgggttc agattgatgc caattcctca gtccttcatg atgaattcat tgctcacagg     240
   cttggattaa ttcccctcat tagtgatgac attgtggaca agctgcagta ctctcgggac     300
   tgcacatgtg aggagttctg ccccgagtgc tcggtggagt tcaccctcga tgtgcggtgc     360
   aatgaagacc agacgcgaca tgtcacgtct cgagacctca tctccaacag cccccgggtc     420
   attccggtga catcccggaa ccgagataat gaccccaatg actacgtgga gcaggatgac     480
   atcctcatcg tcaagttgag aaagggccag gagctgagac ttcgagccta tgccaaaaag     540
   ggctttggca aggagcatgc caagtggaac cctactgcag gggtggcttt tgaatacgat     600
   ccagacaatg ccctgaggac cacagtgtac cccaagcccg aggaatggcc aaagagtgag     660
   tactcggagc tggatgagga tgagtcgcag gctccctatg accccaacgg caagccagaa     720
   aggttttact acaatgtgga gtcctgtggc tctctgcgtc ctgaaaccat tgtcctgtca     780
   gccctctcag gattgaagaa gaaactgagt gatttacaaa ctcaattaag ccacgagatc     840
   cagagtgatg tgctaaccat aaattaactg cagcttgcct gcttcagcaa aaacggagat     900
   tcaggccagc agctggatat gggggtctct cttcagactc ttctcgtttc tgagaatcta     960
   gtctactgtt ggttgagctt cttggcagga catcagtacc aactagaagt gggtcataga    1020
   tagattacca gggatgcagt ggtgtttagg caggataggt ctttactggc cctgactgct    1080
   gttaataatt ggcagcagtg ctccccagat cccagaaggt ccctgctgga gtgtttccag    1140
   tgcacctgta ggaacaacta gacttctctc ctggttagtc cagctcttta ctctaaaccc    1200
   tttctgtcca aaatgagtca ttttcagttg taccttagat gtctggtgtt gaggatcaag    1260
   tgccatagcc tttattcagg gggcctataa acccttccag ttcttgcccc agggctggcc    1320
   tgctagcgct tcaaattccc aggtgtccct aatttgagaa gtaacctttt ggaatagcag    1380
   gagcaagggc ccctcccaca tacacaggag gagtatttca tttctcctta atgaaggctc    1440
   tggccctaac ccctcagcac tgtctccaga taggaacatg cacaaagcag ttaattaggc    1500
   agcctggaga aaaccagaga tccagtacag aaaggaaagg atatttattg attaacagaa    1560
   gttgtctttt taaaagtgtt tatttttgca ataaagagca caacataaaa aaaaaa        1616
<210> 54
   <211> 2338
   <212> DNA
   <213> Homo sapiens
<400> 54
   gctccgcgct tctgcttccg gcggcgctgg tggtggcgcg cggcgcggcg cggcgatggc 60
   ggcgggtggc agcgatccgc gggctggcga cgtagaggag gacgcctcac agctcatctt 120
   tcctaaagag tttgaaacag ctgagacact tctaaattca gaagttcata tgcttctgga 180
   acatcgaaag cagcagaatg agagtgcaga ggacgaacag gagctctcag aagtcttcat 240
   gaaaacatta aactacacag cccgtttcag tcgtttcaaa aacagagaga ccattgccag 300
   tgttcgtagc ttgctactcc agaaaaagct tcataagttt gagttggcct gtttggccaa 360
   cctttgccca gagactgctg aggagtccaa ggctctaatc ccaagcttgg agggacggtt 420
   tgaagatgag gagctgcagc agattcttga tgatatccag acaaagcgca gctttcagta 480
   ttaatctcca aacatcactg ctgctcggag aaaccacatc cccaggcata acaccacctt 540
   cccactgtct ggggctgact tgcacagaaa ttctgttgaa gacagttgag aattcctttg 600
   gagaaaacag cccagcttgg cgtggggtta ggttgctgtt tcaaataact cacaggccca 660
   ggtgacatgg aatcttggag cagccttgtg cagtggcagc cagtggcttc ctgaacgtgc 720
   ctctgcgaag tgtgagatga ggggtcacat aaccacactg ttgactacct cattcctggt 780
   ttttggcctc cacatcatct tttttcttaa tatttcatgt tttaatttca gggtgtttat 840
   actttttgaa actagaccag aagatagtag actttataga gaaagaccag ttttacctag 900
   atactaaagg aagaattaaa ccgctgttag tttgaaatgc tttttttttt tttttttaaa 960
   tggagatagg gtcttaactc ttgtccaggc tggaggagtg cagtcgtaca gtcatggctc 1020
   actgaagtct tgaccccgct gcctcagcct cccaaataac tggggccaca ggtgtgcacc 1080
   acaactctca gctaattttt aaaatttttt atagaggtgg ggttttacta tgctgtccag 1140
   actggtctta aactcctggg ctcaagtgat ccccctgcct tggcctccca aactggtgag 1200
   attacaggca tgagccacca caactggcct gaaattctta aaggatggga gtgtcgatga 1260
   cagcaccttg gcatcgttgt gcctaacctg ggagacggaa gaagcacgcc atgggaagtg 1320
   tttacacttg gggacaagtg ctaagtattg tggagcccat agccccttga gatagatggc 1380
   tactttgcct ttcttcttga actgtcttgc agaatgtgga tttggggtaa gtggtcttga 1440
   aggattcatt tagtcaccct caaattaaga tttttacttc atctttcttg ggcctgcacc 1500
   tccaagataa caaagaagaa gcaatggtcg tgccaaagag gtccacaacc aggtgtgcac 1560
   tgttcactgc agcccatttg ctgtatgaac tgtggttgtt gtgtgcccaa tgacaaggct 1620
   actaagaaat tcatcatttg aaacgtagag gccgcagcag tcagcgatgt ttctgaaatg 1680
   agcatccttg acgcctgtgt acttcccagg ctggatgtga agctacatta ccatgtgagt 1740
   tgtgccattc acagcacagt ggtgaggaat tgagctcatg aagcaggcaa ggaccgaaca 1800
   cctccacccc aacgtagacc tgcaggtgct gccccatgac ctccaccaaa gcccatataa 1860
   ggagcggagt tgttaaggac tgaagaaaaa cttctctgga gaaaaataaa attgcaattc     1920
   tacttaaaaa aatttttttt tttttttact tcataggcca ggcttgaagt tctgaacact     1980
   ttgaagtctc caattatgag agatccagtc taagcctctg gcctgctaat tagcaataag     2040
   tgctttattt ggaaggaggg agtcatccac tcttgagcca ctgcagtgaa gtcacttgat     2100
   ctcagtctgg gggaaaacac ttcaatagct aaacattcta gctttgattt ttctgaaggg     2160
   aatacacttg ttttcaattt tggggttttt ctttggggca cttgcttgac tctgtatgaa     2220
   cttgtgatcc aaggaaaaag gagaaagaac agtgttggct tttaaaatca ggatggtttt     2280
   atgtttgcta cgaaataagg caagaataaa aaattcttat ttttaaaaaa aaaaaaaa       2338
<210> 55
   <211> 566
   <212> DNA
   <213> Homo sapiens
<400> 55
   aaaatacgaa ttttttattc ttgccttatt tcgtagcaaa cataaaacca tcctgatttt      60
   aaaagccaac actgttcttt ctcctttttc cttggatcac aagttcatac agagtcaagc     120
   aagtgcccca aagaaaaacc ccaaaattga aaacaagtgt attcccttca gaaaaatcaa     180
   agctagaatg tttagctatt gaagtgtttt cccccagact gagatcaagt gacttcactg     240
   cagtggctca agagtggatg actccctcct tccaaataaa gcacttattg ctaattagca     300
   ggccagaggc ttagactgga tctctcataa ttggagactt caaagtgttc agaacttcaa     360
   gcctggccta tgaagtaaaa aaaaaaaaaa aattttttta agtagaattg caattttatt     420
   tttctccaga gaagtttttc ttcagtcctt aacaactccg ctccttatat gggctttggt     480
   ggaggtcatg gggcagcacc tgcaggtcta cgttggggtg gaggtgttcg gtccttgcct     540
   gcttcatgag ctcaatttct caccac                                           566
<210> 56
   <211> 903
   <212> DNA
   <213> Homo sapiens
<220>
   <221> misc_feature
   <222> (359)..(359)
   <223> n is a, c, g, or t
<220>
   <221> misc_feature
   <222> (518)..(518)
   <223> n is a, c, g, or t
<220>
   <221> misc_feature
   <222> (594)..(594)
   <223> n is a, c, g, or t
<220>
   <221> misc_feature
   <222> (599)..(599)
   <223> n is a, c, g, or t
<220>
   <221> misc_feature
   <222> (602)..(602)
   <223> n is a, c, g, or t
<220>
   <221> misc_feature
   <222> (625)..(625)
   <223> n is a, c, g, or t
<220>
   <221> misc_feature
   <222> (647)..(647)
   <223> n is a, c, g, or t
<220>
   <221> misc_feature
   <222> (650)..(650)
   <223> n is a, c, g, or t
<220>
   <221> misc_feature
   <222> (664)..(665)
   <223> n is a, c, g, or t
<220>
   <221> misc_feature
   <222> (724)..(724)
   <223> n is a, c, g, or t
<400> 56
   cctcaccgtg ctggtgtccc atacgatgac cccaccgacc agatgtttgt gttctttcca        60
   gaggagccca aggtgggcat caagaccatc aaggtgtact gccagcgcat gcaggaggag       120
   aacatcacac gggctctcat cgtggtgcag cagggcatga caccctccgc caagcagtcc       180
   ctggtcgaca tggcccccaa gtacatcctg gagcagtttc tgcagcagga gctgctcatc       240
   aacatcacgg agcacgagct agtccctgag cacgtcgtca tgaccaagga ggaggtgaca       300
   gagctgctgg cccgatacag taagctccga gagaaccagc tgcccaggat ccaggcggng       360
   gaccctgtgg cgcgctactt tgggataaag cgtgggcagg tggtgaagat catccggccc       420
   agtgagacgg ctggcaggta catcacctac cggctggtgc agtagctacc gcctgacagc       480
   ccctagaggc ggacacacag cgacccccat ccctgcanga caaacgcccc tgccctgcca       540
   gaatccggcc cccacagctc tcacggctgc tgctcctctg gactccccag gcangtggnc       600
   tncaccccac gtctcccgtc ctggngtgag ggcttctgtg ggccagnccn gccccaatca       660
   cctnngtgat tgtgcgagat gcagcctcag aaggaaacaa gcccccagag ggaggtcacc       720
   tggnggcagc tggtgccggg tctcacccaa accacgcttg gtcccctctg tgggggtttg       780
   ggggcggggc tccaccaagc actgttttct cctggccctt ggctttcacc ctttttttcc      840
   ttctcggggg cctgatggtg ggggccccgc ccgctcgggt tttattcctt caaaccggcc       900
   cgc                                                                     903
<210> 57
   <211> 1248
   <212> **DNA**
   <213> Homo sapiens
<400> 57
   ggcggcggcg gcggaggctg ccatggacga cgaggaggag acgtaccggc tctggaaaat      60
   ccgcaagacc atcatgcagc tgtgccacga ccgtggctat ctggtgaccc aggacgagct     120
   tgaccagacc ctggaggagt tcaaagccca atttggggac aagccgagtg aggggcggcc     180
   gcggcgcacg gacctcaccg tgctggtggc ccacaacgat gaccccaccg accagatgtt     240
   tgtgttcttt ccagaggagc ccaaggtggg catcaagacc atcaaggtgt actgccagcg     300
   catgcaggag gagaacatca cacgggctct catcgtggtg cagcagggca tgacaccctc     360
   cgccaagcag tccctggtcg acatggcccc caagtacatc ctggagcagt ttctgcagca     420
   ggagctgctc atcaacatca cggagcacga gctagtccct gagcacgtcg tcatgaccaa     480
   ggaggaggtg acagagctgc tggcccgata taagctccga gagaaccagc tgcccaggat     540
   ccaggcgggg gaccctgtgg cgcgctactt tgggataaag cgtgggcagg tggtgaagat     600
   catccggccc agtgagacgg ctggcaggta catcacctac cggctggtgc agtagctacc     660
   gcctgacagc ccctagaggc ggacacacag cgacccccat ccctgcagga caaacgcccc     720
   tgccctgcca gaatccggcc cccacagctc tcacggctgc tgctcctctg gactccccaa     780
   ggcaggtggc ctccacccac gttctcccgt cctggggtga ggcttcctgt ggcccagccc     840
   gccccattca cctgtggatt tgtgcgagat gcagcctcag aaggaacaag gcccccagag     900
   ggaggtcacc tgggggcagc tggtgccggg tcttcaccca gaccacgctg ggtcccctct     960
   gttgggggtt tggggtccgg gtctcccacc agccactgct tcctcctggg ccctcggcct    1020
   tccacccctc gtcttccctc cctcgggggc cctgatgcgt ggcggccccc acccggcctc    1080
   ggctctttac tccattcaca gccgtgcacg cgctcaagcc accagggtgc gagatgccag    1140
   ctctggagtt ctcggttgtt gtaggaggtt gggtgttttc aaatggtaaa gatgttttga    1200
   gcaaataaat ttgcttgata cagaaaaaaa aaaaaaaaaa aaaaaaaa                 1248
<210> 58
   <211> 588
   <212> DNA
   <213> Homo sapiens
<400> 58
   cgcaggcgca agataagcta ggagccgcgc gagtcgtagt gtcgctgttt gcgggtctcc        60
   gcgcgggacc ggggcgcagc ggggtcgctg aggcgagggt gtcatgtcag acaacgagga       120
   caattttgat ggcgacgact ttgatgatgt ggaggaggat gaagggctag atgacttgga       180
   gaatgccgaa gaggaaggcc aggagaatgt cgagatcctc ccctctgggg agcgaccgca       240
   ggccaaccag aagcgaatca ccacaccata catgaccaag tacgagcgag cccgcgtgct       300
   gggcacccga gcgctccaga ttgcgatgtg tgcccctgtg atggtggagc tggaggggga       360
   gacagatcct ctgctcattg ccatgaagga actcaaggcc cgaaagatcc ccatcatcat       420
   tcgccgttac ctgccagatg ggagctatga agactggggg gtggacgagc tcatcatcac       480
   cgactgagct ggagtcatct tcctgccctt gccccatgcc caattttcat tctcacttta       540
   tatgtgtaaa taataaaata ttcaactttc aaaaaaaaaa aaaaaaaa                    588
<210> 59
   <211> 852
   <212> DNA
   <213> Homo sapiens
<400> 59
<210> 60
   <211> 867
   <212> DNA
   <213> Homo sapiens
<400> 60
   ccctcactaa agggacaaaa gctggagctc caccgcggtg cggccgctct agaactagtg      60
   gatcccccgg gctgcaggtc cggtcttgtc cacgctaggg ggtgcacgta ctcccaactg     120
   tggtcgcgct ctcacccctt ctgctgctct cgtggccccc tcgcgatggc gggcatcctg     180
   tttgaggata ttttcgatgt gaaggatatt gacccggagg ccaagaagtt tgaccgagtg     240
   tctcgactgc attgtgagag tgaatctttc aagatggatc taatcttaga tgtaaacatt     300
   caaatttacc ctgtagactt gggtgacaag tttcggttgg tcatagctag taccttgtat     360
   gaagatggta ccctggatga tggtgaatac aaccccactg atgataggcc ttccagggct     420
   gaccagtttg agtatgtaat gtatggaaaa gtgtacagga ttgagggaga tgaaacttct     480
   actgaagcag caacacgcct ctctgcgtac gtgtcctatg ggggcctgct catgaggctg      540
   cagggggatg ccaacaacct gcatggattc gaggtggact ccagagttta tctcctgatg    600
   aagaagctag ccttctgaac ctcgcctgaa gccagcctct ctgccaagtc actcaggtca     660
   tgggcattgt tcaagcctga gtggcagccg ctcttgctca cctgttgagg aagggctggc     720
   tcactgtcca ccgtggcggc atctttaact ggcctccact caatgggaaa ctgactcgcc     780
   tgtgaaagac acagtgggag agctgaaaat gaatcagaag ctttatgtat atgattttta     840
   aattaaactt tactttttca gactgcc                                         867
<210> 61
   <211> 960
   <212> DNA
   <213> Homo sapiens
<220>
   <221> misc_feature
   <222> (631)..(631)
   <223> n is a, c, g, or t
<220>
   <221> misc_feature
   <222> (646)..(646)
   <223> n is a, c, g, or t
<220>
   <221> misc_feature
   <222> (663)..(663)
   <223> n is a, c, g, or t
<220>
   <221> misc_feature
   <222> (670)..(670)
   <223> n is a, c, g, or t
<220>
   <221> misc_feature
   <222> (740)..(740)
   <223> n is a, c, g, or t
<220>
   <221> misc_feature
   <222> (742)..(742)
   <223> n is a, c, g, or t
<220>
   <221> misc_feature
   <222> (803)..(803)
   <223> n is a, c, g, or t
<220>
   <221> misc_feature
   <222> (806)..(806)
   <223> n is a, c, g, or t
<220>
   <221> misc_feature
   <222> (821)..(821)
   <223> n is a, c, g, or t
<220>
   <221> misc_feature
   <222> (838)..(838)
   <223> n is a, c, g, or t
<220>
   <221> misc_feature
   <222> (846)..(846)
   <223> n is a, c, g, or t
<220>
   <221> misc_feature
   <222> (870)..(870)
   <223> n is a, c, g, or t
<220>
   <221> misc_feature
   <222> (874)..(874)
   <223> n is a, c, g, or t
<220>
   <221> misc_feature
   <222> (892)..(892)
   <223> n is a, c, g, or t
<220>
   <221> misc_feature
   <222> (901)..(901)
   <223> n is a, c, g, or t
<220>
   <221> misc_feature
   <222> (910)..(910)
   <223> n is a, c, g, or t
<220>
   <221> misc_feature
   <222> (940)..(940)
   <223> n is a, c, g, or t
<220>
   <221> misc_feature
   <222> (943)..(943)
   <223> n is a, c, g, or t
<220>
   <221> misc_feature
   <222> (957)..(957)
   <223> n is a, c, g, or t
<400> 61
   gggggcactg gccttcaacc aagccaagcg ccttggcttc tgtcctggat gctcagtgtt      60
   ttgcggttta cgatgcgcat cgttttcccg gctctggcac cagatccata ctattctgta     120
   aactgaggcg gcgatgccca aaaattggaa gcaattactc taagatatca aaagattgaa     180
   gcaagccatg aaacctctgc gcggagagag ccgagcccga ggcggcgggg agccagcacg     240
   tgtgcgcagc tccgccctct tttggcgaag cacctaggcc tggcccctcc cgcgacctgt     300
   agcgcggcgg agcaagcgca gaaggctggg agggctgcgc gggctgcgcg tcgccatgga     360
   gcccgacggg acttacgagc cgggcttcgt gggtattcgc ttctgccagg aatgtaacaa     420
   catgctgtac cccaaggaag acaaggagaa ccgcattctg ctctacgcgt gccggaactg     480
   tgatttccag caggaggccg acaacagctg catctatgtc aacaagatca cgcacgaagt     540
   ggacgaactg acccagatta tcgccgacgt gtcccaggac cccacgttgc cgcggaccga     600
   ggaccacccg tgccaaaagt gcggccacaa ngaggctgtg ttcttncagt cacacagtgc     660
   gcnggccgan gacccatgcg cctttactac gtgtgcacaa gccccacact tgcgggcacc     720
   gctggaccga atgacctctn tnttccccga tgtaataaac accaaattca tgcgtgaaaa     780
   aaaaaaaaaa aactttcatc cgncanctca tgatatattc naccggaatt ccgaccgnac     840
   ctgagngtac actttcttta tggcgataan ctgngatatg gctactgtct gngaatgttc     900
   ntcatcccan atcaccgact aaggaaactg ggcatagacn atnatatggt ggtatcnttc     960
<210> 62
   <211> 652
   <212> DNA
   <213> Homo sapiens
<400> 62
   ggtggcggcg gcggcggacc cttggggtct ggacgcaacg gcggcgggag catgaacgcc 60
   cctccagcct tcgagtcgtt cttgctcttc gagggcgaga agaagatcac cattaacaag 120
   gacaccaagg tacccaatgc ctgtttattc accatcaaca aagaagacca cacactggga 180
   aacatcatta aatcacaact cctaaaagac ccgcaagtgc tatttgctgg ctacaaagtc 240
   ccccacccct tggagcacaa gatcatcatc cgagtgcaga ccacgccgga ctacagcccc 300
   caggaagcct ttaccaacgc catcaccgac ctcatcagtg agctgtccct gctggaggag 360
   cgctttcggg tggccataaa agacaagcag gaaggaattg agtaggggcc agagggggct 420
   ctgctcggcc tgtgagcccc gttcctacct gtgcctgacc ctccgctcca ggtaccacac 480
   cgaggagagc ggcggtccca gccatggccc gccttgtggc acccctcacc ctgacaccga 540
   cgtgttggca cccctcaccc tgacaccgac gtgtcctgta catagattag gttttatatt 600
   cctaataaag tatagcggga gagacctgga aaaaacaaca acaaaaaaac ct 652
<210> 63
   <211> 433
   <212> DNA
   <213> Homo sapiens
<400> 63
   cacgaggcga gtcgttcttg ctcttcgagg gcgagaagat caccattaac aaggacacca 60
   aggtacccaa tgcctgttta ttcaccatca acaaagaaga ccacacactg ggaaacatca 120
   ttaaatcacg tgcctgcttc cccttcgcct tctgccgtga ttgtcagttt cctgaggcct 180
   ccccagccac gcttcctgta cagcctgcag aactctgccc cagagcacat cagctatgtg 240
   ccccagctct caaacgacac cttgtccggg gaggctcacc ctgtccacct tcacgctgga 300
   gcagcctcta ggccagttca gcagccacaa catctctgac ttggatacca tctggctggt 360
   ggtggccctc agcaacgcca cccagagctt cacggcccca cggacaaacc aggacatccc 420
   tgctcctgcc aac 433
<210> 64
   <211> 494
   <212> DNA
   <213> Homo sapiens
<400> 64
   tgggggggca gggtctcact ctgtcaccca ggctggagtg cagtggtgcg atcatagctc 60
   actgcggcct cgacctccca ggctcaaggc tctcagattc tcagacccca ccctggggaa 120
   ggatggagga tgaggacaag cttcccaggt ccacatgaca gagggactca ggattggcaa 180
   tttggcctgt gtccagcttt gctgctcaga gagctcagat ccccacccct caattcactc 240
   ccttgtgtgg aagggagtga ccctcacttt ctagcacaca cagggaagag ggcttggcta 300
   agagcaccca ggaggtcagg gcaggactgg ggattagatc acacccaaat gcaagctctg 360
   tggcactcct ggtatagccc tgctgtcccc ctgtagcctg gccctgggaa gggggctccc 420
   agacttcaca ccccctcacc tctttatcca ggggaccggc tgtctcttcc tccttcatcc 480
   ccagcctctg tcag 494
<210> 65
   <211> 959
   <212> DNA
   <213> Homo sapiens
<400> 65
   gtaccggtcc ggaattcccg ggatctcgac acctggacta gccgggttgt atttggaaac 60
   gcggagtgag tttttccgtg ctgtgtaggg gctaacaatg gacacccaga aggacgttca 120
   acctccaaag cagcaaccaa tgatatatat ctgtggagag tgtcacacag aaaatgaaat 180
   aaaatctagg gatccaatca gatgcagaga atgtggatac agaataatgt acaagaaaag 240
   gactaaaaga ttggtcgttt ttgatgctcg atgaatgctg ggaattcaga ggaatgtctt 300
   cacttatact tggatttgct ctcttcccat ttctgattgt tgtatagctt tcgattttgc 360
   ttacagtagt tcccccttat cttcgggaga tacattccaa ggcccccagt gaactcctga 420
   aacctcaaac agtaccaaac ctttatacac tgttttttcc atatatatat acctatgata 480
   aagtataatg tataaattaa gcatagcaag agattaataa taatgtaata gaacaatgat 540
   aacatactat aataaaagtt atgtgaatgt ggttggtctc tcttgctttc aaaatatctt 600
   cttgtacagt actcacctat tttagaatgt ggttgactac aggtaaccaa aaccacagaa 660
   agggaaactt tggatgmggg gggcactact gtacttagga atacaactat atacatatga 720
   ttttattttt aagaccatat tatatttggg tatctactaa tattttgtat aaagcaattt 780
   tttgttccat tacgtgactt tttgttttat tgtatatgta atttaacaca caataaaggg 840
   taaagttgct tcccmaacca cagttttaat caaaacctag aatcatctgc agtccttgtt 900
   aaaaatgcag gtttctagaa ccctctgaag ttctgattaa ataaatttwt tgcaaacca 959
<210> 66
   <211> 971
   <212> DNA
   <213> Homo sapiens
<400> 66
   gcactgtgcg tcatgtacca gcgccggaag ttggtctcga cacctggact agccgggttg      60
   tatttggaaa cgcggagtga gtttttccgt gctgtgtagg ggctaacaat ggacacccag     120
   aaggacgttc aacctccaaa gcagcaacca atgatatata tctgtggaga gtgtcacaca     180
   gaaaatgaaa taaaatctag ggatccaatc agatgcagag aatgtggata cagaataatg     240
   tacaagaaaa ggactaaaag attggtcgtt tttgatgctc gatgaatgct gggaattcag     300
   aggaatgtct tcacttatac ttggatttgc tctcttccca tttctgattg ttgtatagct     360
   ttcgattttg cttacagtag ttccccctta tcttcgggag atacattcca aggcccccag     420
   tgaactcctg aaacctcaaa cagtaccaaa cctttataca ctgttttttc catatatata     480
   tacctatgat aaagtataat gtataaatta agcatagcaa gagattaata ataatgtaat     540
   agaacaatga taacatacta taataaaagt tatgtgaatg tggttggtct ctcttgcttt     600
   caaaatatct tcttgtacag tactcaccta ttttagaatg tggttgacta caggtaacca     660
   aaaccacaga aagggaaact ttggatgagg ggggcactac tgtacttagg aatacaacta     720
   tatacatatg attttatttt taagaccata ttatatttgg gtatctacta atattttgta     780
   taaagcaatt ttttgttcca ttacgtgact ttttgtttta ttgtatatgt aatttaacac     840
   acaataaagg gtaaagttgc ttccccaaac cacactttta atcaaaacct agaatcatct     900
   gcagtccttg ttaaaaatgc aggtttctag aaccctctga agttctgatt aaataaattt     960
   attgcaaacc a                                                          971
<210> 67
   <211> 469
   <212> DNA
   <213> Homo sapiens
<400> 67
   tttttttttt gaacactgtg cccagctaat ttattaaatt ttgttgtaga gatggggtct      60
   cattatgatg cccaggcggg tcttgaattt ctggactcaa gtgatcctcc tgccttggcc     120
   tcccaaagtc ctgggattat aggggtgagc cctgttgccc ggcctaaatc tggctcttca     180
   gattccgaga gagagggtac aggagcctct ctgctcagcc tgaagaacac ccagaaggag     240
   ggaacctcaa agagccccga gatggaaggc tctcctggca gcgctcctgc aggggtgcct     300
   gtggaacagt gtgccccagg gctaggagag ttcatggccc agtctgcttc actgggctgc     360
   cacagtgtga aagctgggcc tagacctggc tcctgacatc ctagtgaggg ctgggactta     420
   gtggttctga aagaccttta ctggatggtt cttccagagt ggggtatgg                 469
<210> 68
   <211> 423
   <212> DNA
   <213> Homo sapiens
<400> 68
   gagtctggga cgcgccgccg ccatgatcat ccctgtacgc tgcttcactt gtggcaagat      60
   cgtcggcaac aagtgggagg cttacctggg gctgctgcag gccgagtaca ccgaggggga     120
   tgcgctggat gccctgggcc tgaagcgcta ctgctgccgc cggatgctgc tggcccacgt     180
   ggacctgatc gagaagctgc tcaattatgc acccctggag aagtgaccac gctggaaccc    240
   acccacccgc tgtgctgacc atgggccctg agcgtcctgc cccgaattca cgaggctgag     300
   gcatccggga gctggcgtaa tgcctggccg cagtgtgtgt gtatcccata ccccactctg     360
   gaaggaacca tccagtaaag gtctttcaga aacaaaaaaa aaaaaaaaaa aaaaaaaaaa     420
   aaa                                                                   423
<210> 69
   <211> 4237
   <212> DNA
   <213> Homo sapiens
<400> 69
   agattcgggc agggagcggg cggaaccttt ctaccgcgtc tctagctaac acgcacggcg      60
   gggacagttt aggcctccgc gcaccgttcg ccgggagtct tgcagtttgc ttggtgcagg 120
   gaaggcgggc gcggaggttc tatctgtttc ttcctccttc gtgagcagca tggacgtgct 180
   agcggaggag tttgggaacc tgactccgga gcagctggcg gcgccgatcc cgactgtaga 240
   ggaaaaatgg aggctgcttc cagcattttt aaaggtgaaa ggccttgtga aacagcatat 300
   agattcattt aactatttca ttaatgtaga gataaagaag ataatgaaag ccaatgaaaa 360
   ggttacaagt gacgctgacc ctatgtggta cttaaaatat cttaatatct atgttgggct 420
   tcctgatgtt gaagaaagct tcaatgtaac tagaccagtg tcccctcatg agtgccgttt 480
   gagagacatg acatactctg cccctattac agtggatatt gaatataccc gaggcagcca 540
   gaggatcatc cgcaatgcct tacctatcgg cagaatgccc ataatgctac gtagttcaaa 600
   ctgtgttctt acaggaaaaa cgccagcaga atttgccaaa ctgaacgaat gtcccttaga 660
   tccaggtggc tacttcattg ttaaaggagt agaaaaagtt attcttatcc aagagcagct 720
   gtctaagaac aggatcatcg tggaggctga tagaaaaggg gctgttggag cttcagttac 780
   cagctctacc catgagaaaa aaagcagaac caatatggct gtgaaacaag gacgatttta 840
   tttgaggcat aatactttgt cagaagatat acccattgtc atcatattta aggccatggg 900
   tgttgagagt gaccaggaaa ttgtgcagat gattggaaca gaggagcacg tgatggctgc 960
   atttgggccc agtctggaag agtgccagaa agctcagatt ttcacacaga tgcaggcatt 1020
   aaaatatata gggaacaaag taagaaggca aaggatgtgg ggaggtggac caaagaaaac 1080
   caaaatagaa gaagcaagag agctcctggc ttccaccatt ctgacccatg tcccagttaa 1140
   ggaattcaat ttccgagcca aatgtatcta tactgcagtg atggtgcgaa gagttattct 1200
   ggcccaagga gataataaag ttgacgacag agattattat ggtaacaagc gactggaatt 1260
   ggcaggacag cttttatctc ttctttttga agacttgttc aaaaaattta attctgaaat 1320
   gaaaaagatt gccgaccagg tgattcctaa gcaaagagca gcccagtttg atgttgtcaa 1380
   acacatgcgc caagaccaga tcaccaatgg catggtgaat gctatttcta ccggaaattg 1440
   gtctttaaag agatttaaaa tggaccgcca gggtgtaacc caagtgctgt ctcgcttgtc 1500
   atatatatcc gcactgggca tgatgacaag aatctcttcc cagtttgaaa aaacgagaaa 1560
   agtgagtggt cctcgctccc tccagccatc tcagtgggga atgctgtgtc cttcggacac 1620
   tcctgaagga gaggcatgtg gtttggttaa aaacttggcc cttatgacac acatcacaac 1680
   tgatatggaa gatggaccca ttgttaaatt agccagtaac ttgggagtag aagatgtgaa 1740
   tttattatgt ggggaagagc tctcttaccc aaatgtgttt cttgtctttc ttaatggtaa 1800
   catcttaggt gtcattcgag accacaaaaa gctagtgaat acatttcgac tcatgagaag 1860
   agcaggatat atcaatgaat ttgtttccat ctcaacaaat cttacagatc gatgtgtcta 1920
   tatttcttct gatgggggaa ggctatgcag accctacata attgtcaaga aacagaagcc 1980
   agcagtcaca aataaacata tggaagagct ggcccaaggg tacaggaatt ttgaagattt 2040
   cttacatgag agtctggttg aatatttaga tgtgaatgaa gaaaatgatt gtaacattgc 2100
   actgtacgaa cacacaatta ataaagacac cacccacttg gagattgaac ccttcactct 2160
   tctcggcgtg tgtgctggac ttatcccata ccctcaccat aaccagtcac cgagaaacac 2220
   ttatcagtgt gccatgggga aacaagccat gggtactata ggatacaacc agcgaaacag 2280
   aattgatact ctcatgtatc tactagcata tccacaaaaa cccatggtta agacaaaaac 2340
   cattgaattg atagaatttg agaaactgcc agctggacag aatgcaacag ttgctgtgat 2400
   gagctatagt ggctatgata ttgaagatgc tcttgtttta aacaaggcct ctttagacag 2460
   aggctttggg cgttgccttg tatataaaaa tgctaaatgt acgttgaaac gatacaccaa 2520
   tcagactttt gataaagtga tggggcccat gttggatgct gctacaagga aacctatctg 2580
   gcgacatgaa atcttagatg cagatggtat ttgttctcca ggtgagaaag tagaaaacaa 2640
   acaagtgctt gtaaataagt ccatgcccac agtgactcag attcctttgg aaggaagtaa 2700
   tgtaccacag caaccacagt acaaagatgt acccataacc tacaaaggag caacagactc 2760
   atatattgaa aaagtgatga tatcttcaaa tgctgaagat gcttttctga tcaaaatgct 2820
   gctgagacag acaaggcgtc cagaaattgg agacaaattc agcagtcgtc atgggcaaaa 2880
   aggtgtttgt ggcttgatcg tcccccagga agacatgcca ttttgtgatt ctggcatctg 2940
   tccggacatc atcatgaacc cacacggctt cccatcacga atgacggtgg ggaagctcat 3000
   tgagctgctg gctggcaagg ccggtgtgct ggacggcaga ttccactacg gcactgcgtt 3060
   tggaggcagt aaagtgaagg atgtgtgtga ggacctcgtt cgccatggtt ataactactt 3120
   ggggaaagac tatgttacat ccggcatcac aggtgagccc ttcgaagcat acatctattt 3180
   tggccccgtg tactatcaga agctgaaaca catggtgcta gataaaatgc atgcccgggc 3240
   ccggggccca cgagccgtcc ttaccaggca acccactgaa ggacggtctc gtgatggtgg 3300
   cttgcgtctc ggggaaatgg aacgtgactg tttaatcggt tatggagcca gtatgctttt 3360
   gctagagaga ctaatgattt caagtgatgc ctttgaggtt gatgtctgtg ggcagtgtgg 3420
   acttctgggg tattctggct ggtgccatta ctgcaagtca tcctgccacg tgtcttccct 3480
   ccgtattccg tatgcctgca agctgctctt ccaggaacta cagtctatga acatcatccc 3540
   caggttaaaa ctgtccaagt acaatgaatg aggatggaaa aaatgattat taaagagaac 3600
   aagtgataca tccaatgcaa cggaaagcag aagggattta ggactacgtc tcctcctgtg 3660
   aagaattccc ttgcgtattc tctctctaaa acaaccaaaa aaaaatggag aggcttttta 3720
   tatactctaa gactggctaa acaaccttga tcattgagcc tcgagccatg ggagagatgc 3780
   tgaccatgtg gactgcaagg ctgcttgatt cacagatgga tgtgacctaa aggataaata 3840
   agctattact tatgtgctga tctcttgaca ttcactcatt agaagacctt actccttcaa 3900
   gcaaatgttt ggggtcaaat ttaccatatc ttctggctaa ccatattcaa gattcttctg 3960
   aaacttggag gatgtaaaga atccatttga tttggtcagc ctggcttttg tcgtggtggc 4020
   tggctcggat aaattttccc aacaattaaa tcttgccttt acacacccaa actttgtaat 4080
   tttagtcttg gtgaaatata atgaatttgt tcctaccttg tcaagcaaga atgtcgtctt 4140
   ctcctatgga ctcaattgct attattttaa acctgcatga ttgtaccatg caatactatt 4200
   cgttaaatat ctgaatctag aaaaaaaaaa aaaaaaa 4237
<210> 70
   <211> 1843
   <212> DNA
   <213> Homo sapiens
<220>
   <221> misc_feature
   <222> (578)..(578)
   <223> n is a, c, g, or t
<220>
   <221> misc_feature
   <222> (599)..(599)
   <223> n is a, c, g, or t
<220>
   <221> misc_feature
   <222> (601)..(601)
   <223> n is a, c, g, or t
<220>
   <221> misc_feature
   <222> (630)..(633)
   <223> n is a, c, g, or t
<220>
   <221> misc_feature
   <222> (685)..(685)
   <223> n is a, c, g, or t
<220>
   <221> misc_feature
   <222> (830)..(830)
   <223> n is a, c, g, or t
<220>
   <221> misc_feature
   <222> (1701)..(1702)
   <223> n is a, c, g, or t
<220>
   <221> misc_feature
   <222> (1825)..(1825)
   <223> n is a, c, g, or t
<400> 70
   tcttcggtgg cgatccggtc ctagaaaggg cggtgggctc cacctcggcc tagaagcaga      60
   cgggaccgta ggaagccgtc gcgggaagct cagccgaatt ggagttggag cccccggatt     120
   gcgctgaccc tgagctctca gactccccag tacaatgact caagcagaaa ttaagctctg     180
   ttctttgttg ctgcaagagc attttggaga gattgtagaa aaaattggag tccatctgat     240
   aagaaccggc agccagccac taagagtaat tgcccatgac acaggaacat cactggatca     300
   ggtgaagaaa gccctgtgtg tcctcgtcca acataacctg gtgagttatc aagtgcacaa     360
   acgtggtgtg gtggagtatg aagcccagtg cagccgggta ttgcgaatcg ttagatatcc     420
   ccggtacatc tatactacca aaactctgta cagtgacact ggagagctga ttgttgagga     480
   gcttctgttg aacggcaaac tgacaatgtg cactgttgtg aagaaagtgg cagaccggct     540
   cacagagacc atggaggatg gcaagaccat ggactatnct gaagtatcaa acacatttnt     600
   ncgactggca gacacacact ttgtacaacn nnncccttcg gtacctacca ctgagaattc     660
   agaccctggg ccaccaccac ctgcncccac acttgtcatt aatgaaaagg acatgtacct     720
   ggttcctaaa ctcagcttga tagggaaagg taaaaggagg agatcatctg atgaagatgc     780
   tgctggggag cccaaggcaa gagaccaaaa tatactacag ataacaaggn gcccattcca     840
   gatgatggga tttattggca ggcccaacct tgacagattc cgccaacact tccgtgacca     900
   agccattgtg agcgcagttg ctaacaggat ggaccagaca agcagcgaga ttgtgcgaac     960
   catgctccga atgagtgaga ttaccacttc ctctagtgct cccttcaccc agccattgtt    1020
   ttccaatgag atcttcagat ccctacctgt tggctataac atctctaagc aagttcttga    1080
   tcagtatctc actctgctgg cagatgatcc actagagttt gttggaaagt ctggcgacag    1140
   tggtggagga atgtatgtca tcaacctcca taaggcatta cgatccctag ccacagccac    1200
   tctggagtcc gtcgtacagg agagatttgg gtctcgctgt gctagaatat gccgtctagt    1260
   tttgcagaag aaacacatag agcagaagca agtggaagac tttgcaatga ttctgcaaga    1320
   ggcaaaggat atgctatata agatgctctc aggaaatttc atgtcactcc aggaaattcc    1380
   caaaacacca gaccatgccc catccaggac cttctattta tatactgtga acatcctgtc    1440
   agctgcccga atgttgttcg acaggtgcta caagagcata gccaacttga tagaaaggag    1500
   gcaatttgaa accaaagaga ataagcgtct actagaaaaa tctcagaggg tagaagccat    1560
   cattgcatct atgcaggcta ctggtgcaga ggaagcacag ttacaagaaa tagaggagat    1620
   gatcacagct cctgaacgtc agcagctaga gaccctgaaa cgtaatgtca acaagttgga    1680
   tgccagtgag atccaggtgg nngaaaccat cttcctgctg gagtcttaca ttgagtgcac    1740
   catgaagaga cagtgatcca gaagaagcat cttcctcaga agatctgggg ggatggaaag    1800
   caaaataaag gaggtgcctg gatgnaaaaa aaaaaaaaaa aaa                      1843
<210> 71
   <211> 1621
   <212> DNA
   <213> Homo sapiens
<400> 71
   cggaagccgc cgaaactctg gggtagagtg gcacgcgctt tttgcttttc cgcgtcttct       60
   tcggtggcga tccgcgtcct agaaagggcg gtgggctcca cctcggccta gaaggccagc      120
   gggagccgta ggaagccgtc gcgggaagct cagccgaatt ggagttggag cccccggatt      180
   gcgctgaccc tgagctctca gactccccag tacaatgact caagcagaaa ttaagctctg      240
   ttctttgttg ctgcaagagc attttggaga gattgtagaa aaaattggag tccatctgat      300
   aagaaccggc agccagccac taagagtaat tgcccatgac acaggaacat cactggatca      360
   ggtgaagaaa gccctgtgtg tcctcgtcca acataacctg gtgagttatc aagtgcacaa      420
   acgtggtgtg gtggagtatg aagcccagtg cagccgggta ttgcgaatgc ttagatatcc      480
   ccggtacatc tatactacca aaactctgta cagtgacact ggagagctga ttgttgagga      540
   gcttctgttg aacggcaaac tgacaatgtc agctgttgtg aagaaagtgg cagaccggct      600
   cacagagacc atggaggatg gcaagaccat ggactatgct gaagtatcaa acacatttgt      660
   gcgactggca gacacacact ttgtacaacg ctgcccttcg gtacctacca ctgagaattc      720
   agaccctggg ccaccaccac ctgcccccac acttgtcatt aatgaaaagg acatgtacct      780
   ggttcctaaa ctcagcttga tagggaaagg taaaaggagg agatcatctg atgaagatgc      840
   tgctggggag cccaaggcca agagaccaaa atatactaca gataacaagg agcccattcc      900
   agatgatggg atttattggc aggccaacct tgacagattc caccaacact tccgtgacca      960
   agccattgtg agcgcagttg ctaacaggat ggaccagaca agcagcgaga ttgtgcgaac     1020
   catgctccga atgagtgaga ttaccacttc ctctagtgct cccttcaccc agccattgtc     1080
   ttccaatgag atcttcagat ccctacctgt tggctataac atctctaagc aagttcttga     1140
   tcagtatctc actctgctgg cagatgatcc actagagttt gttggaaagt ctggcgacag     1200
   tggtggagga atgtatgtca tcaacctcca taaggcatta gcatccctag ccacagccac     1260
   tctgaagtcc gtcgtacagg agagatttgg gtctcgctgt gctagaatat tccgtctagt     1320
   tttgcagaag aaacacatag agcagaagca agtggaagac tttgcaatga ttcctgcaaa     1380
   ggaggcaaag gatatgctat ataagatgct ctcagaaaat ttcatgtcac tccaggttgg     1440
   atgccagtga gatccaggtg gacgaaacca tcttcctgct ggagtcttac attgagtgca     1500
   ccatgaagag acagtgatcc agaagaagca tcttcctcag aagatctggg gggatggaaa     1560
   gcaaaataaa ggaggtgcct ggatgcatta aaaaaaaaaa aaaaaaaaaa aaaaaaaaaa     1620
   a                                                                     1621
<210> 72
   <211> 1946
   <212> DNA
   <213> Homo sapiens
<400> 72
   gatggcggcg cccaggccct gccacgcaga cttccgcccg gcgcggagac cgaaggctgg 60
   cggctggtcg cgttgcaggc aacatgtcgg aaggaaacgc cgccggcgag cccagcacgc 120
   cgggagggcc ccgacctctc ctgactgggg cccgggggct catcgggcgg cggccggcgc 180
   ctcccctcac ccccggccgc cttccctcca tccgttccag ggacctcacc ctcgggggag 240
   tcaagaagaa aaccttcacc ccaaatatca tcagtcggaa gatcaaggaa gagcccaagg 300
   aagaagtaac tgtcaagaag gagaagcgtg aaagggacag agaccgacaa cgagaggggc 360
   atggacgagg gcgaggccgt ccagaagtga tccagtctca ctccatcttt gagcagggcc 420
   cagctgaaat gatgaagaaa aaagggaact gggataagac agtggatgtg tcagacatgg 480
   gaccttctca tatcatcaac atcaaaaaag agaagagaga gacagacgaa gaaactaaac 540
   agatcttgcg tatgctggag aaggacgatt tcctcgatga ccccggcctg aggaacgaca 600
   ctcgaaatat gcctgtgcag ctgccgctgg ctcactcagg atggcttttt aaggaagaaa 660
   atgacgaacc agatgttaaa ccttggctgg ctggccccaa ggaagaggac atggaggtgg 720
   acatacctgc tgtgaaagtg aaagaggagc cacgagatga ggaggaagag gccaagatga 780
   aggctcctcc caaagcagcc aggaagactc caggcctccc gaaggatgta tctgtggcag 840
   agctgctgag ggagctgagc ctcaccaagg aagaggaact gctgtttctg cagctgccag 900
   acaccctccc tggccagcca cccacccagg acatcaagcc tatcaagaca gaggtgcagg 960
   gcgaggacgg acaggtggtg ctcatcaagc aggagaaaga ccgagaagcc aaattggcag 1020
   agaatgcttg taccctggct gacctgacag agggtcaggt tggcaagcta ctcatccgca 1080
   agtctggaag ggtgcaactc ctcttgggca aggtgactct ggacgtgacc atgggaactg 1140
   cctgctcctt cctgcaggag ctggtgtccg tgggccttgg agacagtagg acaggggaga 1200
   tgacagtcct gggacacgtg aagcacaaac ttgtatgttc ccctgatttt gaatccctct 1260
   tggatcacaa acaccggtaa aatgagcagg tggaggagga cggcgcctgt gcccacggct 1320
   gctgcctgct ccagacattt tgttcttgaa tctgtgagac ccagaagggg cccactgagc 1380
   ccactcactc cagcctttgg caaccattgt tccaggtccc ccagggcttc ctcccacagc 1440
   agctgtgaat ggcacagtga ccttcctgca gcgtggagat ggcacatcct tgctgctggg 1500
   gacttggccc tgctatttat ttttgtattt atgtcttaat ctcttccact gatgcatcct 1560
   ccaagggtag atggggaggg tctgtgtgaa ggggccggct tctcttggtg cctgctgggt 1620
   tgcaggggca ggaagcgtgt ggactgcagc ttctgctggt gctccccccg tcctcctgga 1680
   ggcagtatag gagagagagc aaggattgag tctgagactt aagcactcgg tcccagcttg 1740
   ccagttcctg gttctgtgtc cttggacaaa ctacctaacc tttctgagcc tcctatacct 1800
   catccgacac aaatggggat gatacctacc tccagggttg gcgtgaggat tcatgggcta 1860
   ttatagatga aaactgcaca aggccagaac cagcaggcac tcaataaacg ttcatgtcct 1920
   ttttctctaa aaaaaaaaaa aaaaaa 1946
<210> 73
   <211> 2640
   <212> DNA
   <213> Homo sapiens
<400> 73
   ggagtttctc caccagcaac atggccgccg cctgagagga gagccgggcc gccgccgtct      60
   ctgcagcccg cgggtaactg ggccgttgcc gccgtccgcg ctcggccccc gcggagagat     120
   cgagctgaag gactgcgcgg ctggctctcc tctagtatgg ccaatgaaga ggatgaccca     180
   gttgtacagg agatcgatgt gtacttggcc aagagtctgg cggaaaagct gtatctattt     240
   cagtaccctg tgcgtccagc ctcgatgacc tacgatgaca ttccgcacct ctcagccaag     300
   atcaagccca agcagcagaa ggtagagctt gagatggcca tcgacaccct gaaccccaac     360
   tattgccgca gcaaagggga gcagattgcg ctgaacgtgg acggggcctg cgccgacgag     420
   accagcacgt attcctcgaa gctgatggac aagcagacct tctgctcttc ccagaccacc     480
   agtaacacat cccgttatgc cgctgcactc tacaggcaag gtgagctcca cctgacacct     540
   ttacatggca tcctgcagct gcggcccagc ttctcctacc tggataaggc tgacgccaag     600
   caccgggaga gggaggcggc caacgaggca ggggactctt cacaggatga ggcggaagac     660
   gatgttaagc agatcacggt gcggttctcc cggccggagt cagagcaggc ccgccagcgc     720
   cgtgtgcagt cctatgagtt cctgcagaag aagcacgcag aggagccctg ggtccacctg     780
   cattactatg gcctgaggga cagtcgctct gagcatgagc gtcagtacct gctgtgcccc     840
   ggctcaagcg gggtggagaa cacggagctc gtcaagtcac ccagtgagta cctgatgatg      900
   ctgatgccac ccagccagga ggaggagaaa gacaagcctg tggcccccag caacgtcctg     960
   tcgatggccc agctgcgcac gctgcccctg gccgatcaga tcaagatcct gatgaagaat    1020
   gtgaaggtca tgccttttgc caacttgatg agcctcctgg gcccctccat cgattccgtg    1080
   gctgttctgc ggggcatcca gaaggtggcg atgttggtcc aagggaactg ggtggtgaag    1140
   agtgacatcc tataccccaa ggactcgtcc agccctcaca gcggcgtgcc tgctgaggtg    1200
   ctctgcaggg gccgagactt cgttatgtgg aagttcacgc agagccgctg ggtggttagg    1260
   aaagaggtgg caaccgtgac caaactctgc gccgaggatg tgaaggactt cctggagcac    1320
   atggccgtgg tgaggatcaa caaaggctgg gagttcattc tgccttatga tggggagttc    1380
   atcaagaagc acccggatgt ggtccagcgg cagcacatgc tgtggacggg tatccaggcc    1440
   aaactggaaa aagtctataa tcttgtaaag gaaaccatgc caaagaagcc ggatgcacaa 1500
   tcagggcctg ccgggctggt ctgtggggac cagcggatcc aagtagccaa aaccaaggcc 1560
   cagcagaacc acgcgttgct ggagcgggag ctgcagcggc ggaaggagca gctgcgggtg 1620
   cctgcggtcc cgcccggtgt gcggatcaag gaggagcccg tgagcgagga gggcgaggag 1680
   gacgaggagc aggaggcgga ggaggagccc atggacactt cccccagcgg cctccacagc 1740
   aagctggcca acgggctgcc tctcgggcgg gctgcgggca cagacagctt caacgggcac 1800
   ccgccccagg gctgcgccag cacccctgtg gctcgggaac tgaaggcctt cgtggaggcc 1860
   acctttcaga gacagtttgt gctcacgctg agcgaactca agcgcctctt caatctgcac 1920
   ttggccagcc tgccccccgg ccacacactc ttcagcggca tctcggaccg catgctacag 1980
   gacacggtgc tggccgccgg ttgcaagcag atactggtgc cttttccccc ccagactgct 2040
   gcttccccgg atgagcagaa ggtgtttgcc ctctgggagt ctggagacat gagtgatcag 2100
   catcgacagg ttttgcttga aattttttcc aaaaattacc gggtacgccg aaacatgatc 2160
   cagtctcggt tgactcaaga gtgtggagaa gatctcagta aacaggaggt ggataaagta 2220
   ctaaaggact gctgtgtaag ctatggtggc atgtggtacc ttaaagggac agtacagtct 2280
   tgacaatagt agcaaactac taacccagca aatctaagcc caaggaagaa gggcggaacc 2340
   agaagtaggg cctcgacttg cttcagacga cacagagcaa gaggaactga ccatctcatg 2400
   acctgtggca ttgcacggtg cagtggacag aagggattat cctcagccag tcgcagggtc 2460
   agcttaagtt agttagatca ctcccagaag agaccagctg ggaccttctt tgcagtacaa 2520
   tttgaaattc ctgatgtatt ttgcttatta tttggtttca ttctcataat aaagagagtg 2580
   tatactgaca tgggcaggat gataaaaaaa aaaaaaaaaa aaaaaaaaaa aaaaaaaaaa 2640
<210> 74
   <211> 2177
   <212> DNA
   <213> Homo sapiens
<400> 74
   ggaagaaggc ccctcggcct cagcagagcg ctatcctcca ctggttaccc gggttccccg      60
   gcttgctacc gggctgctcc gtgcatcttt ccccccaggc gtcaggaact gcgccctcat     120
   ggcggaggtg aaggtgaagg tgcagccgcc tgacgcggat ccggtcgaaa tagaaaacag     180
   gattatagaa ttatgtcacc agttccctca tggaatcaca gaccaagtaa ttcagaatga     240
   aatgcctcat atagaagccc agcagcgggc agtagccatc aataggttgt tgtctatggg     300
   tcagttggat ctcttaagga gcaatacggg ccttttatat agaataaagg actctcagaa     360
   tgctggtaaa atgaagggat ccgataacca agaaaaacta gtatatcaaa tcatagagga     420
   tgcaggaaat aaaggaatat ggagcagaga tatccgctat aaaagtaatt tgccattaac     480
   agaaatcaac aaaattctga agaatctgga aagtaaaaag cttatcaaag ctgttaagtc 540
   tgtagcagcc tcaaaaaaga aggtgtatat gctctataac ctgcagccag accggtctgt 600
   gactggtgga gcctggtaca gtgaccagga ttttgaatct gaatttgtag aggtgcttaa 660
   ccaacagtgt tttaaattcc tacagtccaa ggcagaaaca gcacgagaaa gcaaacagaa 720
   cccaatgata caaagaaata gttcatttgc ctcatcacat gaagtgtgga aatatatctg 780
   cgaattggga atcagtaagg tagagttatc catggaagac attgaaacca tcctgaatac 840
   actcatttat gatggaaaag tggagatgac gattattgct gcaaaagaag gcacagttgg 900
   cagtgtagat ggacacatga aactgtacag ggcagtcaat ccaatcatcc ctcccacagg 960
   tttggtccgg gcaccctgtg gactctgccc ggtttttgat gactgccacg aaggtggtga 1020
   gatttcacca tctaactgta tttacatgac agagtggctc gaattttaat agagagctat 1080
   gaactttatt gacattttgc aaatgaagtt acttagggag cagataattt aattcatgat 1140
   ggaacacgaa atctccttga aagcaaactt cacaataatg gacgtagact tgctgctatg 1200
   aaaacatatt ttttttattt atgaagacta aatttatatt ggtaaaatag ccagtagaat 1260
   atgaaagaaa taaggttagt agtgaaattc attcttcaat aaataaaaca ctttgaaact 1320
   ccggaggacc acatctttca agacttctga tgggtcaagg aaaaagatgc caacataccc 1380
   gtatttacca agtactatga taatggctag agtataaaaa tgttcttttt aaagttattt 1440
   attaagttct tcattggaag ttttttttta tatctggttc actaccacca ttttctgttt 1500
   cctactttct cagtggtttc attgaaaaga aattagaagg ggttaaaggc aggaatagca 1560
   aagagtgcaa acttggggta tgactggggg agagtggaac atgccttttc cgcacaatat 1620
   taattccttt ttgtatcaga aatgttcttt taggagatta tgctaccata cttacttcaa 1680
   acccaatgac tactgtcaag gacatatttt cagtacataa atactatcat tttcattcta 1740
   aagaatattt tcactgttcc ttctttctta aagtcttatg tttcactctt taactcaaat 1800
   gtattctttg ttagaattta ccctagattc ttatttaatg tctgcagtag actgaatgtt 1860
   tgtgtgcccc cagaattcta atgttgaaat ctcatttcca atgtgatggt atttggaggt 1920
   ggggcttttg gtaagtgata ggtcaggaga gtaacagcgc tcatgaatgg gattagtgcc 1980
   cttatataaa gagacccaga gagctccatc accccttctg ccatgtgaaa gggagaagac 2040
   aaacatccac gaaccaggaa gtgggtcctc accagaaaac aaatctgtaa gcaccttgat 2100
   cttggacttc ccagcctcca gaattgtgag aaataaattt ctgttgttga taaaaaaaaa 2160
   aaaaaaaaaa aaaaaaa 2177
<210> 75
   <211> 414
   <212> DNA
   <213> Homo sapiens
<220>
   <221> misc_feature
   <222> (336)..(336)
   <223> n is a, c, g, or t
<220>
   <221> misc_feature
   <222> (406)..(406)
   <223> n is a, c, g, or t
<400> 75
   gtttgtggtg attgtttatt caagaatatt tggaacaaac atccctgctt aatagttact      60
   agttaagatc tatattaaca aattagtttt ctctaacaaa taaacattta aataagttca     120
   aaataaaaca tcttactata aaacaaacga acacattttg ttttgtaaga atttaatgtg     180
   taaataaata cagaaaaaca agcattacag caaccagttt tgctcatgat agcaatttta     240
   aagacacttc aacaaatact tttatgtaag aggctaattc tgcaaaataa tattcacttt     300
   cctatggaat aatttcacag tcccagaatt ttaagncccc aaggggaaga gaaaagaaag     360
   tgggaaaggg aaaccgtttc ccaatttggg gccctttttg gggggntttt ttaa          414
<210> 76
   <211> 3285
   <212> DNA
   <213> Homo sapiens
<400> 76
   actccgcggt cctggtgccg cgtgcaggtc ggtgcgcgct tctcccgagg tggaacgggc      60
   ggcagtcaag cgccggcgtt ctctgccgtc accctttcct tgccggccgg cacttcggct     120
   gcagagtttt gcccacgctt cgagacttag ggagcagtgc ctttcagaat ttgcccactc     180
   atctggtata actggttctg atggctggga ataaaggaag aggacgtgct gcttatacct     240
   ttaatattga ggctgttgga tttagcaaag gtgaaaagtt acctgatgta gtgttgaaac     300
   cacccccact atttcctgat acagattata aaccagtgcc actgaaaaca ggagaaggtg     360
   aagaatatat gctggctttg aaacaggagt tgagagaaac aatgaaaaga atgccttatt     420
   ttattgaaac acctgaagaa agacaagata ttgaaaggta tagtaaaaga tacatgaagg     480
   tatacaagga agaatggata ccagattgga gaagacttcc aagagagatg atgccaagaa     540
   ataaatgtaa aaaagcaggc ccaaaaccca aaaaggcaaa agacgcaggc aaaggcacac     600
   cactcactaa tactgaagat gtgttgaaaa aaatggagga attggaaaaa agaggtgatg     660
   gtgaaaaatc agatgaggaa aatgaagaga aagaaggaag caaagagaaa agtaaagaag     720
   gtgatgatga cgatgacgat gatgccgcag aacaggagga atatgatgaa gaagagcaag     780
   aagaggaaaa tgactacatt aattcatact ttgaagatgg agatgatttt ggcgcagaca     840
   gtgatgacaa catggatgag gcaacctatt aggcatgaaa tttttcaaaa aatattttta 900
   tgatgcagct tctgaacatt tggacagact tgatttgtat tttatttctg ataaggaata 960
   agtacttgtt tctgttgttt tggacaaata gttgttacca aaatattcaa aaccactttg 1020
   agtttacata ctagttacct taaaaattat tccctgacac tctgacattc cttctaaaca 1080
   cctctgccat ctctcttatg tactctcatg ggtttttttg tatgatttga atatgaatgt 1140
   gcctaaagaa tttttgctct cttaatctat gtatacatac ttgaacaaat cattcttgct 1200
   taactgctga tcttttgtaa aactattgct agtcataatg gatttctata acctgaaagt 1260
   tgatataata cagcacgtgg agcactttaa aaacaaagaa acttgaaaat aattttattt 1320
   tttaccttta catggtgtgt tctagactac tgcattatga ctacataaac ctacctgagc 1380
   tcttacaagt acagaattgt gagacagagg ttttgtgatg gaaaacatac acatagggaa 1440
   aaataccaaa ctgagcaata taaatttcac agctcacatc ttagccagta caaagaaact 1500
   ttcacatggg gtgaatatta taatacttga atttgagact taatactgta tatgaattaa 1560
   tcatcccact gtaataactg acatcttcaa atagttgggc ttatgaaaaa tttgactaag 1620
   acattttatt ttatacttta tgtaaatgtg aaataagttc tggtcatata aatatgtaga 1680
   atgcatgtgt gtgaactcta tgaagtgaag tgtggtggcc ttttatgttt cagagtactc 1740
   aagtgtacaa ttgatttata taaaaatgca aaatgaactc aggtcatttt gaaattaata 1800
   ttaatatttt tgttgtttaa ctttaaagct agctccccta actttatata ttttttggga 1860
   aaaaatacct aaaaacctca gcctacaaag actctcagaa atgccaaaga ttttcaagga 1920
   aattcatatt atatatttca aaaatgattt atcaatgtta tctaccaaaa gaaataattt 1980
   tatttttccc ctttggggag atattatcct ctaatatgag aatcagatcc ctagattcta 2040
   tttctaccta tactattaaa ctcaaccttg aacctgagct tggttgtttc tgtgccttag 2100
   tttctccact cgtaaacaat ttcctataaa aattgtttaa acaccaacca aaatttacca 2160
   tatgtattca ttctcttgcc ttaaagatca cttcttaact tacatttggc tctcaccctc 2220
   taaaaatttg tagtttggag agtaactgaa agcacaattt taagaaacat gttttcctca 2280
   caaaatatat tgtaatctga ttttcctatc ttgtattcat gcagaaaatt ggagaaaatg 2340
   tgtattgtct tgtgccattc tgcaatatga atttctcagg aaaaagatgt ttcaatggca 2400
   atactttctt aaataattca agatgggtgt gaggacctat cttattagtt tagtgctgtg 2460
   ttaaccattt gtgaattact tcttgtttaa aggaagttgt caaaaaattt taagattcta 2520
   aatggaatac acgaataaag taagccttag aatagaaacc tcacaaaaag ttgtcatttg 2580
   cggttgataa ttaacatagg tgttttattc tctcaactcc ttagtaccag agccaaatac 2640
   aatttagttt tcagaaatct tatcaatatt gttctattta cctacttata aatctgcaga 2700
   taaccacttg aaccaggcaa atacactgaa gataaaggtt atttcttttt ttagctttga 2760
   atttgtcatg accattttag tcttgcagat agcagggcag ccccttgggg caaggattta 2820
   ctctgggagg taccgttaag agccttcttt ccccctttga aagatccttt tacaatgtta 2880
   aagtatacta gttgcaagaa caagcaggat ttgcaggttg ctttaccagc atgagtctca 2940
   tttttctggc ttaaaatctg ggactgtgaa attattccat aggaaagtga atgttatttt 3000
   gcagaattag cctcttacat aaaagtattt gttgaagtgt ctttaaaatt gctatcatga 3060
   gcaaaactgg ttgctgtaat gcttgttttt ctgtatttat ttacacatta aattcttaca 3120
   aaacaaaatg tgttcgtttg ttttatagta agatgtttta ttttgaactt atttaaatgt 3180
   ttatttgtta gagaaaacta atttgttaat atagatctta actagtaact attaagcagg 3240
   gatgtttgtt ccaaatattc ttgaataaac aatcaccaca aacaa 3285
<210> 77
   <211> 1377
   <212> DNA
   <213> Homo sapiens
<400> 77
   gggagcctgc ggagttcgag accatgctgc tgttctgccc cggctgcggg aacgggctga 60
   tcgtggagga gggacaacgc tgccaccgct tcgcctgcaa cacgtgcccc tacgtgcaca 120
   acatcacccg caaggtaaca aatcggaagt acccaaaact gaaagaagtg gatgatgtgc 180
   ttggtggagc agctgcctgg gagaatgttg actctactgc agagtcgtgt cccaaatgcg 240
   aacatcctcg tgcttacttc atgcagcttc agacccgctc tgcagatgag ccgatgacca 300
   ccttctacaa gtgctgcaat gctcagtgtg gacaccgctg gagggattag ggccaggatg 360
   gcccagctgc cctagtgtgt gcttgccttg tccctcgggg tagatgctta gctggcagta 420
   tgagttgtgt gtcctgaggg tctttgctag tgtggtggaa agataaacct tttgaggtga 480
   agagccaggg ggtcaggaaa tatggcctat ctgccaggca gggtggatga agtcatgaat 540
   gtctgggagt ttttctgtgt ggggaggaga cagagaccca taactaaata tgctctgtgt 600
   aaagtcctat tctttcatct tccactttat tggcagttga cattccctta ctcccgatca 660
   acactcttaa atatttgtac tgtttgtaaa acttagtaca tgtccctaaa atatttaact 720
   gttacttgta aacttgtgta atttattatt tattttaatc aaaattctga atatttcatt 780
   taaatgaaag ttggaatatt gccttctcag tgttttaaaa tagcttaaga ggtagaaagc 840
   agaccccaaa tcacagtgat cccagaatag actacatact tacatttgaa caggctacga 900
   aaatgttaat caccgggatt tcaatttctt tctttattcc tattcttact cccttattca 960
   tttttttgaa cctcccctga tttgcttcct cggttgcttt taaatgtata agatactatt 1020
   tatttattta tttattttat ttatttattt atttatttaa gacagagtct tgttctgtcg 1080
   cccaggctgg agtgcagtgg tacgaattca gctcactgca acctctgcct tccgggttca 1140
   agggattctc ctgtctcagc ctcccaagca gctgggacta caggcatgca ccaccacacc 1200
   tgggtaattt tgcattttta gtagagatgg ggtttctcca tgttggtcgg gctggtctcc 1260
   aactcctgac ctcagataat ctgtctgcct cggcctccta aagtgctggg attacaggcg 1320
   tgagccactg tgtcccacca atctttacat ttaatatgac tattttctta ttgttcg 1377
<210> 78
   <211> 3800
   <212> DNA
   <213> Homo sapiens
<400> 78
   ggcgcgggcg catgcgcagg cgcgggccgg tggggtggcc tggagcggcg tgcgtaatgt      60
   cggcactttg ctggggccgc ggagcggcgg ggctcaaacg agccctacgg ccttgcggcc     120
   gcccgggact ccccggcaaa gaagggaccg ccggtggcgt ctgcggcccc aggaggagct     180
   cgtccgccag cccccaggag caagaccaag accgcaggaa ggactggggc cacgtggagc     240
   tgctggaggt gctccaggcg cgggtgcggc agctgcaggc tgagagcgtg tcggaggtgg     300
   tggtgaacag ggtggatgtg gcgcggctcc cagaatgtgg cagtggagat ggtagcctcc     360
   agccacccag gaaggtccag atgggggcca aggatgccac cccggtgccc tgtggccgct     420
   gggcaaagat actggagaag gataagcgga cccagcagat gcgtatgcag cggttgaagg     480
   cgaagctgca gatgccattc cagagcgggg agttcaaggc gctgaccagg cgcctgcagg     540
   tggagccccg gctcctgagc aagcagatgg ccgggtgcct ggaggactgc acgcgccagg     600
   cccccgagag cccctgggag gagcagctgg cccggctgct gcaggaggcc cctgggaagc     660
   tgagcctcga tgtggagcag gccccgtcgg ggcagcactc gcaggcccag ctctcaggtc     720
   agcagcagag gctcctggcc ttcttcaagt gctgcctgct cactgaccag ctgcccctcg     780
   cccaccacct gctggtcgtc caccacggcc agcggcagaa gcggaagctg ctcacgctgg     840
   acatgtacaa cgccgtgatg cttggctggg cgcggcaggg tgccttcaag gagctggtat     900
   atgtgttatt catggtgaag gatgccggct tgactccgga cctgctgtcc tatgcggctg     960
   ccctccagtg catggggagg caggaccagg acgccgggac catcgaaagg tgtctggaac    1020
   agatgagcca ggaggggctg aagctgcagg cactcttcac cgccgttctg ctgtctgagg    1080
   aggatcgggc cactgttctg aaggccgtgc acaaggtgaa gcccaccttc agcctcccgc    1140
   cgcagctgcc gcccccggtc aacacctcca agctgctcag ggacgtgtat gccaaggatg    1200
   ggcgtgtgtc ctacccgaag ctgcacctgc ccttgaagac cctgcagtgc ctctttgaga    1260
   agcagctcca catggagctg gccagcaggg tgtgcgtggt gtccgtggag aagcccacgt    1320
   tgccaagcaa ggaggtcaag cacgcgcgga agaccctgaa gaccctgcgg gaccaatggg    1380
   agaaagcact gtgccgggcg ctgcgggaga ccaagaaccg cctagagcgc gaggtgtacg    1440
   agggccggtt ctcactttac cccttcctgt gcctgctgga cgagcgcgag gtggtgcgga 1500
   tgctcctgca ggtcctgcag gcgctgcccg cccaaggtga gtccttcacc accctggccc 1560
   gggagctgag tgcgcgcact ttcagccggc acgtggtgca gaggcagcgg gtcagtggcc 1620
   aggtgcaggc gctgcagaac cactacagga agtacctctg cttgctggcc tccgacgccg 1680
   aggtgcccga gccctgcctg ccgcggcagt actgggagga gctgggggcg cccgaggccc 1740
   tgcgggagca gccctggccc ctgccagtgc agatggagct gggcaagctg ctggcggaga 1800
   tgctggtgca ggctacgcag atgccatgca gcctggacaa gccgcatcgt tcctctcggc 1860
   ttgtccccgt gctctaccac gtgtattcct tccgcaacgt ccagcagatc ggcatcctga 1920
   agccgcaccc ggcctacgtg cagctgctgg agaaggccgc ggagcccacg ctgaccttcg 1980
   aggcggtgga tgtacccatg ctttgccccc cgctgccctg gacatcgccg cactctggtg 2040
   ctttcctgct cagccccacc aagctgatgc gcacggtgga aggcgccacg cagcaccagg 2100
   agctgctgga aacctgcccg cccaccgcgc tgcatggcgc actggacgcc ctcacccaac 2160
   tgggcaactg cgcctggcgc gtcaacgggc gcgtgctgga cctggtgctg cagctcttcc 2220
   aggccaaggg ctgcccccag ctaggcgtgc cggccccgcc ctccgaggcg ccccagccgc 2280
   ccgaggccca cctgccgcac agcgccgcgc ccgcccgcaa ggccgagctg cgccgtgagc 2340
   tggcgcactg ccagaaggtg gcccgggaga tgcacagcct gcgggcggag gcgctgtacc 2400
   gcctctcgct ggcgcagcac ctgcgggacc gcgtcttctg gctgccgcac aacatggact 2460
   tccgcggccg cacctacccc tgcccgccgc acttcaacca cctgggcagc gacgtggcgc 2520
   gggccctgct ggagttcgcc cagggccgcc cgctcggccc gcacggcctg gattggctca 2580
   agatccacct ggtcaatctc acggggttga agaagcggga gccgctgcgg aagcgcctgg 2640
   cctttgcgga ggaggtgatg gatgacatcc tggactccgc ggaccaaccc ttgacgggcc 2700
   gaaagtggtg gatgggcgcg gaggaaccct ggcagacgct ggcctgctgt atggaggtgg 2760
   cgaacgctgt gcgcgcctcc gaccctgccg cctatgtctc ccacctcccc gtccatcagg 2820
   acggctcttg caacggcctg cagcattatg ctgctctggg ccgcgacagc gtgggcgccg 2880
   cctccgtcaa cctggagccc tcggatgtgc cgcaggacgt gtacagcggc gtggccgcgc 2940
   aggtggaggt gttccgtagg caggacgccc agcggggcat gcgggtggca caggtgctgg 3000
   aaggtttcat cacccgcaag gtggtgaagc agacggtgat gacggtggtg tacggggtca 3060
   cgcgctatgg cgggcgcctg cagattgaga agcgcctccg ggagctgagc gactttcccc 3120
   aggagttcgt gtgggaggcc tctcactatc tcgtacgcca ggtcttcaag agtctacagg 3180
   agatgttctc ggggacccgg gccatccagc actggctgac cgagagtgcc cgcctcatct 3240
   cccacatggg ctctgtggtg gagtgggtca cacccctggg cgtccccgtc atccagccct 3300
   atcgcctgga ctccaaggtc aagcaaatag gaggtggaat tcagagcatc acctacaccc 3360
   acaacggaga catcagccga aagcccaaca cacgtaagca gaagaacggc ttcccgccca 3420
   acttcatcca ctcgctggac tcctcccaca tgatgctcac cgccctgcac tgctacagga 3480
   agggcctgac cttcgtctct gtgcacgact gttactggac tcacgcagct gatgtctccg 3540
   tcatgaacca ggtgtgccgg gagcagtttg tccgcttgca cagcgagccc atcctgcagg 3600
   acctgtccag attcctggtc aagcggttct gctctgagcc ccagaagatc ttggaggcca 3660
   gccagctgaa ggagacactg caggcggtgc ccaagccagg ggccttcgac ctggagcagg 3720
   tgaagcgttc cacctacttc ttcagctgac accccgtgag ccttgtcagt gtgtaaataa 3780
   agctcttttg ccacccccag 3800
<210> 79
   <211> 698
   <212> DNA
   <213> Homo sapiens
<400> 79
   cggcagccag aagacgcggt cccgcaggtg ctgcgccagc gagaggcggt acagcgcctc 60
   cgcccgcagg ctgtgcatct cccgggccac cttctggcag tgcgccagct cacggcgcag 120
   ctcggccttg cgggcgggcg cggcgctgtg cggcaggtgg gcctcgggcg gctggggcgc 180
   ctcggagggc ggggccggca cgcctagctg gggcagcccc ttggcctgga agagctgcag 240
   caccaggtcc agcacgcgcc cgttgacgcg ccaggcgcag ttgcccagtt gggtgagggc 300
   gtccagtgcg ccatgcagcg cggtgggcgg gcaggtttcc agcagctcct ggtgctgcgt 360
   ggcgccttcc accgtgcgca tcagcttggt ggggctgagc aggaaggcac cagagtgcgg 420
   cgatgtccag ggcagcgggg ggcaaagcat gggtacatcc accgcctcga aggtcagcgt 480
   gggctccgcg gccttctcca gcagctgcac gtaagccggg tgcggcttca tgatgccgat 540
   ctgctggacg tagcggtacg aatacacgag gtagagcacg gtgacaagcc gagaggaacg 600
   atgcggcttg tccacgctgc atggcatctg cgtagcctgc accatcatgt acgccagcat 660
   cttggtcaca tcatcagcac tgacagaggc catggctg 698
<210> 80
   <211> 3861
   <212> DNA
   <213> Homo sapiens
<400> 80
   gcggccgctc tcagcggagg gggcggcccc ggggcccagg cgccgcggcc cggcaccccg       60
   tggaagagcc gcgcgtacag cccgggcatc cagggactac atgaggaaat aattgacttt      120
   tataacttca tgtccccttg tcctgaagaa gcagctatga gaagagaggt ggtgaaacgg      180
   atcgaaactg tggtgaaaga cctttggccg acggctgatg tacagatatt tggcagcttt      240
   agtacaggtc tttatcttcc aactagcgac atagacctgg tggtcttcgg gaaatgggag      300
   cgtcctcctt tacagctgct ggagcaagcc ctgcggaagc acaacgtggc tgagccgtgt      360
   tccatcaaag tccttgacaa ggctacggta ccaataataa agctcacaga tcaggagact      420
   gaagtgaaag ttgacatcag ctttaacatg gagacgggcg tccgggcagc ggagttcatc      480
   aagaattaca tgaagaaata ttcattgctg ccttacttga ttttagtatt gaaacagttc      540
   cttctgcaga gggacctgaa tgaagttttt acaggtggaa ttagctcata cagcctaatt      600
   ttaatggcca ttagctttct acagttgcat ccaagaattg atgcccggag agctgatgaa      660
   aaccttggaa tgcttcttgt agaatttttt gaactctatg ggagaaattt taattacttg      720
   aaaaccggta ttagaatcaa agaaggaggt gcctatatcg ccaaagagga gatcatgaaa      780
   gccatgacca gcgggtacag accgtcgatg ctgtgcattg aggaccccct gctgccaggg      840
   aatgacgttg gccggagctc ctatggcgcc atgcaggtga agcaggtctt cgattatgcc      900
   tacatagtgc tcagccatgc tgtgtcaccg ctggccaggt cctatccaaa cagagacgcc      960
   gaaagtactt taggaagaat catcaaagta actcaggagg tgattgacta ccggaggtgg     1020
   atcaaagaga agtggggcag caaagcccac ccgtcgccag gcatggacag caggatcaag     1080
   atcaaagagc gaatagccac atgcaatggg gagcagacgc agaaccgaga gcccgagtct     1140
   ccctatggcc agcgcttgac tttgtcgctg tccagccccc agctcctgtc ttcaggctcc     1200
   tcggcctctt ctgtgtcttc actttctggg agtgacgttg attcagacac accgccctgc     1260
   acaacgccca gtgtttacca gttcagtctg caagcgccag ctcctctcat ggccggctta     1320
   cccaccgcct tgccaatgcc cagtggcaaa cctcagccca ccacttccag aacactgatc     1380
   atgacaacca acaatcagac caggtttact atacctccac cgaccctagg ggttgctcct     1440
   gttccttgca gacaagctgg tgtagaagga actgcgtctt tgaaagccgt ccaccacatg     1500
   tcttccccgg ccattccctc agcgtccccc aacccgctct cgagccctca tctgtatcat     1560
   aagcagcaca acggcatgaa actgtccatg aagggctctc acggccacac ccaaggcggc     1620
   ggctacagct ctgtgggtag cggaggtgtg cggccccctg tgggcaacag gggacaccac     1680
   cagtataacc gcaccggctg gaggaggaaa aaacacacac acacacggga cagtctgccc     1740
   gtgagcctca gcagataatg gctcctggct gcgtcagcct cccccacccc tctgcagact     1800
   gccccgcggc ctcggccacc ggcaggggaa ccgagaccag caccccgcac gtcagccggg     1860
   ctcgcggcac gcccgccgct gatcactctg catgtttctt cgtgtggtgg tcgcgtccat     1920
   cttcaagaac agctcgttgt gctcatctgt gaagccttat taaacgtgga cgttgttttc     1980
   tgccttccca ggattcttcc ttcagtgctg aggcaggtcg ggctcaggaa ctgcagggac     2040
   gtgaacatgc gcttgcggtt tgaggtagcc gtgtctgttc cttcgcggtt tgctattttc     2100
   atttcctgtt cgtcaaagca gcagaggaga tcaaaccccg ttcgtgtgtc tttcctccac     2160
   ggataagctt gggaggtcat tgttttactg ccctcacatt ttgtttgaaa tttcagaact     2220
   gtttttctat gtaaatattg aaaacttatg atttgtgcaa taactcagat attttttatt     2280
   taatttccta ttttcacata agttatattt aagggaggag ggaatttttt ttaaacaagc     2340
   ttaggtcctt tcccgagctg cattttctaa gttgggtcat cgtgtcggct ggttgtctga     2400
   cgagcatcgt tacaaacacc atgatgaggg gtttggggtt ttattttgat gtcttttctt     2460
   ttggtcggaa gtgagtgaag gagccaggtc gccctgaagg ttttccaaag ggcttggctc     2520
   cagagccacc tggcagactg cccgtggccc tgctgtcggg ccccaggccg ttgtcctgct     2580
   ctgaccacag agttttaatg ttttggtttt cacttctttt aaactggaca acaaatccag     2640
   catttcaagt gccagaagta taactttcta aggagagaag ggttgtcaca ttataaaatc     2700
   tttaggaaaa tgtgaactgg aaaacgcttc ggtcagtttt agtgacatag cctgtgatga     2760
   tgggtctggt gactattatt gcggaccgtg gtacccagtt ttaggaatgt ggagaaagga     2820
   attctgttga ttccgttgag gaatctgtag cgtatgcatt cgttctgtta agagcaaatc     2880
   taggagaagt gcttcagctg cccagtgcgc cgtggggagt gttttaacgg atcgtgtcgc     2940
   aggagagcac agcccagcgt tggggccggg accgctggcg cccgacgtcg gaagcataca     3000
   ggtatactat gcaagtgtat tctgccacaa caaccactgt ctttgttacc tttttttgaa     3060
   caagaatata tccatcctgc ctaaccctga gtttttggag caccacagtt gtcctgggag     3120
   ttggttgcat cttgtaggcc atctgacttc ctgtttttaa aacgggggtc tggtcttgct     3180
   aaacactaca ggtaggttgg tctttgaagt ccactagtgg agaatgtcaa gacaagatac     3240
   ttattaccat gacatctgat gcatgtgcag cagtggggag ttctagattg atctctgaat     3300
   gtgatcgacg cccagcaagg acaagcttta aaatgtctgc ggtctgccct tttgaagcag     3360
   gactggctca ctctgtcatt gggagctgtc agctgcgact gcaggttctc taggaggcat     3420
   tccagaatag agtagcacac tgtgtctgca gttctcgatg accgaaagtt atcaaaaata     3480
   tttaaaatat ttaaattgtg aacctattga taaagaatat ttataaaaac tgatctgtag     3540
   gcctgtacta atctctacgc attagcaata ttgactgtaa acccacatta aggaaaccac     3600
   tacgggtctg gcagtgcgtg tcccgtgggg tgtgcatttt aaaactcgat tcatagacac     3660
   aggtaccatg ttccatttcc gtcatggtga agcaaatgaa ttggcctggc taccactgtg     3720
   gtcgcgtgct acaggtttga caaaaagata tcatgtttcg atttttttgt gtgtggacaa     3780
   caatatggaa gctaaaattg acatattttt atgtaaagtt tttctattct ttgattttta     3840
   ataaactttg gaaaccagtt t                                                3861
<210> 81
   <211> 651
   <212> PRT
   <213> Homo sapiens
<400> 81
   Met Ala Ala Pro Leu Leu His Thr Arg Leu Pro Gly Asp Ala Ala Ala
   1               5                   10                  15
   Ser Ser Ser Ala Val Lys Lys Leu Gly Ala Ser Arg Thr Gly Ile Ser
               20                  25                  30
   Asn Met Arg Ala Leu Glu Asn Asp Phe Phe Asn Ser Pro Pro Arg Lys
           35                  40                  45
   Thr Val Arg Phe Gly Gly Thr Val Thr Glu Val Leu Leu Lys Tyr Lys
       50                  55                  60
   Lys Gly Glu Thr Asn Asp Phe Glu Leu Leu Lys Asn Gln Leu Leu Asp
   65                  70                  75                  80
   Pro Asp Ile Lys Asp Asp Gln Ile Ile Asn Trp Leu Leu Glu Phe Arg
                   85                  90                  95
   Ser Ser Ile Met Tyr Leu Thr Lys Asp Phe Glu Gln Leu Ile Ser Ile
               100                 105                 110
   Ile Leu Arg Leu Pro Trp Leu Asn Arg Ser Gln Thr Val Val Glu Glu
           115                 120                 125
   Tyr Leu Ala Phe Leu Gly Asn Leu Val Ser Ala Gln Thr Val Phe Leu
       130                 135                 140
   Arg Pro Cys Leu Ser Met Ile Ala Ser His Phe Val Pro Pro Arg Val
   145                 150                 155                 160
   Ile Ile Lys Glu Gly Asp Val Asp Val Ser Asp Ser Asp Asp Glu Asp
                   165                 170                 175
   Asp Asn Leu Pro Ala Asn Phe Asp Thr Cys His Arg Ala Leu Gln Ile
               180                 185                 190
   Ile Ala Arg Tyr Val Pro Ser Thr Pro Trp Phe Leu Met Pro Ile Leu
           195                 200                 205
   Val Glu Lys Phe Pro Phe Val Arg Lys Ser Glu Arg Thr Leu Glu Cys
       210                 215                 220
   Tyr Val His Asn Leu Leu Arg Ile Ser Val Tyr Phe Pro Thr Leu Arg
   225                 230                 235                 240
   His Glu Ile Leu Glu Leu Ile Ile Glu Lys Leu Leu Lys Leu Asp Val
                   245                 250                 255
   Asn Ala Ser Arg Gln Gly Ile Glu Asp Ala Glu Glu Thr Ala Thr Gln
               260                 265                 270
   Thr Cys Gly Gly Thr Asp Ser Thr Glu Gly Leu Phe Asn Met Asp Glu
           275                 280                 285
   Asp Glu Glu Thr Glu His Glu Thr Lys Ala Gly Pro Glu Arg Leu Asp
       290                 295                 300
   Gln Met Val His Pro Val Ala Glu Arg Leu Asp Ile Leu Met Ser Leu
   305                 310                 315                 320
   Val Leu Ser Tyr Met Lys Asp Val Cys Tyr Val Asp Gly Lys Val Asp
                   325                 330                 335
   Asn Gly Lys Thr Lys Asp Leu Tyr Arg Asp Leu Ile Asn Ile Phe Asp
               340                 345                 350
   Lys Leu Leu Leu Pro Thr His Ala Ser Cys His Val Gln Phe Phe Met
           355                 360                 365
   Phe Tyr Leu Cys Ser Phe Lys Leu Gly Phe Ala Glu Ala Phe Leu Glu
       370                 375                 380
   His Leu Trp Lys Lys Leu Gln Asp Pro Ser Asn Pro Ala Ile Ile Arg
   385                 390                 395                 400
   Gln Ala Ala Gly Asn Tyr Ile Gly Ser Phe Leu Ala Arg Ala Lys Phe
                   405                 410                 415
   Ile Pro Leu Ile Thr Val Lys Ser Cys Leu Asp Leu Leu Val Asn Trp
               420                 425                 430
   Leu His Ile Tyr Leu Asn Asn Gln Asp Ser Gly Thr Lys Ala Phe Cys
           435                 440                 445
   Asp Val Ala Leu His Gly Pro Phe Tyr Ser Ala Cys Gln Ala Val Phe
       450                 455                 460
   Tyr Thr Phe Val Phe Arg His Lys Gln Leu Leu Ser Gly Asn Leu Lys
   465                 470                 475                 480
   Glu Gly Leu Gln Tyr Leu Gln Ser Leu Asn Phe Glu Arg Ile Val Met
   485 490 495
   Ser Gln Leu Asn Pro Leu Lys Ile Cys Leu Pro Ser Val Val Asn Phe
   500 505 510
   Phe Ala Ala Ile Thr Asn Lys Tyr Gln Leu Val Phe Cys Tyr Thr Ile
   515 520 525
   Ile Glu Arg Asn Asn Arg Gln Met Leu Pro Val Ile Arg Ser Thr Ala
   530 535 540
   Gly Gly Asp Ser Val Gln Ile Cys Thr Asn Pro Leu Asp Thr Phe Phe
   545 550 555 560
   Pro Phe Asp Pro Cys Val Leu Lys Arg Ser Lys Lys Phe Ile Asp Pro
   565 570 575
   Ile Tyr Gln Val Trp Glu Asp Met Ser Ala Glu Glu Leu Gln Glu Phe
   580 585 590
   Lys Lys Pro Met Lys Lys Asp Ile Val Glu Asp Glu Asp Asp Asp Phe
   595 600 605
   Leu Lys Gly Glu Val Pro Gln Asn Asp Thr Val Ile Gly Ile Thr Pro
   610 615 620
   Ser Ser Phe Asp Thr His Phe Arg Ser Pro Ser Ser Ser Val Gly Ser
   625 630 635 640
   Pro Pro Val Leu Tyr Met Gln Pro Ser Pro Leu
   645 650
<210> 82
   <211> 1394
   <212> PRT
   <213> Homo sapiens
<400> 82
<210> 83
   <211> 1079
   <212> PRT
   <213> Homo sapiens
<400> 83
   Met Asp Pro Gly Ser Arg Trp Arg Asn Leu Pro Ser Gly Pro Ser Leu
   1 5 10 15
   Lys His Leu Thr Asp Pro Ser Tyr Gly Ile Pro Arg Glu Gln Gln Lys
   20 25 30
   Ala Ala Leu Gln Glu Leu Thr Arg Ala His Val Glu Ser Phe Asn Tyr
   35 40 45
   Ala Val His Glu Gly Leu Gly Leu Ala Val Gln Ala Asp Ile Asn Trp
   50 55 60
   Ala Val Asn Gly Ile Ser Lys Gly Ile Ile Lys Gln Phe Leu Gly Tyr
   65 70 75 80
   Val Pro Ile Met Val Lys Ser Lys Leu Cys Asn Leu Arg Asn Leu Pro
   85 90 95
   Pro Gln Ala Leu Ile Glu His His Glu Glu Ala Glu Glu Met Gly Gly
   100 105 110
   Tyr Phe Ile Ile Asn Gly Ile Glu Lys Val Ile Arg Met Leu Ile Met
           115                 120                 125
   Pro Arg Arg Asn Phe Pro Ile Ala Met Ile Arg Pro Lys Trp Lys Thr
       130                 135                 140
   Arg Gly Pro Gly Tyr Thr Gln Tyr Gly Val Ser Met His Cys Val Arg
   145                 150                 155                 160
   Glu Glu His Ser Ala Val Asn Met Asn Leu His Tyr Leu Glu Asn Gly
                   165                 170                 175
   Thr Val Met Leu Asn Phe Ile Tyr Arg Lys Glu Leu Phe Phe Leu Pro
               180                 185                 190
   Leu Gly Phe Ala Leu Lys Ala Leu Val Ser Phe Ser Asp Tyr Gln Ile
           195                 200                 205
   Phe Gln Glu Leu Ile Lys Gly Lys Glu Asp Asp Ser Phe Leu Arg Asn
       210                 215                 220
   Ser Val Ser Gln Met Leu Arg Ile Val Met Glu Glu Gly Cys Ser Thr
   225                 230                 235                 240
   Gln Lys Gln Val Leu Asn Tyr Leu Gly Glu Cys Phe Arg Val Lys Leu
                   245                 250                 255
   Asn Val Pro Asp Trp Tyr Pro Asn Glu Gln Ala Ala Glu Phe Leu Phe
               260                 265                 270
   Asn Gln Cys Ile Cys Ile His Leu Lys Ser Asn Thr Glu Lys Phe Tyr
           275                 280                 285
   Met Leu Cys Leu Met Thr Arg Lys Leu Phe Ala Leu Ala Lys Gly Glu
       290                 295                 300
   Cys Met Glu Asp Asn Pro Asp Ser Leu Val Asn Gln Glu Val Leu Thr
   305                 310                 315                 320
   Pro Gly Gln Leu Phe Leu Met Phe Leu Lys Glu Lys Leu Glu Gly Trp
                   325                 330                 335
   Leu Val Ser Ile Lys Ile Ala Phe Asp Lys Lys Ala Gln Lys Thr Ser
               340                 345                 350
   Val Ser Met Asn Thr Asp Asn Leu Met Arg Ile Phe Thr Met Gly Ile
           355                 360                 365
   Asp Leu Thr Lys Pro Phe Glu Tyr Leu Phe Ala Thr Gly Asn Leu Arg
   370 375 380
   Ser Lys Thr Gly Leu Gly Leu Leu Gln Asp Ser Gly Leu Cys Val Val
   385 390 395 400
   Ala Asp Lys Leu Asn Phe Ile Arg Tyr Leu Ser His Phe Arg Cys Val
   405 410 415
   His Arg Gly Ala Asp Phe Ala Lys Met Arg Thr Thr Thr Val Arg Arg
   420 425 430
   Leu Leu Pro Glu Ser Trp Gly Phe Leu Cys Pro Val His Thr Pro Asp
   435 440 445
   Gly Glu Pro Cys Gly Leu Met Asn His Leu Thr Ala Val Cys Glu Val
   450 455 460
   Val Thr Gln Phe Val Tyr Thr Ala Ser Ile Pro Ala Leu Leu Cys Asn
   465 470 475 480
   Leu Gly Val Thr Pro Ile Asp Gly Ala Pro His Arg Ser Tyr Ser Glu
   485 490 495
   Cys Tyr Pro Val Leu Leu Asp Gly Val Met Val Gly Trp Val Asp Lys
   500 505 510
   Asp Leu Ala Pro Gly Ile Ala Asp Ser Leu Arg His Phe Lys Val Leu
   515 520 525
   Arg Glu Lys Arg Ile Pro Pro Trp Met Glu Val Val Leu Ile Pro Met
   530 535 540
   Thr Gly Lys Pro Ser Leu Tyr Pro Gly Leu Phe Leu Phe Thr Thr Pro
   545 550 555 560
   Cys Arg Leu Val Arg Pro Val Gln Asn Leu Ala Leu Gly Lys Glu Glu
   565 570 575
   Leu Ile Gly Thr Met Glu Gln Ile Phe Met Asn Val Ala Ile Phe Glu
   580 585 590
   Asp Glu Val Phe Ala Gly Val Thr Thr His Gln Glu Leu Phe Pro His
   595 600 605
   Ser Leu Leu Ser Val Ile Ala Asn Phe Ile Pro Phe Ser Asp His Asn
   610 615 620
   Gln Ser Pro Arg Asn Met Tyr Gln Cys Gln Met Gly Lys Gln Thr Met
   625 630 635 640
   Gly Phe Pro Leu Leu Thr Tyr Gln Asp Arg Ser Asp Asn Lys Leu Tyr
   645 650 655
   Arg Leu Gln Thr Pro Gln Ser Pro Leu Val Arg Pro Ser Met Tyr Asp
   660 665 670
   Tyr Tyr Asp Met Asp Asn Tyr Pro Ile Gly Thr Asn Ala Ile Val Ala
   675 680 685
   Val Ile Ser Tyr Thr Gly Tyr Asp Met Glu Asp Ala Met Ile Val Asn
   690 695 700
   Lys Ala Ser Trp Glu Arg Gly Phe Ala His Gly Ser Val Tyr Lys Ser
   705 710 715 720
   Glu Phe Ile Asp Leu Ser Glu Lys Ile Lys Gln Gly Asp Ser Ser Leu
   725 730 735
   Val Phe Gly Ile Lys Pro Gly Asp Pro Arg Val Leu Gln Lys Leu Asp
   740 745 750
   Asp Asp Gly Leu Pro Phe Ile Gly Ala Lys Leu Gln Tyr Gly Asp Pro
   755 760 765
   Tyr Tyr Ser Tyr Leu Asn Leu Asn Thr Gly Glu Ser Phe Val Met Tyr
   770 775 780
   Tyr Lys Ser Lys Glu Asn Cys Val Val Asp Asn Ile Lys Val Cys Ser
   785 790 795 800
   Asn Asp Thr Gly Ser Gly Lys Phe Lys Cys Val Cys Ile Thr Met Arg
   805 810 815
   Val Pro Arg Asn Pro Thr Ile Gly Asp Lys Phe Ala Ser Arg His Gly
   820 825 830
   Gln Lys Gly Ile Leu Ser Arg Leu Trp Pro Ala Glu Asp Met Pro Phe
   835 840 845
   Thr Glu Ser Gly Met Val Pro Asp Ile Leu Phe Asn Pro His Gly Phe
   850 855 860
   Pro Ser Arg Met Thr Ile Gly Met Leu Ile Glu Ser Met Ala Gly Lys
   865                 870                 875                 880
   Ser Ala Ala Leu His Gly Leu Cys His Asp Ala Thr Pro Phe Ile Phe
                   885                 890                 895
   Ser Glu Glu Asn Ser Ala Leu Glu Tyr Phe Gly Glu Met Leu Lys Ala
               900                 905                 910
   Ala Gly Tyr Asn Phe Tyr Gly Thr Glu Arg Leu Tyr Ser Gly Ile Ser
           915                 920                 925
   Gly Leu Glu Leu Glu Ala Asp Ile Phe Ile Gly Val Val Tyr Tyr Gln
       930                 935                 940
   Arg Leu Arg His Met Val Ser Asp Lys Phe Gln Val Arg Thr Thr Gly
   945                 950                 955                 960
   Ala Arg Asp Arg Val Thr Asn Gln Pro Ile Gly Gly Arg Asn Val Gln
                   965                 970                 975
   Gly Gly Ile Arg Phe Gly Glu Met Glu Arg Asp Ala Leu Leu Ala His
               980                 985                 990
   Gly Thr Ser Phe Leu Leu His Asp  Arg Leu Phe Asn Cys  Ser Asp Arg
           995                 1000                 1005
   Ser Val  Ala His Val Cys Val  Lys Cys Gly Ser Leu  Leu Ser Pro
       1010                 1015                 1020
   Leu Leu  Glu Lys Pro Pro Pro  Ser Trp Ser Ala Met  Arg Asn Arg
       1025                 1030                 1035
   Lys Tyr  Asn Cys Thr Leu Cys  Ser Arg Ser Asp Thr  Ile Asp Thr
       1040                 1045                 1050
   Val Ser  Val Pro Tyr Val Phe  Arg Tyr Phe Val Ala  Glu Leu Ala
       1055                 1060                 1065
   Ala Met  Asn Ile Lys Val Lys  Leu Asp Val Val
       1070                 1075
<210> 84
   <211> 346
   <212> PRT
   <213> Homo sapiens
<400> 84
<210> 85
   <211> 346
   <212> PRT
   <213> Homo sapiens
<400> 85
<210> 86
   <211> 133
   <212> PRT
   <213> Homo sapiens
<400> 86
   Met Glu Glu Asp Gln Glu Leu Glu Arg Lys Ile Ser Gly Leu Lys Thr
   1               5                   10                  15
   Ser Met Ala Glu Gly Glu Arg Lys Thr Ala Leu Glu Met Val Gln Ala
               20                  25                  30
   Ala Gly Thr Asp Arg His Cys Val Thr Phe Val Leu His Glu Glu Asp
           35                  40                  45
   His Thr Leu Gly Asn Ser Leu Arg Tyr Met Ile Met Lys Asn Pro Glu
       50                  55                  60
   Val Glu Phe Cys Gly Tyr Thr Thr Thr His Pro Ser Glu Ser Lys Ile
   65                  70                  75                  80
   Asn Leu Arg Ile Gln Thr Arg Gly Thr Leu Pro Ala Val Glu Pro Phe
                   85                  90                  95
   Gln Arg Gly Leu Asn Glu Leu Met Asn Val Cys Gln His Val Leu Asp
               100                 105                 110
   Lys Phe Glu Ala Ser Ile Lys Asp Tyr Lys Asp Gln Lys Ala Ser Arg
           115                 120                 125
   Asn Glu Ser Thr Phe
       130
<210> 87
   <211> 1970
   <212> PRT
   <213> Homo sapiens
<400> 87
<210> 88
   <211> 1970
   <212> PRT
   <213> Homo sapiens
<400> 88
<210> 89
   <211> 1174
   <212> PRT
   <213> Homo sapiens
<400> 89
   Met Tyr Asp Ala Asp Glu Asp Met Gln Tyr Asp Glu Asp Asp Asp Glu
   1               5                   10                  15
   Ile Thr Pro Asp Leu Trp Gln Glu Ala Cys Trp Ile Val Ile Ser Ser
               20                  25                  30
   Tyr Phe Asp Glu Lys Gly Leu Val Arg Gln Gln Leu Asp Ser Phe Asp
           35                  40                  45
   Glu Phe Ile Gln Met Ser Val Gln Arg Ile Val Glu Asp Ala Pro Pro
       50                  55                  60
   Ile Asp Leu Gln Ala Glu Ala Gln His Ala Ser Gly Glu Val Glu Glu
   65                  70                  75                  80
   Pro Pro Arg Tyr Leu Leu Lys Phe Glu Gln Ile Tyr Leu Ser Lys Pro
                   85                  90                  95
   Thr His Trp Glu Arg Asp Gly Ala Pro Ser Pro Met Met Pro Asn Glu
               100                 105                 110
   Ala Arg Leu Arg Asn Leu Thr Tyr Ser Ala Pro Leu Tyr Val Asp Ile
           115                 120                 125
   Thr Lys Thr Val Ile Lys Glu Gly Glu Glu Gln Leu Gln Thr Gln His
       130                 135                 140
   Gln Lys Thr Phe Ile Gly Lys Ile Pro Ile Met Leu Arg Ser Thr Tyr
   145                 150                 155                 160
   Cys Leu Leu Asn Gly Leu Thr Asp Arg Asp Leu Cys Glu Leu Asn Glu
                   165                 170                 175
   Cys Pro Leu Asp Pro Gly Gly Tyr Phe Ile Ile Asn Gly Ser Glu Lys
               180                 185                 190
   Val Leu Ile Ala Gln Glu Lys Met Ala Thr Asn Thr Val Tyr Val Phe
           195                 200                 205
   Ala Lys Lys Asp Ser Lys Tyr Ala Tyr Thr Gly Glu Cys Arg Ser Cys
       210                 215                 220
   Leu Glu Asn Ser Ser Arg Pro Thr Ser Thr Ile Trp Val Ser Met Leu
   225                 230                 235                 240
   Ala Arg Gly Gly Gln Gly Ala Lys Lys Ser Ala Ile Gly Gln Arg Ile
                   245                 250                 255
   Val Ala Thr Leu Pro Tyr Ile Lys Gln Glu Val Pro Ile Ile Ile Val
               260                 265                 270
   Phe Arg Ala Leu Gly Phe Val Ser Asp Arg Asp Ile Leu Glu His Ile
           275                 280                 285
   Ile Tyr Asp Phe Glu Asp Pro Glu Met Met Glu Met Val Lys Pro Ser
       290                 295                 300
   Leu Asp Glu Ala Phe Val Ile Gln Glu Gln Asn Val Ala Leu Asn Phe
   305                 310                 315                 320
   Ile Gly Ser Arg Gly Ala Lys Pro Gly Val Thr Lys Glu Lys Arg Ile
                   325                 330                 335
   Lys Tyr Ala Lys Glu Val Leu Gln Lys Glu Met Leu Pro His Val Gly
               340                 345                 350
   Val Ser Asp Phe Cys Glu Thr Lys Lys Ala Tyr Phe Leu Gly Tyr Met
           355                 360                 365
   Val His Arg Leu Leu Leu Ala Ala Leu Gly Arg Arg Glu Leu Asp Asp
       370                 375                 380
   Arg Asp His Tyr Gly Asn Lys Arg Leu Asp Leu Ala Gly Pro Leu Leu
   385                 390                 395                 400
   Ala Phe Leu Phe Arg Gly Met Phe Lys Asn Leu Leu Lys Glu Val Arg
                   405                 410                 415
   Ile Tyr Ala Gln Lys Phe Ile Asp Arg Gly Lys Asp Phe Asn Leu Glu
               420                 425                 430
   Leu Ala Ile Lys Thr Arg Ile Ile Ser Asp Gly Leu Lys Tyr Ser Leu
           435                 440                 445
   Ala Thr Gly Asn Trp Gly Asp Gln Lys Lys Ala His Gln Ala Arg Ala
       450                 455                 460
   Gly Val Ser Gln Val Leu Asn Arg Leu Thr Phe Ala Ser Thr Leu Ser
   465 470 475 480
   His Leu Arg Arg Leu Asn Ser Pro Ile Gly Arg Asp Gly Lys Leu Ala
   485 490 495
   Lys Pro Arg Gln Leu His Asn Thr Leu Trp Gly Met Val Cys Pro Ala
   500 505 510
   Glu Thr Pro Glu Gly His Ala Val Gly Leu Val Lys Asn Leu Ala Leu
   515 520 525
   Met Ala Tyr Ile Ser Val Gly Ser Gln Pro Ser Pro Ile Leu Glu Phe
   530 535 540
   Leu Glu Glu Trp Ser Met Glu Asn Leu Glu Glu Ile Ser Pro Ala Ala
   545 550 555 560
   Ile Ala Asp Ala Thr Lys Ile Phe Val Asn Gly Cys Trp Val Gly Ile
   565 570 575
   His Lys Asp Pro Glu Gln Leu Met Asn Thr Leu Arg Lys Leu Arg Arg
   580 585 590
   Gln Met Asp Ile Ile Val Ser Glu Val Ser Met Ile Arg Asp Ile Arg
   595 600 605
   Glu Arg Glu Ile Arg Ile Tyr Thr Asp Ala Gly Arg Ile Cys Arg Pro
   610 615 620
   Leu Leu Ile Val Glu Lys Gln Lys Leu Leu Leu Lys Lys Arg His Ile
   625 630 635 640
   Asp Gln Leu Lys Glu Arg Glu Tyr Asn Asn Tyr Ser Trp Gln Asp Leu
   645 650 655
   Val Ala Ser Gly Val Val Glu Tyr Ile Asp Thr Leu Glu Glu Glu Thr
   660 665 670
   Val Met Leu Ala Met Thr Pro Asp Asp Leu Gln Glu Lys Glu Val Ala
   675 680 685
   Tyr Cys Ser Thr Tyr Thr His Cys Glu Ile His Pro Ser Met Ile Leu
   690 695 700
   Gly Val Cys Ala Ser Ile Ile Pro Phe Pro Asp His Asn Gln Ser Pro
   705 710 715 720
   Arg Asn Thr Tyr Gln Ser Ala Met Gly Lys Gln Ala Met Gly Val Tyr
                   725                 730                 735
   Ile Thr Asn Phe His Val Arg Met Asp Thr Leu Ala His Val Leu Tyr
               740                 745                 750
   Tyr Pro Gln Lys Pro Leu Val Thr Thr Arg Ser Met Glu Tyr Leu Arg
           755                 760                 765
   Phe Arg Glu Leu Pro Ala Gly Ile Asn Ser Ile Val Ala Ile Ala Ser
       770                 775                 780
   Tyr Thr Gly Tyr Asn Gln Glu Asp Ser Val Ile Met Asn Arg Ser Ala
   785                 790                 795                 800
   Val Asp Arg Gly Phe Phe Arg Ser Val Phe Tyr Arg Ser Tyr Lys Glu
                   805                 810                 815
   Gln Glu Ser Lys Lys Gly Phe Asp Gln Glu Glu Val Phe Glu Lys Pro
               820                 825                 830
   Thr Arg Glu Thr Cys Gln Gly Met Arg His Ala Ile Tyr Asp Lys Leu
           835                 840                 845
   Asp Asp Asp Gly Leu Ile Ala Pro Gly Val Arg Val Ser Gly Asp Asp
       850                 855                 860
   Val Ile Ile Gly Lys Thr Val Thr Leu Pro Glu Asn Glu Asp Glu Leu
   865                 870                 875                 880
   Glu Ser Thr Asn Arg Arg Tyr Thr Lys Arg Asp Cys Ser Thr Phe Leu
                   885                 890                 895
   Arg Thr Ser Glu Thr Gly Ile Val Asp Gln Val Met Val Thr Leu Asn
               900                 905                 910
   Gln Glu Gly Tyr Lys Phe Cys Lys Ile Arg Val Arg Ser Val Arg Ile
           915                 920                 925
   Pro Gln Ile Gly Asp Lys Phe Ala Ser Arg His Gly Gln Lys Gly Thr
       930                 935                 940
   Cys Gly Ile Gln Tyr Arg Gln Glu Asp Met Pro Phe Thr Cys Glu Gly
   945                 950                 955                 960
   Ile Thr Pro Asp Ile Ile Ile Asn Pro His Ala Ile Pro Ser Arg Met
                   965                 970                 975
   Thr Ile Gly His Leu Ile Glu Cys Leu Gln Gly Lys Val Ser Ala Asn
   980 985 990
   Lys Gly Glu Ile Gly Asp Ala Thr Pro Phe Asn Asp Ala Val Asn Val
   995 1000 1005
   Gln Lys Ile Ser Asn Leu Leu Ser Asp Tyr Gly Tyr His Leu Arg
   1010 1015 1020
   Gly Asn Glu Val Leu Tyr Asn Gly Phe Thr Gly Arg Lys Ile Thr
   1025 1030 1035
   Ser Gln Ile Phe Ile Gly Pro Thr Tyr Tyr Gln Arg Leu Lys His
   1040 1045 1050
   Met Val Asp Asp Lys Ile His Ser Arg Ala Arg Gly Pro Ile Gln
   1055 1060 1065
   Ile Leu Asn Arg Gln Pro Met Glu Gly Arg Ser Arg Asp Gly Gly
   1070 1075 1080
   Leu Arg Phe Gly Glu Met Glu Arg Asp Cys Gln Ile Ala His Gly
   1085 1090 1095
   Ala Ala Gln Phe Leu Arg Glu Arg Leu Phe Glu Ala Ser Asp Pro
   1100 1105 1110
   Tyr Gln Val His Val Cys Asn Leu Cys Gly Ile Met Ala Ile Ala
   1115 1120 1125
   Asn Thr Arg Thr His Thr Tyr Glu Cys Arg Gly Cys Arg Asn Lys
   1130 1135 1140
   Thr Gln Ile Ser Leu Val Arg Met Pro Tyr Ala Cys Lys Leu Leu
   1145 1150 1155
   Phe Gln Glu Leu Met Ser Met Ser Ile Ala Pro Arg Met Met Ser
   1160 1165 1170
   Val
<210> 90
   <211> 275
   <212> PRT
   <213> Homo sapiens
<400> 90
   Met Pro Tyr Ala Asn Gln Pro Thr Val Arg Ile Thr Glu Leu Thr Asp
   1               5                   10                  15
   Glu Asn Val Lys Phe Ile Ile Glu Asn Thr Asp Leu Ala Val Ala Asn
               20                  25                  30
   Ser Ile Arg Arg Val Phe Ile Ala Glu Val Pro Ile Ile Ala Ile Asp
           35                  40                  45
   Trp Val Gln Ile Asp Ala Asn Ser Ser Val Leu His Asp Glu Phe Ile
       50                  55                  60
   Ala His Arg Leu Gly Leu Ile Pro Leu Ile Ser Asp Asp Ile Val Asp
   65                  70                  75                  80
   Lys Leu Gln Tyr Ser Arg Asp Cys Thr Cys Glu Glu Phe Cys Pro Glu
                   85                  90                  95
   Cys Ser Val Glu Phe Thr Leu Asp Val Arg Cys Asn Glu Asp Gln Thr
               100                 105                 110
   Arg His Val Thr Ser Arg Asp Leu Ile Ser Asn Ser Pro Arg Val Ile
           115                 120                 125
   Pro Val Thr Ser Arg Asn Arg Asp Asn Asp Pro Asn Asp Tyr Val Glu
       130                 135                 140
   Gln Asp Asp Ile Leu Ile Val Lys Leu Arg Lys Gly Gln Glu Leu Arg
   145                 150                 155                 160
   Leu Arg Ala Tyr Ala Lys Lys Gly Phe Gly Lys Glu His Ala Lys Trp
                   165                 170                 175
   Asn Pro Thr Ala Gly Val Ala Phe Glu Tyr Asp Pro Asp Asn Ala Leu
               180                 185                 190
   Arg His Thr Val Tyr Pro Lys Pro Glu Glu Trp Pro Lys Ser Glu Tyr
           195                 200                 205
   Ser Glu Leu Asp Glu Asp Glu Ser Gln Ala Pro Tyr Asp Pro Asn Gly
       210                 215                 220
   Lys Pro Glu Arg Phe Tyr Tyr Asn Val Glu Ser Cys Gly Ser Leu Arg
   225                 230                 235                 240
   Pro Glu Thr Ile Val Leu Ser Ala Leu Ser Gly Leu Lys Lys Lys Leu
   245 250 255
   Ser Asp Leu Gln Thr Gln Leu Ser His Glu Ile Gln Ser Asp Val Leu
   260 265 270
   Thr Ile Asn
   275
<210> 91
   <211> 275
   <212> PRT
   <213> Homo sapiens
<400> 91
   Met Pro Tyr Ala Asn Gln Pro Thr Val Arg Ile Thr Glu Leu Thr Asp
   1               5                   10                  15
   Glu Asn Val Lys Phe Ile Ile Glu Asn Thr Asp Leu Ala Val Ala Asn
               20                  25                  30
   Ser Ile Arg Arg Val Phe Ile Ala Glu Val Pro Ile Ile Ala Ile Asp
           35                  40                  45
   Trp Val Gln Ile Asp Ala Asn Ser Ser Val Leu His Asp Glu Phe Ile
       50                  55                  60
   Ala His Arg Leu Gly Leu Ile Pro Leu Ile Ser Asp Asp Ile Val Asp
   65                  70                  75                  80
   Lys Leu Gln Tyr Ser Arg Asp Cys Thr Cys Glu Glu Phe Cys Pro Glu
                   85                  90                  95
   Cys Ser Val Glu Phe Thr Leu Asp Val Arg Cys Asn Glu Asp Gln Thr
               100                 105                 110
   Arg His Val Thr Ser Arg Asp Leu Ile Ser Asn Ser Pro Arg Val Ile
           115                 120                 125
   Pro Val Thr Ser Arg Asn Arg Asp Asn Asp Pro Asn Asp Tyr Val Glu
       130                 135                 140
   Gln Asp Asp Ile Leu Ile Val Lys Leu Arg Lys Gly Gln Glu Leu Arg
   145                 150                 155                 160
   Leu Arg Ala Tyr Ala Lys Lys Gly Phe Gly Lys Glu His Ala Lys Trp
                   165                 170                 175
   Asn Pro Thr Ala Gly Val Ala Phe Glu Tyr Asp Pro Asp Asn Ala Leu
   180 185 190
   Arg His Thr Val Tyr Pro Lys Pro Glu Glu Trp Pro Lys Ser Glu Tyr
   195 200 205
   Ser Glu Leu Asp Glu Asp Glu Ser Gln Ala Pro Tyr Asp Pro Asn Gly
   210 215 220
   Lys Pro Glu Arg Phe Tyr Tyr Asn Val Glu Ser Cys Gly Ser Leu Arg
   225 230 235 240
   Pro Glu Thr Ile Val Leu Ser Ala Leu Ser Gly Leu Lys Lys Lys Leu
   245 250 255
   Ser Asp Leu Gln Thr Gln Leu Ser His Glu Ile Gln Ser Asp Val Leu
   260 265 270
   Thr Ile Asn
   275
<210> 92
   <211> 275
   <212> PRT
   <213> Homo sapiens
<400> 92
   Met Pro Tyr Ala Asn Gln Pro Thr Val Arg Ile Thr Glu Leu Thr Asp
   1               5                   10                  15
   Glu Asn Val Lys Phe Ile Ile Glu Asn Thr Asp Leu Ala Val Ala Asn
               20                  25                  30
   Ser Ile Arg Arg Val Phe Ile Ala Glu Val Pro Ile Ile Ala Ile Asp
           35                  40                  45
   Trp Val Gln Ile Asp Ala Asn Ser Ser Val Leu His Asp Glu Phe Ile
       50                  55                  60
   Ala His Arg Leu Gly Leu Ile Pro Leu Ile Ser Asp Asp Ile Val Asp
   65                  70                  75                  80
   Lys Leu Gln Tyr Ser Arg Asp Cys Thr Cys Glu Glu Phe Cys Pro Glu
                   85                  90                  95
   Cys Ser Val Glu Phe Thr Leu Asp Val Arg Cys Asn Glu Asp Gln Thr
               100                 105                 110
   Arg His Val Thr Ser Arg Asp Leu Ile Ser Asn Ser Pro Arg Val Ile
   115 120 125
   Pro Val Thr Ser Arg Asn Arg Asp Asn Asp Pro Asn Asp Tyr Val Glu
   130 135 140
   Gln Asp Asp Ile Leu Ile Val Lys Leu Arg Lys Gly Gln Glu Leu Arg
   145 150 155 160
   Leu Arg Ala Tyr Ala Lys Lys Gly Phe Gly Lys Glu His Ala Lys Trp
   165 170 175
   Asn Pro Thr Ala Gly Val Ala Phe Glu Tyr Asp Pro Asp Asn Ala Leu
   180 185 190
   Arg His Thr Val Tyr Pro Lys Pro Glu Glu Trp Pro Lys Ser Glu Tyr
   195 200 205
   Ser Glu Leu Asp Glu Asp Glu Ser Gln Ala Pro Tyr Asp Pro Asn Gly
   210 215 220
   Lys Pro Glu Arg Phe Tyr Tyr Asn Val Glu Ser Cys Gly Ser Leu Arg
   225 230 235 240
   Pro Glu Thr Ile Val Leu Ser Ala Leu Ser Gly Leu Lys Lys Lys Leu
   245 250 255
   Ser Asp Leu Gln Thr Gln Leu Ser His Glu Ile Gln Ser Asp Val Leu
   260 265 270
   Thr Ile Asn
   275
<210> 93
   <211> 142
   <212> PRT
   <213> Homo sapiens
<400> 93
   Met Ala Ala Gly Gly Ser Asp Pro Arg Ala Gly Asp Val Glu Glu Asp
   1               5                   10                  15
   Ala Ser Gln Leu Ile Phe Pro Lys Glu Phe Glu Thr Ala Glu Thr Leu
               20                  25                  30
   Leu Asn Ser Glu Val His Met Leu Leu Glu His Arg Lys Gln Gln Asn
           35                  40                  45
   Glu Ser Ala Glu Asp Glu Gln Glu Leu Ser Glu Val Phe Met Lys Thr
       50                  55                  60
   Leu Asn Tyr Thr Ala Arg Phe Ser Arg Phe Lys Asn Arg Glu Thr Ile
   65                  70                  75                  80
   Ala Ser Val Arg Ser Leu Leu Leu Gln Lys Lys Leu His Lys Phe Glu
                   85                  90                  95
   Leu Ala Cys Leu Ala Asn Leu Cys Pro Glu Thr Ala Glu Glu Ser Lys
               100                 105                 110
   Ala Leu Ile Pro Ser Leu Glu Gly Arg Phe Glu Asp Glu Glu Leu Gln
           115                 120                 125
   Gln Ile Leu Asp Asp Ile Gln Thr Lys Arg Ser Phe Gln Tyr
       130                 135                 140
<210> 94
   <211> 142
   <212> PRT
   <213> Homo sapiens
<400> 94
   Met Ala Ala Gly Gly Ser Asp Pro Arg Ala Gly Asp Val Glu Glu Asp
   1               5                   10                  15
   Ala Ser Gln Leu Ile Phe Pro Lys Glu Phe Glu Thr Ala Glu Thr Leu
               20                  25                  30
   Leu Asn Ser Glu Val His Met Leu Leu Glu His Arg Lys Gln Gln Asn
           35                  40                  45
   Glu Ser Ala Glu Asp Glu Gln Glu Leu Ser Glu Val Phe Met Lys Thr
       50                  55                  60
   Leu Asn Tyr Thr Ala Arg Phe Ser Arg Phe Lys Asn Arg Glu Thr Ile
   65                  70                  75                  80
   Ala Ser Val Arg Ser Leu Leu Leu Gln Lys Lys Leu His Lys Phe Glu
                   85                  90                  95
   Leu Ala Cys Leu Ala Asn Leu Cys Pro Glu Thr Ala Glu Glu Ser Lys
               100                 105                 110
   Ala Leu Ile Pro Ser Leu Glu Gly Arg Phe Glu Asp Glu Glu Leu Gln
           115                 120                 125
   Gln Ile Leu Asp Asp Ile Gln Thr Lys Arg Ser Phe Gln Tyr
             130                 135                 140
<210> 95
   <211> 210
   <212> PRT
   <213> Homo sapiens
<400> 95
   Met Asp Asp Glu Glu Glu Thr Tyr Arg Leu Trp Lys Ile Arg Lys Thr
   1               5                   10                  15
   Ile Met Gln Leu Cys His Asp Arg Gly Tyr Leu Val Thr Gln Asp Glu
               20                  25                  30
   Leu Asp Gln Thr Leu Glu Glu Phe Lys Ala Gln Phe Gly Asp Lys Pro
           35                  40                  45
   Ser Glu Gly Arg Pro Arg Arg Thr Asp Leu Thr Val Leu Val Ala His
       50                  55                  60
   Asn Asp Asp Pro Thr Asp Gln Met Phe Val Phe Phe Pro Glu Glu Pro
   65                  70                  75                  80
   Lys Val Gly Ile Lys Thr Ile Lys Val Tyr Cys Gln Arg Met Gln Glu
                   85                  90                  95
   Glu Asn Ile Thr Arg Ala Leu Ile Val Val Gln Gln Gly Met Thr Pro
               100                 105                 110
   Ser Ala Lys Gln Ser Leu Val Asp Met Ala Pro Lys Tyr Ile Leu Glu
           115                 120                 125
   Gln Phe Leu Gln Gln Glu Leu Leu Ile Asn Ile Thr Glu His Glu Leu
       130                 135                 140
   Val Pro Glu His Val Val Met Thr Lys Glu Glu Val Thr Glu Leu Leu
   145                 150                 155                 160
   Ala Arg Tyr Lys Leu Arg Glu Asn Gln Leu Pro Arg Ile Gln Ala Gly
                   165                 170                 175
   Asp Pro Val Ala Arg Tyr Phe Gly Ile Lys Arg Gly Gln Val Val Lys
               180                 185                 190
   Ile Ile Arg Pro Ser Glu Thr Ala Gly Arg Tyr Ile Thr Tyr Arg Leu
           195                 200                 205
   Val Gln
   210
<210> 96
   <211> 210
   <212> PRT
   <213> Homo sapiens
<400> 96
   Met Asp Asp Glu Glu Glu Thr Tyr Arg Leu Trp Lys Ile Arg Lys Thr
   1               5                   10                  15
   Ile Met Gln Leu Cys His Asp Arg Gly Tyr Leu Val Thr Gln Asp Glu
               20                  25                  30
   Leu Asp Gln Thr Leu Glu Glu Phe Lys Ala Gln Phe Gly Asp Lys Pro
           35                  40                  45
   Ser Glu Gly Arg Pro Arg Arg Thr Asp Leu Thr Val Leu Val Ala His
       50                  55                  60
   Asn Asp Asp Pro Thr Asp Gln Met Phe Val Phe Phe Pro Glu Glu Pro
   65                  70                  75                  80
   Lys Val Gly Ile Lys Thr Ile Lys Val Tyr Cys Gln Arg Met Gln Glu
                   85                  90                  95
   Glu Asn Ile Thr Arg Ala Leu Ile Val Val Gln Gln Gly Met Thr Pro
               100                 105                 110
   Ser Ala Lys Gln Ser Leu Val Asp Met Ala Pro Lys Tyr Ile Leu Glu
           115                 120                 125
   Gln Phe Leu Gln Gln Glu Leu Leu Ile Asn Ile Thr Glu His Glu Leu
       130                 135                 140
   Val Pro Glu His Val Val Met Thr Lys Glu Glu Val Thr Glu Leu Leu
   145                 150                 155                 160
   Ala Arg Tyr Lys Leu Arg Glu Asn Gln Leu Pro Arg Ile Gln Ala Gly
                   165                 170                 175
   Asp Pro Val Ala Arg Tyr Phe Gly Ile Lys Arg Gly Gln Val Val Lys
               180                 185                 190
   Ile Ile Arg Pro Ser Glu Thr Ala Gly Arg Tyr Ile Thr Tyr Arg Leu
           195                 200                 205
   Val Gln
   210
<210> 97
   <211> 127
   <212> PRT
   <213> Homo sapiens
<400> 97
   Met Ser Asp Asn Glu Asp Asn Phe Asp Gly Asp Asp Phe Asp Asp Val
   1               5                   10                  15
   Glu Glu Asp Glu Gly Leu Asp Asp Leu Glu Asn Ala Glu Glu Glu Gly
               20                  25                  30
   Gln Glu Asn Val Glu Ile Leu Pro Ser Gly Glu Arg Pro Gln Ala Asn
           35                  40                  45
   Gln Lys Arg Ile Thr Thr Pro Tyr Met Thr Lys Tyr Glu Arg Ala Arg
       50                  55                  60
   Val Leu Gly Thr Arg Ala Leu Gln Ile Ala Met Cys Ala Pro Val Met
   65                  70                  75                  80
   Val Glu Leu Glu Gly Glu Thr Asp Pro Leu Leu Ile Ala Met Lys Glu
                   85                  90                  95
   Leu Lys Ala Arg Lys Ile Pro Ile Ile Ile Arg Arg Tyr Leu Pro Asp
               100                 105                 110
   Gly Ser Tyr Glu Asp Trp Gly Val Asp Glu Leu Ile Ile Thr Asp
           115                 120                 125
<210> 98
   <211> 172
   <212> PRT
   <213> Homo sapiens
<400> 98
   Met Phe Tyr His Ile Ser Leu Glu His Glu Ile Leu Leu His Pro Arg
   1               5                   10                  15
   Tyr Phe Gly Pro Asn Leu Leu Asn Thr Val Lys Gln Lys Leu Phe Thr
               20                  25                  30
   Glu Val Glu Gly Thr Cys Thr Gly Lys Tyr Gly Phe Val Ile Ala Val
           35                  40                  45
   Thr Thr Ile Asp Asn Ile Gly Ala Gly Val Ile Gln Pro Gly Arg Gly
       50                  55                  60
   Phe Val Leu Tyr Pro Val Lys Tyr Lys Ala Ile Val Phe Arg Pro Phe
   65 70 75 80
   Lys Gly Glu Val Val Asp Ala Val Val Thr Gln Val Asn Lys Val Gly
   85 90 95
   Leu Phe Thr Glu Ile Gly Pro Met Ser Cys Phe Ile Ser Arg His Ser
   100 105 110
   Ile Pro Ser Glu Met Glu Phe Asp Pro Asn Ser Asn Pro Pro Cys Tyr
   115 120 125
   Lys Thr Met Asp Glu Asp Ile Val Ile Gln Gln Asp Asp Glu Ile Arg
   130 135 140
   Leu Lys Ile Val Gly Thr Arg Val Asp Lys Asn Asp Ile Phe Ala Ile
   145 150 155 160
   Gly Ser Leu Met Asp Asp Tyr Leu Gly Leu Val Ser
   165 170
<210> 99
   <211> 150
   <212> PRT
   <213> Homo sapiens
<400> 99
   Met Ala Gly Ile Leu Phe Glu Asp Ile Phe Asp Val Lys Asp Ile Asp
   1 5 10 15
   Pro Glu Gly Lys Lys Phe Asp Arg Val Ser Arg Leu His Cys Glu Ser
   20 25 30
   Glu Ser Phe Lys Met Asp Leu Ile Leu Asp Val Asn Ile Gln Ile Tyr
   35 40 45
   Pro Val Asp Leu Gly Asp Lys Phe Arg Leu Val Ile Ala Ser Thr Leu
   50 55 60
   Tyr Glu Asp Gly Thr Leu Asp Asp Gly Glu Tyr Asn Pro Thr Asp Asp
   65 70 75 80
   Arg Pro Ser Arg Ala Asp Gln Phe Glu Tyr Val Met Tyr Gly Lys Val
   85 90 95
   Tyr Arg Ile Glu Gly Asp Glu Thr Ser Thr Glu Ala Ala Thr Arg Leu
   100 105 110
   Ser Ala Tyr Val Ser Tyr Gly Gly Leu Leu Met Arg Leu Gln Gly Asp
   115 120 125
   Ala Asn Asn Leu His Gly Phe Glu Val Asp Ser Arg Val Tyr Leu Leu
   130 135 140
   Met Lys Lys Leu Ala Phe
   145 150
<210> 100
   <211> 125
   <212> PRT
   <213> Homo sapiens
<400> 100
   Met Glu Pro Asp Gly Thr Tyr Glu Pro Gly Phe Val Gly Ile Arg Phe
   1 5 10 15
   Cys Gln Glu Cys Asn Asn Met Leu Tyr Pro Lys Glu Asp Lys Glu Asn
   20 25 30
   Arg Ile Leu Leu Tyr Ala Cys Arg Asn Cys Asp Tyr Gln Gln Glu Ala
   35 40 45
   Asp Asn Ser Cys Ile Tyr Val Asn Lys Ile Thr His Glu Val Asp Glu
   50 55 60
   Leu Thr Gln Ile Ile Ala Asp Val Ser Gln Asp Pro Thr Leu Pro Arg
   65 70 75 80
   Thr Glu Asp His Pro Cys Gln Lys Cys Gly His Lys Glu Ala Val Phe
   85 90 95
   Phe Gln Ser His Ser Ala Arg Ala Glu Asp Ala Met Arg Leu Tyr Tyr
   100 105 110
   Val Cys Thr Ala Pro His Cys Gly His Arg Trp Thr Glu
   115 120 125
<210> 101
   <211> 117
   <212> PRT
   <213> Homo sapiens
<400> 101
   Met Asn Ala Pro Pro Ala Phe Glu Ser Phe Leu Leu Phe Glu Gly Glu
   1 5 10 15
   Lys Lys Ile Thr Ile Asn Lys Asp Thr Lys Val Pro Asn Ala Cys Leu
   20 25 30
   Phe Thr Ile Asn Lys Glu Asp His Thr Leu Gly Asn Ile Ile Lys Ser
   35 40 45
   Gln Leu Leu Lys Asp Pro Gln Val Leu Phe Ala Gly Tyr Lys Val Pro
   50 55 60
   His Pro Leu Glu His Lys Ile Ile Ile Arg Val Gln Thr Thr Pro Asp
   65 70 75 80
   Tyr Ser Pro Gln Glu Ala Phe Thr Asn Ala Ile Thr Asp Leu Ile Ser
   85 90 95
   Glu Leu Ser Leu Leu Glu Glu Arg Phe Arg Val Ala Ile Lys Asp Lys
   100 105 110
   Gln Glu Gly Ile Glu
   115
<210> 102
   <211> 115
   <212> PRT
   <213> Homo sapiens
<400> 102
   Met Asn Ala Pro Pro Ala Phe Glu Ser Phe Leu Leu Phe Glu Gly Glu
   1 5 10 15
   Lys Ile Thr Ile Asn Lys Asp Thr Lys Val Pro Lys Ala Cys Leu Phe
   20 25 30
   Thr Ile Asn Lys Glu Asp His Thr Leu Gly Asn Ile Ile Lys Ser Gln
   35 40 45
   Leu Leu Lys Asp Pro Gln Val Leu Phe Ala Gly Tyr Lys Val Pro His
   50 55 60
   Pro Leu Glu His Lys Ile Ile Ile Arg Val Gln Thr Thr Pro Asp Tyr
   65 70 75 80
   Ser Pro Gln Glu Ala Phe Thr Asn Ala Ile Thr Asp Leu Ile Ser Glu
   85 90 95
   Leu Ser Leu Leu Glu Glu Arg Phe Arg Thr Cys Leu Leu Pro Leu Arg
   100 105 110
   Leu Leu Pro
           115
<210> 103
   <211> 115
   <212> PRT
   <213> Homo sapiens
<400> 103
   Met Asn Ala Pro Pro Ala Phe Glu Ser Phe Leu Leu Phe Glu Gly Glu
   1               5                   10                  15
   Lys Ile Thr Ile Asn Lys Asp Thr Lys Val Pro Asn Ala Cys Leu Phe
               20                  25                  30
   Thr Ile Asn Lys Glu Asp His Thr Leu Gly Asn Ile Ile Lys Ser Gln
           35                  40                  45
   Leu Leu Lys Asp Pro Gln Val Leu Phe Ala Gly Tyr Lys Val Pro His
       50                  55                  60
   Pro Leu Glu His Lys Ile Ile Ile Arg Val Gln Thr Thr Pro Asp Tyr
   65                  70                  75                  80
   Ser Pro Gln Glu Ala Phe Thr Asn Ala Ile Thr Asp Leu Ile Ser Glu
                   85                  90                  95
   Leu Ser Leu Leu Glu Glu Arg Phe Arg Thr Cys Leu Leu Pro Leu Arg
               100                 105                 110
   Leu Leu Pro
           115
<210> 104
   <211> 58
   <212> PRT
   <213> Homo sapiens
<400> 104
   Met Asp Thr Gln Lys Asp Val Gln Pro Pro Lys Gln Gln Pro Met Ile
   1               5                   10                  15
   Tyr Ile Cys Gly Glu Cys His Thr Glu Asn Glu Ile Lys Ser Arg Asp
               20                  25                  30
   Pro Ile Arg Cys Arg Glu Cys Gly Tyr Arg Ile Met Tyr Lys Lys Arg
           35                  40                  45
   Thr Lys Arg Leu Val Val Phe Asp Ala Arg
   50 55
<210> 105
   <211> 58
   <212> PRT
   <213> Homo sapiens
<400> 105
   Met Asp Thr Gln Lys Asp Val Gln Pro Pro Lys Gln Gln Pro Met Ile
   1 5 10 15
   Tyr Ile Cys Gly Glu Cys His Thr Glu Asn Glu Ile Lys Ser Arg Asp
   20 25 30
   Pro Ile Arg Cys Arg Glu Cys Gly Tyr Arg Ile Met Tyr Lys Lys Arg
   35 40 45
   Thr Lys Arg Leu Val Val Phe Asp Ala Arg
   50 55
<210> 106
   <211> 67
   <212> PRT
   <213> Homo sapiens
<400> 106
   Met Ile Ile Pro Val Arg Cys Phe Thr Cys Gly Lys Ile Val Gly Asn
   1 5 10 15
   Lys Trp Glu Ala Tyr Leu Gly Leu Leu Gln Ala Glu Tyr Thr Glu Gly
   20 25 30
   Asp Ala Leu Asp Ala Leu Gly Leu Lys Arg Tyr Cys Cys Arg Arg Met
   35 40 45
   Leu Leu Ala His Val Asp Leu Ile Glu Lys Leu Leu Asn Tyr Ala Pro
   50 55 60
   Leu Glu Lys
   65
<210> 107
   <211> 67
   <212> PRT
   <213> Homo sapiens
<400> 107
   Met Ile Ile Pro Val Arg Cys Phe Thr Cys Gly Lys Ile Val Gly Asn
   1               5                   10                  15
   Lys Trp Glu Ala Tyr Leu Gly Leu Leu Gln Ala Glu Tyr Thr Glu Gly
               20                  25                  30
   Asp Ala Leu Asp Ala Leu Gly Leu Lys Arg Tyr Cys Cys Arg Arg Met
           35                  40                  45
   Leu Leu Ala His Val Asp Leu Ile Glu Lys Leu Leu Asn Tyr Ala Pro
       50                  55                  60
   Leu Glu Lys
   65
<210> 108
   <211> 1075
   <212> PRT
   <213> Homo sapiens
<400> 108
   Met Lys Ala Asn Glu Lys Val Thr Ser Asp Ala Asp Pro Met Trp Tyr
   1               5                   10                  15
   Leu Lys Tyr Leu Asn Ile Tyr Val Gly Leu Pro Asp Val Glu Glu Ser
               20                  25                  30
   Phe Asn Val Thr Arg Pro Val Ser Pro His Glu Cys Arg Leu Arg Asp
           35                  40                  45
   Met Thr Tyr Ser Ala Pro Ile Thr Val Asp Ile Glu Tyr Thr Arg Gly
       50                  55                  60
   Ser Gln Arg Ile Ile Arg Asn Ala Leu Pro Ile Gly Arg Met Pro Ile
   65                  70                  75                  80
   Met Leu Arg Ser Ser Asn Cys Val Leu Thr Gly Lys Thr Pro Ala Glu
                   85                  90                  95
   Phe Ala Lys Leu Asn Glu Cys Pro Leu Asp Pro Gly Gly Tyr Phe Ile
               100                 105                 110
   Val Lys Gly Val Glu Lys Val Ile Leu Ile Gln Glu Gln Leu Ser Lys
           115                 120                 125
   Asn Arg Ile Ile Val Glu Ala Asp Arg Lys Gly Ala Val Gly Ala Ser
       130                 135                 140
   Val Thr Ser Ser Thr His Glu Lys Lys Ser Arg Thr Asn Met Ala Val
   145                 150                 155                 160
   Lys Gln Gly Arg Phe Tyr Leu Arg His Asn Thr Leu Ser Glu Asp Ile
                   165                 170                 175
   Pro Ile Val Ile Ile Phe Lys Ala Met Gly Val Glu Ser Asp Gln Glu
               180                 185                 190
   Ile Val Gln Met Ile Gly Thr Glu Glu His Val Met Ala Ala Phe Gly
           195                 200                 205
   Pro Ser Leu Glu Glu Cys Gln Lys Ala Gln Ile Phe Thr Gln Met Gln
       210                 215                 220
   Ala Leu Lys Tyr Ile Gly Asn Lys Val Arg Arg Gln Arg Met Trp Gly
   225                 230                 235                 240
   Gly Gly Pro Lys Lys Thr Lys Ile Glu Glu Ala Arg Glu Leu Leu Ala
                   245                 250                 255
   Ser Thr Ile Leu Thr His Val Pro Val Lys Glu Phe Asn Phe Arg Ala
               260                 265                 270
   Lys Cys Ile Tyr Thr Ala Val Met Val Arg Arg Val Ile Leu Ala Gln
           275                 280                 285
   Gly Asp Asn Lys Val Asp Asp Arg Asp Tyr Tyr Gly Asn Lys Arg Leu
       290                 295                 300
   Glu Leu Ala Gly Gln Leu Leu Ser Leu Leu Phe Glu Asp Leu Phe Lys
   305                 310                 315                 320
   Lys Phe Asn Ser Glu Met Lys Lys Ile Ala Asp Gln Val Ile Pro Lys
                   325                 330                 335
   Gln Arg Ala Ala Gln Phe Asp Val Val Lys His Met Arg Gln Asp Gln
               340                 345                 350
   Ile Thr Asn Gly Met Val Asn Ala Ile Ser Thr Gly Asn Trp Ser Leu
           355                 360                 365
   Lys Arg Phe Lys Met Asp Arg Gln Gly Val Thr Gln Val Leu Ser Arg
       370                 375                 380
   Leu Ser Tyr Ile Ser Ala Leu Gly Met Met Thr Arg Ile Ser Ser Gln
   385                 390                 395                 400
   Phe Glu Lys Thr Arg Lys Val Ser Gly Pro Arg Ser Leu Gln Pro Ser
   405 410 415
   Gln Trp Gly Met Leu Cys Pro Ser Asp Thr Pro Glu Gly Glu Ala Cys
   420 425 430
   Gly Leu Val Lys Asn Leu Ala Leu Met Thr His Ile Thr Thr Asp Met
   435 440 445
   Glu Asp Gly Pro Ile Val Lys Leu Ala Ser Asn Leu Gly Val Glu Asp
   450 455 460
   Val Asn Leu Leu Cys Gly Glu Glu Leu Ser Tyr Pro Asn Val Phe Leu
   465 470 475 480
   Val Phe Leu Asn Gly Asn Ile Leu Gly Val Ile Arg Asp His Lys Lys
   485 490 495
   Leu Val Asn Thr Phe Arg Leu Met Arg Arg Ala Gly Tyr Ile Asn Glu
   500 505 510
   Phe Val Ser Ile Ser Thr Asn Leu Thr Asp Arg Cys Val Tyr Ile Ser
   515 520 525
   Ser Asp Gly Gly Arg Leu Cys Arg Pro Tyr Ile Ile Val Lys Lys Gln
   530 535 540
   Lys Pro Ala Val Thr Asn Lys His Met Glu Glu Leu Ala Gln Gly Tyr
   545 550 555 560
   Arg Asn Phe Glu Asp Phe Leu His Glu Ser Leu Val Glu Tyr Leu Asp
   565 570 575
   Val Asn Glu Glu Asn Asp Cys Asn Ile Ala Leu Tyr Glu His Thr Ile
   580 585 590
   Asn Lys Asp Thr Thr His Leu Glu Ile Glu Pro Phe Thr Leu Leu Gly
   595 600 605
   Val Cys Ala Gly Leu Ile Pro Tyr Pro His His Asn Gln Ser Pro Arg
   610 615 620
   Asn Thr Tyr Gln Cys Ala Met Gly Lys Gln Ala Met Gly Thr Ile Gly
   625 630 635 640
   Tyr Asn Gln Arg Asn Arg Ile Asp Thr Leu Met Tyr Leu Leu Ala Tyr
   645 650 655
   Pro Gln Lys Pro Met Val Lys Thr Lys Thr Ile Glu Leu Ile Glu Phe
               660                 665                 670
   Glu Lys Leu Pro Ala Gly Gln Asn Ala Thr Val Ala Val Met Ser Tyr
           675                 680                 685
   Ser Gly Tyr Asp Ile Glu Asp Ala Leu Val Leu Asn Lys Ala Ser Leu
       690                 695                 700
   Asp Arg Gly Phe Gly Arg Cys Leu Val Tyr Lys Asn Ala Lys Cys Thr
   705                 710                 715                 720
   Leu Lys Arg Tyr Thr Asn Gln Thr Phe Asp Lys Val Met Gly Pro Met
                   725                 730                 735
   Leu Asp Ala Ala Thr Arg Lys Pro Ile Trp Arg His Glu Ile Leu Asp
               740                 745                 750
   Ala Asp Gly Ile Cys Ser Pro Gly Glu Lys Val Glu Asn Lys Gln Val
           755                 760                 765
   Leu Val Asn Lys Ser Met Pro Thr Val Thr Gln Ile Pro Leu Glu Gly
       770                 775                 780
   Ser Asn Val Pro Gln Gln Pro Gln Tyr Lys Asp Val Pro Ile Thr Tyr
   785                 790                 795                 800
   Lys Gly Ala Thr Asp Ser Tyr Ile Glu Lys Val Met Ile Ser Ser Asn
                   805                 810                 815
   Ala Glu Asp Ala Phe Leu Ile Lys Met Leu Leu Arg Gln Thr Arg Arg
               820                 825                 830
   Pro Glu Ile Gly Asp Lys Phe Ser Ser Arg His Gly Gln Lys Gly Val
           835                 840                 845
   Cys Gly Leu Ile Val Pro Gln Glu Asp Met Pro Phe Cys Asp Ser Gly
       850                 855                 860
   Ile Cys Pro Asp Ile Ile Met Asn Pro His Gly Phe Pro Ser Arg Met
   865                 870                 875                 880
   Thr Val Gly Lys Leu Ile Glu Leu Leu Ala Gly Lys Ala Gly Val Leu
                   885                 890                 895
   Asp Gly Arg Phe His Tyr Gly Thr Ala Phe Gly Gly Ser Lys Val Lys
               900                 905                 910
   Asp Val Cys Glu Asp Leu Val Arg His Gly Tyr Asn Tyr Leu Gly Lys
   915 920 925
   Asp Tyr Val Thr Ser Gly Ile Thr Gly Glu Pro Leu Glu Ala Tyr Ile
   930 935 940
   Tyr Phe Gly Pro Val Tyr Tyr Gln Lys Leu Lys His Met Val Leu Asp
   945 950 955 960
   Lys Met His Ala Arg Ala Arg Gly Pro Arg Ala Val Leu Thr Arg Gln
   965 970 975
   Pro Thr Glu Gly Arg Ser Arg Asp Gly Gly Leu Arg Leu Gly Glu Met
   980 985 990
   Glu Arg Asp Cys Leu Ile Gly Tyr Gly Ala Ser Met Leu Leu Leu Glu
   995 1000 1005
   Arg Leu Met Ile Ser Ser Asp Ala Phe Glu Val Asp Val Cys Gly
   1010 1015 1020
   Gln Cys Gly Leu Leu Gly Tyr Ser Gly Trp Cys His Tyr Cys Lys
   1025 1030 1035
   Ser Ser Cys His Val Ser Ser Leu Arg Ile Pro Tyr Ala Cys Lys
   1040 1045 1050
   Leu Leu Phe Gln Glu Leu Gln Ser Met Asn Ile Ile Pro Arg Leu
   1055 1060 1065
   Lys Leu Ser Lys Tyr Asn Glu
   1070 1075
<210> 109
   <211> 534
   <212> PRT
   <213> Homo sapiens
<400> 109
   Met Thr Gln Ala Glu Ile Lys Leu Cys Ser Leu Leu Leu Gln Glu His
   1 5 10 15
   Phe Gly Glu Ile Val Glu Lys Ile Gly Val His Leu Ile Arg Thr Gly
   20 25 30
   Ser Gln Pro Leu Arg Val Ile Ala His Asp Thr Gly Thr Ser Leu Asp
   35 40 45
   Gln Val Lys Lys Ala Leu Cys Val Leu Val Gln His Asn Leu Val Ser
       50                  55                  60
   Tyr Gln Val His Lys Arg Gly Val Val Glu Tyr Glu Ala Gln Cys Ser
   65                  70                  75                  80
   Arg Val Leu Arg Met Leu Arg Tyr Pro Arg Tyr Ile Tyr Thr Thr Lys
                   85                  90                  95
   Thr Leu Tyr Ser Asp Thr Gly Glu Leu Ile Val Glu Glu Leu Leu Leu
               100                 105                 110
   Asn Gly Lys Leu Thr Met Ser Ala Val Val Lys Lys Val Ala Asp Arg
           115                 120                 125
   Leu Thr Glu Thr Met Glu Asp Gly Lys Thr Met Asp Tyr Ala Glu Val
       130                 135                 140
   Ser Asn Thr Phe Val Arg Leu Ala Asp Thr His Phe Val Gln Arg Cys
   145                 150                 155                 160
   Pro Ser Val Pro Thr Thr Glu Asn Ser Asp Pro Gly Pro Pro Pro Pro
                   165                 170                 175
   Ala Pro Thr Leu Val Ile Asn Glu Lys Asp Met Tyr Leu Val Pro Lys
               180                 185                 190
   Leu Ser Leu Ile Gly Lys Gly Lys Arg Arg Arg Ser Ser Asp Glu Asp
           195                 200                 205
   Ala Ala Gly Glu Pro Lys Ala Lys Arg Pro Lys Tyr Thr Thr Asp Asn
       210                 215                 220
   Lys Glu Pro Ile Pro Asp Asp Gly Ile Tyr Trp Gln Ala Asn Leu Asp
   225                 230                 235                 240
   Arg Phe His Gln His Phe Arg Asp Gln Ala Ile Val Ser Ala Val Ala
                   245                 250                 255
   Asn Arg Met Asp Gln Thr Ser Ser Glu Ile Val Arg Thr Met Leu Arg
               260                 265                 270
   Met Ser Glu Ile Thr Thr Ser Ser Ser Ala Pro Phe Thr Gln Pro Leu
           275                 280                 285
   Ser Ser Asn Glu Ile Phe Arg Ser Leu Pro Val Gly Tyr Asn Ile Ser
       290                 295                 300
   Lys Gln Val Leu Asp Gln Tyr Leu Thr Leu Leu Ala Asp Asp Pro Leu
   305                 310                 315                 320
   Glu Phe Val Gly Lys Ser Gly Asp Ser Gly Gly Gly Met Tyr Val Ile
                   325                 330                 335
   Asn Leu His Lys Ala Leu Ala Ser Leu Ala Thr Ala Thr Leu Glu Ser
               340                 345                 350
   Val Val Gln Glu Arg Phe Gly Ser Arg Cys Ala Arg Ile Phe Arg Leu
           355                 360                 365
   Val Leu Gln Lys Lys His Ile Glu Gln Lys Gln Val Glu Asp Phe Ala
       370                 375                 380
   Met Ile Pro Ala Lys Glu Ala Lys Asp Met Leu Tyr Lys Met Leu Ser
   385                 390                 395                 400
   Glu Asn Phe Met Ser Leu Gln Glu Ile Pro Lys Thr Pro Asp His Ala
                   405                 410                 415
   Pro Ser Arg Thr Phe Tyr Leu Tyr Thr Val Asn Ile Leu Ser Ala Ala
               420                 425                 430
   Arg Met Leu Leu His Arg Cys Tyr Lys Ser Ile Ala Asn Leu Ile Glu
           435                 440                 445
   Arg Arg Gln Phe Glu Thr Lys Glu Asn Lys Arg Leu Leu Glu Lys Ser
       450                 455                 460
   Gln Arg Val Glu Ala Ile Ile Ala Ser Met Gln Ala Thr Gly Ala Glu
   465                 470                 475                 480
   Glu Ala Gln Leu Gln Glu Ile Glu Glu Met Ile Thr Ala Pro Glu Arg
                   485                 490                 495
   Gln Gln Leu Glu Thr Leu Lys Arg Asn Val Asn Lys Leu Asp Ala Ser
               500                 505                 510
   Glu Ile Gln Val Asp Glu Thr Ile Phe Leu Leu Glu Ser Tyr Ile Glu
           515                 520                 525
   Cys Thr Met Lys Arg Gln
       530
<210> 110
   <211> 534
   <212> PRT
   <213> Homo sapiens
<400> 110
   Met Thr Gln Ala Glu Ile Lys Leu Cys Ser Leu Leu Leu Gln Glu His
   1               5                   10                  15
   Phe Gly Glu Ile Val Glu Lys Ile Gly Val His Leu Ile Arg Thr Gly
               20                  25                  30
   Ser Gln Pro Leu Arg Val Ile Ala His Asp Thr Gly Thr Ser Leu Asp
           35                  40                  45
   Gln Val Lys Lys Ala Leu Cys Val Leu Val Gln His Asn Leu Val Ser
       50                  55                  60
   Tyr Gln Val His Lys Arg Gly Val Val Glu Tyr Glu Ala Gln Cys Ser
   65                  70                  75                  80
   Arg Val Leu Arg Met Leu Arg Tyr Pro Arg Tyr Ile Tyr Thr Thr Lys
                   85                  90                  95
   Thr Leu Tyr Ser Asp Thr Gly Glu Leu Ile Val Glu Glu Leu Leu Leu
               100                 105                 110
   Asn Gly Lys Leu Thr Met Ser Ala Val Val Lys Lys Val Ala Asp Arg
           115                 120                 125
   Leu Thr Glu Thr Met Glu Asp Gly Lys Thr Met Asp Tyr Ala Glu Val
       130                 135                 140
   Ser Asn Thr Phe Val Arg Leu Ala Asp Thr His Phe Val Gln Arg Cys
   145                 150                 155                 160
   Pro Ser Val Pro Thr Thr Glu Asn Ser Asp Pro Gly Pro Pro Pro Pro
                   165                 170                 175
   Ala Pro Thr Leu Val Ile Asn Glu Lys Asp Met Tyr Leu Val Pro Lys
               180                 185                 190
   Leu Ser Leu Ile Gly Lys Gly Lys Arg Arg Arg Ser Ser Asp Glu Asp
           195                 200                 205
   Ala Ala Gly Glu Pro Lys Ala Lys Arg Pro Lys Tyr Thr Thr Asp Asn
       210                 215                 220
   Lys Glu Pro Ile Pro Asp Asp Gly Ile Tyr Trp Gln Ala Asn Leu Asp
   225 230 235 240
   Arg Phe His Gln His Phe Arg Asp Gln Ala Ile Val Ser Ala Val Ala
   245 250 255
   Asn Arg Met Asp Gln Thr Ser Ser Glu Ile Val Arg Thr Met Leu Arg
   260 265 270
   Met Ser Glu Ile Thr Thr Ser Ser Ser Ala Pro Phe Thr Gln Pro Leu
   275 280 285
   Ser Ser Asn Glu Ile Phe Arg Ser Leu Pro Val Gly Tyr Asn Ile Ser
   290 295 300
   Lys Gln Val Leu Asp Gln Tyr Leu Thr Leu Leu Ala Asp Asp Pro Leu
   305 310 315 320
   Glu Phe Val Gly Lys Ser Gly Asp Ser Gly Gly Gly Met Tyr Val Ile
   325 330 335
   Asn Leu His Lys Ala Leu Ala Ser Leu Ala Thr Ala Thr Leu Glu Ser
   340 345 350
   Val Val Gln Glu Arg Phe Gly Ser Arg Cys Ala Arg Ile Phe Arg Leu
   355 360 365
   Val Leu Gln Lys Lys His Ile Glu Gln Lys Gln Val Glu Asp Phe Ala
   370 375 380
   Met Ile Pro Ala Lys Glu Ala Lys Asp Met Leu Tyr Lys Met Leu Ser
   385 390 395 400
   Glu Asn Phe Met Ser Leu Gln Glu Ile Pro Lys Thr Pro Asp His Ala
   405 410 415
   Pro Ser Arg Thr Phe Tyr Leu Tyr Thr Val Asn Ile Leu Ser Ala Ala
   420 425 430
   Arg Met Leu Leu His Arg Cys Tyr Lys Ser Ile Ala Asn Leu Ile Glu
   435 440 445
   Arg Arg Gln Phe Glu Thr Lys Glu Asn Lys Arg Leu Leu Glu Lys Ser
   450 455 460
   Gln Arg Val Glu Ala Ile Ile Ala Ser Met Gln Ala Thr Gly Ala Glu
   465 470 475 480
   Glu Ala Gln Leu Gln Glu Ile Glu Glu Met Ile Thr Ala Pro Glu Arg
                   485                 490                 495
   Gln Gln Leu Glu Thr Leu Lys Arg Asn Val Asn Lys Leu Asp Ala Ser
               500                 505                 510
   Glu Ile Gln Val Asp Glu Thr Ile Phe Leu Leu Glu Ser Tyr Ile Glu
           515                 520                 525
   Cys Thr Met Lys Arg Gln
       530
<210> 111
   <211> 398
   <212> PRT
   <213> Homo sapiens
<400> 111
   Met Ser Glu Gly Asn Ala Ala Gly Glu Pro Ser Thr Pro Gly Gly Pro
   1               5                   10                  15
   Arg Pro Leu Leu Thr Gly Ala Arg Gly Leu Ile Gly Arg Arg Pro Ala
               20                  25                  30
   Pro Pro Leu Thr Pro Gly Arg Leu Pro Ser Ile Arg Ser Arg Asp Leu
           35                  40                  45
   Thr Leu Gly Gly Val Lys Lys Lys Thr Phe Thr Pro Asn Ile Ile Ser
       50                  55                  60
   Arg Lys Ile Lys Glu Glu Pro Lys Glu Glu Val Thr Val Lys Lys Glu
   65                  70                  75                  80
   Lys Arg Glu Arg Asp Arg Asp Arg Gln Arg Glu Gly His Gly Arg Gly
                   85                  90                  95
   Arg Gly Arg Pro Glu Val Ile Gln Ser His Ser Ile Phe Glu Gln Gly
               100                 105                 110
   Pro Ala Glu Met Met Lys Lys Lys Gly Asn Trp Asp Lys Thr Val Asp
           115                 120                 125
   Val Ser Asp Met Gly Pro Ser His Ile Ile Asn Ile Lys Lys Glu Lys
       130                 135                 140
   Arg Glu Thr Asp Glu Glu Thr Lys Gln Ile Leu Arg Met Leu Glu Lys
   145                 150                 155                 160
   Asp Asp Phe Leu Asp Asp Pro Gly Leu Arg Asn Asp Thr Arg Asn Met
                   165                 170                 175
   Pro Val Gln Leu Pro Leu Ala His Ser Gly Trp Leu Phe Lys Glu Glu
               180                 185                 190
   Asn Asp Glu Pro Asp Val Lys Pro Trp Leu Ala Gly Pro Lys Glu Glu
           195                 200                 205
   Asp Met Glu Val Asp Ile Pro Ala Val Lys Val Lys Glu Glu Pro Arg
       210                 215                 220
   Asp Glu Glu Glu Glu Ala Lys Met Lys Ala Pro Pro Lys Ala Ala Arg
   225                 230                 235                 240
   Lys Thr Pro Gly Leu Pro Lys Asp Val Ser Val Ala Glu Leu Leu Arg
                   245                 250                 255
   Glu Leu Ser Leu Thr Lys Glu Glu Glu Leu Leu Phe Leu Gln Leu Pro
               260                 265                 270
   Asp Thr Leu Pro Gly Gln Pro Pro Thr Gln Asp Ile Lys Pro Ile Lys
           275                 280                 285
   Thr Glu Val Gln Gly Glu Asp Gly Gln Val Val Leu Ile Lys Gln Glu
       290                 295                 300
   Lys Asp Arg Glu Ala Lys Leu Ala Glu Asn Ala Cys Thr Leu Ala Asp
   305                 310                 315                 320
   Leu Thr Glu Gly Gln Val Gly Lys Leu Leu Ile Arg Lys Ser Gly Arg
                   325                 330                 335
   Val Gln Leu Leu Leu Gly Lys Val Thr Leu Asp Val Thr Met Gly Thr
               340                 345                 350
   Ala Cys Ser Phe Leu Gln Glu Leu Val Ser Val Gly Leu Gly Asp Ser
           355                 360                 365
   Arg Thr Gly Glu Met Thr Val Leu Gly His Val Lys His Lys Leu Val
       370                 375                 380
   Cys Ser Pro Asp Phe Glu Ser Leu Leu Asp His Lys His Arg
   385                 390                 395
<210> 112
   <211> 708
   <212> PRT
   <213> Homo sapiens
<400> 112
   Met Ala Asn Glu Glu Asp Asp Pro Val Val Gln Glu Ile Asp Val Tyr
   1               5                   10                  15
   Leu Ala Lys Ser Leu Ala Glu Lys Leu Tyr Leu Phe Gln Tyr Pro Val
               20                  25                  30
   Arg Pro Ala Ser Met Thr Tyr Asp Asp Ile Pro His Leu Ser Ala Lys
           35                  40                  45
   Ile Lys Pro Lys Gln Gln Lys Val Glu Leu Glu Met Ala Ile Asp Thr
       50                  55                  60
   Leu Asn Pro Asn Tyr Cys Arg Ser Lys Gly Glu Gln Ile Ala Leu Asn
   65                  70                  75                  80
   Val Asp Gly Ala Cys Ala Asp Glu Thr Ser Thr Tyr Ser Ser Lys Leu
                   85                  90                  95
   Met Asp Lys Gln Thr Phe Cys Ser Ser Gln Thr Thr Ser Asn Thr Ser
               100                 105                 110
   Arg Tyr Ala Ala Ala Leu Tyr Arg Gln Gly Glu Leu His Leu Thr Pro
           115                 120                 125
   Leu His Gly Ile Leu Gln Leu Arg Pro Ser Phe Ser Tyr Leu Asp Lys
       130                 135                 140
   Ala Asp Ala Lys His Arg Glu Arg Glu Ala Ala Asn Glu Ala Gly Asp
   145                 150                 155                 160
   Ser Ser Gln Asp Glu Ala Glu Asp Asp Val Lys Gln Ile Thr Val Arg
                   165                 170                 175
   Phe Ser Arg Pro Glu Ser Glu Gln Ala Arg Gln Arg Arg Val Gln Ser
               180                 185                 190
   Tyr Glu Phe Leu Gln Lys Lys His Ala Glu Glu Pro Trp Val His Leu
           195                 200                 205
   His Tyr Tyr Gly Leu Arg Asp Ser Arg Ser Glu His Glu Arg Gln Tyr
       210                 215                 220
   Leu Leu Cys Pro Gly Ser Ser Gly Val Glu Asn Thr Glu Leu Val Lys
   225                 230                 235                 240
   Ser Pro Ser Glu Tyr Leu Met Met Leu Met Pro Pro Ser Gln Glu Glu
                   245                 250                 255
   Glu Lys Asp Lys Pro Val Ala Pro Ser Asn Val Leu Ser Met Ala Gln
               260                 265                 270
   Leu Arg Thr Leu Pro Leu Ala Asp Gln Ile Lys Ile Leu Met Lys Asn
           275                 280                 285
   Val Lys Val Met Pro Phe Ala Asn Leu Met Ser Leu Leu Gly Pro Ser
       290                 295                 300
   Ile Asp Ser Val Ala Val Leu Arg Gly Ile Gln Lys Val Ala Met Leu
   305                 310                 315                 320
   Val Gln Gly Asn Trp Val Val Lys Ser Asp Ile Leu Tyr Pro Lys Asp
                   325                 330                 335
   Ser Ser Ser Pro His Ser Gly Val Pro Ala Glu Val Leu Cys Arg Gly
               340                 345                 350
   Arg Asp Phe Val Met Trp Lys Phe Thr Gln Ser Arg Trp Val Val Arg
           355                 360                 365
   Lys Glu Val Ala Thr Val Thr Lys Leu Cys Ala Glu Asp Val Lys Asp
       370                 375                 380
   Phe Leu Glu His Met Ala Val Val Arg Ile Asn Lys Gly Trp Glu Phe
   385                 390                 395                 400
   Ile Leu Pro Tyr Asp Gly Glu Phe Ile Lys Lys His Pro Asp Val Val
                   405                 410                 415
   Gln Arg Gln His Met Leu Trp Thr Gly Ile Gln Ala Lys Leu Glu Lys
               420                 425                 430
   Val Tyr Asn Leu Val Lys Glu Thr Met Pro Lys Lys Pro Asp Ala Gln
           435                 440                 445
   Ser Gly Pro Ala Gly Leu Val Cys Gly Asp Gln Arg Ile Gln Val Ala
       450                 455                 460
   Lys Thr Lys Ala Gln Gln Asn His Ala Leu Leu Glu Arg Glu Leu Gln
   465                 470                 475                 480
   Arg Arg Lys Glu Gln Leu Arg Val Pro Ala Val Pro Pro Gly Val Arg
                   485                 490                 495
   Ile Lys Glu Glu Pro Val Ser Glu Glu Gly Glu Glu Asp Glu Glu Gln
               500                 505                 510
   Glu Ala Glu Glu Glu Pro Met Asp Thr Ser Pro Ser Gly Leu His Ser
           515                 520                 525
   Lys Leu Ala Asn Gly Leu Pro Leu Gly Arg Ala Ala Gly Thr Asp Ser
       530                 535                 540
   Phe Asn Gly His Pro Pro Gln Gly Cys Ala Ser Thr Pro Val Ala Arg
   545                 550                 555                 560
   Glu Leu Lys Ala Phe Val Glu Ala Thr Phe Gln Arg Gln Phe Val Leu
                   565                 570                 575
   Thr Leu Ser Glu Leu Lys Arg Leu Phe Asn Leu His Leu Ala Ser Leu
               580                 585                 590
   Pro Pro Gly His Thr Leu Phe Ser Gly Ile Ser Asp Arg Met Leu Gln
           595                 600                 605
   Asp Thr Val Leu Ala Ala Gly Cys Lys Gln Ile Leu Val Pro Phe Pro
       610                 615                 620
   Pro Gln Thr Ala Ala Ser Pro Asp Glu Gln Lys Val Phe Ala Leu Trp
   625                 630                 635                 640
   Glu Ser Gly Asp Met Ser Asp Gln His Arg Gln Val Leu Leu Glu Ile
                   645                 650                 655
   Phe Ser Lys Asn Tyr Arg Val Arg Arg Asn Met Ile Gln Ser Arg Leu
               660                 665                 670
   Thr Gln Glu Cys Gly Glu Asp Leu Ser Lys Gln Glu Val Asp Lys Val
           675                 680                 685
   Leu Lys Asp Cys Cys Val Ser Tyr Gly Gly Met Trp Tyr Leu Lys Gly
       690                 695                 700
   Thr Val Gln Ser
   705
<210> 113
   <211> 316
   <212> PRT
   <213> Homo sapiens
<400> 113
   Met Ala Glu Val Lys Val Lys Val Gln Pro Pro Asp Ala Asp Pro Val
   1               5                   10                  15
   Glu Ile Glu Asn Arg Ile Ile Glu Leu Cys His Gln Phe Pro His Gly
               20                  25                  30
   Ile Thr Asp Gln Val Ile Gln Asn Glu Met Pro His Ile Glu Ala Gln
           35                  40                  45
   Gln Arg Ala Val Ala Ile Asn Arg Leu Leu Ser Met Gly Gln Leu Asp
       50                  55                  60
   Leu Leu Arg Ser Asn Thr Gly Leu Leu Tyr Arg Ile Lys Asp Ser Gln
   65                  70                  75                  80
   Asn Ala Gly Lys Met Lys Gly Ser Asp Asn Gln Glu Lys Leu Val Tyr
                   85                  90                  95
   Gln Ile Ile Glu Asp Ala Gly Asn Lys Gly Ile Trp Ser Arg Asp Ile
               100                 105                 110
   Arg Tyr Lys Ser Asn Leu Pro Leu Thr Glu Ile Asn Lys Ile Leu Lys
           115                 120                 125
   Asn Leu Glu Ser Lys Lys Leu Ile Lys Ala Val Lys Ser Val Ala Ala
       130                 135                 140
   Ser Lys Lys Lys Val Tyr Met Leu Tyr Asn Leu Gln Pro Asp Arg Ser
   145                 150                 155                 160
   Val Thr Gly Gly Ala Trp Tyr Ser Asp Gln Asp Phe Glu Ser Glu Phe
                   165                 170                 175
   Val Glu Val Leu Asn Gln Gln Cys Phe Lys Phe Leu Gln Ser Lys Ala
               180                 185                 190
   Glu Thr Ala Arg Glu Ser Lys Gln Asn Pro Met Ile Gln Arg Asn Ser
           195                 200                 205
   Ser Phe Ala Ser Ser His Glu Val Trp Lys Tyr Ile Cys Glu Leu Gly
       210                 215                 220
   Ile Ser Lys Val Glu Leu Ser Met Glu Asp Ile Glu Thr Ile Leu Asn
   225                 230                 235                 240
   Thr Leu Ile Tyr Asp Gly Lys Val Glu Met Thr Ile Ile Ala Ala Lys
                   245                 250                 255
   Glu Gly Thr Val Gly Ser Val Asp Gly His Met Lys Leu Tyr Arg Ala
               260                 265                 270
   Val Asn Pro Ile Ile Pro Pro Thr Gly Leu Val Arg Ala Pro Cys Gly
           275                 280                 285
   Leu Cys Pro Val Phe Asp Asp Cys His Glu Gly Gly Glu Ile Ser Pro
       290                 295                 300
   Ser Asn Cys Ile Tyr Met Thr Glu Trp Leu Glu Phe
   305                 310                 315
<210> 114
   <211> 223
   <212> PRT
   <213> Homo sapiens
<400> 114
   Met Ala Gly Asn Lys Gly Arg Gly Arg Ala Ala Tyr Thr Phe Asn Ile
   1               5                   10                  15
   Glu Ala Val Gly Phe Ser Lys Gly Glu Lys Leu Pro Asp Val Val Leu
               20                  25                  30
   Lys Pro Pro Pro Leu Phe Pro Asp Thr Asp Tyr Lys Pro Val Pro Leu
           35                  40                  45
   Lys Thr Gly Glu Gly Glu Glu Tyr Met Leu Ala Leu Lys Gln Glu Leu
       50                  55                  60
   Arg Glu Thr Met Lys Arg Met Pro Tyr Phe Ile Glu Thr Pro Glu Glu
   65                  70                  75                  80
   Arg Gln Asp Ile Glu Arg Tyr Ser Lys Arg Tyr Met Lys Val Tyr Lys
                   85                  90                  95
   Glu Glu Trp Ile Pro Asp Trp Arg Arg Leu Pro Arg Glu Met Met Pro
               100                 105                 110
   Arg Asn Lys Cys Lys Lys Ala Gly Pro Lys Pro Lys Lys Ala Lys Asp
           115                 120                 125
   Ala Gly Lys Gly Thr Pro Leu Thr Asn Thr Glu Asp Val Leu Lys Lys
       130                 135                 140
   Met Glu Glu Leu Glu Lys Arg Gly Asp Gly Glu Lys Ser Asp Glu Glu
   145                 150                 155                 160
   Asn Glu Glu Lys Glu Gly Ser Lys Glu Lys Ser Lys Glu Gly Asp Asp
                   165                 170                 175
   Asp Asp Asp Asp Asp Ala Ala Glu Gln Glu Glu Tyr Asp Glu Glu Glu
               180                 185                 190
   Gln Glu Glu Glu Asn Asp Tyr Ile Asn Ser Tyr Phe Glu Asp Gly Asp
           195                 200                 205
   Asp Phe Gly Ala Asp Ser Asp Asp Asn Met Asp Glu Ala Thr Tyr
       210                 215                 220
<210> 115
   <211> 223
   <212> PRT
   <213> Homo sapiens
<400> 115
   Met Ala Gly Asn Lys Gly Arg Gly Arg Ala Ala Tyr Thr Phe Asn Ile
   1 5 10 15
   Glu Ala Val Gly Phe Ser Lys Gly Glu Lys Leu Pro Asp Val Val Leu
   20 25 30
   Lys Pro Pro Pro Leu Phe Pro Asp Thr Asp Tyr Lys Pro Val Pro Leu
   35 40 45
   Lys Thr Gly Glu Gly Glu Glu Tyr Met Leu Ala Leu Lys Gln Glu Leu
   50 55 60
   Arg Glu Thr Met Lys Arg Met Pro Tyr Phe Ile Glu Thr Pro Glu Glu
   65 70 75 80
   Arg Gln Asp Ile Glu Arg Tyr Ser Lys Arg Tyr Met Lys Val Tyr Lys
   85 90 95
   Glu Glu Trp Ile Pro Asp Trp Arg Arg Leu Pro Arg Glu Met Met Pro
   100 105 110
   Arg Asn Lys Cys Lys Lys Ala Gly Pro Lys Pro Lys Lys Ala Lys Asp
   115 120 125
   Ala Gly Lys Gly Thr Pro Leu Thr Asn Thr Glu Asp Val Leu Lys Lys
   130 135 140
   Met Glu Glu Leu Glu Lys Arg Gly Asp Gly Glu Lys Ser Asp Glu Glu
   145 150 155 160
   Asn Glu Glu Lys Glu Gly Ser Lys Glu Lys Ser Lys Glu Gly Asp Asp
   165 170 175
   Asp Asp Asp Asp Asp Ala Ala Glu Gln Glu Glu Tyr Asp Glu Glu Glu
   180 185 190
   Gln Glu Glu Glu Asn Asp Tyr Ile Asn Ser Tyr Phe Glu Asp Gly Asp
   195 200 205
   Asp Phe Gly Ala Asp Ser Asp Asp Asn Met Asp Glu Ala Thr Tyr
   210 215 220
<210> 116
   <211> 108
   <212> PRT
   <213> Homo sapiens
<400> 116
   Met Leu Leu Phe Cys Pro Gly Cys Gly Asn Gly Leu Ile Val Glu Glu
   1               5                   10                  15
   Gly Gln Arg Cys His Arg Phe Ser Cys Asn Thr Cys Pro Tyr Val His
               20                  25                  30
   Asn Ile Thr Arg Lys Val Thr Asn Arg Lys Tyr Pro Lys Leu Lys Glu
           35                  40                  45
   Val Asp Asp Val Leu Gly Gly Ala Ala Ala Trp Glu Asn Val Asp Ser
       50                  55                  60
   Thr Ala Glu Ser Cys Pro Lys Cys Glu His Pro Arg Ala Tyr Phe Met
   65                  70                  75                  80
   Gln Leu Gln Thr Arg Ser Ala Asp Glu Pro Met Thr Thr Phe Tyr Lys
                   85                  90                  95
   Cys Cys Asn Ala Gln Cys Gly His Arg Trp Arg Asp
               100                 105
<210> 117
   <211> 1230
   <212> PRT
   <213> Homo sapiens
<400> 117
   Met Ser Ala Leu Cys Trp Gly Arg Gly Ala Ala Gly Leu Lys Arg Ala
   1               5                   10                  15
   Leu Arg Pro Cys Gly Arg Pro Gly Leu Pro Gly Lys Glu Gly Thr Ala
               20                  25                  30
   Gly Gly Val Cys Gly Pro Arg Arg Ser Ser Ser Ala Ser Pro Gln Glu
           35                  40                  45
   Gln Asp Gln Asp Arg Arg Lys Asp Trp Gly His Val Glu Leu Leu Glu
       50                  55                  60
   Val Leu Gln Ala Arg Val Arg Gln Leu Gln Ala Glu Ser Val Ser Glu
   65                  70                  75                  80
   Val Val Val Asn Arg Val Asp Val Ala Arg Leu Pro Glu Cys Gly Ser
                   85                  90                  95
   Gly Asp Gly Ser Leu Gln Pro Pro Arg Lys Val Gln Met Gly Ala Lys
               100                 105                 110
   Asp Ala Thr Pro Val Pro Cys Gly Arg Trp Ala Lys Ile Leu Glu Lys
           115                 120                 125
   Asp Lys Arg Thr Gln Gln Met Arg Met Gln Arg Leu Lys Ala Lys Leu
       130                 135                 140
   Gln Met Pro Phe Gln Ser Gly Glu Phe Lys Ala Leu Thr Arg Arg Leu
   145                 150                 155                 160
   Gln Val Glu Pro Arg Leu Leu Ser Lys Gln Met Ala Gly Cys Leu Glu
                   165                 170                 175
   Asp Cys Thr Arg Gln Ala Pro Glu Ser Pro Trp Glu Glu Gln Leu Ala
               180                 185                 190
   Arg Leu Leu Gln Glu Ala Pro Gly Lys Leu Ser Leu Asp Val Glu Gln
           195                 200                 205
   Ala Pro Ser Gly Gln His Ser Gln Ala Gln Leu Ser Gly Gln Gln Gln
       210                 215                 220
   Arg Leu Leu Ala Phe Phe Lys Cys Cys Leu Leu Thr Asp Gln Leu Pro
   225                 230                 235                 240
   Leu Ala His His Leu Leu Val Val His His Gly Gln Arg Gln Lys Arg
                   245                 250                 255
   Lys Leu Leu Thr Leu Asp Met Tyr Asn Ala Val Met Leu Gly Trp Ala
               260                 265                 270
   Arg Gln Gly Ala Phe Lys Glu Leu Val Tyr Val Leu Phe Met Val Lys
           275                 280                 285
   Asp Ala Gly Leu Thr Pro Asp Leu Leu Ser Tyr Ala Ala Ala Leu Gln
       290                 295                 300
   Cys Met Gly Arg Gln Asp Gln Asp Ala Gly Thr Ile Glu Arg Cys Leu
   305                 310                 315                 320
   Glu Gln Met Ser Gln Glu Gly Leu Lys Leu Gln Ala Leu Phe Thr Ala
                   325                 330                 335
   Val Leu Leu Ser Glu Glu Asp Arg Ala Thr Val Leu Lys Ala Val His
               340                 345                 350
   Lys Val Lys Pro Thr Phe Ser Leu Pro Pro Gln Leu Pro Pro Pro Val
           355                 360                 365
   Asn Thr Ser Lys Leu Leu Arg Asp Val Tyr Ala Lys Asp Gly Arg Val
       370                 375                 380
   Ser Tyr Pro Lys Leu His Leu Pro Leu Lys Thr Leu Gln Cys Leu Phe
   385                 390                 395                 400
   Glu Lys Gln Leu His Met Glu Leu Ala Ser Arg Val Cys Val Val Ser
                   405                 410                 415
   Val Glu Lys Pro Thr Leu Pro Ser Lys Glu Val Lys His Ala Arg Lys
               420                 425                 430
   Thr Leu Lys Thr Leu Arg Asp Gln Trp Glu Lys Ala Leu Cys Arg Ala
           435                 440                 445
   Leu Arg Glu Thr Lys Asn Arg Leu Glu Arg Glu Val Tyr Glu Gly Arg
       450                 455                 460
   Phe Ser Leu Tyr Pro Phe Leu Cys Leu Leu Asp Glu Arg Glu Val Val
   465                 470                 475                 480
   Arg Met Leu Leu Gln Val Leu Gln Ala Leu Pro Ala Gln Gly Glu Ser
                   485                 490                 495
   Phe Thr Thr Leu Ala Arg Glu Leu Ser Ala Arg Thr Phe Ser Arg His
               500                 505                 510
   Val Val Gln Arg Gln Arg Val Ser Gly Gln Val Gln Ala Leu Gln Asn
           515                 520                 525
   His Tyr Arg Lys Tyr Leu Cys Leu Leu Ala Ser Asp Ala Glu Val Pro
       530                 535                 540
   Glu Pro Cys Leu Pro Arg Gln Tyr Trp Glu Glu Leu Gly Ala Pro Glu
   545                 550                 555                 560
   Ala Leu Arg Glu Gln Pro Trp Pro Leu Pro Val Gln Met Glu Leu Gly
                   565                 570                 575
   Lys Leu Leu Ala Glu Met Leu Val Gln Ala Thr Gln Met Pro Cys Ser
               580                 585                 590
   Leu Asp Lys Pro His Arg Ser Ser Arg Leu Val Pro Val Leu Tyr His
           595                 600                 605
   Val Tyr Ser Phe Arg Asn Val Gln Gln Ile Gly Ile Leu Lys Pro His
       610                 615                 620
   Pro Ala Tyr Val Gln Leu Leu Glu Lys Ala Ala Glu Pro Thr Leu Thr
   625                 630                 635                 640
   Phe Glu Ala Val Asp Val Pro Met Leu Cys Pro Pro Leu Pro Trp Thr
                   645                 650                 655
   Ser Pro His Ser Gly Ala Phe Leu Leu Ser Pro Thr Lys Leu Met Arg
               660                 665                 670
   Thr Val Glu Gly Ala Thr Gln His Gln Glu Leu Leu Glu Thr Cys Pro
           675                 680                 685
   Pro Thr Ala Leu His Gly Ala Leu Asp Ala Leu Thr Gln Leu Gly Asn
       690                 695                 700
   Cys Ala Trp Arg Val Asn Gly Arg Val Leu Asp Leu Val Leu Gln Leu
   705                 710                 715                 720
   Phe Gln Ala Lys Gly Cys Pro Gln Leu Gly Val Pro Ala Pro Pro Ser
                   725                 730                 735
   Glu Ala Pro Gln Pro Pro Glu Ala His Leu Pro His Ser Ala Ala Pro
               740                 745                 750
   Ala Arg Lys Ala Glu Leu Arg Arg Glu Leu Ala His Cys Gln Lys Val
           755                 760                 765
   Ala Arg Glu Met His Ser Leu Arg Ala Glu Ala Leu Tyr Arg Leu Ser
       770                 775                 780
   Leu Ala Gln His Leu Arg Asp Arg Val Phe Trp Leu Pro His Asn Met
   785                 790                 795                 800
   Asp Phe Arg Gly Arg Thr Tyr Pro Cys Pro Pro His Phe Asn His Leu
                   805                 810                 815
   Gly Ser Asp Val Ala Arg Ala Leu Leu Glu Phe Ala Gln Gly Arg Pro
               820                 825                 830
   Leu Gly Pro His Gly Leu Asp Trp Leu Lys Ile His Leu Val Asn Leu
           835                 840                 845
   Thr Gly Leu Lys Lys Arg Glu Pro Leu Arg Lys Arg Leu Ala Phe Ala
       850                 855                 860
   Glu Glu Val Met Asp Asp Ile Leu Asp Ser Ala Asp Gln Pro Leu Thr
   865                 870                 875                 880
   Gly Arg Lys Trp Trp Met Gly Ala Glu Glu Pro Trp Gln Thr Leu Ala
                   885                 890                 895
   Cys Cys Met Glu Val Ala Asn Ala Val Arg Ala Ser Asp Pro Ala Ala
               900                 905                 910
   Tyr Val Ser His Leu Pro Val His Gln Asp Gly Ser Cys Asn Gly Leu
           915                 920                 925
   Gln His Tyr Ala Ala Leu Gly Arg Asp Ser Val Gly Ala Ala Ser Val
       930                 935                 940
   Asn Leu Glu Pro Ser Asp Val Pro Gln Asp Val Tyr Ser Gly Val Ala
   945                 950                 955                 960
   Ala Gln Val Glu Val Phe Arg Arg Gln Asp Ala Gln Arg Gly Met Arg
                   965                 970                 975
   Val Ala Gln Val Leu Glu Gly Phe Ile Thr Arg Lys Val Val Lys Gln
               980                 985                 990
   Thr Val Met Thr Val Val Tyr Gly  Val Thr Arg Tyr Gly  Gly Arg Leu
           995                 1000                 1005
   Gln Ile  Glu Lys Arg Leu Arg  Glu Leu Ser Asp Phe  Pro Gln Glu
       1010                 1015                 1020
   Phe Val  Trp Glu Ala Ser His  Tyr Leu Val Arg Gln  Val Phe Lys
       1025                 1030                 1035
   Ser Leu  Gln Glu Met Phe Ser  Gly Thr Arg Ala Ile  Gln His Trp
       1040                 1045                 1050
   Leu Thr  Glu Ser Ala Arg Leu  Ile Ser His Met Gly  Ser Val Val
       1055                 1060                 1065
   Glu Trp  Val Thr Pro Leu Gly  Val Pro Val Ile Gln  Pro Tyr Arg
       1070                 1075                 1080
   Leu Asp  Ser Lys Val Lys Gln  Ile Gly Gly Gly Ile  Gln Ser Ile
       1085                 1090                 1095
   Thr Tyr  Thr His Asn Gly Asp  Ile Ser Arg Lys Pro  Asn Thr Arg
       1100                 1105                 1110
   Lys Gln  Lys Asn Gly Phe Pro  Pro Asn Phe Ile His  Ser Leu Asp
       1115                 1120                 1125
   Ser Ser  His Met Met Leu Thr  Ala Leu His Cys Tyr  Arg Lys Gly
       1130                 1135                 1140
   Leu Thr  Phe Val Ser Val His  Asp Cys Tyr Trp Thr  His Ala Ala
       1145                 1150                 1155
   Asp Val  Ser Val Met Asn Gln  Val Cys Arg Glu Gln  Phe Val Arg
       1160                 1165                 1170
   Leu His  Ser Glu Pro Ile Leu  Gln Asp Leu Ser Arg  Phe Leu Val
       1175                 1180                 1185
   Lys Arg  Phe Cys Ser Glu Pro  Gln Lys Ile Leu Glu  Ala Ser Gln
       1190                 1195                 1200
   Leu Lys  Glu Thr Leu Gln Ala  Val Pro Lys Pro Gly  Ala Phe Asp
       1205                 1210                 1215
   Leu Glu  Gln Val Lys Arg Ser  Thr Tyr Phe Phe Ser
       1220                 1225                 1230
<210> 118
   <211> 1230
   <212> PRT
   <213> Homo sapiens
<400> 118
   Met Ser Ala Leu Cys Trp Gly Arg Gly Ala Ala Gly Leu Lys Arg Ala
   1               5                   10                  15
   Leu Arg Pro Cys Gly Arg Pro Gly Leu Pro Gly Lys Glu Gly Thr Ala
               20                  25                  30
   Gly Gly Val Cys Gly Pro Arg Arg Ser Ser Ser Ala Ser Pro Gln Glu
           35                  40                  45
   Gln Asp Gln Asp Arg Arg Lys Asp Trp Gly His Val Glu Leu Leu Glu
       50                  55                  60
   Val Leu Gln Ala Arg Val Arg Gln Leu Gln Ala Glu Ser Val Ser Glu
   65                  70                  75                  80
   Val Val Val Asn Arg Val Asp Val Ala Arg Leu Pro Glu Cys Gly Ser
                   85                  90                  95
   Gly Asp Gly Ser Leu Gln Pro Pro Arg Lys Val Gln Met Gly Ala Lys
               100                 105                 110
   Asp Ala Thr Pro Val Pro Cys Gly Arg Trp Ala Lys Ile Leu Glu Lys
           115                 120                 125
   Asp Lys Arg Thr Gln Gln Met Arg Met Gln Arg Leu Lys Ala Lys Leu
       130                 135                 140
   Gln Met Pro Phe Gln Ser Gly Glu Phe Lys Ala Leu Thr Arg Arg Leu
   145                 150                 155                 160
   Gln Val Glu Pro Arg Leu Leu Ser Lys Gln Met Ala Gly Cys Leu Glu
                   165                 170                 175
   Asp Cys Thr Arg Gln Ala Pro Glu Ser Pro Trp Glu Glu Gln Leu Ala
               180                 185                 190
   Arg Leu Leu Gln Glu Ala Pro Gly Lys Leu Ser Leu Asp Val Glu Gln
           195                 200                 205
   Ala Pro Ser Gly Gln His Ser Gln Ala Gln Leu Ser Gly Gln Gln Gln
       210                 215                 220
   Arg Leu Leu Ala Phe Phe Lys Cys Cys Leu Leu Thr Asp Gln Leu Pro
   225                 230                 235                 240
   Leu Ala His His Leu Leu Val Val His His Gly Gln Arg Gln Lys Arg
   245 250 255
   Lys Leu Leu Thr Leu Asp Met Tyr Asn Ala Val Met Leu Gly Trp Ala
   260 265 270
   Arg Gln Gly Ala Phe Lys Glu Leu Val Tyr Val Leu Phe Met Val Lys
   275 280 285
   Asp Ala Gly Leu Thr Pro Asp Leu Leu Ser Tyr Ala Ala Ala Leu Gln
   290 295 300
   Cys Met Gly Arg Gln Asp Gln Asp Ala Gly Thr Ile Glu Arg Cys Leu
   305 310 315 320
   Glu Gln Met Ser Gln Glu Gly Leu Lys Leu Gln Ala Leu Phe Thr Ala
   325 330 335
   Val Leu Leu Ser Glu Glu Asp Arg Ala Thr Val Leu Lys Ala Val His
   340 345 350
   Lys Val Lys Pro Thr Phe Ser Leu Pro Pro Gln Leu Pro Pro Pro Val
   355 360 365
   Asn Thr Ser Lys Leu Leu Arg Asp Val Tyr Ala Lys Asp Gly Arg Val
   370 375 380
   Ser Tyr Pro Lys Leu His Leu Pro Leu Lys Thr Leu Gln Cys Leu Phe
   385 390 395 400
   Glu Lys Gln Leu His Met Glu Leu Ala Ser Arg Val Cys Val Val Ser
   405 410 415
   Val Glu Lys Pro Thr Leu Pro Ser Lys Glu Val Lys His Ala Arg Lys
   420 425 430
   Thr Leu Lys Thr Leu Arg Asp Gln Trp Glu Lys Ala Leu Cys Arg Ala
   435 440 445
   Leu Arg Glu Thr Lys Asn Arg Leu Glu Arg Glu Val Tyr Glu Gly Arg
   450 455 460
   Phe Ser Leu Tyr Pro Phe Leu Cys Leu Leu Asp Glu Arg Glu Val Val
   465 470 475 480
   Arg Met Leu Leu Gln Val Leu Gln Ala Leu Pro Ala Gln Gly Glu Ser
   485 490 495
   Phe Thr Thr Leu Ala Arg Glu Leu Ser Ala Arg Thr Phe Ser Arg His
   500 505 510
   Val Val Gln Arg Gln Arg Val Ser Gly Gln Val Gln Ala Leu Gln Asn
   515 520 525
   His Tyr Arg Lys Tyr Leu Cys Leu Leu Ala Ser Asp Ala Glu Val Pro
   530 535 540
   Glu Pro Cys Leu Pro Arg Gln Tyr Trp Glu Glu Leu Gly Ala Pro Glu
   545 550 555 560
   Ala Leu Arg Glu Gln Pro Trp Pro Leu Pro Val Gln Met Glu Leu Gly
   565 570 575
   Lys Leu Leu Ala Glu Met Leu Val Gln Ala Thr Gln Met Pro Cys Ser
   580 585 590
   Leu Asp Lys Pro His Arg Ser Ser Arg Leu Val Pro Val Leu Tyr His
   595 600 605
   Val Tyr Ser Phe Arg Asn Val Gln Gln Ile Gly Ile Leu Lys Pro His
   610 615 620
   Pro Ala Tyr Val Gln Leu Leu Glu Lys Ala Ala Glu Pro Thr Leu Thr
   625 630 635 640
   Phe Glu Ala Val Asp Val Pro Met Leu Cys Pro Pro Leu Pro Trp Thr
   645 650 655
   Ser Pro His Ser Gly Ala Phe Leu Leu Ser Pro Thr Lys Leu Met Arg
   660 665 670
   Thr Val Glu Gly Ala Thr Gln His Gln Glu Leu Leu Glu Thr Cys Pro
   675 680 685
   Pro Thr Ala Leu His Gly Ala Leu Asp Ala Leu Thr Gln Leu Gly Asn
   690 695 700
   Cys Ala Trp Arg Val Asn Gly Arg Val Leu Asp Leu Val Leu Gln Leu
   705 710 715 720
   Phe Gln Ala Lys Gly Cys Pro Gln Leu Gly Val Pro Ala Pro Pro Ser
   725 730 735
   Glu Ala Pro Gln Pro Pro Glu Ala His Leu Pro His Ser Ala Ala Pro
   740 745 750
   Ala Arg Lys Ala Glu Leu Arg Arg Glu Leu Ala His Cys Gln Lys Val
   755 760 765
   Ala Arg Glu Met His Ser Leu Arg Ala Glu Ala Leu Tyr Arg Leu Ser
   770 775 780
   Leu Ala Gln His Leu Arg Asp Arg Val Phe Trp Leu Pro His Asn Met
   785 790 795 800
   Asp Phe Arg Gly Arg Thr Tyr Pro Cys Pro Pro His Phe Asn His Leu
   805 810 815
   Gly Ser Asp Val Ala Arg Ala Leu Leu Glu Phe Ala Gln Gly Arg Pro
   820 825 830
   Leu Gly Pro His Gly Leu Asp Trp Leu Lys Ile His Leu Val Asn Leu
   835 840 845
   Thr Gly Leu Lys Lys Arg Glu Pro Leu Arg Lys Arg Leu Ala Phe Ala
   850 855 860
   Glu Glu Val Met Asp Asp Ile Leu Asp Ser Ala Asp Gln Pro Leu Thr
   865 870 875 880
   Gly Arg Lys Trp Trp Met Gly Ala Glu Glu Pro Trp Gln Thr Leu Ala
   885 890 895
   Cys Cys Met Glu Val Ala Asn Ala Val Arg Ala Ser Asp Pro Ala Ala
   900 905 910
   Tyr Val Ser His Leu Pro Val His Gln Asp Gly Ser Cys Asn Gly Leu
   915 920 925
   Gln His Tyr Ala Ala Leu Gly Arg Asp Ser Val Gly Ala Ala Ser Val
   930 935 940
   Asn Leu Glu Pro Ser Asp Val Pro Gln Asp Val Tyr Ser Gly Val Ala
   945 950 955 960
   Ala Gln Val Glu Val Phe Arg Arg Gln Asp Ala Gln Arg Gly Met Arg
   965 970 975
   Val Ala Gln Val Leu Glu Gly Phe Ile Thr Arg Lys Val Val Lys Gln
   980 985 990
   Thr Val Met Thr Val Val Tyr Gly Val Thr Arg Tyr Gly Gly Arg Leu
   995 1000 1005
   Gln Ile  Glu Lys Arg Leu Arg  Glu Leu Ser Asp Phe  Pro Gln Glu
       1010                 1015                 1020
   Phe Val  Trp Glu Ala Ser His  Tyr Leu Val Arg Gln  Val Phe Lys
       1025                 1030                 1035
   Ser Leu  Gln Glu Met Phe Ser  Gly Thr Arg Ala Ile  Gln His Trp
       1040                 1045                 1050
   Leu Thr  Glu Ser Ala Arg Leu  Ile Ser His Met Gly  Ser Val Val
       1055                 1060                 1065
   Glu Trp  Val Thr Pro Leu Gly  Val Pro Val Ile Gln  Pro Tyr Arg
       1070                 1075                 1080
   Leu Asp  Ser Lys Val Lys Gln  Ile Gly Gly Gly Ile  Gln Ser Ile
       1085                 1090                 1095
   Thr Tyr  Thr His Asn Gly Asp  Ile Ser Arg Lys Pro  Asn Thr Arg
       1100                 1105                 1110
   Lys Gln  Lys Asn Gly Phe Pro  Pro Asn Phe Ile His  Ser Leu Asp
       1115                 1120                 1125
   Ser Ser  His Met Met Leu Thr  Ala Leu His Cys Tyr  Arg Lys Gly
       1130                 1135                 1140
   Leu Thr  Phe Val Ser Val His  Asp Cys Tyr Trp Thr  His Ala Ala
       1145                 1150                 1155
   Asp Val  Ser Val Met Asn Gln  Val Cys Arg Glu Gln  Phe Val Arg
       1160                 1165                 1170
   Leu His  Ser Glu Pro Ile Leu  Gln Asp Leu Ser Arg  Phe Leu Val
       1175                 1180                 1185
   Lys Arg  Phe Cys Ser Glu Pro  Gln Lys Ile Leu Glu  Ala Ser Gln
       1190                 1195                 1200
   Leu Lys  Glu Thr Leu Gln Ala  Val Pro Lys Pro Gly  Ala Phe Asp
       1205                 1210                 1215
   Leu Glu  Gln Val Lys Arg Ser  Thr Tyr Phe Phe Ser
       1220                 1225                 1230
<210> 119
   <211> 541
   <212> PRT
   <213> Homo sapiens
<400> 119
   Met Ser Pro Cys Pro Glu Glu Ala Ala Met Arg Arg Glu Val Val Lys
   1               5                   10                  15
   Arg Ile Glu Thr Val Val Lys Asp Leu Trp Pro Thr Ala Asp Val Gln
               20                  25                  30
   Ile Phe Gly Ser Phe Ser Thr Gly Leu Tyr Leu Pro Thr Ser Asp Ile
           35                  40                  45
   Asp Leu Val Val Phe Gly Lys Trp Glu Arg Pro Pro Leu Gln Leu Leu
       50                  55                  60
   Glu Gln Ala Leu Arg Lys His Asn Val Ala Glu Pro Cys Ser Ile Lys
   65                  70                  75                  80
   Val Leu Asp Lys Ala Thr Val Pro Ile Ile Lys Leu Thr Asp Gln Glu
                   85                  90                  95
   Thr Glu Val Lys Val Asp Ile Ser Phe Asn Met Glu Thr Gly Val Arg
               100                 105                 110
   Ala Ala Glu Phe Ile Lys Asn Tyr Met Lys Lys Tyr Ser Leu Leu Pro
           115                 120                 125
   Tyr Leu Ile Leu Val Leu Lys Gln Phe Leu Leu Gln Arg Asp Leu Asn
       130                 135                 140
   Glu Val Phe Thr Gly Gly Ile Ser Ser Tyr Ser Leu Ile Leu Met Ala
   145                 150                 155                 160
   Ile Ser Phe Leu Gln Leu His Pro Arg Ile Asp Ala Arg Arg Ala Asp
                   165                 170                 175
   Glu Asn Leu Gly Met Leu Leu Val Glu Phe Phe Glu Leu Tyr Gly Arg
               180                 185                 190
   Asn Phe Asn Tyr Leu Lys Thr Gly Ile Arg Ile Lys Glu Gly Gly Ala
           195                 200                 205
   Tyr Ile Ala Lys Glu Glu Ile Met Lys Ala Met Thr Ser Gly Tyr Arg
       210                 215                 220
   Pro Ser Met Leu Cys Ile Glu Asp Pro Leu Leu Pro Gly Asn Asp Val
   225 230 235 240
   Gly Arg Ser Ser Tyr Gly Ala Met Gln Val Lys Gln Val Phe Asp Tyr
   245 250 255
   Ala Tyr Ile Val Leu Ser His Ala Val Ser Pro Leu Ala Arg Ser Tyr
   260 265 270
   Pro Asn Arg Asp Ala Glu Ser Thr Leu Gly Arg Ile Ile Lys Val Thr
   275 280 285
   Gln Glu Val Ile Asp Tyr Arg Arg Trp Ile Lys Glu Lys Trp Gly Ser
   290 295 300
   Lys Ala His Pro Ser Pro Gly Met Asp Ser Arg Ile Lys Ile Lys Glu
   305 310 315 320
   Arg Ile Ala Thr Cys Asn Gly Glu Gln Thr Gln Asn Arg Glu Pro Glu
   325 330 335
   Ser Pro Tyr Gly Gln Arg Leu Thr Leu Ser Leu Ser Ser Pro Gln Leu
   340 345 350
   Leu Ser Ser Gly Ser Ser Ala Ser Ser Val Ser Ser Leu Ser Gly Ser
   355 360 365
   Asp Val Asp Ser Asp Thr Pro Pro Cys Thr Thr Pro Ser Val Tyr Gln
   370 375 380
   Phe Ser Leu Gln Ala Pro Ala Pro Leu Met Ala Gly Leu Pro Thr Ala
   385 390 395 400
   Leu Pro Met Pro Ser Gly Lys Pro Gln Pro Thr Thr Ser Arg Thr Leu
   405 410 415
   Ile Met Thr Thr Asn Asn Gln Thr Arg Phe Thr Ile Pro Pro Pro Thr
   420 425 430
   Leu Gly Val Ala Pro Val Pro Cys Arg Gln Ala Gly Val Glu Gly Thr
   435 440 445
   Ala Ser Leu Lys Ala Val His His Met Ser Ser Pro Ala Ile Pro Ser
   450 455 460
   Ala Ser Pro Asn Pro Leu Ser Ser Pro His Leu Tyr His Lys His Asn
   465 470 475 480
   Gly Met Lys Leu Ser Met Lys Gly Ser His Gly His Thr Gln Gly Gly
   485 490 495
   Gly Tyr Ser Ser Val Gly Ser Gly Gly Val Arg Pro Pro Val Gly Asn
   500 505 510
   Arg Gly His His Gln Tyr Asn Arg Thr Gly Trp Arg Arg Lys Lys His
   515 520 525
   Thr His Thr Arg Asp Ser Leu Pro Val Ser Leu Ser Arg
   530 535 540
<210> 120
   <211> 1135
   <212> PRT
   <213> Homo sapiens
<400> 120
   Met Asp Pro Gly Ser Arg Trp Arg Asn Leu Pro Ser Gly Pro Ser Leu
   1 5 10 15
   Lys His Leu Thr Asp Pro Ser Tyr Gly Ile Pro Arg Glu Gln Gln Lys
   20 25 30
   Ala Ala Leu Gln Glu Leu Thr Arg Ala His Val Glu Ser Phe Asn Tyr
   35 40 45
   Ala Val His Glu Gly Leu Gly Leu Ala Val Gln Ala Ile Pro Pro Phe
   50 55 60
   Glu Phe Ala Phe Lys Asp Glu Arg Ile Ser Phe Thr Ile Leu Asp Ala
   65 70 75 80
   Val Ile Ser Pro Pro Thr Val Pro Lys Gly Thr Ile Cys Lys Glu Ala
   85 90 95
   Asn Val Tyr Pro Ala Glu Cys Arg Gly Arg Arg Ser Thr Tyr Arg Gly
   100 105 110
   Lys Leu Thr Ala Asp Ile Asn Trp Ala Val Asn Gly Ile Ser Lys Gly
   115 120 125
   Ile Ile Lys Gln Phe Leu Gly Tyr Val Pro Ile Met Val Lys Ser Lys
   130 135 140
   Leu Cys Asn Leu Arg Asn Leu Pro Pro Gln Ala Leu Ile Glu His His
   145 150 155 160
   Glu Glu Ala Glu Glu Met Gly Gly Tyr Phe Ile Ile Asn Gly Ile Glu
                   165                 170                 175
   Lys Val Ile Arg Met Leu Ile Met Pro Arg Arg Asn Phe Pro Ile Ala
               180                 185                 190
   Met Ile Arg Pro Lys Trp Lys Thr Arg Gly Pro Gly Tyr Thr Gln Tyr
           195                 200                 205
   Gly Val Ser Met His Cys Val Arg Glu Glu His Ser Ala Val Asn Met
       210                 215                 220
   Asn Leu His Tyr Leu Glu Asn Gly Thr Val Met Leu Asn Phe Ile Tyr
   225                 230                 235                 240
   Arg Lys Glu Leu Phe Phe Leu Pro Leu Gly Phe Ala Leu Lys Ala Leu
                   245                 250                 255
   Val Ser Phe Ser Asp Tyr Gln Ile Phe Gln Glu Leu Ile Lys Gly Lys
               260                 265                 270
   Glu Asp Asp Ser Phe Leu Arg Asn Ser Val Ser Gln Met Leu Arg Ile
           275                 280                 285
   Val Met Glu Glu Gly Cys Ser Thr Gln Lys Gln Val Leu Asn Tyr Leu
       290                 295                 300
   Gly Glu Cys Phe Arg Val Lys Leu Asn Val Pro Asp Trp Tyr Pro Asn
   305                 310                 315                 320
   Glu Gln Ala Ala Glu Phe Leu Phe Asn Gln Cys Ile Cys Ile His Leu
                   325                 330                 335
   Lys Ser Asn Thr Glu Lys Phe Tyr Met Leu Cys Leu Met Thr Arg Lys
               340                 345                 350
   Leu Phe Ala Leu Ala Lys Gly Glu Cys Met Glu Asp Asn Pro Asp Ser
           355                 360                 365
   Leu Val Asn Gln Glu Val Leu Thr Pro Gly Gln Leu Phe Leu Met Phe
       370                 375                 380
   Leu Lys Glu Lys Leu Glu Gly Trp Leu Val Ser Ile Lys Ile Ala Phe
   385                 390                 395                 400
   Asp Lys Lys Ala Gln Lys Thr Ser Val Ser Met Asn Thr Asp Asn Leu
                   405                 410                 415
   Met Arg Ile Phe Thr Met Gly Ile Asp Leu Thr Lys Pro Phe Glu Tyr
               420                 425                 430
   Leu Phe Ala Thr Gly Asn Leu Arg Ser Lys Thr Gly Leu Gly Leu Leu
           435                 440                 445
   Gln Asp Ser Gly Leu Cys Val Val Ala Asp Lys Leu Asn Phe Ile Arg
       450                 455                 460
   Tyr Leu Ser His Phe Arg Cys Val His Arg Gly Ala Asp Phe Ala Lys
   465                 470                 475                 480
   Met Arg Thr Thr Thr Val Arg Arg Leu Leu Pro Glu Ser Trp Gly Phe
                   485                 490                 495
   Leu Cys Pro Val His Thr Pro Asp Gly Glu Pro Cys Gly Leu Met Asn
               500                 505                 510
   His Leu Thr Ala Val Cys Glu Val Val Thr Gln Phe Val Tyr Thr Ala
           515                 520                 525
   Ser Ile Pro Ala Leu Leu Cys Asn Leu Gly Val Thr Pro Ile Asp Gly
       530                 535                 540
   Ala Pro His Arg Ser Tyr Ser Glu Cys Tyr Pro Val Leu Leu Asp Gly
   545                 550                 555                 560
   Val Met Val Gly Trp Val Asp Lys Asp Leu Ala Pro Gly Ile Ala Asp
                   565                 570                 575
   Ser Leu Arg His Phe Lys Val Leu Arg Glu Lys Arg Ile Pro Pro Trp
               580                 585                 590
   Met Glu Val Val Leu Ile Pro Met Thr Gly Lys Pro Ser Leu Tyr Pro
           595                 600                 605
   Gly Leu Phe Leu Phe Thr Thr Pro Cys Arg Leu Val Arg Pro Val Gln
       610                 615                 620
   Asn Leu Ala Leu Gly Lys Glu Glu Leu Ile Gly Thr Met Glu Gln Ile
   625                 630                 635                 640
   Phe Met Asn Val Ala Ile Phe Glu Asp Glu Val Phe Ala Gly Val Thr
                   645                 650                 655
   Thr His Gln Glu Leu Phe Pro His Ser Leu Leu Ser Val Ile Ala Asn
               660                 665                 670
   Phe Ile Pro Phe Ser Asp His Asn Gln Ser Pro Arg Asn Met Tyr Gln
           675                 680                 685
   Cys Gln Met Gly Lys Gln Thr Met Gly Phe Pro Leu Leu Thr Tyr Gln
       690                 695                 700
   Asp Arg Ser Asp Asn Lys Leu Tyr Arg Leu Gln Thr Pro Gln Ser Pro
   705                 710                 715                 720
   Leu Val Arg Pro Ser Met Tyr Asp Tyr Tyr Asp Met Asp Asn Tyr Pro
                   725                 730                 735
   Ile Gly Thr Asn Ala Ile Val Ala Val Ile Ser Tyr Thr Gly Tyr Asp
               740                 745                 750
   Met Glu Asp Ala Met Ile Val Asn Lys Ala Ser Trp Glu Arg Gly Phe
           755                 760                 765
   Ala His Gly Ser Val Tyr Lys Ser Glu Phe Ile Asp Leu Ser Glu Lys
       770                 775                 780
   Ile Lys Gln Gly Asp Ser Ser Leu Val Phe Gly Ile Lys Pro Gly Asp
   785                 790                 795                 800
   Pro Arg Val Leu Gln Lys Leu Asp Asp Asp Gly Leu Pro Phe Ile Gly
                   805                 810                 815
   Ala Lys Leu Gln Tyr Gly Asp Pro Tyr Tyr Ser Tyr Leu Asn Leu Asn
               820                 825                 830
   Thr Gly Glu Ser Phe Val Met Tyr Tyr Lys Ser Lys Glu Asn Cys Val
           835                 840                 845
   Val Asp Asn Ile Lys Val Cys Ser Asn Asp Thr Gly Ser Gly Lys Phe
       850                 855                 860
   Lys Cys Val Cys Ile Thr Met Arg Val Pro Arg Asn Pro Thr Ile Gly
   865                 870                 875                 880
   Asp Lys Phe Ala Ser Arg His Gly Gln Lys Gly Ile Leu Ser Arg Leu
                   885                 890                 895
   Trp Pro Ala Glu Asp Met Pro Phe Thr Glu Ser Gly Met Val Pro Asp
               900                 905                 910
   Ile Leu Phe Asn Pro His Gly Phe Pro Ser Arg Met Thr Ile Gly Met
           915                 920                 925
   Leu Ile Glu Ser Met Ala Gly Lys Ser Ala Ala Leu His Gly Leu Cys
       930                 935                 940
   His Asp Ala Thr Pro Phe Ile Phe Ser Glu Glu Asn Ser Ala Leu Glu
   945                 950                 955                 960
   Tyr Phe Gly Glu Met Leu Lys Ala Ala Gly Tyr Asn Phe Tyr Gly Thr
                   965                 970                 975
   Glu Arg Leu Tyr Ser Gly Ile Ser Gly Leu Glu Leu Glu Ala Asp Ile
               980                 985                 990
   Phe Ile Gly Val Val Tyr Tyr Gln  Arg Leu Arg His Met  Val Ser Asp
           995                 1000                1005
   Lys Phe  Gln Val Arg Thr Thr  Gly Ala Arg Asp Arg  Val Thr Asn
       1010                 1015                1020
   Gln Pro  Ile Gly Gly Arg Asn  Val Gln Gly Gly Ile  Arg Phe Gly
       1025                 1030                1035
   Glu Met  Glu Arg Asp Ala Leu  Leu Ala His Gly Thr  Ser Phe Leu
       1040                 1045                1050
   Leu His  Asp Arg Leu Phe Asn  Cys Ser Asp Arg Ser  Val Ala His
       1055                 1060                1065
   Val Cys  Val Lys Cys Gly Ser  Leu Leu Ser Pro Leu  Leu Glu Lys
       1070                 1075                1080
   Pro Pro  Pro Ser Trp Ser Ala  Met Arg Asn Arg Lys  Tyr Asn Cys
       1085                 1090                1095
   Thr Leu  Cys Ser Arg Ser Asp  Thr Ile Asp Thr Val  Ser Val Pro
       1100                 1105                1110
   Tyr Val  Phe Arg Tyr Phe Val  Ala Glu Leu Ala Ala  Met Asn Ile
       1115                 1120                1125
   Lys Val  Lys Leu Asp Val Val
       1130                 1135
<210> 121
   <211> 122
   <212> PRT
   <213> Homo sapiens
<400> 121
   Met Glu Glu Asp Gln Glu Leu Glu Arg Lys Ala Ile Glu Glu Leu Leu
   1               5                   10                  15
   Lys Glu Ala Lys Arg Gly Lys Thr Arg Ala Glu Thr Met Gly Pro Met
               20                  25                  30
   Gly Trp Met Lys Cys Pro Leu Ala Ser Thr Asn Lys Arg Phe Leu Ile
           35                  40                  45
   Asn Thr Ile Lys Asn Thr Leu Pro Ser His Lys Glu Gln Asp His Glu
       50                  55                  60
   Gln Lys Glu Gly Asp Lys Glu Pro Ala Lys Ser Gln Ala Gln Lys Glu
   65                  70                  75                  80
   Glu Asn Pro Lys Lys His Arg Ser His Pro Tyr Lys His Ser Phe Arg
                   85                  90                  95
   Ala Arg Gly Ser Ala Ser Tyr Ser Pro Pro Arg Lys Arg Ser Ser Gln
               100                 105                 110
   Asp Lys Tyr Glu Lys Arg Ser Asn Arg Arg
           115                 120
<210> 122
   <211> 117
   <212> PRT
   <213> Homo sapiens
<400> 122
   Met Asn Ala Pro Pro Ala Phe Glu Ser Phe Leu Leu Phe Glu Gly Glu
   1               5                   10                  15
   Lys Lys Ile Thr Ile Asn Lys Asp Thr Lys Val Pro Asn Ala Cys Leu
               20                  25                  30
   Phe Thr Ile Asn Lys Glu Asp His Thr Leu Gly Asn Ile Ile Lys Ser
           35                  40                  45
   Gln Leu Leu Lys Asp Pro Gln Val Leu Phe Ala Gly Tyr Lys Val Pro
       50                  55                  60
   His Pro Leu Glu His Lys Ile Ile Ile Arg Val Gln Thr Thr Pro Asp
   65                  70                  75                  80
   Tyr Ser Pro Gln Glu Ala Phe Thr Asn Ala Ile Thr Asp Leu Ile Ser
                   85                  90                  95
   Glu Leu Ser Leu Leu Glu Glu Arg Phe Arg Val Ala Ile Lys Asp Lys
               100                 105                 110
   Gln Glu Gly Ile Glu
           115
<210> 123
   <211> 1133
   <212> PRT
   <213> Homo sapiens
<400> 123
<210> 124
   <211> 362
   <212> PRT
   <213> Homo sapiens
<400> 124
   Met Leu Leu Val Glu Phe Phe Glu Leu Tyr Gly Arg Asn Phe Asn Tyr
   1 5 10 15
   Leu Lys Thr Gly Ile Arg Ile Lys Glu Gly Gly Ala Tyr Ile Ala Lys
               20                  25                  30
   Glu Glu Ile Met Lys Ala Met Thr Ser Gly Tyr Arg Pro Ser Met Leu
           35                  40                  45
   Cys Ile Glu Asp Pro Leu Leu Pro Gly Asn Asp Val Gly Arg Ser Ser
       50                  55                  60
   Tyr Gly Ala Met Gln Val Lys Gln Val Phe Asp Tyr Ala Tyr Ile Val
   65                  70                  75                  80
   Leu Ser His Ala Val Ser Pro Leu Ala Arg Ser Tyr Pro Asn Arg Asp
                   85                  90                  95
   Ala Glu Ser Thr Leu Gly Arg Ile Ile Lys Val Thr Gln Glu Val Ile
               100                 105                 110
   Asp Tyr Arg Arg Trp Ile Lys Glu Lys Trp Gly Ser Lys Ala His Pro
           115                 120                 125
   Ser Pro Gly Met Asp Ser Arg Ile Lys Ile Lys Glu Arg Ile Ala Thr
       130                 135                 140
   Cys Asn Gly Glu Gln Thr Gln Asn Arg Glu Pro Glu Ser Pro Tyr Gly
   145                 150                 155                 160
   Gln Arg Leu Thr Leu Ser Leu Ser Ser Pro Gln Leu Leu Ser Ser Gly
                   165                 170                 175
   Ser Ser Ala Ser Ser Val Ser Ser Leu Ser Gly Ser Asp Val Asp Ser
               180                 185                 190
   Asp Thr Pro Pro Cys Thr Thr Pro Ser Val Tyr Gln Phe Ser Leu Gln
           195                 200                 205
   Ala Pro Ala Pro Leu Met Ala Gly Leu Pro Thr Ala Leu Pro Met Pro
       210                 215                 220
   Ser Gly Lys Pro Gln Pro Thr Thr Ser Arg Thr Leu Ile Met Thr Thr
   225                 230                 235                 240
   Asn Asn Gln Thr Arg Phe Thr Ile Pro Pro Pro Thr Leu Gly Val Ala
                   245                 250                 255
   Pro Val Pro Cys Arg Gln Ala Gly Val Glu Gly Thr Ala Ser Leu Lys
               260                 265                 270
   Ala Val His His Met Ser Ser Pro Ala Ile Pro Ser Ala Ser Pro Asn
           275                 280                 285
   Pro Leu Ser Ser Pro His Leu Tyr His Lys Gln His Asn Gly Met Lys
       290                 295                 300
   Leu Ser Met Lys Gly Ser His Gly His Thr Gln Gly Gly Gly Tyr Ser
   305                 310                 315                 320
   Ser Val Gly Ser Gly Gly Val Arg Pro Pro Val Gly Asn Arg Gly His
                   325                 330                 335
   His Gln Tyr Asn Arg Thr Gly Trp Arg Arg Lys Lys His Thr His Thr
               340                 345                 350
   Arg Asp Ser Leu Pro Val Ser Leu Ser Arg
           355                 360
<210> 125
   <211> 115
   <212> PRT
   <213> Homo sapiens
<400> 125
   Met Asn Ala Pro Pro Ala Phe Glu Ser Phe Leu Leu Phe Glu Gly Glu
   1 5 10 15
   Lys Ile Thr Ile Asn Lys Asp Thr Lys Val Pro Lys Ala Cys Leu Phe
   20 25 30
   Thr Ile Asn Lys Glu Asp His Thr Leu Gly Asn Ile Ile Lys Ser Gln
   35 40 45
   Leu Leu Lys Asp Pro Gln Val Leu Phe Ala Gly Tyr Lys Val Pro His
   50 55 60
   Pro Leu Glu His Lys Ile Ile Ile Arg Val Gln Thr Thr Pro Asp Tyr
   65 70 75 80
   Ser Pro Gln Glu Ala Phe Thr Asn Ala Ile Thr Asp Leu Ile Ser Glu
   85 90 95
   Leu Ser Leu Leu Glu Glu Arg Phe Arg Thr Cys Leu Leu Pro Leu Arg
   100 105 110
   Leu Leu Pro
   115
<210> 126
   <211> 542
   <212> PRT
   <213> Homo sapiens
<400> 126
   Met Ser Pro Cys Pro Glu Glu Ala Ala Met Arg Arg Glu Val Val Lys
   1               5                   10                  15
   Arg Ile Glu Thr Val Val Lys Asp Leu Trp Pro Thr Ala Asp Val Gln
               20                  25                  30
   Ile Phe Gly Ser Phe Ser Thr Gly Leu Tyr Leu Pro Thr Ser Asp Ile
           35                  40                  45
   Asp Leu Val Val Phe Gly Lys Trp Glu Arg Pro Pro Leu Gln Leu Leu
       50                  55                  60
   Glu Gln Ala Leu Arg Lys His Asn Val Ala Glu Pro Cys Ser Ile Lys
   65                  70                  75                  80
   Val Leu Asp Lys Ala Thr Val Pro Ile Ile Lys Leu Thr Asp Gln Glu
                   85                  90                  95
   Thr Glu Val Lys Val Asp Ile Ser Phe Asn Met Glu Thr Gly Val Arg
               100                 105                 110
   Ala Ala Glu Phe Ile Lys Asn Tyr Met Lys Lys Tyr Ser Leu Leu Pro
           115                 120                 125
   Tyr Leu Ile Leu Val Leu Lys Gln Phe Leu Leu Gln Arg Asp Leu Asn
       130                 135                 140
   Glu Val Phe Thr Gly Gly Ile Ser Ser Tyr Ser Leu Ile Leu Met Ala
   145                 150                 155                 160
   Ile Ser Phe Leu Gln Leu His Pro Arg Ile Asp Ala Arg Arg Ala Asp
                   165                 170                 175
   Glu Asn Leu Gly Met Leu Leu Val Glu Phe Phe Glu Leu Tyr Gly Arg
               180                 185                 190
   Asn Phe Asn Tyr Leu Lys Thr Gly Ile Arg Ile Lys Glu Gly Gly Ala
           195                 200                 205
   Tyr Ile Ala Lys Glu Glu Ile Met Lys Ala Met Thr Ser Gly Tyr Arg
   210 215 220
   Pro Ser Met Leu Cys Ile Glu Asp Pro Leu Leu Pro Gly Asn Asp Val
   225 230 235 240
   Gly Arg Ser Ser Tyr Gly Ala Met Gln Val Lys Gln Val Phe Asp Tyr
   245 250 255
   Ala Tyr Ile Val Leu Ser His Ala Val Ser Pro Leu Ala Arg Ser Tyr
   260 265 270
   Pro Asn Arg Asp Ala Glu Ser Thr Leu Gly Arg Ile Ile Lys Val Thr
   275 280 285
   Gln Glu Val Ile Asp Tyr Arg Arg Trp Ile Lys Glu Lys Trp Gly Ser
   290 295 300
   Lys Ala His Pro Ser Pro Gly Met Asp Ser Arg Ile Lys Ile Lys Glu
   305 310 315 320
   Arg Ile Ala Thr Cys Asn Gly Glu Gln Thr Gln Asn Arg Glu Pro Glu
   325 330 335
   Ser Pro Tyr Gly Gln Arg Leu Thr Leu Ser Leu Ser Ser Pro Gln Leu
   340 345 350
   Leu Ser Ser Gly Ser Ser Ala Ser Ser Val Ser Ser Leu Ser Gly Ser
   355 360 365
   Asp Val Asp Ser Asp Thr Pro Pro Cys Thr Thr Pro Ser Val Tyr Gln
   370 375 380
   Phe Ser Leu Gln Ala Pro Ala Pro Leu Met Ala Gly Leu Pro Thr Ala
   385 390 395 400
   Leu Pro Met Pro Ser Gly Lys Pro Gln Pro Thr Thr Ser Arg Thr Leu
   405 410 415
   Ile Met Thr Thr Asn Asn Gln Thr Arg Phe Thr Ile Pro Pro Pro Thr
   420 425 430
   Leu Gly Val Ala Pro Val Pro Cys Arg Gln Ala Gly Val Glu Gly Thr
   435 440 445
   Ala Ser Leu Lys Ala Val His His Met Ser Ser Pro Ala Ile Pro Ser
   450 455 460
   Ala Ser Pro Asn Pro Leu Ser Ser Pro His Leu Tyr His Lys Gln His
   465                 470                 475                 480
   Asn Gly Met Lys Leu Ser Met Lys Gly Ser His Gly His Thr Gln Gly
                   485                 490                 495
   Gly Gly Tyr Ser Ser Val Gly Ser Gly Gly Val Arg Pro Pro Val Gly
               500                 505                 510
   Asn Arg Gly His His Gln Tyr Asn Arg Thr Gly Trp Arg Arg Lys Lys
           515                 520                 525
   His Thr His Thr Arg Asp Ser Leu Pro Val Ser Leu Ser Arg
       530                 535                 540

## Description

### FIELD AND BACKGROUND OF THE INVENTION

The present invention, in some embodiments thereof, relates to a quinolone compound such as CX-5461 for treating autoimmune diseases and, more particularly, for treating multiple sclerosis.

Autoimmune diseases are caused by an autoimmune response, *i.e.,* an immune response directed to a substance in the body of the subject. The characteristics of the autoimmune diseases vary and depend on the site affected by the autoimmune response.

Multiple sclerosis (MS) is the most common demyelinating disease of the central nervous system (CNS) affecting young adults (disease onset between 20 to 40 years of age) and is the third leading cause for disability after trauma and rheumatic diseases, with an estimated annual cost 34,000 USD per patient (total life time cost of 2.2 million USD per patient).

The disease is characterized by destruction of myelin, associated with death of oligodendrocytes and axonal loss. The main pathologic finding in MS is the presence of infiltrating mononuclear cells, predominantly T lymphocytes and macrophages, which surpass the blood brain barrier and induce an active inflammation within the brain and spinal cord. The neurological symptoms that characterize MS include complete or partial vision loss, diplopia, sensory symptoms, motor weakness that can worsen to complete paralysis, bladder dysfunction and cognitive deficits, which eventually may lead to a significant disability. The associated multiple inflammatory foci lead to myelin destruction, plaques of demyelination, gliosis and axonal loss within the brain and spinal cord and are the reasons contribute to the clinical manifestations of neurological disability.

The etiology of MS is not fully understood. The disease develops in genetically predisposed subjects exposed to yet undefined environmental factors and the pathogenesis involves autoimmune mechanisms associated with autoreactive T cells against myelin antigens. It is well established that not one dominant gene determines genetic susceptibility to develop MS, but rather many genes, each with different influence, are involved.

Clinically, in 85 % of MS patients the illness is initiated with a relapsing-remitting course (RRMS), and in about 10-15 % of MS patients have an a-priori primary progressive course (PPMS) without relapses. RRMS is characterized by inflammatory attacks associated with neurological deficits with periods of remissions between the relapses that vary in time. After a period of 10 years, about 50 % of RRMS patients will progress to a secondary progressive MS (SPMS) course, characterized by permanent neurological dysfunction, with or without relapses and progressive disability.

Benign MS (BMS) is a clinical variant of RRMS in which the patients develop low neurological disability if at all after a disease duration of at least 10 years. Accordingly, this group of MS patients do not experience devastating accumulating disability over-time and when these patients are examined neurologically and scored by the Expanded Disability Status Scale (EDSS) they receive a score that is equal to or lower than 3.0. This low EDSS score signifies mild disability and when this low disability occurs more than 10 years after disease onset, the course of MS is defined as benign (Pittock SJ and Rodriguez M, 2008; Costelloe, L., et al., 2008). Prediction of patients that will have BMS is currently impossible and the definition of these patients is retrospective. The molecular events accountable for the BMS variant of disease are not understood.

WO 2008/081435 publication discloses methods and kits for predicting the prognosis of a subject diagnosed with multiple sclerosis and methods of selecting a treatment regimen of a subject diagnosed with multiple sclerosis.

Achiron A, et al., 2007 (Clinical and Experimental Immunology, 149: 235-242) describe genes of the zinc-ion binding and cytokine activity regulation pathways which predict outcome in relapsing-remitting multiple sclerosis.

WO 2010/113096 discloses methods of predicting clinical course and treating multiple sclerosis.

Current approved drugs for the treatment of MS are either general antiinflammatory agents or immunomodulators and consequently result only in moderate beneficial effects suppressing disease activity.

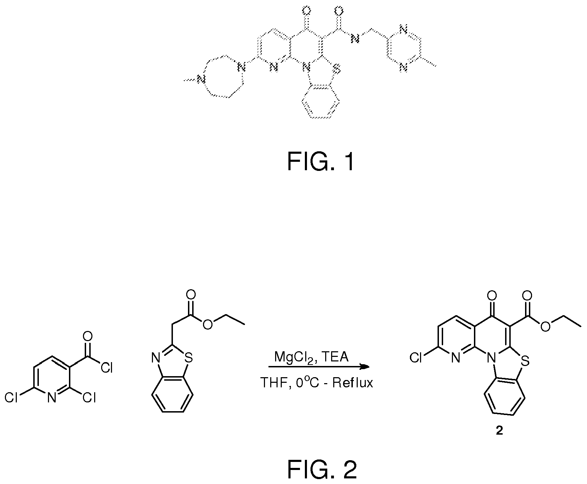

CX-5461 (Figure 1) is a small molecule that was designed to selectively inhibit rRNA synthesis by inhibiting RNA Polymerase I (Pol I), without affecting mRNA synthesis by RNA Polymerase II (Pol II), and without inhibiting DNA replication or protein synthesis (Russell J, Zomerdijk JC. RNA-polymerase-I-directed rDNA transcription, life and works. Trends Biochem Sci 30:87-96, 2005; Drygin D, et al. The RNA polymerase I transcription machinery: an emerging target for the treatment of cancer. Annu Rev Pharmacol Toxicol 50:131-156, 2010). The inhibition of Pol I results in nucleolar stress which causes the release of ribosomal proteins (RP) from the nucleolus and subsequent activation of p53, resulting in cell apoptosis (Kalita K, et al. Inhibition of nucleolar transcription as a trigger for neuronal apoptosis. J Neurochem 105:2286-2299, 2008). In a previous study (Drygin D, et al. Targeting RNA polymerase I with an oral small molecule CX-5461 inhibits ribosomal RNA synthesis and solid tumor growth. Cancer Res 71:1418-1430, 2011) the antiproliferative activity of CX-5461 was studied in cell lines and it was shown that CX-5461 inhibited Pol I activity in human cancer cell lines.

Recent studies indicate that disruption of the SL1/rDNA complex by CX-5461 results from the interference between SL1 and rDNA. SL1, a protein complex containing TATA binding protein-associated factors, is responsible for Pol I promoter specificity. SL1 performs important tasks in the transcription complex assembly, mediating specific interactions between the rDNA promoter region and the Pol I enzyme complex, thereby recruiting Pol I, together with a collection of Pol I-associated factors like RRN3 to rDNA (Cavanaugh A, et al. Mammalian Rrn3 is required for the formation of a transcription competent preinitiation complex containing RNA polymerase I. Gene Expr 14:131-147, 2008).

Additional background art include Leuenroth SJ and Crews CM (Triptolide-induced transcriptional arrest is associated with changes in nuclear substructure. Cancer Res. 2008; 68:5257-5266); Liu Y, et al. (Triptolide, a component of Chinese herbal medicine, modulates the functional phenotype of dendritic cells. Transplantation. 2007; 84:1517-1526); Wang Y, et al. (Triptolide modulates T-cell inflammatory responses and ameliorates experimental autoimmune encephalomyelitis. J Neurosci Res. 2008; 86:2441-2449; EP 0983256; PCT/US1998/008562; WO9852933A1; Alice H. Cavanaugh, et al., 2002 (Rrn3 Phosphorylation is a regulatory checkpoint for ribosome biogenesis J. Biol. Chem., 2002; 277: 27423 - 27432); PCT Pub. No. WO03081201A2.

### SUMMARY OF THE INVENTION

According to an aspect of some embodiments of the present disclosure there is provided a method of treating an autoimmune disease in a subject, comprising:
administering to the subject a therapeutically effective amount of a compound of Formula (I), 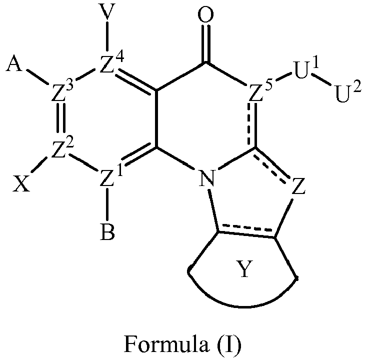
   or a pharmaceutically acceptable salt or ester thereof;
   wherein ------ indicates an optionally unsaturated bond;
   each of B, X, A or V is absent if Z¹, Z², Z³ and Z⁴, respectively, is N; and
   each of B, X, A and V is independently H, halo, azido, -CN, -CF₃, -CONR¹R², - NR¹R²,-SR², -OR², -R³, -W, -L-W, -W⁰, -L-N(R) -W⁰, A² or A³, when each of Z¹, Z², Z³ and Z⁴, respectively, is C;
   Z is O, S, CR⁴₂, NR⁴CR⁴, CR⁴NR⁴, CR⁴, NR⁴ or N;
   each of Z¹, Z², Z³ and Z⁴ is independently C or N, provided any three N are non-adjacent;
   Z⁵ is C; or Z⁵ may be N when Z is N;
   Y is an optionally substituted 5-6 membered carbocyclic or heterocyclic ring;
   U¹ is -C(=T)N(R)-, -C(=T)N(R)O-, -C(=T)-, -SO₂N(R)-, -SO₂N(R)N(R⁰)-, -SO₂-, or - SO₃-, where T is O, S, or NH; or U¹ may be a bond when Z⁵ is N or U² is H;
   U² is H, or C3-C7 cycloalkyl, C1-C10 alkyl, C1-C10 heteroalkyl, C2-C10 alkenyl or C2-C10 heteroalkenyl group, each of which may be optionally substituted with one or more halogens, =O, or an optionally substituted 3-7 membered carbocyclic or heterocyclic ring; or U² is -W, -L-W, -L-N(R)-W⁰, A² or A³;
   in each -NR¹R², R¹ and R² together with N may form an optionally substituted azacyclic ring, optionally containing an additional heteroatom selected from N, O and S as a ring member;
   R¹ is H or C1-C6 alkyl, optionally substituted with one or more halogens, or =O;
   R² is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
   R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
   each R⁴ is independently H, or C1-C6 alkyl; or R⁴ may be -W, -L-W or -L-N(R)-W⁰; each R and R⁰ is independently H or C1-C6 alkyl;
   L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl;
   W is an optionally substituted 4-7 membered azacyclic ring, optionally containing an additional heteroatom selected from N, O and S as a ring member;
   W⁰ is an optionally substituted 3-4 membered carbocyclic ring, or a C1-C6 alkyl group substituted with from 1 to 4 fluorine atoms;
   provided one of U², V, A, X and B is a secondary amine A² or a tertiary amine A³, wherein
   the secondary amine A² is -NH-W⁰, and
   the tertiary amine A³ is a fully saturated and optionally substituted six-membered or seven-membered azacyclic ring optionally containing an additional heteroatom selected from N, O or S as a ring member, or the tertiary amine A³ is a partially unsaturated or aromatic optionally substituted five-membered azacyclic ring, optionally containing an additional heteroatom selected from N, O or S as a ring member;
thereby treating the autoimmune disease in the subject.

According to some embodiments of the disclosure, the Z¹ is N, and each of Z², Z³ and Z⁴ is C.

According to some embodiments of the disclosure, the U is -W or -L-W, where W is an optionally substituted 5-6 membered unsaturated or aromatic azacyclic ring, optionally containing an additional heteroatom selected from N, O and S; or W is an optionally substituted 5-7 membered saturated azacyclic ring containing an additional heteroatom selected from N and S.

According to some embodiments of the disclosure, the U² is -L-N(R)-W⁰. According to some embodiments of the disclosure, the Y is an optionally substituted phenyl ring.

According to some embodiments of the disclosure, with the proviso that when Z¹ is N, Z² and Z⁴ are C, Z⁵ is C, U¹ is -C(O)NH-, U² is -L-W, and L is an ethylene linker, one of V, A, and X is independently an optionally substituted aryl, heteroaryl, or 7-membered azacyclic ring, optionally containing an additional heteroatom selected from N, O and S as a ring member, if W is pyrrolidin-1-yl, N-methyl-pyrrolidin-2-yl, piperidin-1-yl or morpholin-1-yl

According to an aspect of some embodiments of the present disclosure there is provided a method of treating an autoimmune disease in a subject, comprising:
administering to the subject a therapeutically effective amount of a compound of Formula (II), 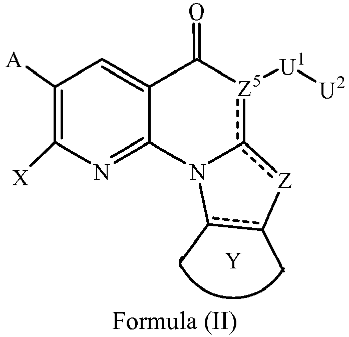
   or a pharmaceutically acceptable salt or ester thereof;
   wherein ----- indicates an optionally unsaturated bond;
   each of A and X is independently H, halo, azido, -CN, -CF₃, -CONR¹R², -NR¹R², -SR², - OR², -R³, -W, -L-W, -W⁰, -L-N(R)-W⁰, A² or A³;
   Z is O,S, CR⁴₂, NR⁴CR⁴, CR⁴NR⁴ or NR⁴;
   Y is an optionally substituted 5-6 membered carbocyclic or heterocyclic ring;
   U¹ is -C(=T)N(R)-, -C(=T)N(R)O-, -C(=T)-, -SO₂N(R)-, -SO₂N(R)N(R⁰)-, -SO₂-, or - SO₃-, where T is O, S, or NH; or U¹ may be a bond when U2 is H;
   U² is H, or C3-C7 cycloalkyl, C1-C10 alkyl, C1-C10 heteroalkyl, C2-C10 alkenyl or C2-C10 heteroalkenyl group, each of which may be optionally substituted with one or more halogens, =O, or an optionally substituted 3-7 membered carbocyclic or heterocyclic ring; or U² is -W, -L-W, -L-N(R)-W⁰, A² or A³;
   in each -NR¹R², R¹ and R² together with N may form an optionally substituted azacyclic ring, optionally containing an additional heteroatom selected from N, O and S as a ring member;
   R¹ is H or C1-C6 alkyl, optionally substituted with one or more halogens, or =O;
   R2 is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
   R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
   each R⁴ is independently H, or C1-C6 alkyl; or R⁴ may be -W, -L-W or -L-N(R)-W0; each R and R⁰ is independently H or C1-C6 alkyl;
   L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl;
   W is an optionally substituted 4-7 membered azacyclic ring, optionally containing an additional heteroatom selected from N, O and S as a ring member;
   W⁰ is an optionally substituted 3-4 membered carbocyclic ring, or a C1-C6 alkyl group substituted with from 1 to 4 fluorine atoms;
   provided that one of U², A, and X is a secondary amine A² or a tertiary amine A³, wherein
   the secondary amine A² is -NH-W⁰, and
   the tertiary amine A³ is a fully saturated and optionally substituted six-membered or seven-membered azacyclic ring optionally containing an additional heteroatom selected from N, O or S as a ring member, or the tertiary amine A³ is a partially unsaturated or aromatic optionally substituted five-membered azacyclic ring optionally containing an additional heteroatom selected from N, O or S as a ring member;
thereby treating the autoimmune disease in the subject.

According to some embodiments of the disclosure, the at least one of A and X is a tertiary amine A³.

According to some embodiments of the disclosure, the A³ is selected from the group consisting of imidazole, imidazoline, pyrroline, piperidine, piperazine, morpholine, thiomorpholine and homopiperazine.

According to some embodiments of the disclosure, the U¹ is a -C(=T)N(R)-, T is O, and U² is -L-W or -L-N(R)-W⁰.

According to some embodiments of the disclosure, with the proviso that when U¹ is -C(O)NH-, U² is -L-W, and L is an ethylene linker, one of A and X is independently an optionally substituted aryl, heteroaryl, or 7-membered azacyclic ring, optionally containing an additional heteroatom selected from N, O and S as a ring member, if W is pyrrolidin-1-yl, N-methyl-pyrrolidin-2-yl, piperidin-1-yl or morpholin-1-yl.

According to an aspect of some embodiments of the present disclosure there is provided a method of treating an autoimmune disease in a subject, comprising:
administering to the subject a therapeutically effective amount of a compound of Formula (III), 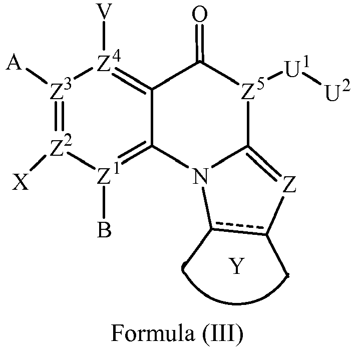
   or a pharmaceutically acceptable salt or ester thereof;
   wherein ---- indicates an optionally unsaturated bond; and
   each of B, X, A or V is absent if Z¹, Z², Z³ and Z⁴, respectively, is N; and
   each of B, X, A and V is independently H, halo, azido, -CN, -CF₃, -CONR¹R², -NR¹R², - SR², -OR², -R³, -W, -L-W, -W⁰, -L-N(R)-W⁰, A² or A³, when each of Z¹, Z², Z³ and Z⁴, respectively, is C;
   each of Z¹, Z², Z³ and Z⁴ is independently C or N, provided any three N are non-adjacent;
   Y is an optionally substituted 5-6 membered carbocyclic or heterocyclic ring;
   U¹ is -C(=T)N(R)-, -C(=T)N(R)O-, -C(=T)-, -SO₂N(R)-, -SO₂N(R)N(R⁰)-, -SO₂-, or - SO₃-, where T is O, S, or NH; or U¹ may be a bond when Z⁵ is N or U² is H;
   U² is H, or C3-C7 cycloalkyl, C1-C10 alkyl, C1-C10 heteroalkyl, C2-C10 alkenyl or C2-C10 heteroalkenyl group, each of which may be optionally substituted with one or more halogens, =O, or an optionally substituted 3-7 membered carbocyclic or heterocyclic ring; or U² is -W, -L-W, -L-N(R) -W⁰, A² or A³;
   in each -NR¹R², R¹ and R² together with N may form an optionally substituted azacyclic ring, optionally containing an additional heteroatom selected from N, O and S as a ring member;
   R¹ is H or C1-C6 alkyl, optionally substituted with one or more halogens, or =O;
   R² is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
   R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
   each R⁴ is independently H, or C1-C6 alkyl; or R⁴ may be -W, -L-W or -L-N(R)-W⁰; each R and R⁰ is independently H or C1-C6 alkyl;
   L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl;
   W is an optionally substituted 4-7 membered azacyclic ring, optionally containing an additional heteroatom selected from N, O and S as a ring member;
   W⁰ is an optionally substituted 3-4 membered carbocyclic ring, or a C1-C6 alkyl group substituted with from 1 to 4 fluorine atoms;
   provided that one of U², V, A, X and B is a secondary amine A² or a tertiary amine A3, wherein
   the secondary amine A² is -NH-W⁰, and
   the tertiary amine A³ is a fully saturated and optionally substituted six-membered or seven-membered azacyclic ring optionally containing an additional heteroatom selected from N, O or S as a ring member, or the tertiary amine A³ is a partially unsaturated or aromatic optionally substituted five-membered azacyclic ring, optionally containing an additional heteroatom selected from N, O or S as a ring member,
thereby treating the autoimmune disease in the subject.

According to an aspect of some embodiments of the present disclosure there is provided a method of treating an autoimmune disease in a subject, comprising:
administering to the subject a therapeutically effective amount of a compound of Formula (IV), 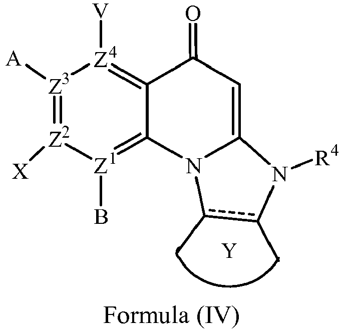
   or a pharmaceutically acceptable salt or ester thereof;
   wherein ----- indicates an optionally unsaturated bond;
   each of B, X, A or V is absent if Z¹, Z², Z³ and Z⁴ respectively, is N; and each of B, X, A and V is independently H, halo, azido, -CN, -CF₃, -CONR¹R²,-NR¹R², -SR², -OR², -R³, -W, -L-W, -W⁰, -L-N(R)-W⁰, A² or A³, when each of Z¹, Z², Z³ and Z⁴, respectively, is C;
   each of Z¹, Z², Z³ and Z⁴ is independently C or N, provided any three N are non-adjacent;
   Y is an optionally substituted 5-6 membered carbocyclic or heterocyclic ring;
   in each NR¹R², R¹ and R² together with N may form an optionally substituted azacyclic ring, optionally containing an additional heteroatom selected from N, O and S as a ring member;
   R¹ is H or C1-C6 alkyl, optionally substituted with one or more halogens, or =O;
   R² is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
   R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
   R⁴ is -W, -L-W or -L-N(R)-W0; and
   each R is independently H or C1-C6 alkyl;
   L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl;
   W is an optionally substituted 4-7 membered azacyclic ring, optionally containing an additional heteroatom selected from N, O and S as a ring member; and
   W⁰ is an optionally substituted 3-4 membered carbocyclic ring, or a C1-C6 alkyl group substituted with from 1 to 4 fluorine atoms,
thereby treating the autoimmune disease in the subject.

According to an aspect of some embodiments of the present disclosure there is provided a method of treating an autoimmune disease in a subject, comprising:
administering to the subject a therapeutically effective amount of a compound of Formula (V), 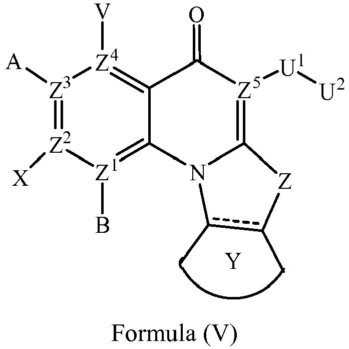
   or a pharmaceutically acceptable salt or ester thereof;
   ---- indicates an optionally unsaturated bond;
   A and V independently are H, halo, azido, -CN, -CF₃, -CONR¹R², -NR¹R², -SR², -OR², - R³, -W, -L-W, -W⁰, -L-N(R)-W⁰, A² or A³,
   Z is O, S, CR⁴₂, NR⁴CR⁴, CR⁴NR⁴ or NR⁴;
   Y is an optionally substituted 5-6 membered carbocyclic or heterocyclic ring;
   U¹ is -C(=T)N(R)-, -C(=T)N(R)O-, -C(=T)-, -SO₂N(R)-, -SO₂N(R)N(R⁰)-, -SO₂-, or - SO₃-, where T is O, S, or NH; or U¹ may be a bond when U2 is H;
   U² is H, or C3-C7 cycloalkyl, C1-C10 alkyl, C1-C10 heteroalkyl, C2-C10 alkenyl or C2-C10 heteroalkenyl group, each of which may be optionally substituted with one or more halogens, =O, or an optionally substituted 3-7 membered carbocyclic or heterocyclic ring; or U² is -W, -L-W or -L-N(R)-W⁰, A² or A³;
   in each -NR¹R², R¹ and R² together with N may form an optionally substituted azacyclic ring, optionally containing an additional heteroatom selected from N, O and S as a ring member;
   R¹ is H or C1-C6 alkyl, optionally substituted with one or more halogens, or =O;
   R² is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
   R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
   each R⁴ is independently H, or C1-C6 alkyl; or R4 may be -W, -L-W or -L-N(R)-W⁰;
   each R and R⁰ is independently H or C1-C6 alkyl;
   L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl; W is an optionally substituted 4-7 membered azacyclic ring, optionally containing an additional heteroatom selected from N, O and S as a ring member;
   W⁰ is an optionally substituted 3-4 membered carbocyclic ring, or a C1-C6 alkyl group substituted with from 1 to 4 fluorine atoms;
   provided one of U², A, and V is a secondary amine A² or a tertiary amine A3, wherein the secondary amine A² is -NH-W0, and
   the tertiary amine A³ is a fully saturated and optionally substituted six-membered or seven-membered azacyclic ring optionally containing an additional heteroatom selected from N, O or S as a ring member, or the tertiary amine A³ is a partially unsaturated or aromatic optionally substituted five-membered azacyclic ring optionally containing an additional heteroatom selected from N, O or S as a ring member,
thereby treating the autoimmune disease in the subject.

According to the present invention there is provided a compound of Formula (VI), 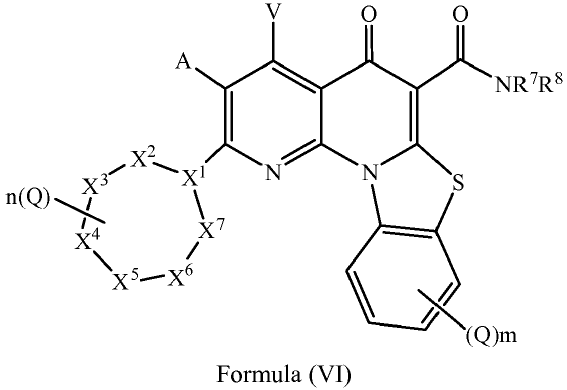 or a pharmaceutically acceptable salt or ester thereof;
wherein:
X¹ is CH or N;
X², X³, X⁴, X⁵, X⁶ and X⁷ independently are NR⁴, CH₂, CHQ or C(Q)₂, provided that: (i) zero, one or two of X², X³, X⁴, X⁵, X⁶ and X⁷ are NR⁴; (ii) when X¹ is N, both of X² and X⁷ are not NR⁴; (iii) when X¹ is N, X³ and X⁶ are not NR⁴; and (iv) when X¹ is CH and two of X², X³, X⁴, X⁵, X⁶ and X⁷ are NR⁴, the two NR⁴ are located at adjacent ring positions or are separated by two or more other ring positions;
A and V independently are H, halo, azido, -CN, -CF₃, -CONR¹R², -NR¹R², -SR², -OR², - R³, -W, -L-W, -W⁰, or -L-N(R)-W⁰;
each Q is independently halo, azido, -CN, -CF₃, -CONR¹R², -NR¹R², -SR², -OR², -R³, - W, -L-W, -W⁰, or -L-N(R)-W⁰;
in each -NR¹R², R¹ and R² together with N may form an optionally substituted azacyclic ring, optionally containing one additional heteroatom selected from N, O and S as a ring member;
R¹ is H or C1-C6 alkyl, optionally substituted with one or more halogens, or =O; R² is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
each R⁴ is independently H, or C1-C6 alkyl; or R4 may be -W, -L-W or -L-N(R)-W⁰; each R is independently H or C1-C6 alkyl;
R⁷ is H and R⁸ is C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring; or in - NR⁷R⁸, R⁷ and R⁸ together with N may form an optionally substituted azacyclic ring, optionally containing an additional heteroatom selected from N, O and S as a ring member;
m is 0, 1, 2, 3 or 4;
n is 0, 1, 2, 3, 4, or 5;
L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl; W is an optionally substituted 4-7 membered azacyclic ring, optionally containing an additional heteroatom selected from N, O and S as a ring member; and W⁰ is an optionally substituted 3-4 membered carbocyclic ring, or a C1-C6 alkyl group substituted with from 1 to 4 fluorine atoms, for use in treating multiple sclerosis in a subject.

According to some embodiments of the invention, the X¹ is CH and two of X², X³, X⁴, X⁵, X⁶ and X⁷ are NR⁴.

According to some embodiments of the invention, the X¹ is CH and one of X², X³, X⁴, X⁵, X⁶ and X⁷ are NR⁴.

According to some embodiments of the invention, the X¹ is CH and none of X², X³, X⁴, X⁵, X⁶ and X⁷ are NR⁴.

According to some embodiments of the invention, the X¹ is N and none of X², X³, X⁴, X⁵, X⁶ and X⁷ are NR⁴.

According to some embodiments of the invention, the X¹ is N and one of X⁴ or X⁵ is NR⁴.

According to an aspect of some embodiments of the present invention there is provided a compound of Formula (VIII), 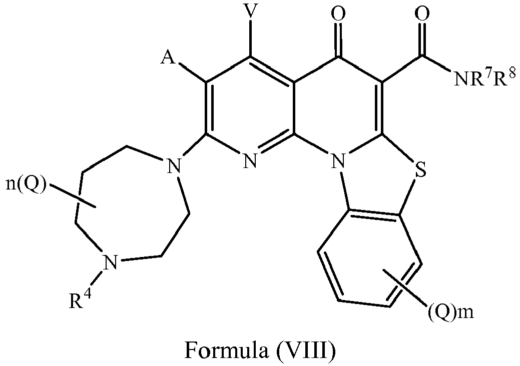 or a pharmaceutically acceptable salt or ester thereof;
wherein:
A and V independently are H, halo, azido, -CN, -CF₃, -CONR¹R², -NR¹R², -SR², -OR², - R³, -W, -L-W, -W⁰, or -L-N(R)-W⁰;
each Q is independently halo, azido, -CN, -CF₃, -CONR¹R², -NR¹R², -SR², -OR², -R³, - W, -L-W, -W⁰, or -L-N(R)-W⁰;
in each -NR¹R², R¹ and R² together with N may form an optionally substituted azacyclic ring, optionally containing an additional heteroatom selected from N, O and S as a ring member;
R¹ is H or C1-C6 alkyl, optionally substituted with one or more halogens, or =O;
R² is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
each R⁴ is independently H, or C1-C6 alkyl; or R4 may be -W, -L-W or -L-N(R)-W⁰; each R is independently H or C1-C6 alkyl;
R⁷ is H and R⁸ is C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring; or in - NR⁷R⁸, R⁷ and R⁸ together with N may form an optionally substituted azacyclic ring, optionally containing an additional heteroatom selected from N, O and S as a ring member;
m is 0, 1, 2, 3 or 4;
n is 0, 1, 2, 3, 4 or 5;
p is 0, 1, 2 or 3;
L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl;
W is an optionally substituted 4-7 membered azacyclic ring, optionally containing an additional heteroatom selected from N, O and S as a ring member; and
W⁰ is an optionally substituted 3-4 membered carbocyclic ring, or a C1-C6 alkyl group substituted with from 1 to 4 fluorine atoms, for use in treating multiple sclerosis in a subject.

According to some embodiments of the invention, the R⁷ is H and R⁸ is a C₁₋₄ alkyl substituted with an optionally substituted aromatic heterocyclic ring.

According to some embodiments of the invention, the optionally substituted aromatic heterocyclic ring is selected from pyridine, pyrimidine, pyrazine, imidazole, pyrrolidine, and thiazole.

According to some embodiments of the invention, the R⁷ and R⁸ together with N in -NR⁷R⁸ form an optionally substituted azacyclic ring selected from the group consisting of morpholine, thiomorpholine, piperidine or piperazine ring.

According to an aspect of some embodiments of the present invention there is provided a compound of Formula (VII) 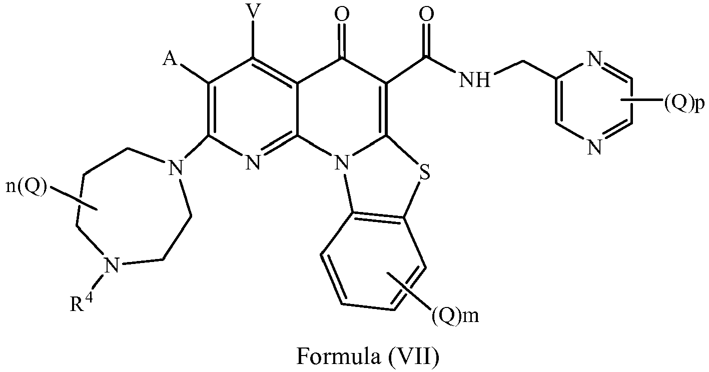
or a pharmaceutically acceptable salt or ester thereof;
A and V independently are H, halo, azido, -CN, -CF₃, -CONR1R2, -NR1R2, -SR², -OR², -R³, -W, -L-W, -W⁰, or -L-N(R)-W⁰;
each Q is independently halo, azido, -CN, -CF₃, -CONR¹R², -NR¹R², -SR², -OR², -R³, - W, -L-W, -W⁰, or -L-N(R)-W⁰;
in each -NR¹R², R¹ and R² together with N may form an optionally substituted azacyclic ring, optionally containing an additional heteroatom selected from N, O and S as a ring member;
R¹ is H or C1-C6 alkyl, optionally substituted with one or more halogens, or =O;
R2 is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
each R⁴ is independently H, or C1-C6 alkyl; or R⁴ may be -W, -L-W or -L-N(R)-W⁰;
each R is independently H or C1-C6 alkyl;
m is 0, 1, 2, 3 or 4;
n is 0, 1, 2, 3, 4 or 5;
p is 0, 1, 2 or 3;
L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl;
W is an optionally substituted 4-7 membered azacyclic ring, optionally containing an additional heteroatom selected from N, O and S as a ring member; and
W⁰ is an optionally substituted 3-4 membered carbocyclic ring, or a C1-C6 alkyl group substituted with from 1 to 4 fluorine atoms, for use in treating multiple sclerosis in a subject.

According to some embodiments of the invention, the A and V are independently H or halo.

According to some embodiments of the invention, the R⁴ is H or C1-4 alkyl.

According to some embodiments of the invention, the m and n are each 0.

According to some embodiments of the invention, the p is 0 or 1.

According to some embodiments of the invention, the compound having the structure: 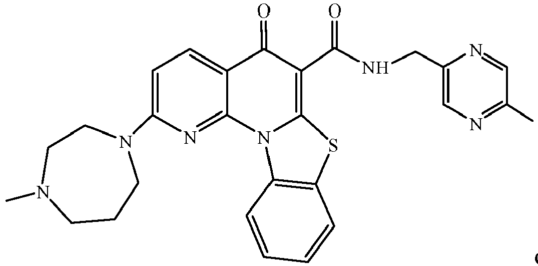 or a pharmaceutically acceptable salt or ester thereof.

According to an aspect of some embodiments of the present invention there is provided a compound selected from the group consisting of: 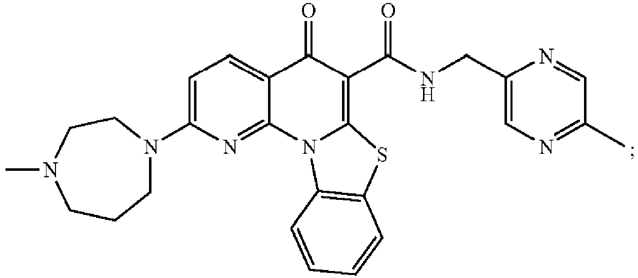 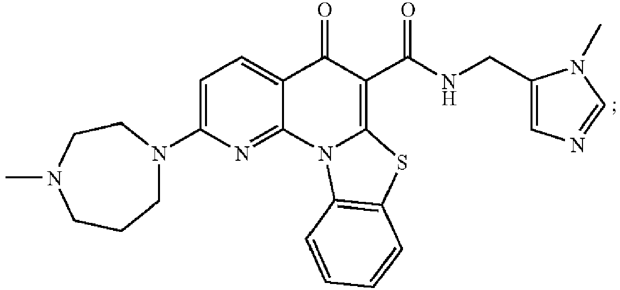 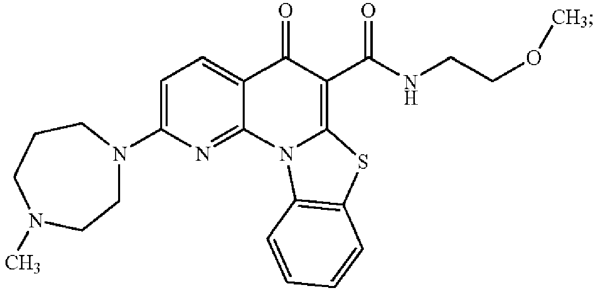 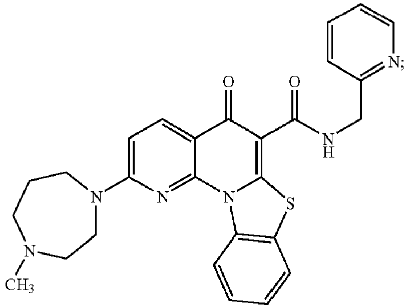 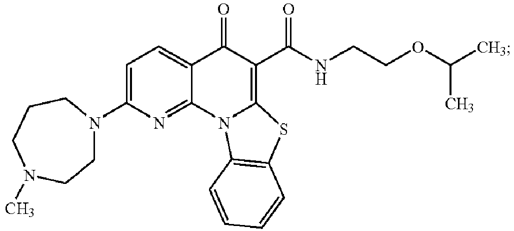 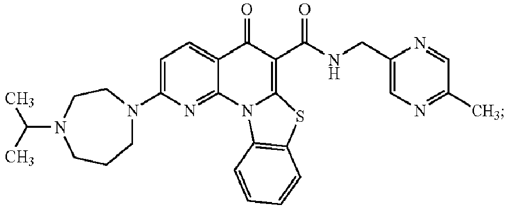 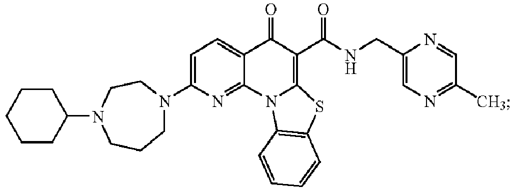 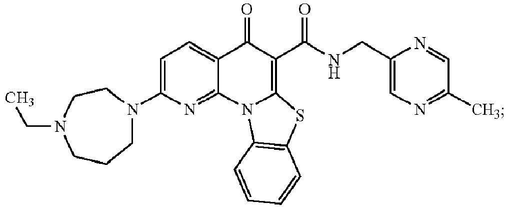 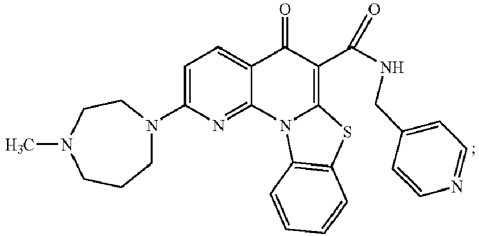 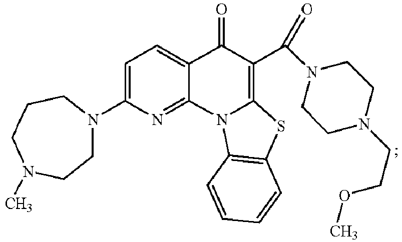 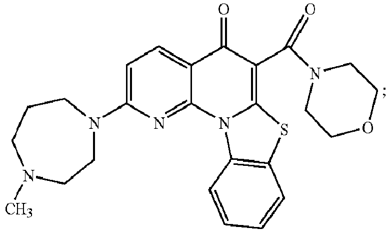 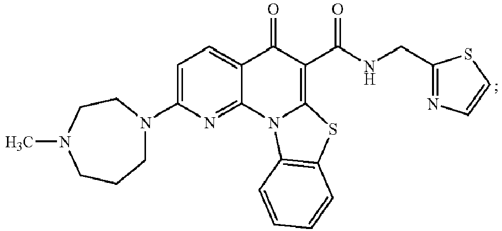 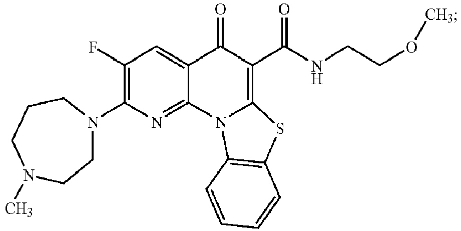 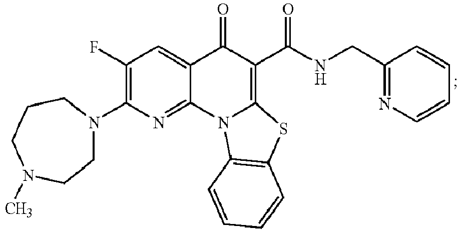 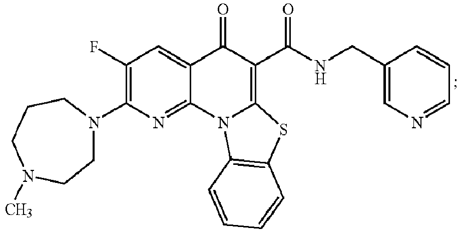 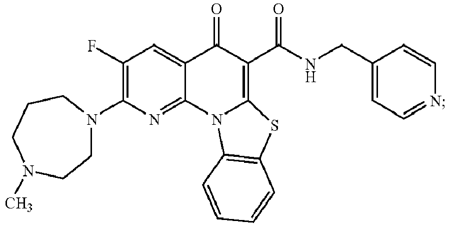 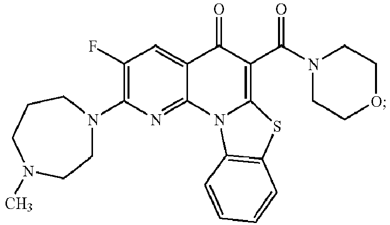 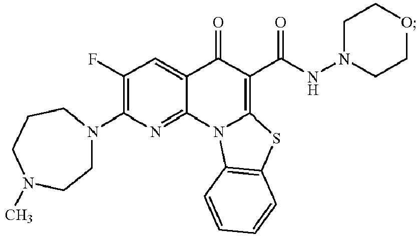 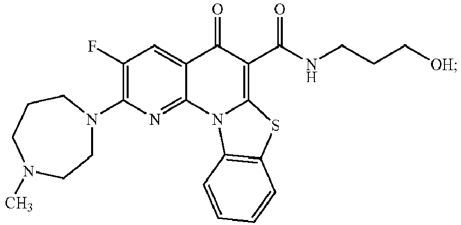 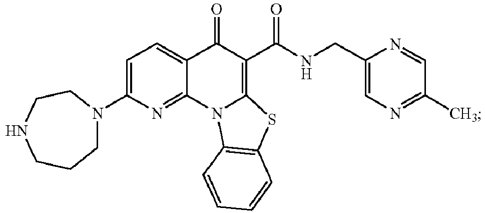 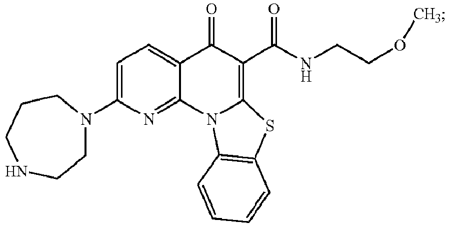 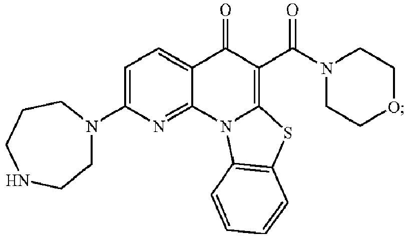 and 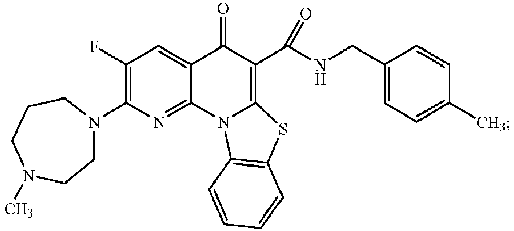 or a pharmaceutically acceptable salt or ester thereof, for use in treating multiple sclerosis in a subject.

According to an aspect of some embodiments of the present invention there is provided a method of monitoring treatment efficiency of a subject having an autoimmune disease, the method comprising:
(a) treating the subject with the compound according to the method of some embodiments of the invention, and
(b) comparing a level of expression of least one gene involved in the RNA polymerase I pathway in a cell of the subject following the treating with the compound to a level of expression of the at least one gene in a cell of the subject prior to the treating the subject with the compound,
   (i) wherein a decrease above a predetermined threshold in the level of expression of the at least one gene following the treating with the compound relative to the level of expression of the at least one gene prior to the treating with the compound indicates that the compound is efficient for treating the subject;
   (ii) wherein an increase above a predetermined threshold in the level of expression of the at least one gene following the treating with the compound relative to the level of expression of the at least one gene prior to the treating with the compound indicates that the compound is not efficient for treating the subject; or
   (iii) wherein when a level of expression of the at least one gene following the treating with the compound is identical or changed below a predetermined threshold as compared to prior to the treating with the compound then the treatment is not efficient for treating the subject
   thereby monitoring treatment efficiency of the subject having an autoimmune disease.

According to some embodiments of the invention, the compound is 2-(4-methyl-1,4-diazepan-1-yl)- N-((5-methylpyrazin-2-yl)methyl)-5-oxo-5H-benzo[4,5]thiazolo[3,2-a][1,8]naphthyridine-6-carboxamide as depicted in Formula

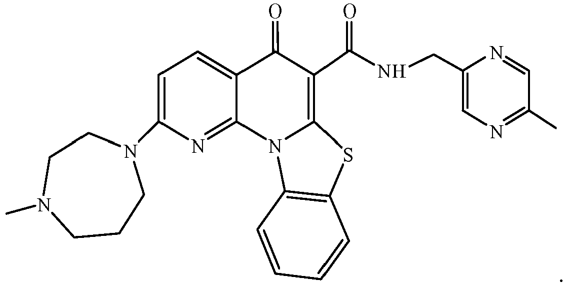

According to the invention, the autoimmune disease is multiple sclerosis.

According to some embodiments of the invention, the compound is 2-(4-methyl-1,4-diazepan-1-yl)- N-((5-methylpyrazin-2-yl)methyl)-5-oxo-5H-benzo[4,5]thiazolo[3,2-a][1,8]naphthyridine-6-carboxamide as depicted in Formula 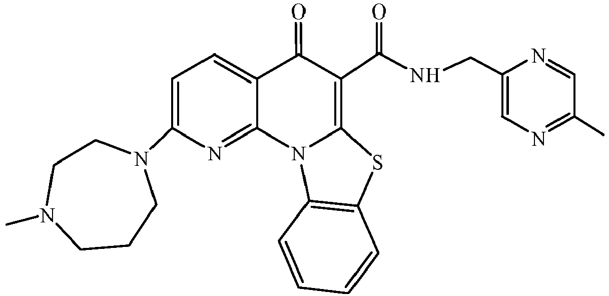 and wherein the autoimmune disease is multiple sclerosis.

According to some embodiments of the invention, the multiple sclerosis is a relapsing-remitting course of multiple sclerosis.

According to some embodiments of the invention, the multiple sclerosis is a progressive course of multiple sclerosis.

According to some embodiments of the invention, the multiple sclerosis is a benign multiple sclerosis.

According to some embodiments of the invention, the multiple sclerosis is a progressive-relapsing course.

According to some embodiments of the invention, administering the compound is performed following diagnosis of the autoimmune disease.

According to some embodiments of the invention, the autoimmune disease is multiple sclerosis and whereas the diagnosis comprises appearance of brain lesions characteristics of the multiple sclerosis.

According to some embodiments of the invention, the compound is provided at a concentration range of about 0.081-1.62 mg/kg/day.

According to some embodiments of the invention, the compound is provided at a concentration range of about 0.81-1.215 mg/kg/day.

According to some embodiments of the invention, the compound is provided at a concentration of about 1.01 mg/kg/day.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a schematic illustration of the chemical structure of CX-5461;
FIG. 2 is a schematic illustration of the chemical formation of ethyl 2-chloro-5-oxo-5H-benzo[4,5]thiazolo[3,2-a][1,8]naphthyridine-6-carboxylate (2);

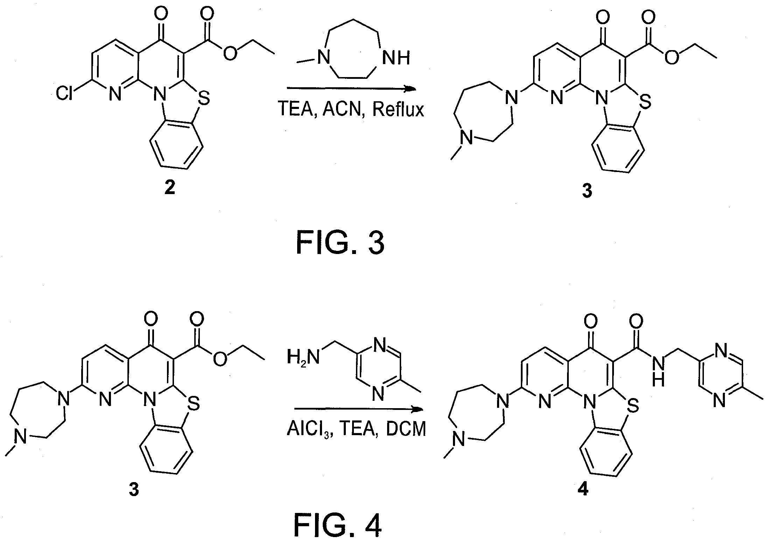

FIG. 3 is a schematic illustration depicting the chemical conversion of ethyl 2-chloro-5-oxo-5H-benzo[4,5]thiazolo[3,2-a][1,8]naphthyridine-6-carboxylate (2) to ethyl 2-(4-methyl-1,4-diazepan-1-yl)-5-oxo-5H-benzo[4,5]thiazolo[3,2-a][1,8]naphthyridine-6-carboxylate (3);
FIG. 4 is a is a schematic illustration depicting the chemical conversion of ethyl 2-(4-methyl-1,4-diazepan-1-yl)-5-oxo-5H-benzo[4,5]thiazolo[3,2-a][1,8]naphthyridine-6-carboxylate (3) to 2-(4-methyl-1,4-diazepan-1-yl)-N-((5-methylpyrazin-2-yl)methyl)-5-oxo-5H-benzo[4,5]thiazolo[3,2-a][1,8]naphthyridine-6-carboxamide(4);

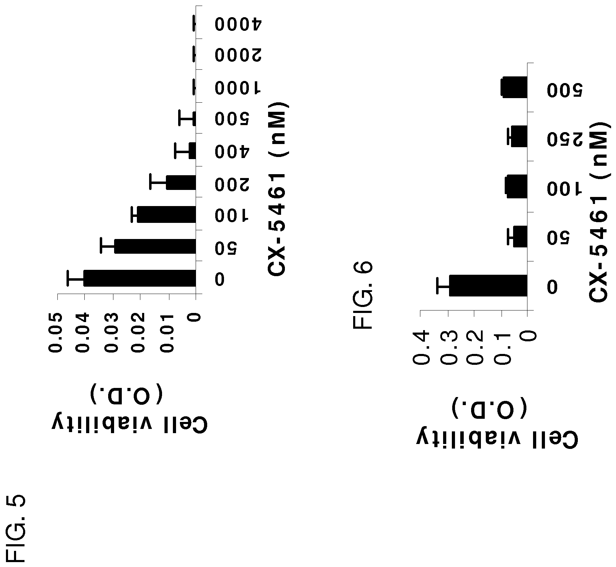

FIG. 5 is a histogram depicting the effect of CX-5461 on viability of mouse splenocytes. Mouse splenocytes were plated in DMEM+10 % FCS+P/S+Q and 10 µg/ml PHA (250,000 cell/well) in the presence of increased concentrations of CX-5461 (50-4000 nM) for 72 hours. Cells cultured with PHA alone served as controls. Following incubation cell viability was determined by XTT assay;
FIG. 6 is a histogram depicting the effect of CX-5461 on viability of human PBMCs. Human PBMCs were plated in DMEM+10 % FCS+P/S+Q and 20 µg/ml PHA (400,000 cell/well) in the presence of increased concentrations of CX-5461 (50-500 nM) for 72 hours. Cells cultured with PHA alone served as controls. After incubation cell viability was determined by XTT assay;

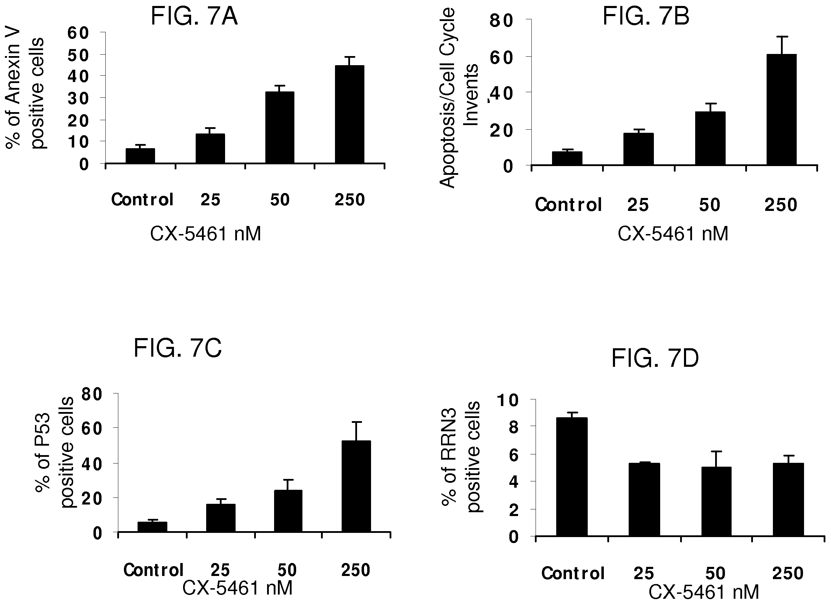

FIGs. 7A-D are histograms depicting the effects of CX-5461 on apoptosis of human PBMCs. Human PBMCs were plated in DMEM+10 % FCS+P/S+Q (400,000 cell/well) in the presence of elevated concentration of CX-5461 (25-250 nM) for 24 hours. Cells cultured with vehicle alone served as controls. After incubation the percent of cells positive for Annexin V (Figure 7A), P53 (Figure 7C), RRN3 (Figure 7D) and total apoptotic cells was determined by flow cytometry (Figure 7B);

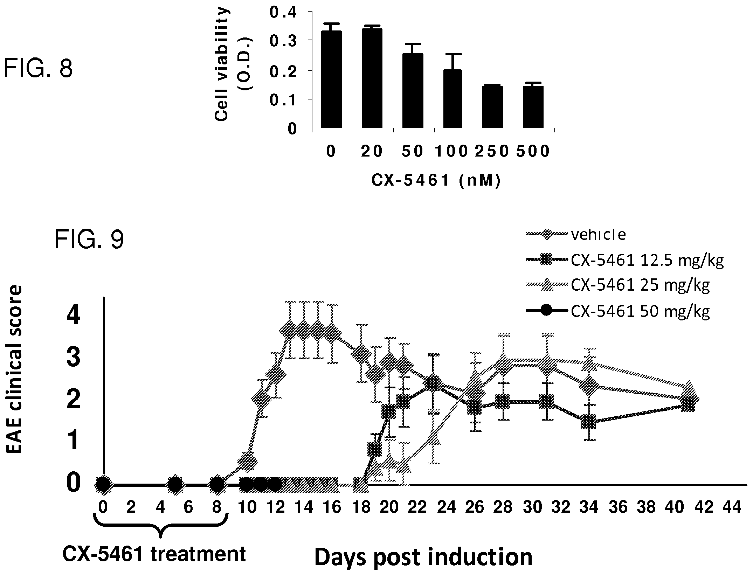

FIG. 8 is a histogram depicting the effects of CX-5461 on viability of neuronal progenitor cells (NPCs). Mouse NPCs were plated in neurospheres medium containing basic FGF and EGF at 20 ng/ml each (26,000 cell/well) in the presence of increased concentrations of CX-5461 (20-500 nM) for 72 hours. Cells cultured without CX-5461 served as controls. Following incubation cell viability was determined by XTT assay;
FIG. 9 is a graph depicting the EAE clinical score in mice treated with CX-5461 on the same day when EAE was induced in the mice. Note the delayed onset and suppressed severity of MOG induced EAE by various concentrations of CX-5461 administered from EAE induction.

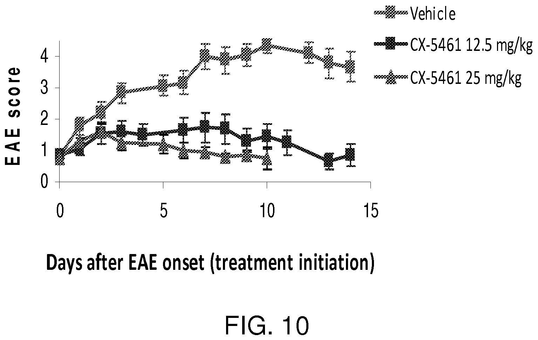

FIG. 10 is a graph depicting the EAE clinical score in mice treated with CX-5461 after EAE onset in the mice. Note the suppression of MOG induced EAE by CX-5461 treatment CX-5461 administered from EAE clinical onset.

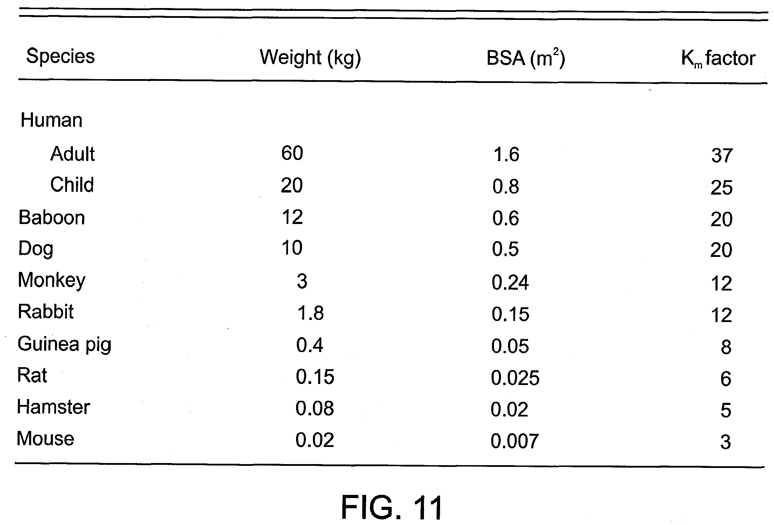

FIG. 11 is art accepted conversion Table approved by the FDA for determination of human equivalent dose (HED) based on body surface area (BSA) (Reagan-Shaw S., et al., FASEB J. 22:659-661 (2007)). To convert dose in mg/kg to dose in mg/m², multiply by Kₘ value. The human equivalent dose is calculated as follows: HED (mg/kg) = Animal dose (mg/kg) multiplied by (Animal Kₘ/human Kₘ).

### DESCRIPTION OF SPECIFIC EMBODIMENTS OF THE INVENTION

The present invention, in some embodiments thereof, relates to a quinolone compound such as CX-5461 for treating autoimmune diseases and, more particularly, for treating multiple sclerosis

The present inventors have uncovered that 2-(4-methyl-1,4-diazepan-1-yl)-N-((5-methylpyrazin-2-yl)methyl)-5-oxo-5H-benzo[4,5]thiazolo[3,2-a][1,8]naphthyridine-6-carboxamide (also known as "CX-5461"), which is a potent RNA Polymerase I (Pol I) inhibitor, can be used to treat autoimmune diseases such as multiple sclerosis.

As shown in the Examples section which follows, the present inventors uncovered that the quinolone compound CX-5461 can reduce the viability of immune cells (e.g., splenocytes) in a dose-related manner (Example 2, Figure 5), reduce the viability of normal (e.g., healthy) PBMCs (Example 3, Figure 6), and induce apoptosis of PBMCs obtained from multiple sclerosis subjects (Example 4, Figures 7A-B). In addition, the present inventors have uncovered that CX-5461 increases the level of p53 in PBMCs obtained from multiple sclerosis subjects (Example 4, Figure 7C) and decreases the number of RRN3-positive cells in PBMCs obtained from multiple sclerosis subjects (Example 4, Figure 7D). The present inventors have further tested the viability of neuronal progenitor cells (NPCs) in the presence of the CX-5461 compound, and found that CX-5461 does not affect NPCs' viability at a concentration lower than 20 nM, and does not suppress more than 50% viability at concentrations higher than 50 nM (Example 5, Figure 8). Moreover, the present inventors performed *in-vivo* experiments in which they tested the ability of CX-5461 to treat EAE, animal model of multiple sclerosis. Thus, as shown in Example 6 of the Examples section which follows, when CX-5461 was administered to the animals along with MOG-induction, *i.e.,* before development of symptoms of EAE mice (a multiple sclerosis animal model), the animals exhibited delayed onset of multiple sclerosis symptoms as compared to control animals which were not treated with the CX-5461 compound (Example 6, Table 2). In addition, the CX-5461 compound also significantly decreased the maximal and cumulative clinical scores of multiple sclerosis in the EAE mice (Example 6, Table 2, Figure 9), demonstrating the feasibility of the CX-5461 compound to treat multiple sclerosis patients by reducing severity of disease, and to delay onset or deterioration of disease. Moreover, when CX-5461 was administered in a dose of 12.5 mg/kg/day to mice upon disease onset (when the mice exhibited multiple sclerosis clinical score of 0.5 or 1.0), the treated mice exhibited significantly lower EAE scores as compared to the control vehicle group (Example 7 and Figure 10), with a significantly reduced cumulative or maximal EAE score (Example 7, Table 3). Furthermore, the present inventors have shown that a dose of 25 mg/kg/day of CX-5461 is toxic to mice, as evidenced by a change in their fur color and brightness and loss of weight. These results demonstrate that treatment with the CX-5461 compound (e.g., in dose of about 1.01 mg/kg/day) upon onset of disease significantly decreases the severity of the disease.

According to an aspect of some embodiments of the invention, there is provided a quinolone compound, for use in treating multiple sclerosis in the subject.

According to an aspect of some embodiments of the invention, the quinolone compound is for use in treating multiple sclerosis in a subject.

According to some embodiments of the present disclosure, the quinolone compound has a general structure of Formula (I), 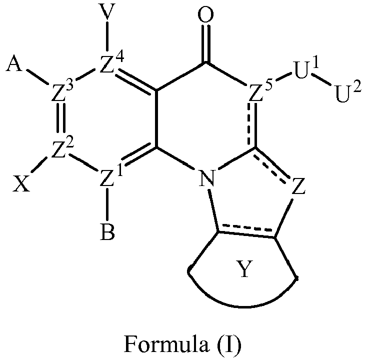
or a pharmaceutically acceptable salt or ester thereof;
wherein ------ indicates an optionally unsaturated bond;
each of B, X, A or V is absent if Z¹, Z², Z³ and Z⁴, respectively, is N; and
each of B, X, A and V is independently H, halo, azido, -CN, -CF₃, -CONR¹R², - NR¹R², -SR², -OR², -R³, -W, -L-W, -W⁰, -L-N(R) -W⁰, A² or A³, when each of Z¹, Z², Z³ and Z⁴, respectively, is C;
Z is O, S, CR⁴₂, NR⁴CR⁴, CR⁴NR⁴, CR⁴, NR⁴ or N;
each of Z¹, Z², Z³ and Z⁴ is independently C or N, provided any three N are non-adjacent;
Z⁵ is C; or Z⁵ may be N when Z is N;
Y is an optionally substituted 5-6 membered carbocyclic or heterocyclic ring;
U¹ is -C(=T)N(R)-, -C(=T)N(R)O-, -C(=T)-, -SO₂N(R)-, -SO₂N(R)N(R⁰)-, -SO₂-, or - SO₃-, where T is O, S, or NH; or U¹ may be a bond when Z⁵ is N or U² is H;
U² is H, or C3-C7 cycloalkyl, C1-C10 alkyl, C1-C10 heteroalkyl, C2-C10 alkenyl or C2-C10 heteroalkenyl group, each of which may be optionally substituted with one or more halogens, =O, or an optionally substituted 3-7 membered carbocyclic or heterocyclic ring; or U² is -W, -L-W, -L-N(R)-W⁰, A² or A³;
in each -NR¹R², R¹ and R² together with N may form an optionally substituted azacyclic ring, optionally containing an additional heteroatom selected from N, O and S as a ring member;
R¹ is H or C1-C6 alkyl, optionally substituted with one or more halogens, or =O;
R² is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
each R⁴ is independently H, or C1-C6 alkyl; or R⁴ may be -W, -L-W or -L-N(R)-W⁰; each R and R⁰ is independently H or C1-C6 alkyl;
L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl;
W is an optionally substituted 4-7 membered azacyclic ring, optionally containing an additional heteroatom selected from N, O and S as a ring member;
W⁰ is an optionally substituted 3-4 membered carbocyclic ring, or a C1-C6 alkyl group substituted with from 1 to 4 fluorine atoms;
provided one of U², V, A, X and B is a secondary amine A² or a tertiary amine A³, wherein
the secondary amine A² is -NH-W⁰, and
the tertiary amine A³ is a fully saturated and optionally substituted six-membered or seven-membered azacyclic ring optionally containing an additional heteroatom selected from N, O or S as a ring member, or the tertiary amine A³ is a partially unsaturated or aromatic optionally substituted five-membered azacyclic ring, optionally containing an additional heteroatom selected from N, O or S as a ring member;
thereby treating the autoimmune disease in the subject.

According to some embodiments of the disclosure, Z¹ is N, and each of Z², Z³ and Z⁴ is C.

According to some embodiments of the disclosure, U is -W or -L-W, where W is an optionally substituted 5-6 membered unsaturated or aromatic azacyclic ring, optionally containing an additional heteroatom selected from N, O and S; or W is an optionally substituted 5-7 membered saturated azacyclic ring containing an additional heteroatom selected from N and S.

According to some embodiments of the disclosure, U² is -L-N(R)-W⁰.

According to some embodiments of the disclosure, Y is an optionally substituted phenyl ring.

According to some embodiments of the disclosure, the compound with the proviso that when Z¹ is N, Z² and Z⁴ are C, Z⁵ is C, U¹ is -C(O)NH-, U² is -L-W, and L is an ethylene linker, one of V, A, and X is independently an optionally substituted aryl, heteroaryl, or 7-membered azacyclic ring, optionally containing an additional heteroatom selected from N, O and S as a ring member, if W is pyrrolidin-1-yl, N-methyl-pyrrolidin-2-yl, piperidin-1-yl or morpholin-1-yl

According to some embodiments of the disclosure, the quinolone compound has a general structure of Formula (II), 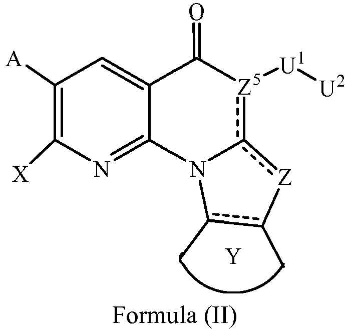
or a pharmaceutically acceptable salt or ester thereof;
wherein ----- indicates an optionally unsaturated bond;
each of A and X is independently H, halo, azido, -CN, -CF₃, -CONR¹R², -NR¹R², -SR², - OR², -R³, -W, -L-W, -W⁰, -L-N(R)-W⁰, A² or A³;
Z is O,S, CR⁴₂, NR⁴CR⁴, CR⁴NR⁴ or NR⁴;
Y is an optionally substituted 5-6 membered carbocyclic or heterocyclic ring;
U¹ is -C(=T)N(R)-, -C(=T)N(R)O-, -C(=T)-, -SO₂N(R)-, -SO₂N(R)N(R⁰)-, -SO₂-, or - SO₃-, where T is O, S, or NH; or U¹ may be a bond when U2 is H;
U² is H, or C3-C7 cycloalkyl, C1-C10 alkyl, C1-C10 heteroalkyl, C2-C10 alkenyl or C2-C10 heteroalkenyl group, each of which may be optionally substituted with one or more halogens, =O, or an optionally substituted 3-7 membered carbocyclic or heterocyclic ring; or U² is -W, -L-W, -L-N(R)-W⁰, A² or A³;
in each -NR¹R², R¹ and R² together with N may form an optionally substituted azacyclic ring, optionally containing an additional heteroatom selected from N, O and S as a ring member;
R¹ is H or C1-C6 alkyl, optionally substituted with one or more halogens, or =O;
R2 is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
each R⁴ is independently H, or C1-C6 alkyl; or R⁴ may be -W, -L-W or -L-N(R)-W0; each R and R⁰ is independently H or C1-C6 alkyl;
L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl;
W is an optionally substituted 4-7 membered azacyclic ring, optionally containing an additional heteroatom selected from N, O and S as a ring member;
W⁰ is an optionally substituted 3-4 membered carbocyclic ring, or a C1-C6 alkyl group substituted with from 1 to 4 fluorine atoms;
provided that one of U², A, and X is a secondary amine A² or a tertiary amine A³, wherein
the secondary amine A² is -NH-W⁰, and
the tertiary amine A³ is a fully saturated and optionally substituted six-membered or seven-membered azacyclic ring optionally containing an additional heteroatom selected from N, O or S as a ring member, or the tertiary amine A³ is a partially unsaturated or aromatic optionally substituted five-membered azacyclic ring optionally containing an additional heteroatom selected from N, O or S as a ring member, thereby treating the autoimmune disease in the subject.

According to some embodiments of the disclosure, with the proviso that when U¹ is -C(O)NH-, U² is -L-W, and L is an ethylene linker, one of A and X is independently an optionally substituted aryl, heteroaryl, or 7-membered azacyclic ring, optionally containing an additional heteroatom selected from N, O and S as a ring member, if W is pyrrolidin-1-yl, N-methyl-pyrrolidin-2-yl, piperidin-1-yl or morpholin-1-yl.

According to some embodiments of the disclosure, at least one of A and X is a tertiary amine A³.

According to some embodiments of the disclosure, A³ is selected from the group consisting of imidazole, imidazoline, pyrroline, piperidine, piperazine, morpholine, thiomorpholine and homopiperazine.

According to some embodiments of the disclosure, U¹ is a -C(=T)N(R)-, T is O, and U² is -L-W or -L-N(R)-W⁰.

According to some embodiments of the disclosure, the quinolone compound has a general structure of Formula (III), 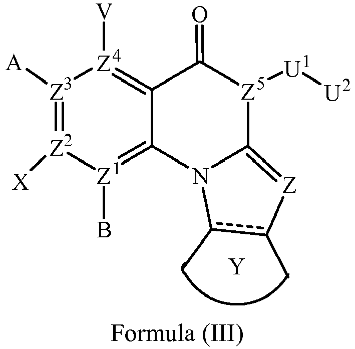
or a pharmaceutically acceptable salt or ester thereof;
wherein ---- indicates an optionally unsaturated bond; and
each of B, X, A or V is absent if Z¹, Z², Z³ and Z⁴, respectively, is N; and
each of B, X, A and V is independently H, halo, azido, -CN, -CF₃, -CONR¹R², -NR¹R², - SR², -OR², -R³, -W, -L-W, -W⁰, -L-N(R)-W⁰, A² or A³, when each of Z¹, Z², Z³ and Z⁴, respectively, is C;
each of Z¹, Z², Z³ and Z⁴ is independently C or N, provided any three N are non-adjacent;
Y is an optionally substituted 5-6 membered carbocyclic or heterocyclic ring;
U¹ is -C(=T)N(R)-, -C(=T)N(R)O-, -C(=T)-, -SO₂N(R)-, -SO₂N(R)N(R⁰)-, -SO₂-, or - SO₃-, where T is O, S, or NH; or U¹ may be a bond when Z⁵ is N or U² is H;
U² is H, or C3-C7 cycloalkyl, C1-C10 alkyl, C1-C10 heteroalkyl, C2-C10 alkenyl or C2-C10 heteroalkenyl group, each of which may be optionally substituted with one or more halogens, =O, or an optionally substituted 3-7 membered carbocyclic or heterocyclic ring; or U² is -W, -L-W, -L-N(R) -W⁰, A² or A³;
in each -NR¹R², R¹ and R² together with N may form an optionally substituted azacyclic ring, optionally containing an additional heteroatom selected from N, O and S as a ring member;
R¹ is H or C1-C6 alkyl, optionally substituted with one or more halogens, or =O;
R² is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
each R⁴ is independently H, or C1-C6 alkyl; or R⁴ may be -W, -L-W or -L-N(R)-W⁰; each R and R⁰ is independently H or C1-C6 alkyl;
L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl;
W is an optionally substituted 4-7 membered azacyclic ring, optionally containing an additional heteroatom selected from N, O and S as a ring member;
W⁰ is an optionally substituted 3-4 membered carbocyclic ring, or a C1-C6 alkyl group substituted with from 1 to 4 fluorine atoms;
provided that one of U², V, A, X and B is a secondary amine A² or a tertiary amine A3, wherein
the secondary amine A² is -NH-W⁰, and
the tertiary amine A³ is a fully saturated and optionally substituted six-membered or seven-membered azacyclic ring optionally containing an additional heteroatom selected from N, O or S as a ring member, or the tertiary amine A³ is a partially unsaturated or aromatic optionally substituted five-membered azacyclic ring, optionally containing an additional heteroatom selected from N, O or S as a ring member, thereby treating the autoimmune disease in the subject.

According to some embodiments of the disclosure, the quinolone compound has a general structure of Formula (IV), 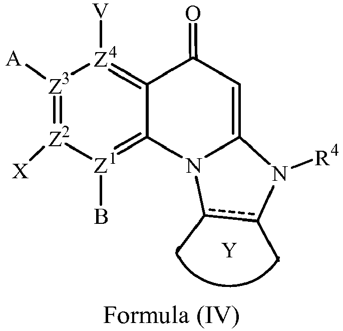
or a pharmaceutically acceptable salt or ester thereof;
wherein ----- indicates an optionally unsaturated bond;
each of B, X, A or V is absent if Z¹, Z², Z³ and Z⁴ respectively, is N; and
each of B, X, A and V is independently H, halo, azido, -CN, -CF₃, -CONR¹R², - NR¹R², -SR², -OR², -R³, -W, -L-W, -W⁰, -L-N(R)-W⁰, A² or A³, when each of Z¹, Z², Z³ and Z⁴, respectively, is C;
each of Z¹, Z², Z³ and Z⁴ is independently C or N, provided any three N are non-adjacent;
Y is an optionally substituted 5-6 membered carbocyclic or heterocyclic ring;
in each NR¹R², R¹ and R² together with N may form an optionally substituted azacyclic ring, optionally containing an additional heteroatom selected from N, O and S as a ring member;
R¹ is H or C1-C6 alkyl, optionally substituted with one or more halogens, or =O;
R² is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
R⁴ is -W, -L-W or -L-N(R)-W0; and
each R is independently H or C1-C6 alkyl;
L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl; W is an optionally substituted 4-7 membered azacyclic ring, optionally containing an additional heteroatom selected from N, O and S as a ring member; and W⁰ is an optionally substituted 3-4 membered carbocyclic ring, or a C1-C6 alkyl group substituted with from 1 to 4 fluorine atoms,
thereby treating the autoimmune disease in the subject.

According to some embodiments of the disclosure, the quinolone compound has a general structure of Formula (V), 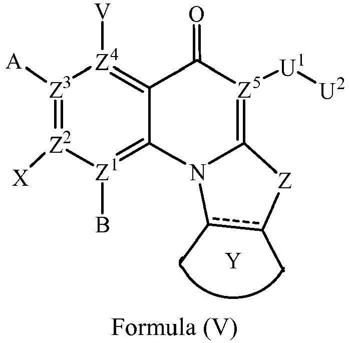
or a pharmaceutically acceptable salt or ester thereof;
---- indicates an optionally unsaturated bond;
A and V independently are H, halo, azido, -CN, -CF₃, -CONR¹R², -NR¹R², -SR², -OR², - R³, -W, -L-W, -W⁰, -L-N(R)-W⁰, A² or A³;
Z is O, S, CR⁴₂, NR⁴CR⁴, CR⁴NR⁴ or NR⁴;
Y is an optionally substituted 5-6 membered carbocyclic or heterocyclic ring;
U¹ is -C(=T)N(R)-, -C(=T)N(R)O-, -C(=T)-, -SO₂N(R)-, -SO₂N(R)N(R⁰)-, -SO₂-, or - SO₃-, where T is O, S, or NH; or U¹ may be a bond when U2 is H;
U² is H, or C3-C7 cycloalkyl, C1-C10 alkyl, C1-C10 heteroalkyl, C2-C10 alkenyl or C2-C10 heteroalkenyl group, each of which may be optionally substituted with one or more halogens, =O, or an optionally substituted 3-7 membered carbocyclic or heterocyclic ring; or U² is -W, -L-W or -L-N(R)-W⁰, A² or A³;
in each -NR¹R², R¹ and R² together with N may form an optionally substituted azacyclic ring, optionally containing an additional heteroatom selected from N, O and S as a ring member;
R¹ is H or C1-C6 alkyl, optionally substituted with one or more halogens, or =O;
R² is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
each R⁴ is independently H, or C1-C6 alkyl; or R4 may be -W, -L-W or -L-N(R)-W⁰; each R and R⁰ is independently H or C1-C6 alkyl;
L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl;
W is an optionally substituted 4-7 membered azacyclic ring, optionally containing an additional heteroatom selected from N, O and S as a ring member;
W⁰ is an optionally substituted 3-4 membered carbocyclic ring, or a C1-C6 alkyl group substituted with from 1 to 4 fluorine atoms;
provided one of U², A, and V is a secondary amine A² or a tertiary amine A3, wherein the secondary amine A² is -NH-W0, and
the tertiary amine A³ is a fully saturated and optionally substituted six-membered or seven-membered azacyclic ring optionally containing an additional heteroatom selected from N, O or S as a ring member, or the tertiary amine A³ is a partially unsaturated or aromatic optionally substituted five-membered azacyclic ring optionally containing an additional heteroatom selected from N, O or S as a ring member,
thereby treating the autoimmune disease in the subject.

According to the present invention, the quinolone compound has a general structure of Formula (VI), 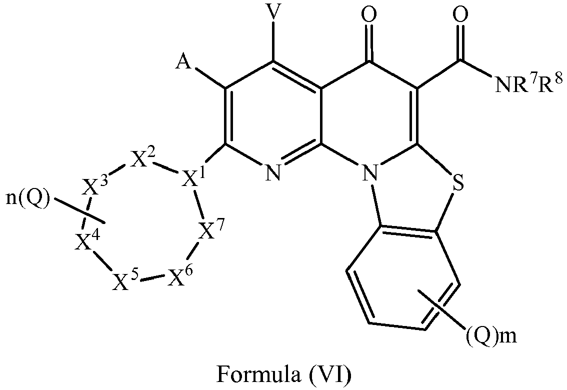 or a pharmaceutically acceptable salt or ester thereof;
wherein:
X¹ is CH or N;
X², X³, X⁴, X⁵, X⁶ and X⁷ independently are NR⁴, CH₂, CHQ or C(Q)₂, provided that: (i) zero, one or two of X², X³, X⁴, X⁵, X⁶ and X⁷ are NR⁴; (ii) when X¹ is N, both of X² and X⁷ are not NR⁴; (iii) when X¹ is N, X³ and X⁶ are not NR⁴; and (iv) when X¹ is CH and two of X², X³, X⁴, X⁵, X⁶ and X⁷ are NR⁴, the two NR⁴ are located at adjacent ring positions or are separated by two or more other ring positions;
A and V independently are H, halo, azido, -CN, -CF₃, -CONR¹R², -NR¹R², -SR², -OR², - R³, -W, -L-W, -W⁰, or -L-N(R)-W⁰;
each Q is independently halo, azido, -CN, -CF₃, -CONR¹R², -NR¹R², -SR², -OR², -R³, - W, -L-W, -W⁰, or -L-N(R)-W⁰;
in each -NR¹R², R¹ and R² together with N may form an optionally substituted azacyclic ring, optionally containing one additional heteroatom selected from N, O and S as a ring member;
R¹ is H or C1-C6 alkyl, optionally substituted with one or more halogens, or =O;
R is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
each R⁴ is independently H, or C1-C6 alkyl; or R4 may be -W, -L-W or -L-N(R)-W⁰; each R is independently H or C1-C6 alkyl;
R⁷ is H and R⁸ is C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring; or in - NR⁷R⁸, R⁷ and R⁸ together with N may form an optionally substituted azacyclic ring, optionally containing an additional heteroatom selected from N, O and S as a ring member;
m is 0, 1, 2, 3 or 4;
n is 0, 1, 2, 3, 4, or 5;
L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl;
W is an optionally substituted 4-7 membered azacyclic ring, optionally containing an additional heteroatom selected from N, O and S as a ring member; and
W⁰ is an optionally substituted 3-4 membered carbocyclic ring, or a C1-C6 alkyl group substituted with from 1 to 4 fluorine atoms,
thereby treating multiple sclerosis in the subject.

According to some embodiments of the invention, X¹ is CH and two of X², X³, X⁴, X⁵, X⁶ and X⁷ are NR⁴.

According to some embodiments of the invention, wherein X¹ is CH and one of X², X³, X⁴, X⁵, X⁶ and X⁷ are NR⁴.

According to some embodiments of the invention, X¹ is CH and none of X², X³, X⁴, X⁵, X⁶ and X⁷ are NR⁴.

According to some embodiments of the invention, wherein X¹ is N and none of X², X³, X⁴, X⁵, X⁶ and X⁷ are NR⁴.

According to some embodiments of the invention, X¹ is N and one of X⁴ or X⁵ is NR⁴.

According to some embodiments of the invention, the quinolone compound has a general structure of Formula (VIII), 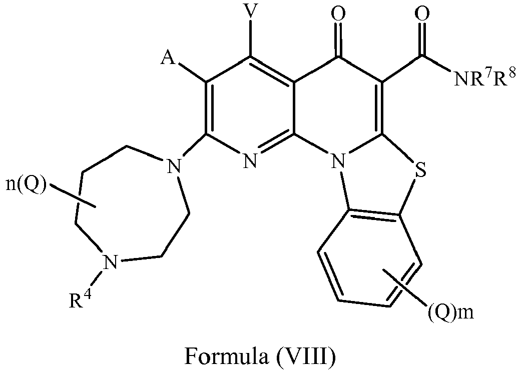 or a pharmaceutically acceptable salt or ester thereof;
wherein:
A and V independently are H, halo, azido, -CN, -CF₃, -CONR¹R², -NR¹R², -SR², -OR², - R³, -W, -L-W, -W⁰, or -L-N(R)-W⁰;
each Q is independently halo, azido, -CN, -CF₃, -CONR¹R², -NR¹R², -SR², -OR², -R³, - W, -L-W, -W⁰, or -L-N(R)-W⁰;
in each -NR¹R², R¹ and R² together with N may form an optionally substituted azacyclic ring, optionally containing an additional heteroatom selected from N, O and S as a ring member;
R¹ is H or C1-C6 alkyl, optionally substituted with one or more halogens, or =O;
R² is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
each R⁴ is independently H, or C1-C6 alkyl; or R4 may be -W, -L-W or -L-N(R)-W⁰; each R is independently H or C1-C6 alkyl;
R⁷ is H and R⁸ is C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring; or in - NR⁷R⁸, R⁷ and R⁸ together with N may form an optionally substituted azacyclic ring, optionally containing an additional heteroatom selected from N, O and S as a ring member;
m is 0, 1, 2, 3 or 4;
n is 0, 1, 2, 3, 4 or 5;
p is 0, 1, 2 or 3;
L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl;
W is an optionally substituted 4-7 membered azacyclic ring, optionally containing an additional heteroatom selected from N, O and S as a ring member; and
W⁰ is an optionally substituted 3-4 membered carbocyclic ring, or a C1-C6 alkyl group substituted with from 1 to 4 fluorine atoms,
thereby treating multiple sclerosis in the subject.

According to some embodiments of the invention, R⁷ is H and R⁸ is a C₁₋₄ alkyl substituted with an optionally substituted aromatic heterocyclic ring.
According to some embodiments of the invention, the optionally substituted aromatic heterocyclic ring is selected from pyridine, pyrimidine, pyrazine, imidazole, pyrrolidine, and thiazole.

According to some embodiments of the invention, R⁷ and R⁸ together with N in - NR⁷R⁸ form an optionally substituted azacyclic ring selected from the group consisting of morpholine, thiomorpholine, piperidine or piperazine ring.

According to some embodiments of the invention, the quinolone compound has a general structure of Formula (VII) 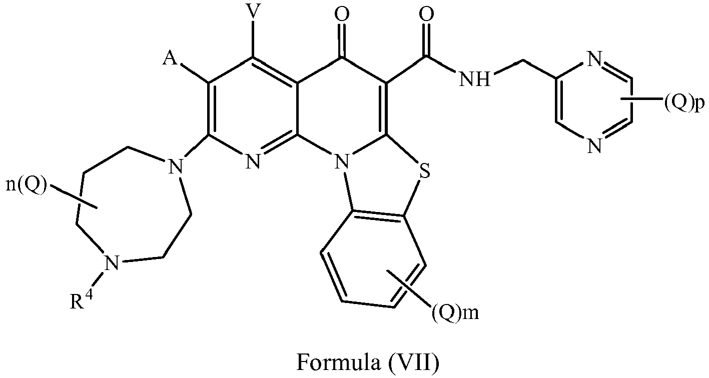
or a pharmaceutically acceptable salt or ester thereof;
A and V independently are H, halo, azido, -CN, -CF₃, -CONR1R2, -NR¹R², -SR², -OR², -R³, -W, -L-W, -W⁰, or -L-N(R)-W⁰;
each Q is independently halo, azido, -CN, -CF₃, -CONR¹R², -NR¹R², -SR², -OR², -R³, - W, -L-W, -W⁰, or -L-N(R)-W⁰;
in each -NR¹R², R¹ and R² together with N may form an optionally substituted azacyclic ring, optionally containing an additional heteroatom selected from N, O and S as a ring member;
R¹ is H or C1-C6 alkyl, optionally substituted with one or more halogens, or =O;
R2 is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
each R⁴ is independently H, or C1-C6 alkyl; or R⁴ may be -W, -L-W or -L-N(R)-W⁰;
each R is independently H or C1-C6 alkyl;
m is 0, 1, 2, 3 or 4;
n is 0, 1, 2, 3, 4 or 5;
p is 0, 1, 2 or 3;
L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl; W is an optionally substituted 4-7 membered azacyclic ring, optionally containing an additional heteroatom selected from N, O and S as a ring member; and W⁰ is an optionally substituted 3-4 membered carbocyclic ring, or a C1-C6 alkyl group substituted with from 1 to 4 fluorine atoms,
thereby treating multiple sclerosis in the subject.

According to some embodiments of the invention, A and V are independently H or halo.

According to some embodiments of the invention, R⁴ is H or C1-4 alkyl.

According to some embodiments of the invention, m and n are each 0.

According to some embodiments of the invention, p is 0 or 1.

According to some embodiments of the invention, the compound having the structure: 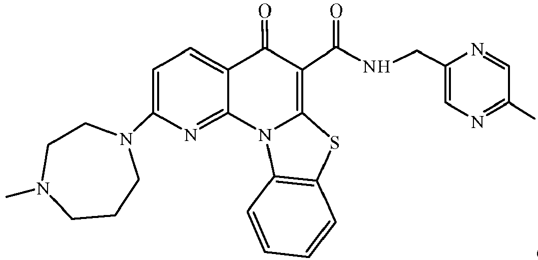 or a pharmaceutically acceptable salt or ester thereof.

According to some embodiments of the invention, the quinolone compound has a structure depicting in a Formula selected from the group consisting of: 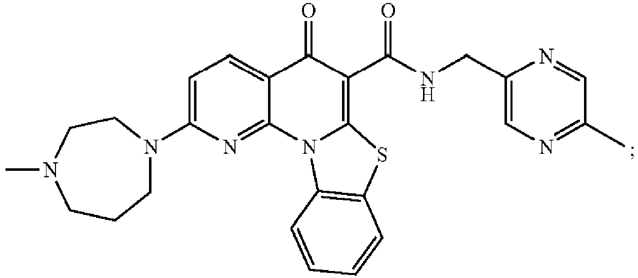 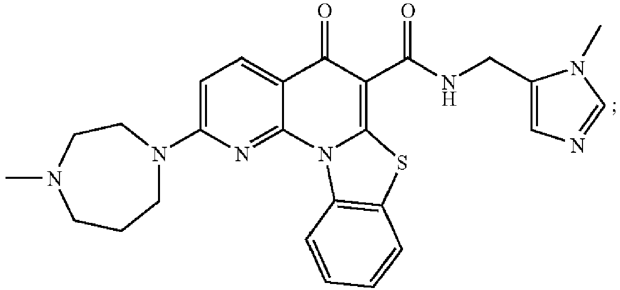 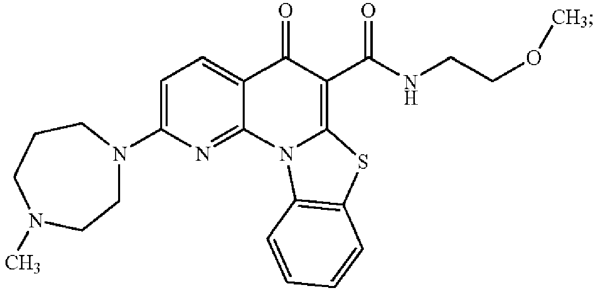 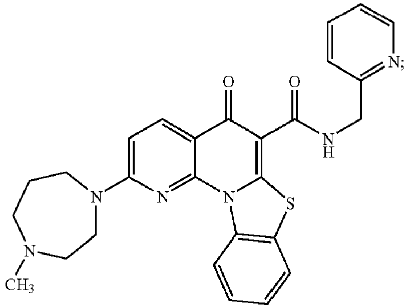 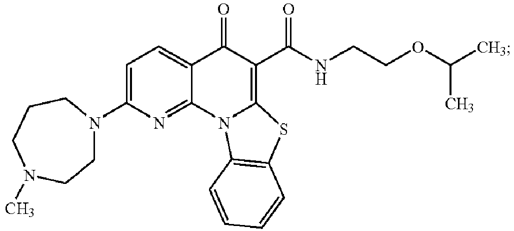 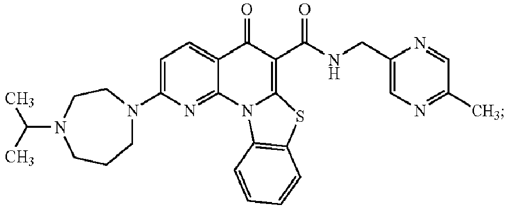 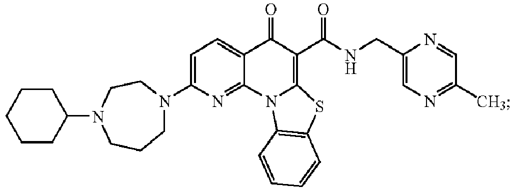 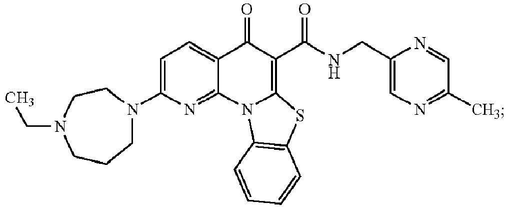 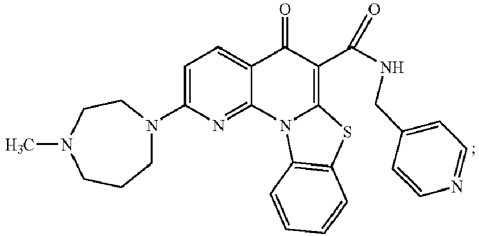 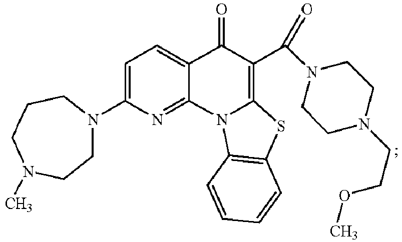 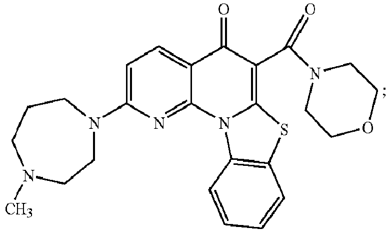 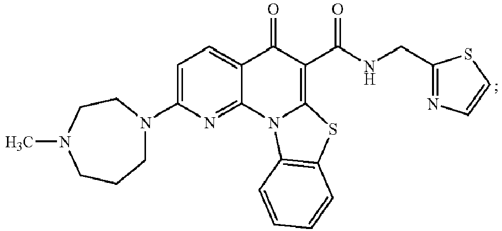 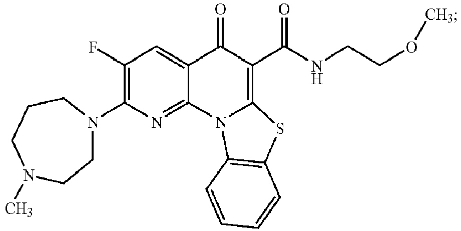 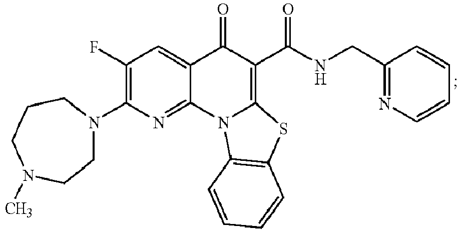 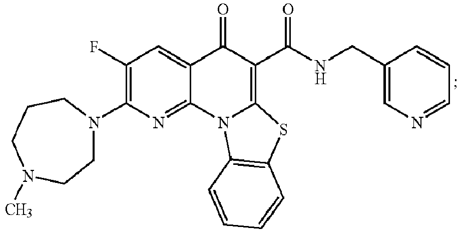 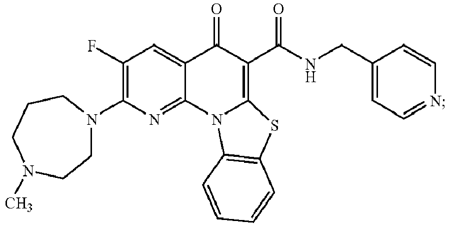 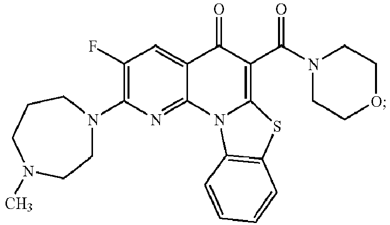 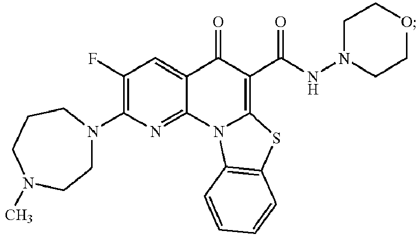 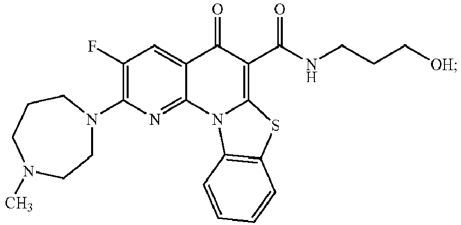 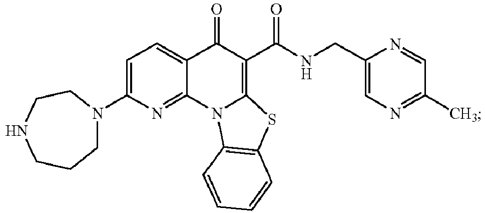 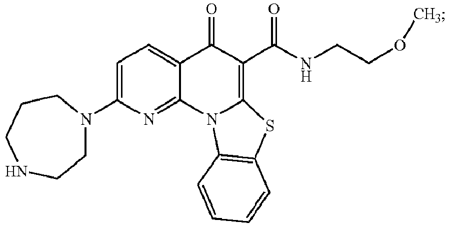 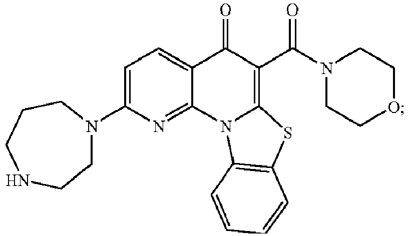 and 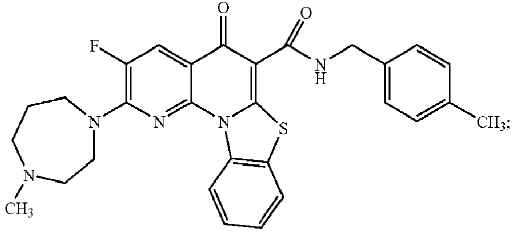 or a pharmaceutically acceptable salt or ester thereof, thereby treating multiple sclerosis in the subject.

According to some embodiments of the invention, the compound is 2-(4-methyl-1,4-diazepan-1-yl)- N-((5-methylpyrazin-2-yl)methyl)-5-oxo-5H-benzo[4,5]thiazolo[3,2-a][1,8]naphthyridine-6-carboxamide as depicted in Formula

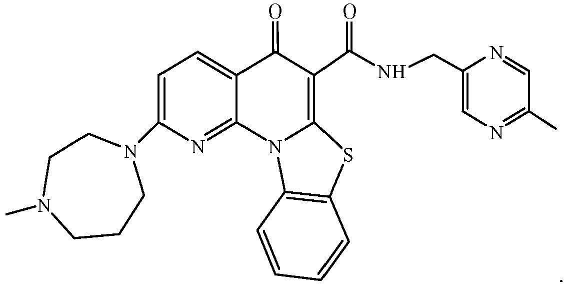

Methods of synthesizing the compound of some embodiments of the invention are known in the art and include for example those described in Nagasawa et al., US Patent Application Publication No. 2009/0093455A1. In addition, Example 1 of the Examples section which follows, provides a detailed description of synthesize of the CX-5461 compound.

For use as pharmaceutical agents, the compound of some embodiments of the invention is sterile.

According to some embodiments of the invention, the compound is purified using known methods.

According to some embodiments of the invention, the compound has 95-99.9% purity.

As used herein the phrase "autoimmune disease" refers to any disease caused by an autoimmune response, *i.e.,* an immune response directed to a substance in the body of the subject.

It should be noted that since autoimmunity can affect any organ or tissue of the subject, e.g., the brain, skin, kidney, lungs, liver, heart, or thyroid of the subject, the clinical expression of the disease depends upon the site affected.

Following is a non-limiting list of autoimmune diseases or disorders (including autoimmune-related diseases or disorders) which can be treated by the compound of the present disclosure: Acute Disseminated Encephalomyelitis (ADEM); Acute necrotizing hemorrhagic leukoencephalitis; Addison's disease; Agammaglobulinemia; Alopecia areata; Amyloidosis; Ankylosing spondylitis; Anti-GBM/Anti-TBM nephritis; Antiphospholipid syndrome (APS); Autoimmune angioedema; Autoimmune aplastic anemia; Autoimmune dysautonomia; Autoimmune hepatitis; Autoimmune hyperlipidemia; Autoimmune immunodeficiency; Autoimmune inner ear disease (AIED); Autoimmune myocarditis; Autoimmune pancreatitis; Autoimmune retinopathy; Autoimmune thrombocytopenic purpura (ATP); Autoimmune thyroid disease; Autoimmune urticaria; Axonal & neuronal neuropathies; Balo disease; Behcet's disease; Bullous pemphigoid; Cardiomyopathy; Castleman disease; Celiac disease; Chagas disease; Chronic inflammatory demyelinating polyneuropathy (CIDP); Chronic recurrent multifocal ostomyelitis (CRMO); Churg-Strauss syndrome; Cicatricial pemphigoid/benign mucosal pemphigoid; Crohn's disease; Cogans syndrome; Cold agglutinin disease; Congenital heart block; Coxsackie myocarditis; CREST disease; Essential mixed cryoglobulinemia; Demyelinating neuropathies; Dermatitis herpetiformis; Dermatomyositis; Devic's disease (neuromyelitis optica); Discoid lupus; Dressler's syndrome; Endometriosis; Eosinophilic fasciitis; Erythema nodosum; Experimental allergic encephalomyelitis; Evans syndrome; Fibrosing alveolitis; Giant cell arteritis (temporal arteritis); Glomerulonephritis; Goodpasture's syndrome; Granulomatosis with Polyangiitis (GPA) see Wegener's; Graves' disease; Guillain-Barre syndrome; Hashimoto's encephalitis; Hashimoto's thyroiditis; Hemolytic anemia; Henoch-Schonlein purpura; Herpes gestationis; Hypogammaglobulinemia; Idiopathic thrombocytopenic purpura (ITP); IgA nephropathy; IgG4-related sclerosing disease; Immunoregulatory lipoproteins; Inclusion body myositis; Insulin-dependent diabetes (type1); Interstitial cystitis; Juvenile arthritis; Juvenile diabetes; Kawasaki syndrome; Lambert-Eaton syndrome; Leukocytoclastic vasculitis; Lichen planus; Lichen sclerosus; Ligneous conjunctivitis; Linear IgA disease (LAD); Lupus (SLE); Lyme disease, chronic; Meniere's disease; Microscopic polyangiitis; Mixed connective tissue disease (MCTD); Mooren's ulcer; Mucha-Habermann disease; Multiple sclerosis; Myasthenia gravis; Myositis; Narcolepsy; Neuromyelitis optica (Devic's); Neutropenia; Ocular cicatricial pemphigoid; Optic neuritis; Palindromic rheumatism; PANDAS (Pediatric Autoimmune Neuropsychiatric Disorders Associated with Streptococcus); Paraneoplastic cerebellar degeneration; Paroxysmal nocturnal hemoglobinuria (PNH); Parry Romberg syndrome; Parsonnage-Turner syndrome; Pars planitis (peripheral uveitis); Pemphigus; Peripheral neuropathy; Perivenous encephalomyelitis; Pernicious anemia; POEMS syndrome; Polyarteritis nodosa; Type I, II, & III autoimmune polyglandular syndromes; Polymyalgia rheumatica; Polymyositis; Postmyocardial infarction syndrome; Postpericardiotomy syndrome; Progesterone dermatitis; Primary biliary cirrhosis; Primary sclerosing cholangitis; Psoriasis; Psoriatic arthritis; Idiopathic pulmonary fibrosis; Pyoderma gangrenosum; Pure red cell aplasia; Raynauds phenomenon; Reflex sympathetic dystrophy; Reiter's syndrome; Relapsing polychondritis; Restless legs syndrome; Retroperitoneal fibrosis; Rheumatic fever; Rheumatoid arthritis; Sarcoidosis; Schmidt syndrome; Scleritis; Scleroderma; Sjogren's syndrome; Sperm & testicular autoimmunity; Stiff person syndrome; Subacute bacterial endocarditis (SBE); Susac's syndrome; Sympathetic ophthalmia; Takayasu's arteritis; Temporal arteritis/Giant cell arteritis; Thrombocytopenic purpura (TTP); Tolosa-Hunt syndrome; Transverse myelitis; Ulcerative colitis; Undifferentiated connective tissue disease (UCTD); Uveitis; Vasculitis; Vesiculobullous dermatosis; Vitiligo; Wegener's granulomatosis (now termed Granulomatosis with Polyangiitis (GPA).

According to the invention, the autoimmune disease is multiple sclerosis.

The diagnosis of "multiple sclerosis" can be made when a subject has experienced at least one neurological attack affecting the central nervous system (CNS) accompanied by demyelinating lesions within the brain or spinal cord, which may have, but not necessarily confirmed by magnetic resonance imaging (MRI). The neurological attack can involve acute or sub-acute neurological symptomatology (attack) manifested by various clinical presentations like unilateral loss of vision, vertigo, ataxia, incoordination, gait difficulties, sensory impairment characterized by paresthesia, dysesthesia, sensory loss, urinary disturbances until incontinence, diplopia, dysarthria, various degrees of motor weakness until paralysis, cognitive decline either as a monosymptomatic or in combination. The symptoms usually remain for several days to few weeks, and then partially or completely resolve.

Further details on the diagnosis of multiple sclerosis according to 2010 McDonald Criteria for Diagnosis of MS are provided in Polman CH., et al., 2011 ("Diagnostic criteria for multiple sclerosis: 2010 revisions to the McDonald criteria" Annals of Neurology, vol. 69 (2): pages 292-302). For example, the diagnosis of multiple sclerosis can be made upon (I): Clinical presentation of ≥2 attacks, with objective clinical evidence of ≥2 lesions or objective clinical evidence of 1 lesion with reasonable historical evidence of a prior attack; (II): Clinical presentation of ≥2 attacks, with objective clinical evidence of 1 lesion, additional data have to include dissemination in space, demonstrated by: ≥1 T2 lesion in at least 2 of 4 MS-typical regions of the CNS (periventricular, juxtacortical, infratentorial, or spinal cord); (III): Clinical presentation of 1 attack, with objective clinical evidence of ≥2 lesions, additional data have to include dissemination in time, demonstrated by: Simultaneous presence of asymptomatic gadolinium-enhancing and nonenhancing lesions at any time; or A new T2 and/or gadolinium-enhancing lesion(s) on follow-up MRI, irrespective of its timing with reference to a baseline scan; (IV): Clinical presentation of 1 attack, additional data have to include dissemination in space and time, demonstrated by: For DIS: ≥1 T2 lesion in at least 2 of 4 MS-typical regions of the CNS (periventricular, juxtacortical, infratentorial, or spinal cord) and for DIT: Simultaneous presence of asymptomatic gadolinium-enhancing and nonenhancing lesions at any time; or A new T2 and/or gadolinium-enhancing lesion(s) on follow-up MRI, irrespective of its timing with reference to a baseline scan; (V): Clinical presentation of Insidious neurological progression suggestive of MS (PPMS), additional data have to include 1 year of disease progression (retrospectively or prospectively determined) plus 2 of 3 of the following criteria: 1. Evidence for DIS in the brain based on ≥1 T2 lesions in the MS-characteristic (periventricular, juxtacortical, or infratentorial) regions 2. Evidence for DIS in the spinal cord based on ≥2 T2 lesions in the cord 3. Positive CSF (isoelectric focusing evidence of oligoclonal bands and/or elevated IgG index).

According to some embodiments of the invention, the subject has relapsing-remitting multiple sclerosis (RRMS).

According to some embodiments of the invention, the subject has a primary progressive multiple sclerosis (PPMS).

According to some embodiments of the invention, the subject has a secondary progressive MS (SPMS).

According to some embodiments of the invention, the subject has benign multiple sclerosis (BMS).

According to some embodiments of the invention, the subject has a progressive-relapsing course of MS.

According to some embodiments of the invention, treating the subject refers to changing the disease course of the subject from a typical RRMS course to a BMS course.

According to some embodiments of the invention, treating the subject refers to suppressing the activity of typical RRMS course.

According to some embodiments of the invention, the compound is 2-(4-methyl-1,4-diazepan-1-yl)-N-((5-methylpyrazin-2-yl)methyl)-5-oxo-5H-benzo[4,5]thiazolo[3,2-a][1,8]naphthyridine-6-carboxamide as depicted in Formula 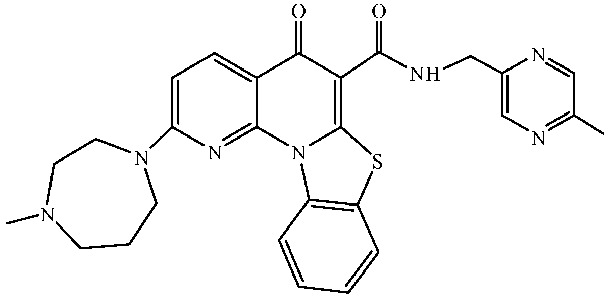 and wherein the autoimmune disease is multiple sclerosis.

According to some embodiments of the invention, administering the compound is performed after diagnosing the subject as having the autoimmune disease.

According to some embodiments of the invention, the autoimmune disease is multiple sclerosis and the diagnosis comprises appearance of brain lesions characteristics of the multiple sclerosis.

According to some embodiments of the invention, the compound prevents the appearance of additional neurological attack(s) and/or brain lesion(s) as compared to the number of neurological attack(s) and/or brain lesion(s) present at time of diagnosing multiple sclerosis.

As used herein the phrase "treating" refers to inhibiting or arresting the development of the autoimmune disease [e.g., multiple sclerosis] and/or causing the reduction, remission, or regression of the autoimmune disease and/or optimally curing the autoimmune disease. Those of skill in the art will understand that various methodologies and assays can be used to assess the development of autoimmune disease, and similarly, various methodologies and assays may be used to assess the reduction, remission or regression of the autoimmune disease.

As used herein, the term "subject" includes mammals, preferably human beings at any age which suffer from the pathology (the autoimmune disease).

According to some embodiments of the invention, the term "subject" encompasses individuals who are at risk to develop the pathology or are suspected of having the pathology.

The compound of some embodiments of the invention can be administered to the subject per se, or in a pharmaceutical composition where it is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the compound of some embodiments of the invention accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition.

Suitable routes of administration may, for example, include oral, rectal, transmucosal, especially transnasal, intestinal or parenteral delivery, including intramuscular, subcutaneous and intramedullary injections as well as intrathecal, direct intraventricular, intracardiac, e.g., into the right or left ventricular cavity, into the common coronary artery, intravenous, inrtaperitoneal, intranasal, or intraocular injections.

According to some embodiments of the invention, the compound is administered by oral administration.

Conventional approaches for drug delivery to the central nervous system (CNS) include: neurosurgical strategies (e.g., intracerebral injection or intracerebroventricular infusion); pharmacological strategies designed to increase the lipid solubility of an agent (e.g., conjugation of water-soluble agents to lipid or cholesterol carriers); and the transitory disruption of the integrity of the BBB by hyperosmotic disruption (resulting from the infusion of a mannitol solution into the carotid artery or the use of a biologically active agent such as an angiotensin peptide). However, each of these strategies has limitations, such as the inherent risks associated with an invasive surgical procedure, a size limitation imposed by a limitation inherent in the endogenous transport systems, potentially undesirable biological side effects associated with the systemic administration of a chimeric molecule comprised of a carrier motif that could be active outside of the CNS, and the possible risk of brain damage within regions of the brain where the BBB is disrupted, which renders it a suboptimal delivery method.

Alternately, one may administer the pharmaceutical composition in a local rather than systemic manner, for example, via injection of the pharmaceutical composition directly into a tissue region of a patient.

The term "tissue" refers to part of an organism consisting of cells designed to perform a function or functions. Examples include, but are not limited to, brain tissue, retina, skin tissue, hepatic tissue, pancreatic tissue, bone, cartilage, connective tissue, blood tissue, muscle tissue, cardiac tissue brain tissue, vascular tissue, renal tissue, pulmonary tissue, gonadal tissue, hematopoietic tissue.

Pharmaceutical compositions of some embodiments of the invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with some embodiments of the invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to some embodiments of the invention are conveniently delivered in the form of an aerosol spray presentation from a pressurized pack or a nebulizer with the use of a suitable propellant, e.g., dichlorodifluoromethane, trichlorofluoromethane, dichlorotetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuos infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition of some embodiments of the invention may also be formulated in rectal compositions such as suppositories or retention enemas, using, e.g., conventional suppository bases such as cocoa butter or other glycerides.

Pharmaceutical compositions suitable for use in context of some embodiments of the invention include compositions wherein the active ingredients are contained in an amount effective to achieve the intended purpose. More specifically, a therapeutically effective amount means an amount of active ingredients (the compound of some embodiments of the invention) effective to prevent, alleviate or ameliorate symptoms of a disorder (e.g., an autoimmune disease such as multiple sclerosis) or prolong the survival of the subject being treated.

Determination of a therapeutically effective amount is well within the capability of those skilled in the art, especially in light of the detailed disclosure provided herein.

For any preparation used in the methods of the invention, the therapeutically effective amount or dose can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to provide tissue or blood levels of the active ingredient which are sufficient to induce or suppress the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

The doses shown herein with respect to the mouse animal model can be converted for the treatment other species such as human and other animals diagnosed with the autoimmune disease. Figure 11 shows an art accepted conversion Table approved by the FDA (Reagan-Shaw S., et al., FASEB J. 22:659-661 (2007)).

The human equivalent dose is calculated as follows: HED (mg/kg) = Animal dose (mg/kg) multiplied by (Animal Kₘ/human Kₘ).

According to some embodiments of the invention, the compound is provided at a concentration range of about 0.081-1.62 mg/kg/day, e.g., about 0.162-1.539 mg/kg/day, 0.243- 1.458 mg/kg/day, 0.324-1.458 mg/kg/day, 0.405-1.377 mg/kg/day, 0.486-1.296 mg/kg/day, 0.567-1.215 mg/kg/day, 0.648-1.134 mg/kg/day, 0.729-1.053 mg/kg/day, 0.81-0.972 mg/kg/day, 0.891-1.215 mg/kg/day, 0.972-1.134 mg/kg/day.

According to some embodiments of the invention, the compound is provided at a concentration range of about 0.81-1.215 mg/kg/day.

According to some embodiments of the invention, the compound is provided at a concentration of about 1.01 mg/kg/day.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

The teachings of the invention can be also used to determine efficiency of the compound of some embodiments of the invention in treating the autoimmune disease (e.g., multiple sclerosis) by determining the effect of the compound on the expression level of the at least one gene of the RNA polymerase I pathway. This can be used to develop a tailored treatment of an autoimmune disease by monitoring drug efficacy. This system is based on measuring the level of genes of the RNA polymerase I pathway during treatment with the compound and the ability to perform an ongoing fine-tuning drug efficacy assessment.

Thus, according to an aspect of some embodiments of the invention, there is provided a method of monitoring treatment efficiency of the compound of some embodiments of the invention, the method comprising:
(a) treating the subject with the compound according to the method of some embodiments of the invention, and
(b) comparing a level of expression of least one gene involved in the RNA polymerase I pathway in a cell of the subject following treating with the compound to a level of expression of the at least one gene in a cell of the subject prior to treating the subject with the compound,

(i) wherein a decrease above a predetermined threshold in the level of expression of the at least one gene following treating with the compound relative to the level of expression of the at least one gene prior to treating with the compound indicates that the compound is efficient for treating the subject;
(ii) wherein an increase above a predetermined threshold in the level of expression of the at least one gene following treating with the compound relative to the level of expression of the at least one gene prior to treating with the compound indicates that the compound is not efficient for treating the subject; or
(iii) wherein when a level of expression of the at least one gene following treating with the compound is identical or changed below a predetermined threshold as compared to prior to treating with the compound then the treatment is not efficient for treating the subject;
thereby monitoring treatment efficiency of the subject having an autoimmune disease.

As used herein, the phrase "level of expression" refers to the degree of gene expression and/or gene product activity in a specific cell. For example, up-regulation or down-regulation of various genes can affect the level of the gene product (*i.e.,* RNA and/or protein) in a specific cell.

It should be noted that the level of expression can be determined in arbitrary absolute units, or in normalized units (relative to known expression levels of a control reference). For example, when using DNA chips, the expression levels are normalized according to the chips' internal controls or by using quantile normalization such as RMA.

As used herein the phrase "a cell of the subject" refers to at least one cell (e.g., an isolated cell), cell culture, cell content and/or cell secreted content which contains RNA and/or proteins of the subject. Examples include a blood cell, a cell obtained from any tissue biopsy [e.g., cerebrospinal fluid, (CSF), brain biopsy], a bone marrow cell, body fluids such as plasma, serum, saliva, spinal fluid, lymph fluid, the external sections of the skin, respiratory, intestinal, and genitourinary tracts, tears, saliva, sputum and milk. According to an embodiment of the invention, the cell is a blood cell (e.g., white blood cells, macrophages, B- and T-lymphocytes, monocytes, neutrophiles, eosinophiles, and basophiles) which can be obtained using a syringe needle from a vein of the subject. It should be noted that the cell may be isolated from the subject (e.g., for *in vitro* detection) or may optionally comprise a cell that has not been physically removed from the subject (e.g., *in vivo* detection).

According to some embodiments of the invention, the white blood cell comprises peripheral blood mononuclear cells (PBMC). The phrase, "peripheral blood mononuclear cells (PBMCs)" as used herein, refers to a mixture of monocytes and lymphocytes. Several methods for isolating white blood cells are known in the art. For example, PBMCs can be isolated from whole blood samples using density gradient centrifugation procedures. Typically, anticoagulated whole blood is layered over the separating medium. At the end of the centrifugation step, the following layers are visually observed from top to bottom: plasma/platelets, PBMCs, separating medium and erythrocytes/granulocytes. The PBMC layer is then removed and washed to remove contaminants (e.g., red blood cells) prior to determining the expression level of the polynucleotide(s) therein.

According to some embodiments of the invention, the level of expression of the gene(s) of the invention is determined using an RNA and/or a protein detection method.

According to some embodiments of the invention, the RNA or protein molecules are extracted from the cell of the subject.

Methods of extracting RNA or protein molecules from cells of a subject are well known in the art. Once obtained, the RNA or protein molecules can be characterized for the expression and/or activity level of various RNA and/or protein molecules using methods known in the arts.

According to some embodiments of the invention, detection of the expression level of the RNA of the Pol I pathway is performed using a probe which specifically hybridizes to a polynucleotide expressed from the gene of the Pol I pathway (e.g., including any alternative spliced form which is known in the art). Examples of methods of detecting RNA molecules in a cell sample include Northern blot analysis, RT-PCR, RNA *in situ* hybridization (using e.g., DNA or RNA probes to hybridize RNA molecules present in the cells or tissue sections), *in situ* RT-PCR (e.g., as described in Nuovo GJ, et al. Am J Surg Pathol. 1993, 17: 683-90; Komminoth P, et al. Pathol Res Pract. 1994, 190: 1017-25), and oligonucleotide microarray (e.g., by hybridization of polynucleotide sequences derived from a sample to oligonucleotides attached to a solid surface [e.g., a glass wafer) with addressable location, such as Affymetrix microarray (Affymetrix®, Santa Clara, CA)].

For example, the level of RRN3 in a sample can be determined by RT-PCR using primers available from Santa Cruz Biotechnology Inc. (sc-106866-PR), or Taqman Gene Expression Assay HS00607907_ml (Applied Biosystems, Foster City, CA, USA), according to manufacturer's recommendation.

According to some embodiments of the invention, detection of the expression level of the protein of the Pol I pathway is performed using an antibody which specifically binds to a polypeptide expressed from the gene of the Pol I pathway (e.g., including any variants thereof which is known in the art). Examples of methods of detecting the level and/or activity of specific protein molecules in a cell sample include Enzyme linked immunosorbent assay (ELISA), Western blot analysis, radio-immunoassay (RIA), Fluorescence activated cell sorting (FACS), immunohistochemical analysis, *in situ* activity assay (using e.g., a chromogenic substrate applied on the cells containing an active enzyme), in vitro activity assays (in which the activity of a particular enzyme is measured in a protein mixture extracted from the cells). For example, in case the detection of the expression level of a secreted protein is desired, ELISA assay may be performed on a sample of fluid obtained from the subject (e.g., serum), which contains cell-secreted content.

As described above, the level of expression of least one gene involved in the RNA polymerase I pathway in a cell of the subject following treating with the compound is compared to the level of expression of the at least one gene in a cell of the subject prior to treating the subject with the compound.

As used herein the phrase "following treating with the compound" refers to any time period after administering the compound to the subject, e.g., from a few minutes to hours, or from a few days to weeks or months after drug administration.

According to some embodiments of the invention the level of expression is determined following administration of the first dose of the compound.

According to some embodiments of the invention the level of expression is determined following administration of any dose of the compound.

As used herein the phrase "prior to treating with the compound" refers to any time period prior administering the compound to the subject, e.g., from a few minutes to hours, or from a few days to weeks or months prior to drug administration.

According to some embodiments of the invention the level of expression is determined prior any dose of the compound (e.g., when the subject is naive to treatment).

According to some embodiments of the invention prior to treating refers to when the subject is first diagnosed with autoimmune disease, e.g., multiple sclerosis.

According to some embodiments of the invention prior to treating refers to when the subject is suspected of having the autoimmune disease (e.g., multiple sclerosis), or diagnosed with probable autoimmune disease (e.g., probable multiple sclerosis).

According to some embodiments of the invention prior to treating refers to upon the onset of the autoimmune disease.

According to some embodiments of the invention the effect of the treatment on the subject can be evaluated by monitoring the level of expression of at least one of the polynucleotides described hereinabove. For example, downregulation in the level of RRN3 in the subject following treatment can be indicative of the positive effect of the treatment on the subject, e.g., switching from a typical RRMS to a BMS course of multiple sclerosis.

As described above, a decrease above a predetermined threshold in the level of expression of the at least one gene following treating with the compound relative to the level of expression of the at least one gene prior to treating with the compound indicates that the compound is efficient for treating the subject.

As used herein the phrase "a decrease above a predetermined threshold" refers to a decrease in the level of expression in the cell of the subject following treating with the compound which is higher than a predetermined threshold such as a about 10 %, e.g., higher than about 20 %, e.g., higher than about 30 %, e.g., higher than about 40 %, e.g., higher than about 50 %, e.g., higher than about 60 %, higher than about 70 %, higher than about 80 %, higher than about 90 %, higher than about 2 times, higher than about three times, higher than about four time, higher than about five times, higher than about six times, higher than about seven times, higher than about eight times, higher than about nine times, higher than about 20 times, higher than about 50 times, higher than about 100 times, higher than about 200 times, higher than about 350, higher than about 500 times, higher than about 1000 times, or more relative to the level of expression prior to treating with the compound.

As described, an increase above a predetermined threshold in the level of expression of the at least one gene following treating with the compound relative to the level of expression of the at least one gene prior to treating with the compound indicates that the compound is not efficient for treating the subject.

As used herein the phrase "an increase above a predetermined threshold" refers to an increase in the level of expression in the cell of the subject following treating with the compound, which is higher than a predetermined threshold such as about 10 %, e.g., higher than about 20 %, e.g., higher than about 30 %, e.g., higher than about 40 %, e.g., higher than about 50 %, e.g., higher than about 60 %, higher than about 70 %, higher than about 80 %, higher than about 90 %, higher than about 2 times, higher than about three times, higher than about four time, higher than about five times, higher than about six times, higher than about seven times, higher than about eight times, higher than about nine times, higher than about 20 times, higher than about 50 times, higher than about 100 times, higher than about 200 times, higher than about 350, higher than about 500 times, higher than about 1000 times, or more relative to the level of expression of the at least one gene prior to treating with the compound.

As described, a level of expression of the at least one gene following treating with the compound which is identical or changed below a predetermined threshold as compared to prior to treating with the compound is indicative that the treatment is not efficient for treating the subject.

As used herein the phrase "changed below a predetermined threshold as compared to prior to treating with the compound" refers to an increase or a decrease in the level of expression in the cell of the subject following treating with the compound, which is lower than a predetermined threshold, such as lower than about 10 times, e.g., lower than about 9 times, e.g., lower than about 8 times, e.g., lower than about 7 times, e.g., lower than about 6 times, e.g., lower than about 5 times, e.g., lower than about 4 times, e.g., lower than about 3 times, e.g., lower than about 2 times, e.g., lower than about 90%, e.g., lower than about 80%, e.g., lower than about 70%, e.g., lower than about 60%, e.g., lower than about 50%, e.g., lower than about 40%, e.g., lower than about 30%, e.g., lower than about 20%, e.g., lower than about 10%, e.g., lower than about 9%, e.g., lower than about 8%, e.g., lower than about 7%, e.g., lower than about 6%, e.g., lower than about 5%, e.g., lower than about 4%, e.g., lower than about 3%, e.g., lower than about 2%, e.g., lower than about 1% relative to the level of expression of the at least one gene prior to treating with the compound. Examples of genes involved in the RNA polymerase I pathway which can be used according to the method of the invention include RRN3, LRPPRC, POLR1B, POLR1C, POLR1D, POLR2A, POLR2B, POLR2C, POLR2D, POLR2E, POLR2E, POLR2F, POLR2G, POLR2H, POLR2I, POLR2J, POLR2J2, MGC13098, POLR2K, POLR2L, POLR3B, POLR3C, POLR3D, POLR3E, POLR3F, POLR3G, POLR3K, POLRMT, POLRMT and POLS.

Sequence information regarding gene products (*i.e.,* RNA transcripts and polypeptide sequences) of the genes of RNA polymerase I pathway and of probes which can be used for detection thereof can be found in Table 1 hereinbelow.

**Table 1**

| ***Genes involved in the RNA polymerase I pathway*** | | | | | |
|---|---|---|---|---|---|
| ***Affymetrix ProbSet*** | ***SEQ ID NO:*** | ***Representativ e Public ID* / *SEQ ID NO:*** | ***Representative polypeptide Public ID* / *SEQ ID NO:*** | ***Gene Symbol*** | ***Gene Title*** |
| 216902_s_at | 1 | AF001549 / 40; NM_ 018427 / 41 | NP_060897/81 | RRN3 | RRN3 RNA polymerase I transcription factor homolog |
| 211971_s_at | 2 | AI653608 / 42; NM_133259 / 43 | NP_573566/82 | LRPPRC | leucine-rich PPR-motif containing |
| 220113_x_at | 3 | NM_019014 / 44 | NP_001131076/8 3/NP_061887/120 | POLR1B | polymerase (RNA) I polypeptide B, 128kDa |
| 207515_ s_at | 4 | NM_004875 / 45 | NP_976035/84 | POLR1C | polymerase (RNA) I polypeptide C, 30kDa |
| 209317_at | 5 | AF008442 / 46 | NP_976035/85 | POLR1C | polymerase (RNA) I polypeptide C, 30kDa |
| 218258_at | 6 | NM_015972 / 47 | NP_057056/86/N P_689918/121 | POLR1D | polymerase (RNA) I polypeptide D, 16kDa |
| 202725_at | 7 | NM_000937 / 48 | NP_000928/87 | POLR2A | polymerase (RNA) II (DNA directed) polypeptide A, 220kDa |
| 217420_s_at | 8 | M21610 / 49 | NP_000928/88 | POLR2A | polymerase (RNA) II (DNA directed) polypeptide A, 220kDa |
| 201803_at | 9 | NM_000938 / 50 | NP_000929/89 | POLR2B | polymerase (RNA) II (DNA directed) polypeptide B, 140kDa |
| 208996_s_at | 10 | BC000409 / 51 | NP_116558/90 | POLR2C | polymerase (RNA) II (DNA directed) polypeptide C, 33kDa |
| 214263_x_at | 11 | AI192781 / 52 | NP_116558/91 | POLR2C | polymerase (RNA) II (DNA directed) polypeptide C, 33kDa |
| 216282_x_at | 12 | AJ224143 / 53 | NP_116558/92 | POLR2C | polymerase (RNA) II (DNA directed) polypeptide C, 33kDa |
| 203664_s_at | 13 | NM_004805 / 54 | NP_004796/93 | POLR2D | polymerase (RNA) II (DNA directed) polypeptide D |
| 214144_at | 14 | BF432147 / 55 | NP_004796/94 | POLR2D | polymerase (RNA) II (DNA directed) polypeptide D |
| 213887_s_at | 15 | AI554759 / 56 | NP_002686/95 | POLR2E | polymerase (RNA) II (DNA directed) polypeptide E, 25kDa |
| 217854_s_at | 16 | BC004441 / 57 | NP_002686/96 | POLR2E | polymerase (RNA) II (DNA directed) polypeptide E, 25kDa |
| 209511_at | 17 | BC003582 / 58 | NP_068809/97 | POLR2F | polymerase (RNA) II (DNA directed) polypeptide F |
| 202306_at | 18 | NM_002696 / 59 | NP_002687/98 | POLR2G | polymerase (RNA) II (DNA directed) polypeptide G |
| 209302_at | 19 | U37689 /60 | NP_006223/99 | POLR2H | polymerase (RNA) II (DNA directed) polypeptide H |
| 212955_s_at | 20 | AL037557 / 61 | NP_006224/100 | POLR2I | polymerase (RNA) II (DNA directed) polypeptide I, 14.5kDa |
| 212782_x_at | 21 | BG335629 / 62 | NP_006225/101 | POLR2J | polymerase (RNA) II (DNA directed) polypeptide J, 13.3kDa |
| 216242_x_at | 22 | AW402635 / 63 | NP_116581/102 | POLR2J2 | DNA directed RNA polymerase II polypeptide J-related gene |
| 214740_at | 23 | BE676209 / 64 | NP_001091084/1 03/NP_006225/12 2/NP_116581/125 | POLR2J2 /// MGC13098 | DNA directed RNA polymerase II polypeptide J-related gene /// hypothetical prote |
| 202634_at | 24 | AL558030 / 65 | NP_005025/104 | POLR2K | polymerase (RNA) II (DNA directed) polypeptide K, 7.0kDa |
| 202635_s_at | 25 | NM_005034 / 66 | NP_005025/105 | POLR2K | polymerase (RNA) II (DNA directed) polypeptide K, 7.0kDa |
| 202586_at | 26 | AA772747 / 67 | NP_066951/106 | POLR2L | polymerase (RNA) II (DNA directed) polypeptide L, 7.6kDa |
| 211730_s_at | 27 | BC005903 / 68 | NP_066951/107 | POLR2L | polymerase (RNA) II (DNA directed) polypeptide L, 7.6kDa /// polymerase (RNA) II |
| 219459_at | 28 | NM_018082 / 69 | NP_ 001154180/1 08/NP_060552/12 3 | POLR3B | polymerase (RNA) III (DNA directed) polypeptideB |
| 209382_at | 29 | U93867 / 70 | NP_006459/109 | POLR3C | polymerase (RNA) III (DNA directed) polypeptide C (62kD) |
| 210573_s_at | 30 | BC004424 / 71 | NP_006459/110 | POLR3C | polymerase (RNA) III (DNA directed) polypeptide C (62kD) |
| 208361_s_at | 31 | NM_001722 / 72 | NP_001713/111 | POLR3D | polymerase (RNA) III (DNA directed) polypeptide D, 44kDa |
| 218016_s_at | 32 | NM_ 018119 / 73 | NP_060589/112 | POLR3E | polymerase (RNA) III (DNA directed) polypeptide E (80kD) |
| 205218_at | 33 | NM_006466 / 74 | NP_006457/113 | POLR3F | polymerase (RNA) III (DNA directed) polypeptide F, 39 kDa |
| 206653_at | 34 | BF062139 / 75 | NP_006458/114 | POLR3G | Polymerase (RNA) III (DNA directed) polypeptide G (32kD) |
| 206654_s_at | 35 | NM_006467 / 76 | NP_006458/115 | POLR3G | polymerase (RNA) III (DNA directed) polypeptide G (32kD) |
| 218866_s_at | 36 | AF060223 / 77 | NP_057394/116 | POLR3K | polymerase (RNA) III (DNA directed) polypeptide K, 12.3 kDa |
| 203782_s_at | 37 | NM_005035 / 78 | NP_005026/117 | POLRMT | polymerase (RNA) mitochondrial (DNA directed) |
| 203783_x_at | 38 | BF057617 / 79 | NP_005026/118 | POLRMT | polymerase (RNA) mitochondrial (DNA directed) |
| 202466_at | 39 | NM_006999/ 80 | NP_ 001165276/1 19/NP_00116527 7/124/NP_008930 /126 | PAPD7 | polymerase (DNA directed) sigma |

Table 1.

According to some embodiments of the invention, the at least one gene involved in the RNA polymerase 1 pathway is selected from the group consisting of POLR1D, LRPPRC, RRN3 and NCL.

According to some embodiments of the invention, the at least one gene involved in the RNA polymerase 1 pathway is RRN3.

According to some embodiments of the invention, the at least one gene involved in the RNA polymerase 1 pathway is LRPPRC.

According to some embodiments of the invention, the at least one gene involved in the RNA polymerase 1 pathway is POLR1D.

According to some embodiments of the invention, the at least one gene involved in the RNA polymerase 1 pathway comprises RRN3 and POLR1D.

According to some embodiments of the invention, the at least one gene involved in the RNA polymerase 1 pathway comprises RRN3 and LRPPRC.

According to some embodiments of the invention, the at least one gene involved in the RNA polymerase 1 pathway comprises POLR1D and LRPPRC.

According to some embodiments of the invention, the at least one gene involved in the RNA polymerase 1 pathway comprises RRN3, LRPPRC and POLR1D.

According to some embodiments of the invention, the at least one gene involved in the RNA polymerase 1 pathway is RRN3 and NCL.

According to some embodiments of the invention, the at least one gene involved in the RNA polymerase 1 pathway is LRPPRC and NCL.

According to some embodiments of the invention, the at least one gene involved in the RNA polymerase 1 pathway is POLR1D and NCL.

According to some embodiments of the invention, the at least one gene involved in the RNA polymerase 1 pathway comprises RRN3, POLR1D and NCL.

According to some embodiments of the invention, the at least one gene involved in the RNA polymerase 1 pathway comprises RRN3, LRPPRC and NCL.

According to some embodiments of the invention, the at least one gene involved in the RNA polymerase 1 pathway comprises POLR1D, LRPPRC and NCL.

According to some embodiments of the invention, the at least one gene involved in the RNA polymerase 1 pathway comprises RRN3, LRPPRC, POLR1D and NCL.

Qualifying the compound as being suitable for treating the autoimmune disease in the subject can be also performed by an *in-vitro* method.

Thus, according to an aspect of some embodiments of the invention there is provided an *in vitro* method of predicting an efficiency of the compound of some embodiments of the invention for treatment of a subject diagnosed with an autoimmune disease, the method is effected by:
(a) contacting cells of the subject with a therapeutically effective amount of the compound; and
(b) comparing a level of expression in the cells of at least one gene involved in the RNA polymerase I pathway following contacting with the compound to a level of expression of the at least one gene in the cells prior to contacting with the compound,
   (i) wherein a decrease above a predetermined threshold in the level of expression of the at least one gene following contacting with the compound relative to the level of expression of the at least one gene prior to contacting with the compound indicates that the treatment is efficient for treating the subject;
   (ii) wherein an increase above a predetermined threshold in the level of expression of the at least one gene following contacting with the compound relative to the level of expression of the at least one gene prior to contacting with the compound indicates that the treatment is not efficient for treating the subject; or
   (iii) wherein when a level of expression of the at least one gene following contacting with the compound is identical or changed below a predetermined threshold as compared to prior to contacting with the compound then the treatment is not efficient treating the subject.
thereby predicting the efficiency of the compound for treatment of the subject diagnosed with the autoimmune disease.

Contacting cells with the compound can be performed by any *in vitro* conditions including for example, adding the compound to cells derived from a subject (e.g., a primary cell culture, a cell line) or to a biological sample comprising same (e.g., a fluid, liquid which comprises the cells) such that the compound is in direct contact with the cells. According to some embodiments of the invention, the cells of the subject are incubated with the compound. The conditions used for incubating the cells are selected for a time period/concentration of cells/concentration of drug/ratio between cells and the compound and the like which enable the compound to induce cellular changes, such as changes in transcription and/or translation rate of specific genes, proliferation rate, differentiation, cell death, necrosis, apoptosis and the like.

Methods of monitoring cellular changes induced by the drugs are known in the art and include for example, the MTT test which is based on the selective ability of living cells to reduce the yellow salt MTT (3-(4, 5- dimethylthiazolyl-2)-2, 5-diphenyltetrazolium bromide) (Sigma, Aldrich St Louis, MO, USA) to a purple-blue insoluble formazan precipitate; the BrDu assay [Cell Proliferation ELISA BrdU colorimetric kit (Roche, Mannheim, Germany]; the TUNEL assay [Roche, Mannheim, Germany]; the Annexin V assay [ApoAlert® Annexin V Apoptosis Kit (Clontech Laboratories, Inc., CA, USA)]; the Senescence associated-β-galactosidase assay (Dimri GP, Lee X, et al. 1995. A biomarker that identifies senescent human cells in culture and in aging skin in vivo. Proc Natl Acad Sci USA 92:9363-9367); as well as various RNA and protein detection methods (which detect level of exprssion and/or activity) which are further described hereinabove.

According to some embodiments of the invention, the cells are incubated under conditions which enable the effect of the compound on cellular processes such as downregulation of the at least one gene of the RNA polymerase I pathway.

Compositions of some embodiments of the invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container, and labeled for treatment of an indicated condition, as is further detailed above.

According to an aspect of some embodiments of the invention there is provided a kit for monitoring efficacy of a compound for treating an autoimmune disease, the comprising the compound of some embodiments of the invention and at least one agent suitable for detecting the expression level of at least one gene of the RNA polymerase I pathway.

According to some embodiments of the invention, the kit further comprises a reference cell obtained from a subject who is responsive to the compound, *i.e*., a subject whose clinical symptoms were improved following treatment with the compound.

According to some embodiments of the invention, the agent suitable for detecting the expression level of at least one gene of the RNA polymerase I pathway is an antibody which specifically binds to a protein encoded by the at least one gene of the RNA polymerase I pathway.

According to some embodiments of the invention, the agent suitable for detecting the expression level of at least one gene of the RNA polymerase I pathway is a polynucleotide probe which specifically hybridizes to an RNA of the at least one gene of the RNA polymerase I pathway.

According to some embodiments of the invention, the compound which is included in the kit is CX-5461 and the agent is suitable for detecting the expression level of RRN3 in cells.

As used herein the term "about" refers to ± 10 %.

The terms "comprises", "comprising", "includes", "including", "having" and their conjugates mean "including but not limited to".

The term "consisting of means "including and limited to".

The term "consisting essentially of' means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention. Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

### EXAMPLE 1

### CX-5461 SYNTHESIS

The title compound 2-(4-methyl-1,4-diazepan-1-yl)-N-((5-methylpyrazin-2-yl)methyl)-5-oxo-5H-benzo[4,5]thiazolo[3,2-a][1,8]naphthyridine-6-carboxamide (CX-5461) (schematic illustration of the chemical structure is shown in Figure 1) was synthesized according to reported procedures (Chua PC, et al. A novel and efficient synthesis of 3-carboxy-4-oxo-1,8-naphthyridines using magnesium chloride. Tetrahedron Letters 49 :4437-4442, 2008; Schwaebe MK, et al. Facile and efficient generation of quinolone amides from esters using aluminum chloride. Tetrahedron Letters 52:1096-1100, 2011; Nagasawa JY, et al. PCT Int. Appl 2009046383, 09, Apr 2009). THF was distilled over Na and benzophenone, acetonitrile was dried over 4Å molecular sieves. All other reagents were used with no further purification. 2,6-dichloronicotinoylchloride, N-methylhomopiperazine, triethylamine, magnesium chloride and 1,8-Diazobicyclo[5.4.0]undec-7-ene (DBU) were all purchased from Sigma Aldrich, ethyl 2-(benzothiazol-2-yl)acetate and (5-methylpyrazin-2-yl)methaneamine were purchased from TCI. ¹H NMR analyses were performed using a Bruker Avance DPX-400 Ultra shield or alternatively Bruker Avance DMX-500. All the chemical shifts are referenced to the residual solvent signal. All MS analyses were performed on a Thermo Scientific LCQ Fleet mass spectrometer with an ESI source. All the spectra were recorded in the positive mode (unless mentioned otherwise) and were analyzed by the Thermo Scientific Xcalibur software.

2,6-dichloronicotinoylchloride (2.42 gr.; 11.5 mmol) was slowly added to a solution containing ethyl 2-(benzothiazol-2-yl)acetate (1.89 mL; 10.4 mmol), magnesium chloride (1.48 gr.; 15.6 mmol) and tetrahydrofuran (25 mL). The resulting suspension was cooled to 0°C and triethylamine (3.50 mL; 25.0 mmol) was added dropwise over 30 minutes. Once the addition was complete, the reaction mixture was allowed to stir while heating to room temperature over 2 hours. The reaction was then warmed to 80°C for 1 hour and was monitored by LCMS. Once the reaction was over, 200 mL of water were added, and the resulting suspension was filtered by a büchner funnel. The remaining solid, was then dissolved in a (3:1; volumne /volume (v:v)) chloroform : dichloromethane mixture (500 mL), washed with water, dried over Na₂SO₄, filtered and the solvent was removed under reduced pressure (Figure 2).

The product was then purified by trituration with cold diethylether to afford ethyl 2-chloro-5-oxo-5H-benzo[4,5]thiazolo[3,2-a][1,8]naphthyridine-6-carboxylate (2) as a beige solid (3.34 gr; 89%). ¹H NMR (400 MHz, *CDCl₃*) δ ppm 1.50 (t, 3H, J=7.2 Hz), 4.52 (q, 2H, J=7.2 Hz), 7.49 (t, 1H, J=7.6 Hz), 7.58 (d, 1H, J=8.4 Hz), 7.61 (t, 1H, J=8.0 Hz), 7.77 (d, 1H, J=8.0 Hz), 8.86 (d, 1H, J=8.0 Hz), 9.55 (d, 1H, J=8.8 Hz); MS (ESI): 359 (M+H)⁺.

To a slurry of ethyl 2-chloro-5-oxo-5H-benzo[4,5]thiazolo[3,2-a][1,8]naphthyridine-6-carboxylate (2) (3.34 gr; 9.3 mmol) in acetonitrile (48 mL) was added N-methylhomopiperazine (1.21 mL; 9.7) and triethylamine (1.35 mL; 9.7 mmol). The mixture was heated under reflux for 24 hours after which additional 0.2 eq of N-methylhomopiperazine (231 µL) and 0.2 eq triethylamine (260µL) were added and stirring was continued for additional 24 hours. The reaction was then cooled to RT and the product was collected by filtration via a büchner funnel, the solid was further washed with cold acetonitrile to afford ethyl 2-(4-methyl-1,4-diazepan-1-yl)-5-oxo-5H-benzo[4,5]thiazolo[3,2-a][1,8]naphthyridine-6-carboxylate (3) as a tan solid (3.34 gr; 81%) (Figure 3). ¹H NMR (500 MHz, *CDCl₃*) δ ppm 9.33 (d, *J* = 7.57 Hz, 1H), 8.48 (d, *J* = 8.96 Hz, 1H), 7.69 (dd, *J* = 7.58, 1.47 Hz, 1H), 7.47-7.38 (m, 2H), 6.69 (d, *J* = 9.00 Hz, 1H), 4.45 (q*, J* = 7.13 Hz, 2H), 4.22-4.05 (m, 2H), 3.93-3.74 (m, 2H), 3.30-3.10 (m, 2H), 3.08-2.93 (m, 2H), 2.66 (s, 3H), 2.50-2.31 (m, 2H), 1.46 (t, *J* = 7.13 Hz, 3H); MS (ESI): 437 (M+H)⁺.

To a solution of ethyl 2-(4-methyl-1,4-diazepan-1-yl)-5-oxo-5H-benzo[4,5]thiazolo[3,2-a][1,8]naphthyridine-6-carboxylate (3) (3.34 gr; 7.66 mmol), (5-methylpyrazin-2-yl)methaneamine (2.87 mL; 25.3 mmol), and DBU (3.45 mL; 23.0 mmol) in dichloromethane (118 mL) was added AlCl₃ (3.06 gr; 23.0) portionwise. The reaction mixture was then left to stir at RT for 45 minutes after which, the reaction was diluted with dichloromethane (118 mL) and NaOH 4N (118 mL), and stirring was continued for additional 20 minutes. The layers were then separated and the organic layer was washed with H₂O (2 x 100 mL), NaCl sat. (1 x 100 mL), dried over Na₂SO_{4,} filtered and the solvent was removed under vaccum. The resulting solid was then purified by trituration with acetonitrile to afford 2-(4-methyl-1,4-diazepan-1-yl)-N-((5-methylpyrazin-2-yl)methyl)-5-oxo-5H-benzo[4,5]thiazolo[3,2-a][1,8]naphthyridine-6-carboxamide(4) as a white yellowish solid (2.948 gr, 75%) (Figure 4). ¹H NMR (500 MHz, *MeOD*) δppm 9.26-9.10 (m, 1H), 8.57 (d, *J* = 1.33 Hz, 1H), 8.52 (d, *J* = 1.33 Hz, 1H), 8.20 (d, *J* = 9.08 Hz, 1H), 7.71-7.64 (m, 1H), 7.37-7.31 (m, 2H), 6.72 (d, *J* = 9.12 Hz, 1H), 4.76 (s, 2H), 3.90-3.77 (m, 2H), 3.76-3.63 (m, 2H), 2.95-2.84 (m, 2H), 2.75-2.68 (m, 2H), 2.57 (s, 3H), 2.45 (s, 3H), 2.10 (m, 2H); MS (ESI): 514 (M+H)⁺.

### EXAMPLE 2

### IN VITRO STUDIES: CX-5461 EFFECTS ON VIABILITY OF MICE SPLENOCYTES

No data regarding the effects of CX-5461 on immune cells is available. Towards the application of CX-5461 in the EAE mouse model of multiple sclerosis (MS), the present inventors have examined CX-5461 effects on the viability of immune cells and a range of effective doses. These experiments were done to provide applicable data for the *in-vivo* animal studies.

### Experimental Methods

***Measurements of cell viability*** - Cells viability was assessed by the 2,3 bis [2-Methoxy-4-nitro-5 -sulfophenyl] -2H-tetrazolium-5 -carboxanilide (XTT) assay (Biological Industries, Kibbutz Beit Hemeek, Israel) which measures the reduction of a tetrazolium component (XTT) into soluble formazan product by the mitochondria of viable cells. The intensity of the dye obtained is proportional to the number of metabolic active cells. Mouse splenocytes were plated in presence of PHA (10 mg/ml) and elevated concentrations of CX-5461 (50-4000 nM) for 72 hours. Cells plated in absence of CX-5461 served as controls. Following incubation, cell viability was measured by 2,3-bis-(2-methoxy-4-nitro-5-sulfophenyl)-2H-tetrazolium-5-carboxanilide (XTT) assay according to manufacturer's instructions (Biological Industries, Kibbutz Beit Hemeek, Israel). The intensity of the dye obtained is proportional to the number of metabolic active cells.

### Experimental Results

As shown in Figure 5, CX-5461 decreased cell viability of splenocyte cells in a dose dependent manner, by 27% at 50 nM to maximal 97-100% at 500 nM and higher concentrations. Accordingly, the range between 50 to 500 nM concentrations was chosen for further experiments.

### EXAMPLE 3

### IN VITRO STUDIES: CX-5461 EFFECTS ON VIABILITY OF HUMAN PERIPHERAL MONONUCLEAR CELLS (PBMCS) - HEALTHY SUBJECTS

No data regarding the effects of CX-5461 on human blood immune cells is available. Towards the application of CX-5461 in cell cultures of PBMCs obtained from MS patients, the present inventors have examined CX-5461 effects on the viability of human PBMCs obtained from healthy subjects in a range of the effective doses, as follows.

### Experimental Procedures

PBMCs (2.0*10⁻⁵ cells/well) from normal, healthy, subjects were plated in the presence of PHA (20 µg/ml) with elevated concentrations of CX-5461 (50-500 nM) for 72 hours. Cells plated in absence of CX-5461 served as controls.

### Experimental Results

As shown in Figure 6, maximal effect of CX-5461 on viability of human PBMCs was detected at a concentration of 50 nM by 35%. Increased concentrations of CX-5461 did not result with further decrease in cell viability.

### EXAMPLE 4

### IN VITRO STUDIES: CX-5461 EFFECTS ON APOPTOSIS OF HUMAN PBMCS FROM MULTIPLE SCLEROSIS PATIENTS

Inhibition of Pol I by CX-5461 results in nucleolar stress which causes the release of ribosomal proteins from the nucleolus and subsequent activation of p53, resulting in cell apoptosis of human cancer cells (Drygin D, et al. Targeting RNA polymerase I with an oral small molecule CX-5461 inhibits ribosomal RNA synthesis and solid tumor growth. Cancer Res 71:1418-1430, 2011). To test the effect of CX-5461 on apoptosis in human PBMCs obtained from MS patients, the present inventors used the minimal effective doses of CX-5461 (at a range of 50 to 250 nM and including a lower concentration of 25 nM as well) obtained from experiments on PBMCs from healthy subjects which are shown in Example 3 hereinabove.

### Experimental Procedures

***Measurement of apoptosis*** - Apoptosis was measured by flow cytometry as follows: (1) percent of Annexin V positive cells; (2) number of cell cycle/apoptotic cells using propidium iodid (PI) staining; (3) percent of P53 positive cells to demonstrate the role of P53 dependent apoptosis mechanism; and (4) percent of RRN3 positive cells used as a reporter for detection of Pol I activity in cells after incubation with CX-5461.

For these purposes the present inventors used mAb anti Annexin V-FITC/propidium iodide (PI, 0.5mg/ml), anti p53 mAb and anti RRN3 mAb (Sigma-Auldrich, USA), stained and presented as percentage of total cell counts. Fluorescence intensity measurements were done after excitation at 488 nm and detection at 520-530 nm for green fluorescence and at 665-685 nm for PI. Apoptotic level of PBMCs obtained from MS patients was measured at baseline and after 24 hours of incubation with increasing concentrations of CX-5461 (25-250 nM).

### Experimental Results

***CX-5461 significantly stimulated apoptosis of PBMCs from MS patients*** - The results shown in Figures 7A-D demonstrate that CX-5461 significantly stimulated apoptosis of PBMCs from MS patients in a dose dependent manner as was demonstrated by the correlative increase in the percent and number of PI and Annexin V stained cells with increased CX-5461 concentrations. The mechanism of apoptosis was mediated by RRN3 as demonstrated by significant (p=0.00003) reduction in its expression and accordingly by stimulation of P53 expression.

### EXAMPLE 5

### IN VITRO STUDIES: CX-5461 EFFECTS ON VIABILITY OF NEURAL PROGENITORS CELLS IN MICE

As MS is a central nervous system disease the present inventors aimed to evaluate the effects of CX-5461 on viability of neuronal cells. The present inventors tested the effective range of CX-5461 concentrations found in the peripheral blood in relation to cell viability found on the regenerative capacity of the neural progenitor cells (NPCs).

### Experimental Methods

***Cell viability assay of neuronal progenitor cells*** - NPCs derived from the subventricular zone of the lateral ventricle of adult mice were allowed to proliferate in the presence of increased concentrations of CX-5461 (20 nM to 500 nM) for 72 hours, and cell viability was tested using XTT assay.

### Experimental Results

***CX-5461 does not affect NPCs viability at a concentration lower than 20 nM, and does not suppress more than 50% viability at concentrations higher than 50 nM-***As shown in Figure 8, CX-5461 at a concentration of 20 nM had no effect on NPCs viability, while a 30% decrease in cell viability was detected at a concentration of 50 nM and ∼50% decrease in cell viability was demonstrated at higher concentrations (100, 250 and 500 nM). These results are similar to the findings shown in Examples 2-4 hereinabove with respect to mice splenocytes and human PBMCs both from healthy subjects and MS patients and suggest that CX-5461 would not result in significant effect (above 50% suppression of viability) at concentration lower than 50 nM and does not affect NPCs viability in concentration lower than 20 nM.

### EXAMPLE 6

### IN VIVO EXPERIMENTS: CX-5461 DELAYES ONSET AND REDUCES CLINICAL SCORES OF MOG INDUCED EAE IN MICE

Myelin oligodendrocyte glycoprotein (MOG) induced experimental autoimmune encephalomyelitis (EAE) is a widely accepted model for studying the clinical and pathological features of MS (Miller, S, Karpus WJ. Experimental autoimmune encephalomyelitis in the mouse. Curr Protoc Immunol Chapter 15: Unit 15.1, 2007). To test the effect of CX-5461 on multiple sclerosis, the present inventors have tested the effect of blocking RNA Pol-I pathway by CX-5461 treatment at the disease induction phase of MOG-induced EAE. For this experiment, CX-5461 was first administered on the day of EAE induction and continued till the animals in the control untreated group developed clinical signs of EAE.

### Experimental Methods

***EAE induction*** - EAE was induced in female C57BL/6 mice, 8 weeks of age, by subcutaneous immunization to the flank of emulsion (1:1 ratio volume/volume) containing 200 mg MOG₃₅₋₅₅ peptide dissolved in phosphate-buffered saline (PBS) (at a concentration 2 mg/ml) and 0.5 mg Mycobacterium Tuberculosis H37Ra (Difco, Detroit, MI) in 100 mL incomplete Freund's adjuvant (Sigma, St. Louis, MO). Pertussis toxin 200 ng/mouse (dissolved in 100 ml PBS (List Biological Laboratories, Campbell, CA) was injected intra-peritoneal on the day of immunization and again two days later. Mice were monitored daily for clinical signs of EAE, scored as follows: 1 -flaccid tail; 1.5 - hind limb weakness; 2 - paralyzed hind limbs and poor righting ability; 2.5 - forelimb weakness, paralyzed hind limbs, paralyzed tail; 3 -four limbs paralysis, inability to right; 4 -quadriplegia; 5.0 - moribund or death of the animal after preceding clinical disease. Animal reaching a score of 4 were scarified using CO₂. Mice were kept in an SPF (Specific Pathogen Free) environment. Animal breeding and maintenance and all experimental protocols were performed in accordance with the Israeli Council for Animal Care guidelines and all experiments were approved by Sheba IRB Animal Care Ethical Committee.

***CX-5461 prophylactic treatment initiated at induction phase of MOG-induced EAE*** - EAE was induced in female C57B1/6J female mice (8 weeks old) as described above. Treatment with CX-5461 was initiated on the day of induction and continued till the clinical signs of EAE appeared in the control animal group (In 2 out of the 7 mice clinical signs of EAE appeared). CX-5461 was administered orally to each animal; the following doses of CX-5461 in 0.2 ml of vehicle were used: 12.5 mg/kg (n = 6 mice), 25 mg/kg (n = 6 mice) and 50 mg/kg (n = 7 mice) in 0.2 ml of vehicle. Vehicle treated mice served as controls (n = 7 mice). Treatment continued for 9 days post induction, until the first sign of EAE (grade 1) appeared in the vehicle animal group (in 2 out of 7 mice). Mice were monitored daily for clinical signs of EAE up to 42 days post induction.

### Experimental Results

***CX-5461 delayed EAE onset*** - The onset of EAE in all CX-5461 treated mice was significantly delayed and occurred 10 days later than the appearance of the disease in the vehicle-control group (Figure 9, and Table 2, below). This effect was evident in all experimental groups and was not dependent on CX-5461 concentration.

**Table 2**

| ***Suppression of EAE score by CX-5461*** | | | | |
|---|---|---|---|---|
| ***Treatment group*** | ***Number of mice*** | ***Disease onset (days p.i.)*** | ***Maximal clinical score*** | ***Cumulative disease score*** |
| Vehicle | 7 | 11.7±1.39 | 4.21±0.32 | 46.43±8.5 |
| 1. CX-5461 12.5 mg/kg/day | 6 | 20.83±1.36 | 2.83±0.49 | 16.25±3.94 |
| 2. CX-5461 25.0 mg/kg/day | 6 | 23.1±1.2 | 3.3±1.0 | 14.1±3.5 |
| P-value (group 1 vs. Vehicle) | | 0.0004 | 0.0208 | 0.0059 |
| P-value (group 2 vs. Vehicle) | | 6.2*10⁻⁵ | 0.05 | 0.0050 |

Table 2: Provided are the disease onset, maximal clinical score and cumulative disease scores of mice treated with CX-5461 on the day of induction of EAE by MOG. "days p.i." - days post induction of EAE.

***CX-5461 decreased EAE severity*** - In addition to the delayed EAE onset, the severity of EAE in CX-5461 treated animals was significantly lower than in the control-vehicle treated animals (Figure 9, Table 2 hereinabove). The cumulative and maximal EAE score was significantly lower in mice treated with CX-5461 12.5 mg/kg (p = 0.006 and p = 0.02, respectively) as well as in mice treated with CX-5461 25.0 mg/kg (p = 0.005 and p = 0.05, respectively) than in the control vehicle treated mice group.

The results of CX-5461 treatment at EAE induction demonstrated that blocking Pol I pathway through treatment with its inhibitor CX-5461 in concentrations of 12.5 and 25.0 mg/kg/day delayed MOG-induced EAE and suppressed its severity. Animals treated with CX-5461 at the concentration of 50 mg/kg/day similarly did not develop EAE. However treatment was toxic to the animals and resulted in death after a mean of 7.6±0.5 days of treatment post -induction. Animals showed change in the color and brightness of their fur, loss of weight and change in behavior (the animals were gathered together and movements were reduced) since day 5 of treatment. This concentration was excluded from further experiments.

### EXAMPLE 7

### IN VIVO EXPERIMENTS: CX-5461 TREATS MOG INDUCED EAE IN MICE

To test the effect of CX-5461 treatment on MOG-induced EAE mice, the present inventors have blocked RNA Pol-I pathway by CX-5461 treatment at the clinical symptomatic phase of MOG-induced EAE. For this experiment, CX-5461 was first administered on the day when the animal developed clinical signs of EAE and continued for the following 14 days.

### Experimental Methods

***EAE induction*** - EAE was induced in mice as described in Example 6, hereinabove.

***CX-5461 treatment initiated at clinical onset of MOG-induced EAE* -** EAE was induced in female C57/6J female mice as described above. Treatment with CX-5461 was administered orally in 0.2 ml of vehicle with the following concentrations: 12.5 mg/kg/d (n = 11 mice) and 25.0 mg/kg/d (n = 8 mice). Vehicle treated mice served as controls (n = 9 mice). CX-5461 treatment was initiated from the day of disease onset (score 0.5 or 1.0) for the following 14 days. The day of disease onset was evaluated for each animal daily.

### Experimental Results

*C**X-5461 decreased EAE severity*** - Both groups of mice treated with CX-5461 at a concentration of 12.5 mg/kg/day and 25.0 mg/kg/day had significantly lower EAE scores as compared to the control vehicle group (Figure 10).

All mice from the 25 mg/kg/day group died after 10.1±0.5 days of CX-5461 treatment as result of toxic effects demonstrated by change in their fur color and brightness and loss of weight. All of these animals (that were treated with 25 mg/kg/day CX-5461) exhibited low EAE score 0.88±0.18 (Figure 10).

In the group of mice treated with CX-5461 12.5 mg/kg/day all the animals survived and remained with a low EAE score, as demonstrated by lower (p=0.0004) maximal and cumulative (p=1.6*10⁻⁵) score compared to vehicle group (Table 3, hereinbelow).

**Table 3**

| ***EAE score after CX-5461 treatment at EAE onset*** | | | | |
|---|---|---|---|---|
| ***Experiment groups*** | ***Number of mice*** | ***EAE Score at Disease onset (start treatment)*** | ***Maximal EAE score*** | ***Cumulative disease score*** |
| Vehicle | 9 | 0.77±0.17 | 4.38±0.2 | 58.16±5.09 |
| CX-5461 12.5 mg/kg/day | 11 | 0.86±0.14 | 2±0.47 | 18.59±4.26 |
| P-value | | 0.7 | 0.0004 | 1.6*10⁻⁵ |

Table 3: Provided are the EAE scores at disease onset, the maximal clinical score and cumulative disease scores of mice treated with CX-5461 on the day of EAE onset (appearance of clinical symptoms).

The results presented in Table 3 and Figure 10 demonstrate that treatment of MOG-EAE model with CX-5461 at a concentration of 12.5 mg/kg/day significantly decreased the severity of the disease.

As the current multiple sclerosis treatments are given intramuscularly, subcutaneously or intravenously with uncontrolled plasma peaks and undesired side effects that decrease adherence to treatment, the use of CX-5461 which is orally bioavailable is likely to improve patient compliance.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

## Claims

1. A compound of Formula (VI), 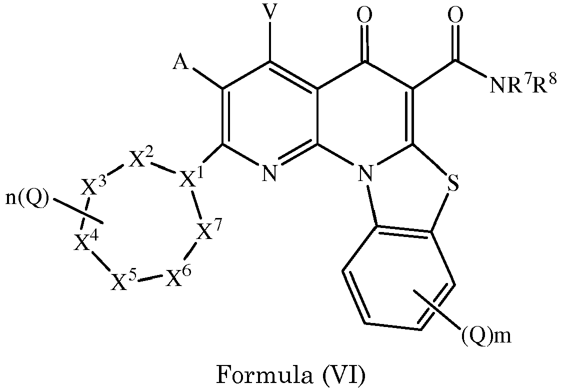 or a pharmaceutically acceptable salt or ester thereof;
wherein:
X¹ is CH or N;
X², X³, X⁴, X⁵, X⁶ and X⁷ independently are NR⁴, CH₂, CHQ or C(Q)₂, provided that: (i) zero, one or two of X², X³, X⁴, X⁵, X⁶ and X⁷ are NR⁴; (ii) when X¹ is N, both of X² and X⁷ are not NR⁴; (iii) when X¹ is N, X³ and X⁶ are not NR⁴; and (iv) when X¹ is CH and two of X², X³, X⁴, X⁵, X⁶ and X⁷ are NR⁴, the two NR⁴ are located at adjacent ring positions or are separated by two or more other ring positions;
A and V independently are H, halo, azido, -CN, -CF₃, -CONR¹R², -NR¹R², -SR², -OR², -R³, -W, -L-W, -W⁰, or -L-N(R)-W⁰;
each Q is independently halo, azido, -CN, -CF₃, -CONR¹R², -NR¹R², -SR², -OR², -R³, -W, -L-W, -W⁰, or -L-N(R)-W⁰;
in each -NR¹R², R¹ and R² together with N may form an optionally substituted azacyclic ring, optionally containing one additional heteroatom selected from N, O and S as a ring member;
R¹ is H or C1-C6 alkyl, optionally substituted with one or more halogens, or =O;
R² is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
each R⁴ is independently H, or C1-C6 alkyl; or R4 may be -W, -L-W or -L-N(R)-W⁰;
each R is independently H or C1-C6 alkyl;
R⁷ is H and R⁸ is C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring; or in -NR⁷R⁸, R⁷ and R⁸ together with N may form an optionally substituted azacyclic ring, optionally containing an additional heteroatom selected from N, O and S as a ring member;
m is 0, 1, 2, 3 or 4;
n is 0, 1, 2, 3, 4, or 5;
L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl;
W is an optionally substituted 4-7 membered azacyclic ring, optionally containing an additional heteroatom selected from N, O and S as a ring member; and
W⁰ is an optionally substituted 3-4 membered carbocyclic ring, or a C1-C6 alkyl group substituted with from 1 to 4 fluorine atoms,
for use in treating multiple sclerosis in a subject.

2. A compound of Formula (VIII), 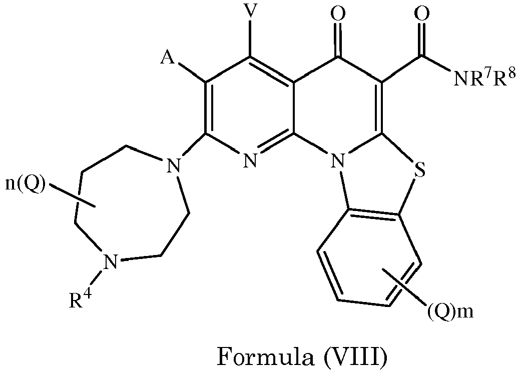 or a pharmaceutically acceptable salt or ester thereof;
wherein:
A and V independently are H, halo, azido, -CN, -CF₃, -CONR¹R², -NR¹R², -SR², -OR², -R³, -W, -L-W, -W⁰, or -L-N(R)-W⁰;
each Q is independently halo, azido, -CN, -CF₃, -CONR¹R², -NR¹R², -SR², -OR², -R³, -W, -L-W, -W⁰, or -L-N(R)-W⁰;
in each -NR¹R², R¹ and R² together with N may form an optionally substituted azacyclic ring, optionally containing an additional heteroatom selected from N, O and S as a ring member;
R¹ is H or C1-C6 alkyl, optionally substituted with one or more halogens, or =O;
R² is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
each R⁴ is independently H, or C1-C6 alkyl; or R4 may be -W, -L-W or -L-N(R)-W⁰;
each R is independently H or C1-C6 alkyl;
R⁷ is H and R⁸ is C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring; or in -NR⁷R⁸, R⁷ and R⁸ together with N may form an optionally substituted azacyclic ring, optionally containing an additional heteroatom selected from N, O and S as a ring member;
m is 0, 1, 2, 3 or 4;
n is 0, 1, 2, 3, 4 or 5;
L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl;
W is an optionally substituted 4-7 membered azacyclic ring, optionally containing an additional heteroatom selected from N, O and S as a ring member; and
W⁰ is an optionally substituted 3-4 membered carbocyclic ring, or a C1-C6 alkyl group substituted with from 1 to 4 fluorine atoms,
for use in treating multiple sclerosis in a subject.

3. A compound for use according to claim 1 or 2, wherein R⁷ is H and R⁸ is a C₁₋₄ alkyl substituted with an optionally substituted aromatic heterocyclic ring.

4. A compound for use according to claim 3, wherein the optionally substituted aromatic heterocyclic ring is selected from pyridine, pyrimidine, pyrazine, imidazole, pyrrolidine, and thiazole.

5. A compound of Formula (VII) 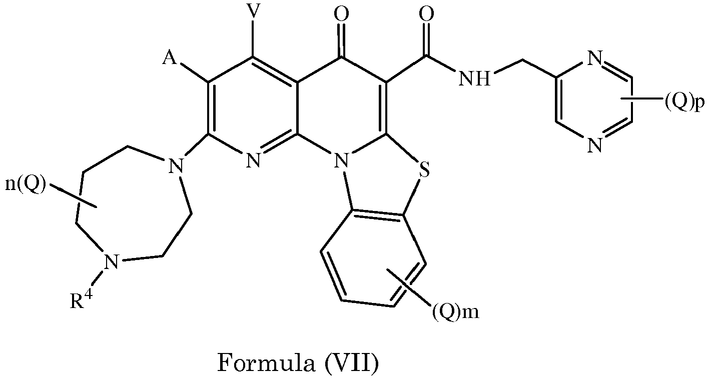
or a pharmaceutically acceptable salt or ester thereof;
A and V independently are H, halo, azido, -CN, -CF₃, -CONR1R2, -NR1R2, - SR², -OR², -R³, -W, -L-W, -W⁰, or -L-N(R)-W⁰;
each Q is independently halo, azido, -CN, -CF₃, -CONR¹R², -NR¹R², -SR², -OR², -R³, -W, -L-W, -W⁰, or -L-N(R)-W⁰;
in each -NR¹R², R¹ and R² together with N may form an optionally substituted azacyclic ring, optionally containing an additional heteroatom selected from N, O and S as a ring member;
R¹ is H or C1-C6 alkyl, optionally substituted with one or more halogens, or =O;
R2 is H, or C1-C10 alkyl, C1-C10 heteroalkyl, C2-C10 alkenyl, or C2-C10 heteroalkenyl, each of which may be optionally substituted with one or more halogens, =O, or an optionally substituted 3-7 membered carbocyclic or heterocyclic ring;
R³ is an optionally substituted C1-C10 alkyl, C2-C10 alkenyl, C5-C10 aryl, or C6-C12 arylalkyl, or a heteroform of one of these, each of which may be optionally substituted with one or more halogens, =O, or an optionally substituted 3-6 membered carbocyclic or heterocyclic ring;
each R⁴ is independently H, or C1-C6 alkyl; or R⁴ may be -W, -L-W or -L-N(R)-W⁰;
each R is independently H or C1-C6 alkyl;
m is 0, 1, 2, 3 or 4;
n is 0, 1, 2, 3, 4 or 5;
p is 0, 1, 2 or 3;
L is a C1-C10 alkylene, C1-C10 heteroalkylene, C2-C10 alkenylene or C2-C10 heteroalkenylene linker, each of which may be optionally substituted with one or more substituents selected from the group consisting of halogen, oxo (=O), or C1-C6 alkyl;
W is an optionally substituted 4-7 membered azacyclic ring, optionally containing an additional heteroatom selected from N, O and S as a ring member; and
W⁰ is an optionally substituted 3-4 membered carbocyclic ring, or a C1-C6 alkyl group substituted with from 1 to 4 fluorine atoms,
for use in treating multiple sclerosis in a subject.

6. A compound for use according to claim 5, wherein p is 0 or 1.

7. A compound for use according to any of claims 1-2, 5, wherein A and V are independently H or halo.

8. A compound for use according to any of claims 1-2, 5, wherein R⁴ is H or C1-4 alkyl.

9. A compound for use according to any of claims 1-2, 5, wherein m and n are each 0.

10. A compound selected from the group consisting of: 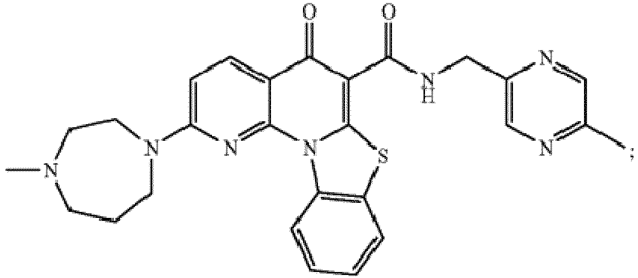 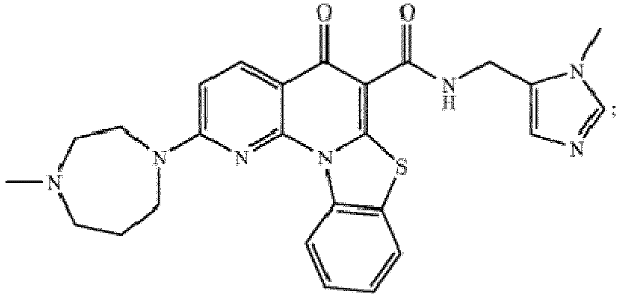 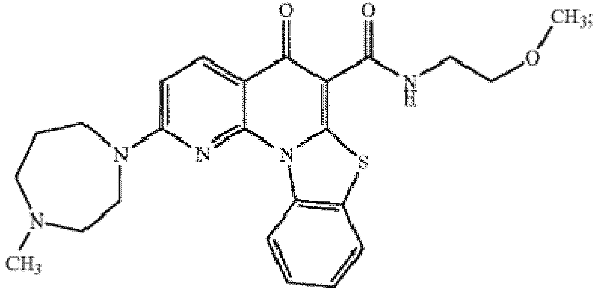 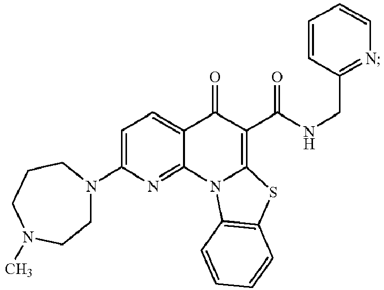 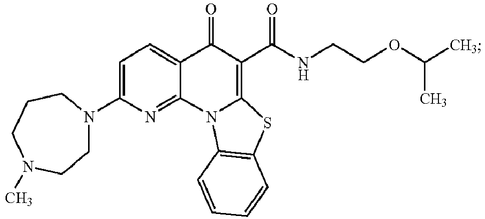 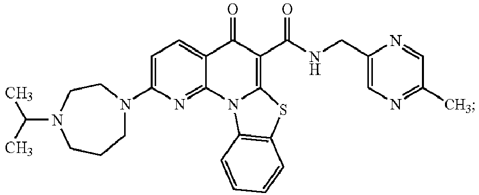 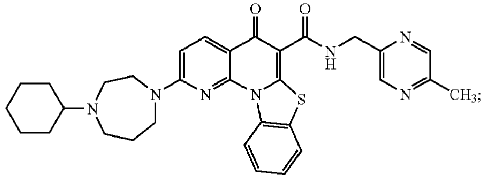 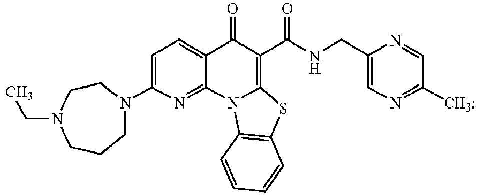 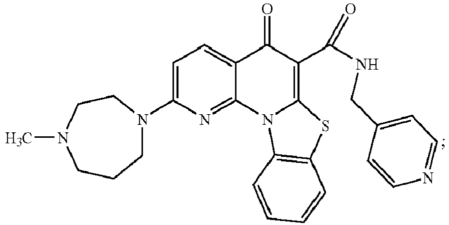 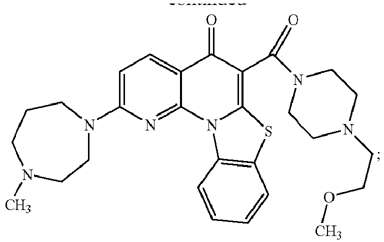 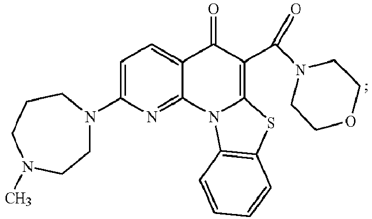 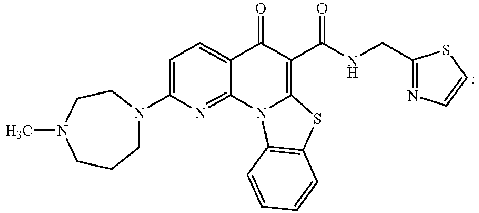 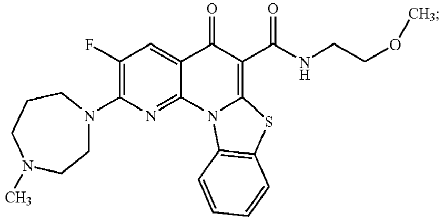 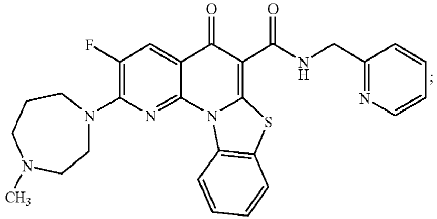 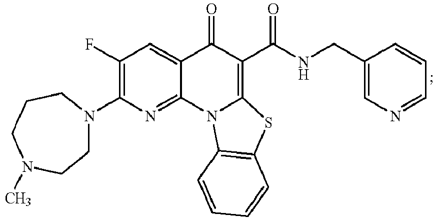 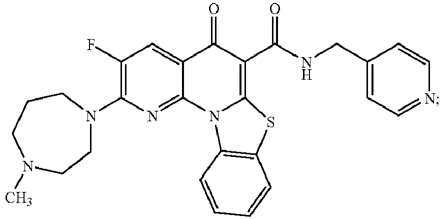 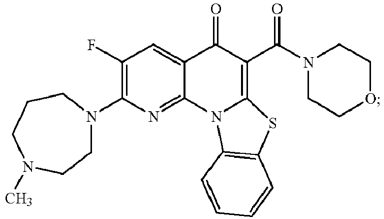 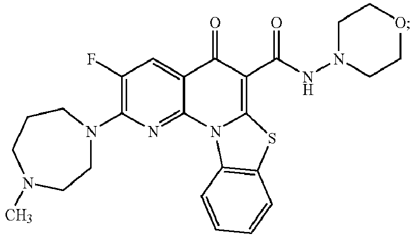 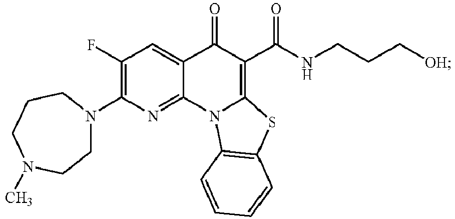 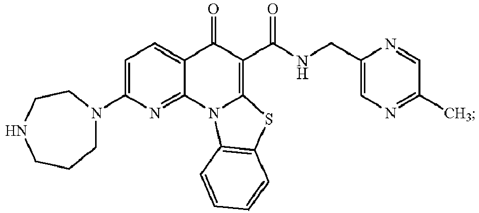 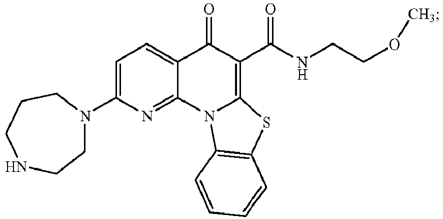 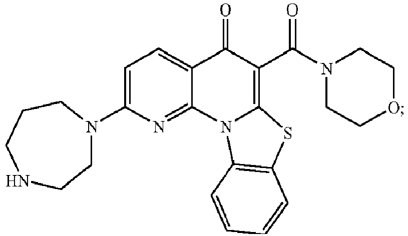 and 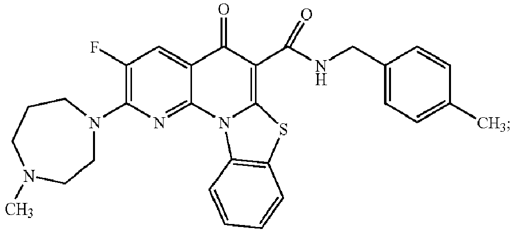 or a pharmaceutically acceptable salt or ester thereof,
for use in treating multiple sclerosis in a subject.

11. A method of monitoring treatment efficiency of a subject having multiple sclerosis, the method comprising:
comparing a level of expression of least one gene involved in the RNA polymerase I pathway in an isolated cell of the subject who has been treated with said compound of any one of claims 1-10 to a level of expression of said at least one gene in a cell of the subject prior to being treated with said compound,
(i) wherein a decrease above a predetermined threshold in said level of expression of said at least one gene following said treating with said compound relative to said level of expression of said at least one gene prior to said treating with said compound indicates that said compound is efficient for treating the subject;
(ii) wherein an increase above a predetermined threshold in said level of expression of said at least one gene following said treating with said compound relative to said level of expression of said at least one gene prior to said treating with said compound indicates that said compound is not efficient for treating the subject; or
(iii) wherein when a level of expression of said at least one gene following said treating with said compound is identical or changed below a predetermined threshold as compared to prior to said treating with said compound then the treatment is not efficient for treating the subject
thereby monitoring treatment efficiency of the subject having multiple sclerosis.

12. A compound for use according to any one of claims 1-10, or the method of claim 11, wherein said compound is 2-(4-methyl-1,4-diazepan-1-yl)-N-((5-methylpyrazin-2-yl)methyl)-5-oxo-5H-benzo[4,5]thiazolo[3,2-a] [1,8]naphthyridine-6-carboxamide as depicted in Formula

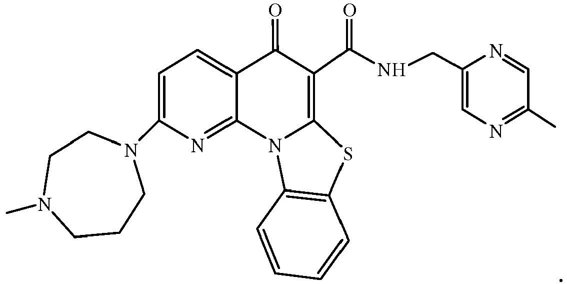

13. A compound for use according to any of claims 1-10 or the method of claim 11 or 12, wherein said multiple sclerosis is a relapsing-remitting course of multiple sclerosis.

14. A compound for use according to any of claims 1-10 or the method of claim 11 or 12, wherein said multiple sclerosis is a progressive course of multiple sclerosis.

15. A compound for use according to any of claims 1-10 or the method of claim 11 or 12, wherein said multiple sclerosis is a benign multiple sclerosis.

## Patentansprüche

1. Verbindung der Formel (VI) 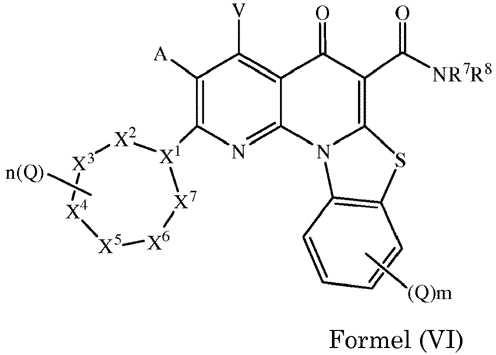 oder ein pharmazeutisch unbedenkliches Salz oder Ester davon;
wobei:
X¹ CH oder N ist;
X², X³, X⁴, X⁵, X⁶ und X⁷ unabhängig NR⁴, CH₂, CHQ oder C(Q)₂ sind, vorausgesetzt dass: (i) null, eins oder zwei von X², X³, X⁴, X⁵, X⁶ und X⁷ NR⁴ sind; (ii) wenn X¹ N ist, beide von X² und X⁷ nicht NR⁴ sind; (iii) wenn X¹ N ist, X³ und X⁶ nicht NR⁴ sind; und (iv) wenn X¹ CH ist und zwei von X², X³, X⁴, X⁵, X⁶ und X⁷ NR⁴ sind, die zwei NR⁴ sich an benachbarten Ringpositionen befinden oder durch zwei oder mehr andere Ringpositionen getrennt sind;
A und V unabhängig H, Halo, Azido, -CN, -CF₃, -CONR¹R², -NR¹R², - SR², -OR², -R³, -W, -L-W, -W⁰ oder -L-N(R)-W⁰ sind;
jedes Q unabhängig Halo, Azido, -CN, -CF₃, -CONR¹R², -NR¹R², -SR², - OR², -R³, -W, -L-W, -W⁰ oder -L-N(R)-W⁰ ist;
in jedem -NR¹R² R¹ und R² zusammen mit N einen optional substituierten azazyklischen Ring bilden können, optional enthaltend ein zusätzliches Heteroatom, ausgewählt aus N, O und S als ein Ringglied;
R¹ H oder C1-C6 Alkyl ist, optional substituiert mit einem oder mehreren Halogenen oder =O;
R² H oder C1-C10 Alkyl, C1-C10 Heteroalkyl, C2-C10 Alkenyl oder C2-C10 Heteroalkenyl ist, von denen jedes optional mit einem oder mehreren Halogenen, =O oder einem optional substituierten 3-7-gliedrigen carbozyklischen oder heterozyklischen Ring substituiert sein kann;
R³ ein optional substituiertes C1-C10 Alkyl, C2-C10 Alkenyl, C5-C10 Aryl oder C6-C12 Arylalkyl oder eine Heteroform von einem davon ist, von denen jedes optional mit einem oder mehreren Halogenen, =O, oder einem optional substituierten 3-6-gliedrigen carbozyklischen oder heterozyklischen Ring substituiert sein kann;
jedes R⁴ unabhängig H oder C1-C6 Alkyl ist; oder R4 -W, -L-W oder -L-N(R)-W⁰ sein kann;
jedes R unabhängig H oder C1-C6 Alkyl ist;
R⁷ H ist und R⁸ C1-C10 Alkyl, C1-C10 Heteroalkyl, C2-C10 Alkenyl oder C2-C10 Heteroalkenyl ist, von denen jedes optional mit einem oder mehreren Halogenen, =O oder einem optional substituierten 3-7-gliedrigen carbozyklischen oder heterozyklischen Ring substituiert sein kann; oder in - NR⁷R⁸ R⁷ und R⁸ zusammen mit N einen optional substituierten azazyklischen Ring, optional enthaltend ein zusätzliches Heteroatom, ausgewählt aus N, O und S als ein Ringglied, bilden können;
m 0, 1, 2, 3 oder 4 ist;
n 0, 1, 2, 3 4 oder 5 ist;
L ein C1-C10 Alkylen, C1-C10 Heteroalkylen, C2-C10 Alkenylen oder C2-C10 Heteroalkenylenlinker ist, von denen jedes optional mit einem oder mehreren Substituenten, ausgewählt aus der Gruppe bestehend aus Halogen, Oxo (=O) oder C1-C6 Alkyl, substituiert sein kann;
W ein optional substituierter 4-7-gliedriger azazyklischer Ring ist, optional enthaltend ein zusätzliches Heteroatom, ausgewählt aus N, O und S als ein Ringglied; und
W⁰ ein optional substituierter 3-4-gliedriger carbozyklischer Ring oder eine C1-C6 Alkylgruppe, substituiert mit von 1 bis 4 Fluoratomen, ist;
zur Verwendung bei der Behandlung von multipler Sklerose bei einem Patienten.

2. Verbindung der Formel (VIII) 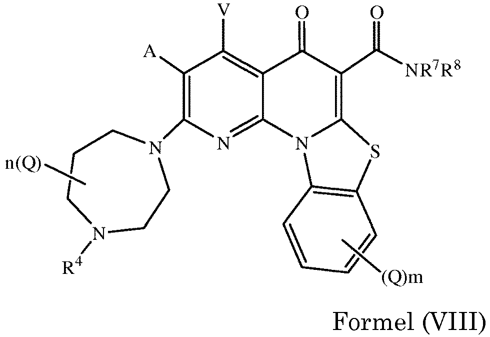 oder ein pharmazeutisch unbedenkliches Salz oder Ester davon;
wobei:
A und V unabhängig H, Halo, Azido, -CN, -CF₃, -CONR¹R², -NR¹R², - SR², -OR², -R³, -W, -L-W, -W⁰ oder -L-N(R)-W⁰ sind;
jedes Q unabhängig Halo, Azido, -CN, -CF₃, -CONR¹R², -NR¹R², -SR², - OR², -R³, -W, -L-W, -W⁰ oder -L-N(R)-W⁰ ist;
in jedem -NR¹R² R¹ und R² zusammen mit N einen optional substituierten azazyklischen Ring bilden können, optional enthaltend ein zusätzliches Heteroatom, ausgewählt aus N, O und S als ein Ringglied;
R¹ H oder C1-C6 Alkyl ist, optional substituiert mit einem oder mehreren Halogenen oder =O;
R² H oder C1-C10 Alkyl, C1-C10 Heteroalkyl, C2-C10 Alkenyl oder C2-C10 Heteroalkenyl ist, von denen jedes optional mit einem oder mehreren Halogenen, =O oder einem optional substituierten 3-7-gliedrigen carbozyklischen oder heterozyklischen Ring substituiert sein kann;
R³ ein optional substituiertes C1-C10 Alkyl, C2-C10 Alkenyl, C5-C10 Aryl oder C6-C12 Arylalkyl oder eine Heteroform von einem dieser ist, von denen jedes optional mit einem oder mehreren Halogenen, =O, oder einem optional substituierten 3-6-gliedrigen carbozyklischen oder heterozyklischen Ring substituiert sein kann;
jedes R⁴ unabhängig H oder C1-C6 Alkyl ist; oder R4 -W, -L-W oder -L-N(R)-W⁰ sein kann;
jedes R unabhängig H oder C1-C6 Alkyl ist;
R⁷ H ist und R⁸ C1-C10 Alkyl, C1-C10 Heteroalkyl, C2-C10 Alkenyl oder C2-C10 Heteroalkenyl ist, von denen jedes optional mit einem oder mehreren Halogenen, =O oder einem optional substituierten 3-7-gliedrigen carbozyklischen oder heterozyklischen Ring substituiert sein kann; oder in - NR⁷R⁸, R⁷ und R⁸ zusammen mit N einen optional substituierten azazyklischen Ring bilden können, optional enthaltend ein zusätzliches Heteroatom, ausgewählt aus N, O und S als ein Ringglied;
m 0, 1, 2, 3 oder 4 ist;
n 0, 1, 2, 3 4 oder 5 ist;
L ein C1-C10 Alkylen, C1-C10 Heteroalkylen, C2-C10 Alkenylen oder C2-C10 Heteroalkenylenlinker ist, von denen jedes optional mit einem oder mehreren Substituenten, ausgewählt aus der Gruppe bestehend aus Halogen, Oxo (=O) oder C1-C6 Alkyl, substituiert sein kann;
W ein optional substituierter 4-7-gliedriger azazyklischer Ring ist, optional enthaltend ein zusätzliches Heteroatom, ausgewählt aus N, O und S als ein Ringglied; und
W⁰ ein optional substituierter 3-4-gliedriger carbozyklischer Ring oder eine C1-C6 Alkylgruppe, substituiert mit von 1 bis 4 Fluoratomen, ist,
zur Verwendung bei der Behandlung von multipler Sklerose bei einem Patienten.

3. Verbindung zur Verwendung nach Anspruch 1 oder 2, wobei R⁷ H ist und R⁸ ein C₁₋₄ Alkyl ist, substituiert mit einem optional substituierten aromatischen heterozyklischen Ring.

4. Verbindung zur Verwendung nach Anspruch 3, wobei der optional substituierte aromatische heterozyklische Ring ausgewählt ist aus Pyridin, Pyrimidin, Pyrazin, Imidazol, Pyrrolidin und Thiazol.

5. Verbindung der Formel (VII) 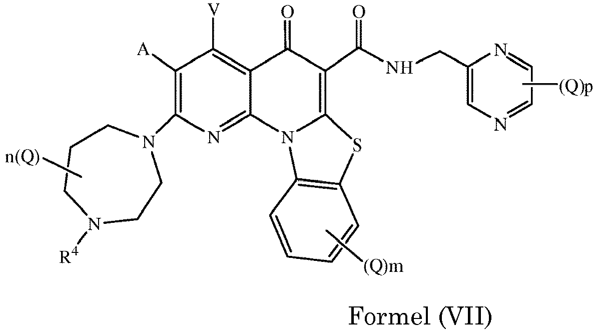
oder ein pharmazeutisch unbedenkliches Salz oder Ester davon;
A und V unabhängig H, Halo, Azido, -CN, -CF₃, -CONR¹R², -NR¹R², - SR², -OR², -R³, -W, -L-W, -W⁰ oder -L-N(R)-W⁰ sind;
jedes Q unabhängig Halo, Azido, -CN, -CF₃, -CONR¹R², -NR¹R², -SR², - OR², -R³, -W, -L-W, -W⁰ oder -L-N(R)-W⁰ ist;
in jedem -NR¹R² R¹ und R² zusammen mit N einen optional substituierten azazyklischen Ring bilden können, optional enthaltend ein zusätzliches Heteroatom, ausgewählt aus N, O und S als ein Ringglied;
R¹ H oder C1-C6 Alkyl ist, optional substituiert mit einem oder mehreren Halogenen oder =O;
R² H oder C1-C10 Alkyl, C1-C10 Heteroalkyl, C2-C10 Alkenyl oder C2-C10 Heteroalkenyl ist, von denen jedes optional mit einem oder mehreren Halogenen, =O oder einem optional substituierten 3-7-gliedrigen carbozyklischen oder heterozyklischen Ring substituiert sein kann;
R³ ein optional substituiertes C1-C10 Alkyl, C2-C10 Alkenyl, C5-C10 Aryl oder C6-C12 Arylalkyl oder eine Heteroform von einem dieser ist, von denen jedes optional mit einem oder mehreren Halogenen, =O, oder einem optional substituierten 3-6-gliedrigen carbozyklischen oder heterozyklischen Ring substituiert sein kann;
jedes R⁴ unabhängig H oder C1-C6 Alkyl ist; oder R4 -W, -L-W oder -L-N(R)-W⁰ sein kann;
jedes R unabhängig H oder C1-C6 Alkyl ist;
m 0, 1, 2, 3 oder 4 ist;
n 0, 1, 2, 3, 4 oder 5 ist;
p 0, 1, 2 oder 3 ist;
L ein C1-C10 Alkylen, C1-C10 Heteroalkylen, C2-C10 Alkenylen oder C2-C10 Heteroalkenylenlinker ist, von denen jedes optional mit einem oder mehreren Substituenten, ausgewählt aus der Gruppe bestehend aus Halogen, Oxo (=O) oder C1-C6 Alkyl, substituiert sein kann;
W ein optional substituierter 4-7-gliedriger azazyklischer Ring ist, optional enthaltend ein zusätzliches Heteroatom, ausgewählt aus N, O und S als ein Ringglied; und
W⁰ ein optional substituierter 3-4-gliedriger carbozyklischer Ring oder eine C1-C6 Alkylgruppe, substituiert mit von 1 bis 4 Fluoratomen, ist,
zur Verwendung bei der Behandlung von multipler Sklerose bei einem Patienten.

6. Verbindung zur Verwendung nach Anspruch 5, wobei p 0 oder 1 ist.

7. Verbindung zur Verwendung nach einem der Ansprüche 1-2, 5, wobei A und V unabhängig H oder Halo sind.

8. Verbindung zur Verwendung nach einem der Ansprüche 1-2, 5, wobei R⁴ H oder C1-4 Alkyl ist.

9. Verbindung zur Verwendung nach einem der Ansprüche 1-2, 5, wobei m und n jeweils 0 sind.

10. Verbindung, ausgewählt aus der Gruppe bestehend aus: 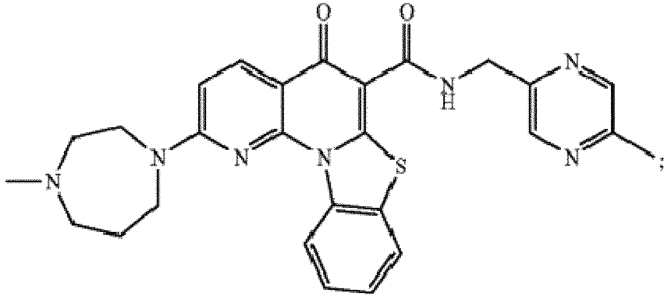 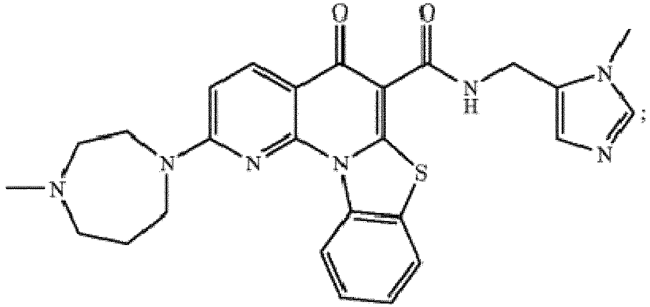 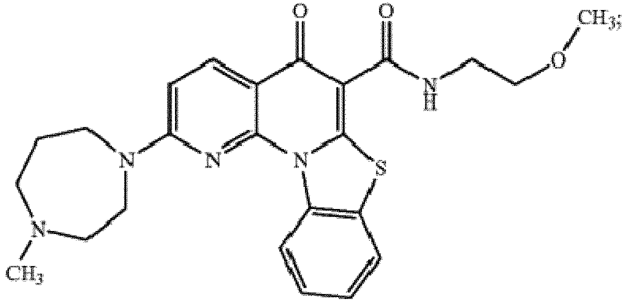 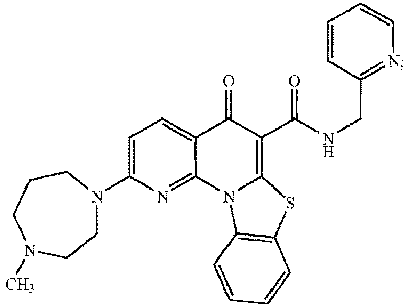 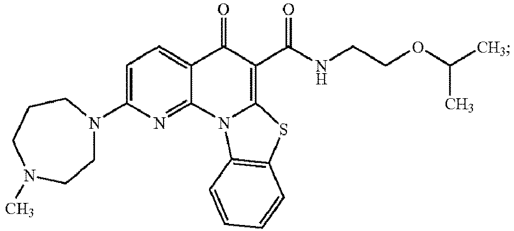 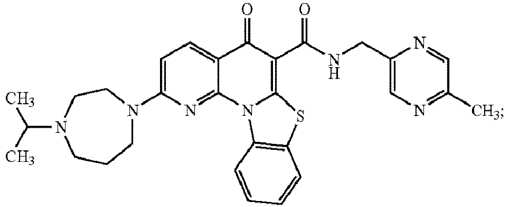 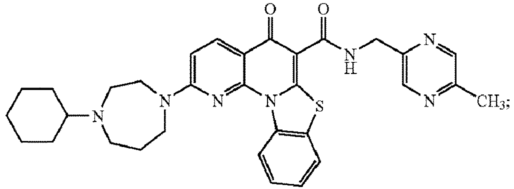 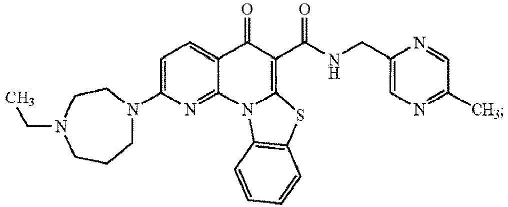 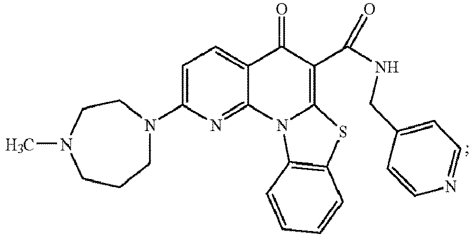 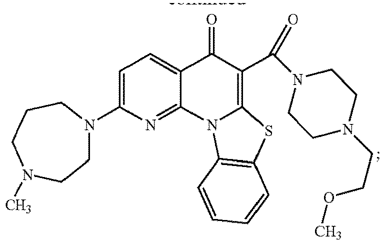 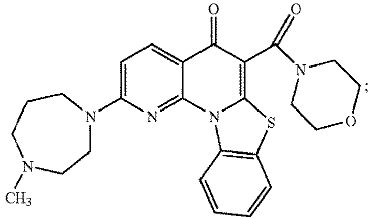 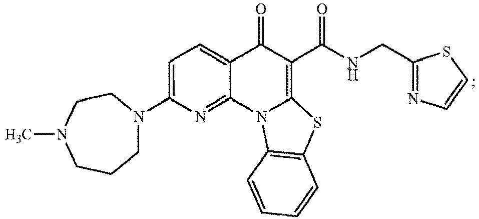 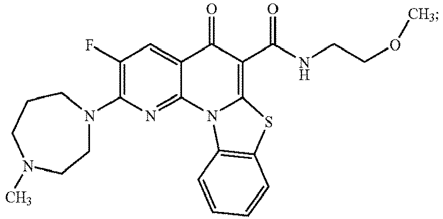 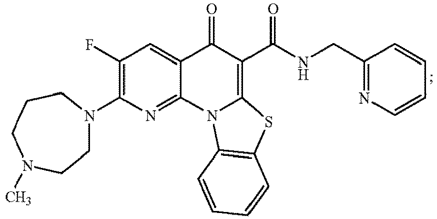 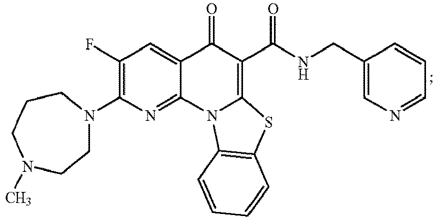 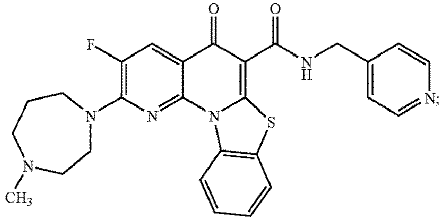 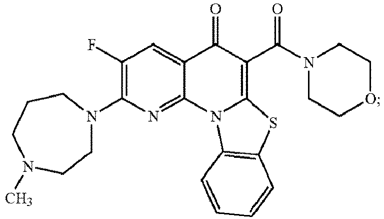 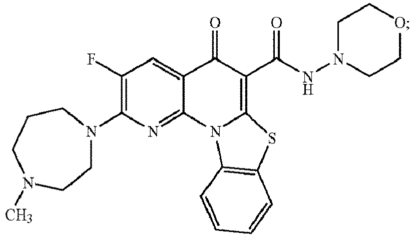 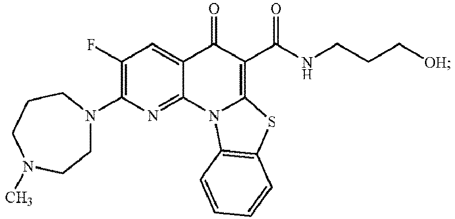 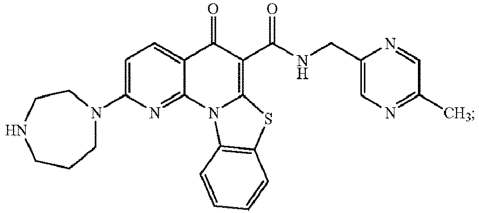 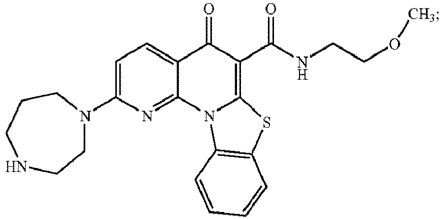 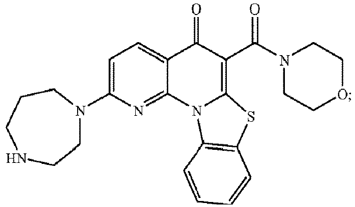 und 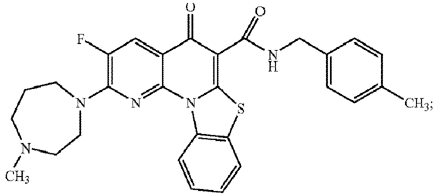
oder ein pharmazeutisch unbedenkliches Salz oder Ester davon,
zur Verwendung bei der Behandlung von multipler Sklerose bei einem Patienten.

11. Verfahren zur Überwachung der Behandlungseffizienz eines Patienten mit multipler Sklerose, das Verfahren umfassend:
Vergleichen eines Expressionsniveaus von mindestens einem Gen, beteiligt an dem RNA-Polymerase I-Weg in einer isolierten Zelle des Patienten, der mit der Verbindung nach einem der Ansprüche 1-10 behandelt wurde, mit einem Expressionsniveau des mindestens einen Gens in einer Zelle des Patienten vor der Behandlung mit der Verbindung,
(i) wobei eine Abnahme über einen vorbestimmten Schwellenwert in dem Expressionsniveau des mindestens einen Gens nach der Behandlung mit der Verbindung relativ zu dem Expressionsniveaudes mindestens einen Gens vor der Behandlung mit der Verbindung darauf hinweist, dass die Verbindung effizient für Behandlung des Patienten ist;
(ii) wobei eine Zunahme über einen vorbestimmten Schwellenwert in dem Expressionsniveau des mindestens einen Gens nach der Behandlung mit der Verbindung relativ zu dem Expressionsniveau des mindestens einen Gens vor der Behandlung mit der Verbindung darauf hinweist, dass die Verbindung ineffizient für Behandlung des Patienten ist; oder
(iii) wobei, wenn ein Expressionsniveau des mindestens einen Gens nach der Behandlung mit der Verbindung identisch oder unter einem vorbestimmten Schwellenwert verändert ist verglichen mit vor der Behandlung mit der Verbindung, dann ist die Behandlung ineffizient für Behandlung des Patienten,
wodurch die Behandlungseffizienz des Patienten mit multipler Sklerose überwacht wird.

12. Verbindung zur Verwendung nach einem der Ansprüche 1-10 oder das Verfahren nach Anspruch 11, wobei die Verbindung 2-(4-Methyl-1,4-diazepan-1-yl)-N-((5-methylpyrazin-2-yl)methyl)-5-oxo-5H-benzo[4,5]thiazol[3,2-a][1,8]naphthyridin-6-carboxamid ist, wie dargestellt in Formel

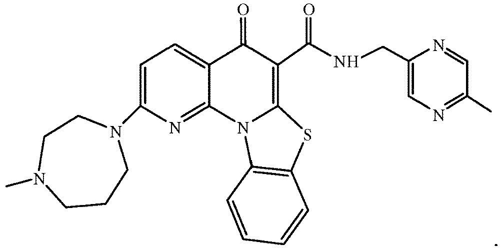

13. Verbindung zur Verwendung nach einem der Ansprüche 1-10 oder das Verfahren nach Anspruch 11 oder 12, wobei die multiple Sklerose ein schubförmig-remittierender Verlauf von multipler Sklerose ist.

14. Verbindung zur Verwendung nach einem der Ansprüche 1-10 oder das Verfahren nach Anspruch 11 oder 12, wobei die multiple Sklerose ein progressiver Verlauf von multipler Sklerose ist.

15. Verbindung zur Verwendung nach einem der Ansprüche 1-10 oder das Verfahren nach Anspruch 11 oder 12, wobei die multiple Sklerose eine gutartige multiple Sklerose ist.

## Revendications

1. Un composé de formule (VI), 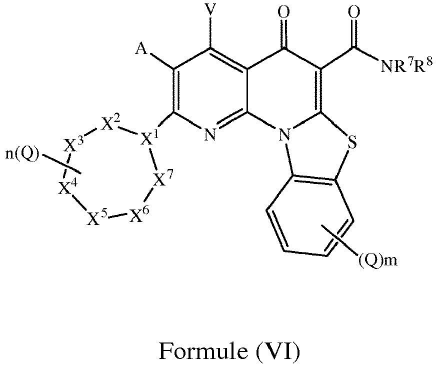 ou un sel ou ester pharmaceutiquement acceptable en dérivant,
formule dans laquelle :
- X¹ est CH ou N ;
- X², X³, X⁴, X⁵, X⁶ et X⁷ , indépendamment l'un de l'autre, sont NR⁴, CH₂, CHQ ou C(Q)₂, à la condition que:
(i) zéro, un ou deux des X², X³, X⁴, X⁵, X⁶ et X⁷ sont NR⁴ ;
(ii) quand X¹ est N, X² et X⁷ ne sont, à la fois, pas NR⁴ ;
(iii) quand X¹ est N, X³ et X⁶ ne sont, à la fois, pas NR⁴ ; et
(iv) quand X¹ est CH et deux des X², X³, X⁴, X⁵, X⁶ et X⁷ sont NR⁴, les deux NR⁴ sont disposés sur deux positions adjacentes du cycle ou sont séparés par deux ou plusieurs autres positions du cycle ;
- A et V, indépendamment l'un de l'autre, sont H, halo, azido, -CN, -CF₃, -CONR¹R², -NR¹R², -SR², -OR², -R³, -W, -L-W, -W⁰ ou -L-N(R)-W⁰ ;
- R¹ est H ou C₁-C₆ alkyl, éventuellement substitué par un ou plusieurs halogènes, ou =O ; chaque Q est indépendamment halo, azido, -CN, -CF₃, -CONR¹R², -NR¹R², -SR², -OR², - R³, -W, -L-W, -W⁰ ou -L-N(R)-W⁰ ;
dans chaque groupement -NR¹R², R¹ et R² ensemble avec N peuvent former un cycle azacyclique éventuellement substitué, contenant éventuellement un heteroatome supplémentaire choisi parmi N, O et S pour faire partie du cycle.
- R² est H ou C₁-C₁₀ alkyl, C₁-C₁₀ hétéroalkyl, C₂-C₁₀ alkényl ou C₂-C₁₀ hétéroalkényl, chacun desquels pouvant être substitués éventuellement par un ou plusieurs halogènes, =O, ou un cycle hétérocyclique ou carbocyclique comportant 3 à 7 atomes éventuellement substitué ;
- R³ est un groupe éventuellement substitué consistant en C₁-C₁₀ alkyl, C₂-C₁₀ alkényl, C₅-C₁₀ aryl, ou C₆-C₁₂ arylalkyl, ou une hétéroforme de l'un d'entre eux, chacun desquels pouvant être substitué éventuellement par un ou plusieurs halogènes, =O, ou un cycle carbocyclique ou hétérocyclique comportant 3 à 6 atomes éventuellement substitué ;
- chaque R⁴ est, indépendamment, H, ou C₁-C₆ alkyl ; ou R⁴ peut être -W, -L-W ou -L-N(R)-W° ;
- chaque R est, indépendamment, H ou C₁-C₆ alkyl ;
- R⁷ est H et R⁸ est C₁-C₁₀ alkyl, C₁-C₁₀ hétéroalkyl, C₂-C₁₀ alkényl ou C₂-C₁₀ hétéroalkényl, chacun desquels pouvant être substitué éventuellement par un ou plusieurs halogènes, =O, ou un cycle hétérocyclique ou carbocyclique comportant 3 à 7 atomes éventuellement substitués ;
ou dans le groupement -NR⁷R⁸, R⁷ et R⁸ ensemble avec l'azote peuvent former un cycle azacyclique éventuellement substitué, contenant éventuellement un hétéroatome supplémentaire choisi parmi N, O et S pour faire partie du cycle ;
m est 0, 1, 2, 3 ou 4 ;
n est 0, 1, 2, 3, 4, ou 5 ;
L est un coupleur C₁-C₁₀ alkyléne, C₁-C₁₀ hétéroalkyléne, C₂-C₁₀ alkényléne ou C₂-C₁₀ hétéroalkényléne, chacun desquels pouvant être substitué éventuellement par un ou plusieurs substituants choisis dans le groupe consistant en : halogene, oxo (=O), ou C₁-C₆ alkyl ; W est un cycle azacyclique comportant 4 à 7 atomes éventuellement substitué, contenant éventuellement un hétéroatome supplémentaire choisi parmi N, O et S pour faire partie du cycle ; et
W° est un cycle carbocyclique comportant 3-4 atomes éventuellement substitué, ou un groupe C₁-C₆ alkyl substitué par 1 à 4 atomes de fluor,
pour une utilisation pour le traitement d'un patient atteint de la sclérose en plaques.

2. Un composé de formule (VIII), 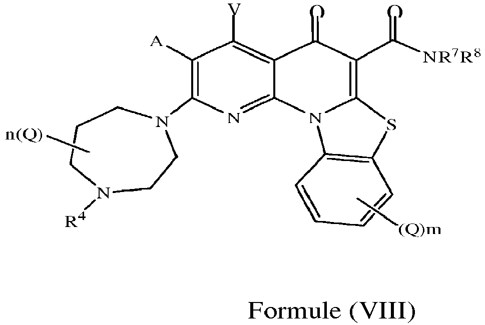 ou un sel ou ester pharmaceutiquement acceptable en dérivant,
formule dans laquelle:
- A et V, indépendamment l'un de l'autre, sont H, halo, azido, -CN, - CF₃, -CONR¹R², - NR¹R², SR², -OR², -R³, -W, -L-W, -W⁰ ou -L-N(R)-W⁰ ;
- chaque Q, indépendamment, est -halo, azido, - CN, -CF₃, -CONR¹R², -NR¹R², -SR², -OR², -R³, -W, -L-W, -W⁰, ou -L-N(R)-W⁰ ;
dans chaque groupement -NR¹R², R¹ et R² ensemble avec l'azote peuvent former un cycle azacyclique éventuellement substitué, contenant éventuellement un hétéroatome supplémentaire choisi parmi N, O et S pour faire partie du cycle ;
- R¹ est H ou C₁-C₆ alkyl, éventuellement substitué par un ou plusieurs halogènes, ou =O ;
- R² est H ou C₁-C₁₀ alkyl, C₁-C₁₀ hétéroalkyl, C₂-C₁₀ alkényl ou C₂-C₁₀ hétéroalkényl, chacun desquels pouvant être substitués éventuellement par un ou plusieurs halogènes, =O, ou un cycle hétérocyclique ou carbocyclique comportant 3 à 7 atomes éventuellement substitués ;
- R³ est un groupe éventuellement substitué consistant en C₁-C₁₀ alkyl, C₂-C₁₀ alkényl, C₅-C₁₀ aryl, ou C₆-C₁₂ arylalkyl, ou une hétéroforme de l'un d'entre eux, chacun desquels pouvant être substitué éventuellement par un ou plusieurs halogènes, =O, ou un cycle carbocyclique ou hétérocyclique comportant 3 à 6 atomes éventuellement substitué ;
- chaque R⁴ est, indépendamment, H, ou C₁-C₆ alkyl ; ou R⁴ peut être -W, -L-W ou -L-N(R)-W⁰ ;
- chaque R est, indépendamment, H ou C₁-C₆ alkyl ;
- R⁷ est H et R⁸ est C₁-C₁₀ alkyl, C₁-C₁₀ hétéroalkyl, C₂-C₁₀ alkényl ou C₂-C₁₀ hétéroalkényl, chacun desquels pouvant être substitué éventuellement par un ou plusieurs halogènes, =O, ou un cycle hétérocyclique ou carbocyclique comportant 3 à 7 atomes éventuellement substitués ;
ou dans un groupement -NR⁷R⁸, R⁷ et R⁸ ensemble avec l'azote peuvent former un cycle azacyclique éventuellement substitué, contenant éventuellement un hétéroatome supplémentaire choisi parmi N, O et S pour faire partie du cycle ;
m est 0, 1, 2, 3 ou 4 ;
n est 0, 1, 2, 3, 4, ou 5 ;
L est un coupleur C₁-C₁₀ alkyléne, C₁-C₁₀ hétéroalkyléne, C₂-C₁₀ alkényléne ou C₂-C₁₀ hétéroalkényléne, chacun desquels pouvant être substitué éventuellement par un ou plusieurs substituants choisis dans le groupe consistant en halogene, oxo (=O), ou C₁-C₆ alkyl ;
W est un cycle azacyclique comportant 4 à 7 atomes éventuellement substitué, contenant éventuellement un hétéroatome supplémentaire choisi parmi N, O et S pour faire partie du cycle ; et
W° est un cycle carbocyclique éventuellement substitué comportant 3-4 atomes, ou un groupe C₁-C₆ alkyl substitué par 1 à 4 atomes de fluor ;
pour une utilisation pour le traitement d'un patient atteint de la sclérose en plaques.

3. Un composé pour une utilisation selon la revendication 1 ou 2, dans laquelle R⁷ est H et R⁸ est un groupe C₁₋₄ alkyl substitué avec un cycle hétérocyclique aromatique éventuellement substitué.

4. Un composé pour une utilisation selon la revendication 3, dans laquelle le cycle hétérocyclique aromatique éventuellement substitué est choisi parmi pyridine, pyrimidine, pyrazine, imidazole, pyrrolidine et thiazole.

5. Un composé de formule (VII) 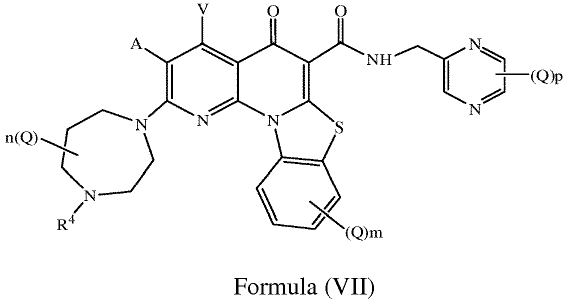 ou un sel ou ester pharmaceutiquement acceptable en dérivant,
formule dans laquelle:
- A et V, indépendamment l'un de l'autre, sont H, halo, azido, -CN, - CF₃, -CONR¹R², - NR¹R², -SR², -OR², -R³, -W, -L-W, -W⁰ ou -L-N(R)-W⁰ ;
- chaque Q, indépendamment, est -halo, azido, -CN, -CF³, -CONR¹R², -NR¹R², -SR², -OR², -R³, -W, -L-W, -W⁰ ou -L-N(R)-W⁰ ;
- dans chaque groupement -NR¹R², R¹ et R² ensemble avec l'azote peuvent former un cycle azacyclique éventuellement substitué, contenant éventuellement un hétéroatome supplémentaire choisi parmi N, O et S pour faire partie du cycle ;
- R¹ est H, ou C₁-C₆ alkyl, éventuellement substitué par un ou plusieurs halogènes, ou =O ;
- R² est H, ou C₁-C₁₀ alkyl, C₁-C₁₀ hétéroalkyl, C₂-C₁₀ alkényl ou C₂-C₁₀ hétéroalkényl, chacun desquels pouvant être substitués éventuellement par un ou plusieurs halogènes =O, ou un cycle hétérocyclique ou carbocyclique comportant 3 à 7 atomes éventuellement substitués ;
- R³ est un groupe éventuellement substitué consistant en C₁-C₁₀ alkyl, C₂-C₁₀ alkényl, C₅-C₁₀ aryl, ou C₆-C₁₂ arylalkyl, ou une hétéroforme de l'un d'entre eux, chacun desquels pouvant être substitué éventuellement par un ou plusieurs halogènes =O, ou un cycle carbocyclique ou hétérocyclique comportant 3 à 6 atomes éventuellement substitué ;
- chaque R⁴ est, indépendamment, H, ou C₁-C₆ alkyl ; ou R⁴ peut être -W, -L-W ou -L-N(R)-W° ;
- chaque R est, indépendamment, H ou C₁-C₆ alkyl ;
m est 0, 1, 2, 3 ou 4 ;
n est 0, 1, 2, 3, 4, ou 5 ;
p est 0, 1, 2 ou 3,
L est un coupleur C₁-C₁₀ alkyléne, C₁-C₁₀ hétéroalkyléne, C₂-C₁₀ alkényléne ou C₂-C₁₀ hétéroalkényléne, chacun desquels pouvant être substitué éventuellement par un ou plusieurs substituants choisis dans le groupe consistant en halogene, oxo (=O), ou C₁-C₆ alkyl ;
W est un cycle azacyclique comportant 4 à 7 atomes éventuellement substitué, contenant éventuellement un hétéroatome supplémentaire choisi parmi N, O et S pour faire partie du cycle ; et
W° est un cycle carbocyclique comportant 3-4 atomes éventuellement substitué, ou un groupe C₁-C₆ alkyl substitué par 1 à 4 atomes de fluor ;
pour une utilisation pour le traitement d'un patient atteint de la sclérose en plaques.

6. Un composé pour une utilisation selon la revendication 5, dans laquelle p est 0 ou 1.

7. Un composé pour une utilisation selon une quelconque des revendications 1 - 2, 5 dans lequel A et V sont, indépendamment l'un de l'autre, H ou halo.

8. Un composé pour une utilisation selon une quelconque des revendications 1 - 2, 5, dans lequel R⁴ est H ou C₁₋₄ alkyl.

9. Un composé pour une utilisation selon une quelconque des revendications 1 - 2, 5 dans lequel m et n sont chacun 0.

10. Un composé choisis dans le groupe consistant en: 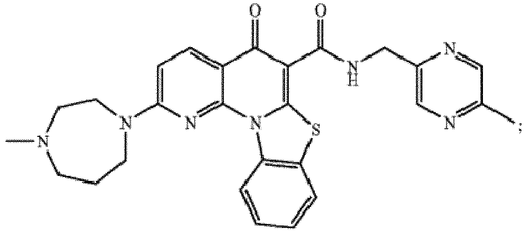 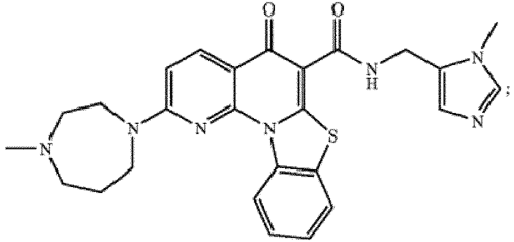 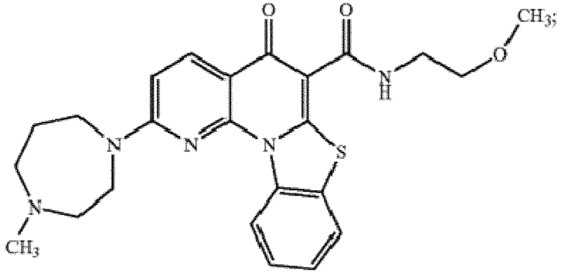 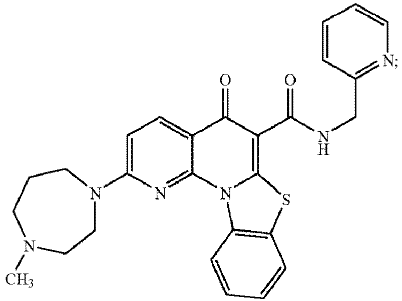 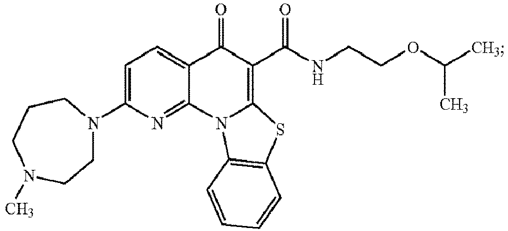 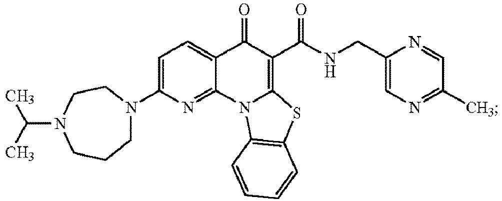 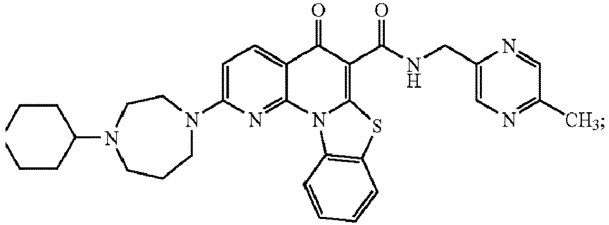 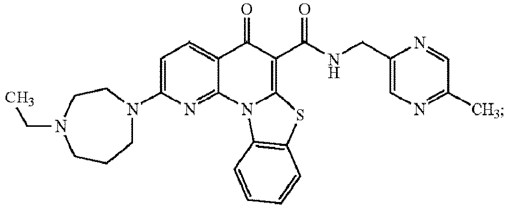 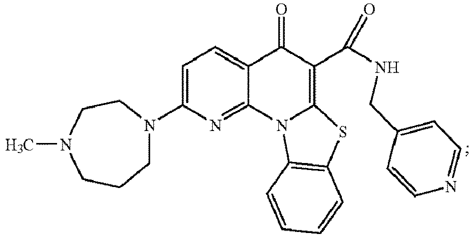 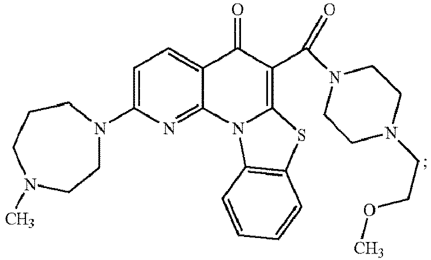 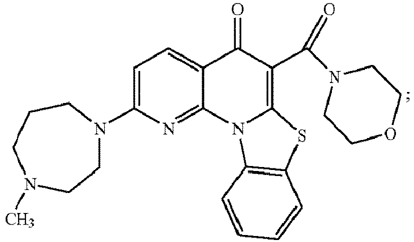 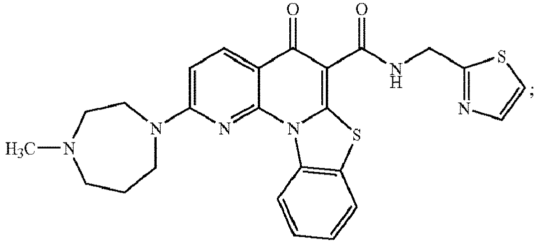 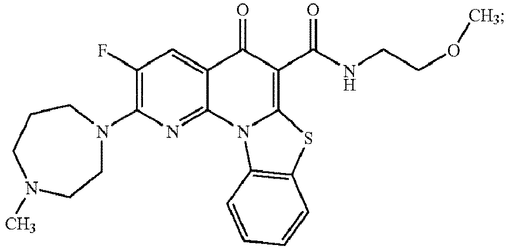 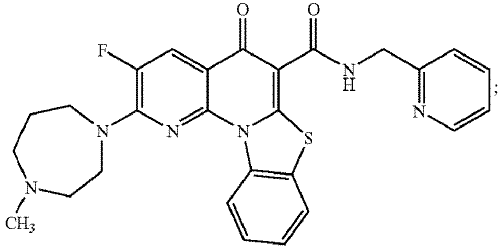 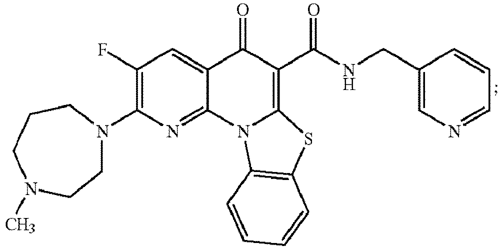 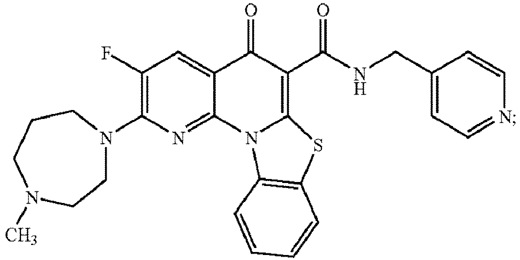 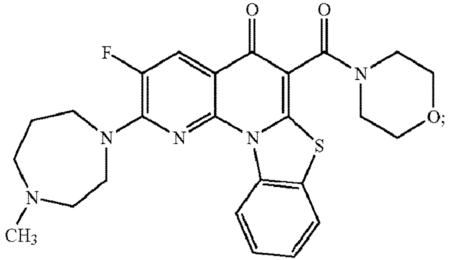 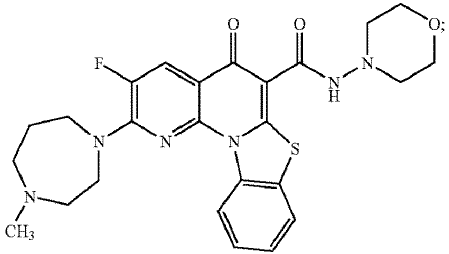 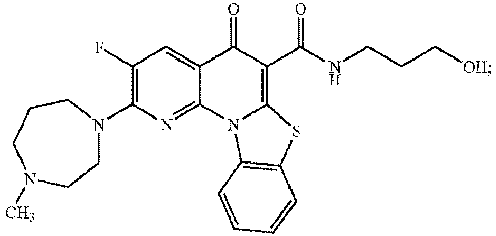 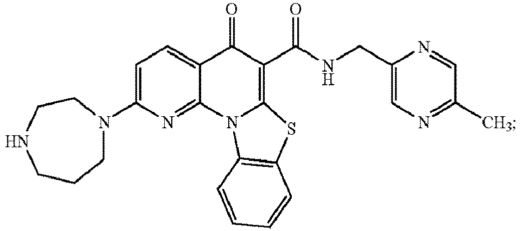 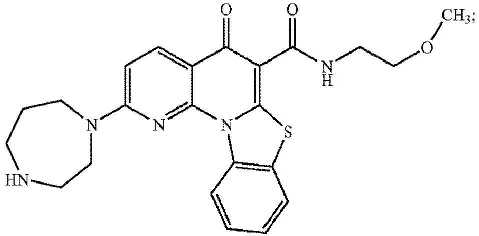 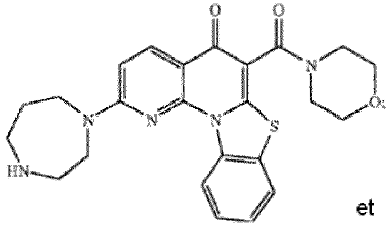 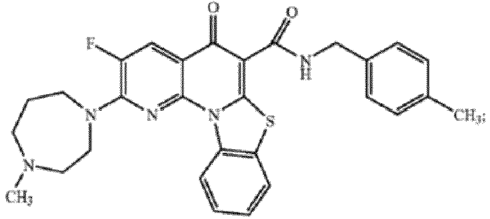 ou un sel ou ester pharmaceutiquement acceptable en dérivant,
pour une utilisation pour le traitement d'un patient atteint de la sclérose en plaques.

11. Un procédé pour contrôler l'efficacité d'un traitement d'un patient atteint de sclérose en plaques, ce procédé comprenant:
- la comparaison d'un niveau d'expression d'au moins un gène impliqué dans une voie de transcription de l'ARN polymérase I dans une cellule isolée d'un patient ayant été traité par ledit composé selon une quelconque des revendications 1 à 10 avec un niveau d'expression dudit au moins un gène dans une cellule du patient préalablement à son traitement par ledit composé,
- (i) où une diminution supérieure à un seuil prédéterminé du dit niveau d'expression dudit au moins un gène après ledit traitement par ledit composé par rapport audit niveau d'expression dudit au moins un gène préalablement au dit traitement par ledit composé signifie que ledit composé agit efficacement sur le traitement du patient ;
- (ii) où une augmentation supérieure à un seuil prédéterminé du dit niveau d'expression dudit au moins un gène après ledit traitement par ledit composé par rapport audit niveau d'expression dudit au moins un gène préalablement au dit traitement par ledit composé signifie que ledit composé n'agit pas efficacement sur le traitement du patient ;
- (iii) où lorsque le niveau d'expression dudit au moins un gène après ledit traitement par ledit composé est identique ou changé au-dessous d'un seuil prédéterminé par comparaison audit niveau d'expression préalablement audit traitement par ledit composé
alors le traitement n'est pas efficace pour traiter le patient ;
ce qui permet de contrôler l'efficacité du traitement sur le patient ayant une sclérose en plaques.

12. Un composé pour une utilisation selon une quelconque des revendications 1-10, ou le procédé selon la revendication 11, lequel composé consiste en 2-(4-méthyl-1,4-diazepane-1-yl)-N-((5-méthylpyrazin-2-yl)-méthyl)-5-oxo-5H-benzo[4,5]thiazolo[3,2-a][1,8]-naphthyridine-6-carboxamide, tel que représentée par la formule

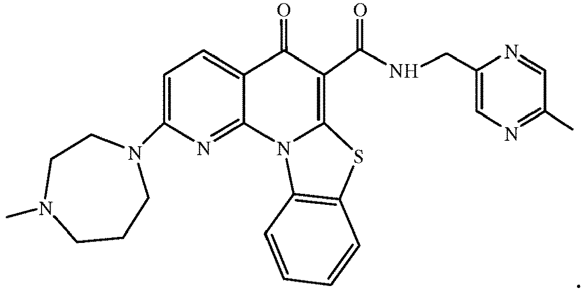

13. Un composé pour une utilisation selon une quelconque des revendications 1-10, ou le procédé selon la revendication 11 ou 12, dans laquelle la sclérose en plaques est une sclérose en plaques récurrente-rémittente.

14. Un composé pour une utilisation selon une quelconque des revendications 1-10, ou le procédé selon la revendication 11 ou 12, dans laquelle la sclérose en plaques est une sclérose en plaques progressive.

15. Un composé pour une utilisation selon une quelconque des revendications 1-10, ou le procédé selon la revendication 11 ou 12, dans laquelle la sclérose en plaques est une sclérose en plaques bénigne.
